# EUROPEAN PATENT APPLICATION

(11) **EP 4 285 933 A1**
(43) Date of publication of application: **06.12.2023**
(21) Application number: 22382517.5
(22) Date of filing: 30.05.2022
(51) Int. Cl.: A61K 47/61, A61P 37/02

(54) **OLIGOSACCHARIDE COMPLEXES AND USES**

(71) Applicant: BioNTech SE, 55131 Mainz (DE); Consejo Superior de Investigaciones Científicas (CSIC), 41013 Sevilla (ES); Universidad de Sevilla, 41013 Sevilla (ES)
(72) Inventor: Moreno Herrero, Jorge, 55131 Mainz (DE); Haas, Heinrich, 55131 Mainz (DE); Erbar, Stephanie, 55131 Mainz (DE); Stahl, Theo Benjamin, 55131 Mainz (DE); Garcia Fernandez, Jose Manuel, 41092 Sevilla (ES); Benito Hernandez, Juan Manuel, 41092 Sevilla (ES); Lopez Fernandez, Jose, 41092 Sevilla (ES); Ortiz Mellet, Maria del Carmen, 41012 Sevilla (ES); de la Cruz Ruiz, Noelia, 41807 Sevilla (ES); Gonzalez Cuesta, Manuel, 41012 Sevilla (ES); Jacobus Ambuludi, Egon Jack, 55131 Mainz (ES); Vlatkovic, Irena, 55131 Mainz (ES)
(74) Representative: Carlos Hernando, Borja

(57) **Abstract**

The present disclosure provides complexes i) a cationic oligosaccharide comprising one or more cationic moieties bonded to a trehalose, a sucrose, or a gluco-n-oligosaccharide moiety, where n is 2-6; ii) a surfactant; and iii) one or more additives selected from: a sterol, a helper lipid, an immunomodulator, and a targeting molecule; and used thereof

## Description

### Background

Targeted delivery and expression of therapeutic and/or prophylactic agents such as nucleic acids present certain challenges due to the instability of nucleic acids and their inability to permeate the cell membrane. Certain approaches, including use of lipid or polymer-based systems, exhibit promise but suffer from drawbacks related to manufacturing difficulties, poor structural definition, and high polydispersity. Still further, many such compositions lack satisfactory safety and efficacy for use in delivery of therapeutic agents.

### Summary

The present disclosure provides, among other things, a surprising and unexpected insight that complexes comprising cationic and/or ionizable oligosaccharides are useful for targeted delivery of biological agents, including small molecules and nucleic acids. For example, in some embodiments, the present disclosure encompasses an insight that such complexes are surprisingly useful for the delivery of RNA. Moreover, complexes of the present disclosure avoid problems associated with other delivery systems by, for example, allowing for lower doses, and further, by avoiding particular adverse events (e.g., increased inflammatory activity manifesting as pain, swelling, and the like) that are associated with alternative and previous delivery systems.

In some embodiments, the present disclosure encompasses an insight that a composition comprising a cationic oligosaccharide, in combination with a surfactant, and one or more additives, for example in particular molar ratios described herein, provide complexes that exhibit improved properties relative to traditional lipid-based complexes or previously reported oligosaccharide compositions.

Previous work related to cationic oligosaccharides focused on their use with DNA. In particular, Carbajo-Gordillo, *et al.,* reported use of certain oligosaccharides in isolation (i.e., without the addition of surfactants or other additives) for DNA nanocomplexing and delivery. See Carbajo-Gordillo, et al., Chem. Comm., 55:8227-8230 (2019). Notably, however, the oligosaccharide-DNA complexes reported by Carbajo-Gordillo, *et al.,* exhibit surprisingly distinct properties from complexes comprising cationic oligosaccharides as reported in the present application, in particular when complexed with RNA. For example, the complexes reported herein, in some embodiments, exhibit different behavior in vivo, such as targeting (i.e., causing expression of RNA in) different systems in the body, and can be prepared with significantly reduced ratios of oligosaccharide to nucleic acid. For at least these reasons, the presently reported complexes exhibit unexpected benefits over those complexes previously reported.

In some embodiments, the present disclosure provides, among other things, a complex comprising i) a cationic oligosaccharide comprising one or more cationic moieties bonded to a trehalose, a sucrose, or a gluco-n-oligosaccharide moiety, where n is 2-6; ii) a surfactant; and iii) one or more additives selected from: a sterol, a helper lipid, an immunomodulator, and a targeting molecule.

In some embodiments, the present disclosure provides a composition comprising an oligosaccharide of formula I: 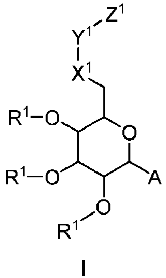 or a pharmaceutically acceptable salt thereof, wherein:
A is A¹, A², or A³: 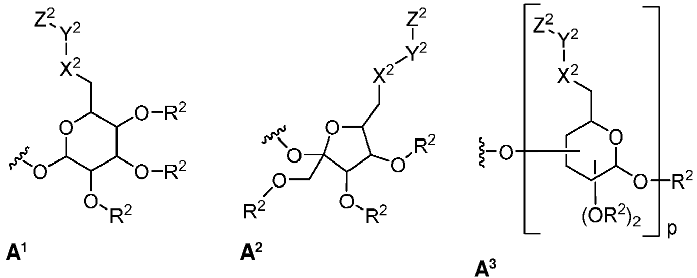
R¹ and R² are each independently selected from H, R^{a}, and -C(O)-R^{a}, wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by -Cy-, -NH-, -NR^{Y}C(O)-, -C(O)NR^{Y}-, -NR^{Y}C(O)O-, -OC(O)NR^{Y}-, -NR^{Y}C(O)NR^{Y}-, -NR^{Y} (S)NR^{Y}-, -C(S)NR^{Y}- - C(O)NR^{Y}SO₂-, -SO₂NR^{Y}C(O)-, -OC(O)O-, -O-, -C(O)-, -OC(O)-, -C(O)O, SO, or SO₂; each R^{Y} is independently selected from H and optionally substituted C₁-C₆ aliphatic; each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, -N⁺(M)₃, 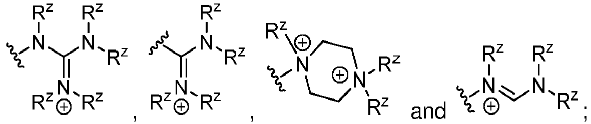
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5.

In some embodiments, the present disclosure provides a method of increasing or causing increased expression of RNA in a target in a subject, the method comprising administering to the subject a complex as described herein.

In some embodiments, the present disclosure provides a method of treating and or providing prophylaxis for a disease, disorder, or condition in a subject comprising administering to the subject a complex as described herein.

### Brief Description of the Drawings

**FIG. 1A** is a bar graph illustrating hydrodynamic diameter of complexes formulated at N/P6 ratios with modRNA and different cationic oligosaccharides.
**FIG. 1B** is a bar graph illustrating quantified bioluminescence over the duration of the experiment and normalized to the JLF41-modRNA group.
**FIG. 2A** is a plot illustrating hydrodynamic diameter of complexes formulated at N/P 6 but with different surfactants and within a surfactant:JLF99 ratio between about 0:1 to about 0.5:1.
**FIG. 2B** is a plot illustrating hydrodynamic diameter of complexes formulated at N/P 6 with either Tween20 (T20) or Tween40 (T40) and within a surfactant:JLF99 ratio between about 0:1 to about 0.5:1.
**FIG. 2C** is a plot illustrating expression of luciferase-encoding modRNA after 24 of transfection of C2C12 cells, N/P6 formulated modRNA with either Tween20 or Tween40 at different ratios of JLF, at 100ng of RNA/well.
**FIG. 2D** is a plot illustrating expression of luciferase-encoding modRNA after 24 of transfection of HepG2 cells, N/P6 formulated modRNA with either Tween20 or Tween40 at different surfactant to JLF99 ratios, at 100ng of RNA/well. Data here represents 3 independent experiments (n=3) with technical triplicates.
**FIG. 2E** is a bar graph illustrating hydrodynamic diameter of complexes formulated at different ratios of JLF99 to surfactant.
**FIG. 2F** is a bar graph illustrating quantified bioluminescence over the whole duration of the experiment represented as area under the curve.
**FIG. 2G** is a plot illustrating dorsal quantified bioluminescence over about 6 days.
**FIG. 2H** is an image illustrating bioluminescence on the ventral axis 6 hours after injection of modRNA into each group of mice.
**FIG. 3A** is a plot illustrating hydrodynamic diameter of example complexes.
**FIG. 3B** is a bar graph illustrating expression of luciferase-encoding modRNA after 24 of transfection of either C2C12, HepG2 or RAW264.7 cells, at 100ng of RNA/well. Data here represents 2 independent experiments (n=2) with technical triplicates.
**FIG. 4A** is a bar graph illustrating hydrodynamic diameter of complexes formed with either a combination of JLF99+Tween20+Cholesterol or JLF99+Tween20+ β-Sitosterol.
**FIG. 4B** is a bar graph illustrating normalized expression of luciferase-encoding modRNA to JLF99 benchmark after 24 hours after of either C2C12, HepG2 or RAW264.7 cells, at 100ng of RNA/well complexes formed with either a combination of JLF99+Tween20+Cholesterol or JLF99+Tween20+ β-Sitosterol.
**FIG. 4C** is a bar graph illustrating hydrodynamic diameter of complexes formed of the combination of JLF99 + Tween20 + β-Sitosterol + Helper Lipid at a molar ratio of 1:0.5:0.5:0.5 with either DOPE, DOPC, DOPS, DSPC, or DSPE.
**FIG. 4D** is a bar graph illustrating normalized expression of luciferase-encoding modRNA to JLF99 benchmark after 24 of transfection of either C2C12, HepG2 or RAW264.7 cells, at 100ng of RNA/well of complexes formed with JLF99 + Tween20 + β-Sitosterol + Helper Lipid at a molar ratio of 1:0.5:0.5:0.5 with either DOPE, DOPC, DOPS, DSPC, or DSPE.
**FIG. 4E** is a bar graph illustrating hydrodynamic diameter of complexes formed of the combination of JLF99 + Tween20 + Sterol + DSPC at a molar ratio of 1:0.5:0.5:0.5 with either β-Sitosterol, Stigmasterol, or Acetate-α-Tocopherol.
**FIG. 4F** is a bar graph illustrating the normalized expression of luciferase-encoding modRNA to JLF99 benchmark 24 hours after of transfection of either C2C12, HepG2 or RAW264.7 cells, at 100ng of RNA/well of complexes formed with JLF99 + Tween20 + Sterol + DSPC at a molar ratio of 1:0.5:0.5:0.5 wherein said sterol is either β-Sitosterol, Stigmasterol, or Acetate-a-Tocopherol.
**FIG. 4G** is a scatter plot illustrating hydrodynamic diameter of complexes formed of the combination of JLF99 + Tween20 + β-Sitosterol+ Helper Lipid at different molar ratios with either DMPC or DSPC.
**FIG. 4H** is a scatter plot illustrating the expression of luciferase-encoding modRNA after 24 of transfection of C2C12 at 100ng of RNA/well of complexes formed with JLF99 + Tween20 + β-Sitosterol + Helper Lipid at different molar ratios with either DMPC or DSPC.
**FIG 41** is a plot illustrating expression of luciferase-encoding modRNA 24 hours after transfection of HepG2 at 100ng of RNA/well of complexes formed with JLF99 + Tween20 + β-Sitosterol + Helper Lipid at different molar ratios with either DMPC or DSPC.

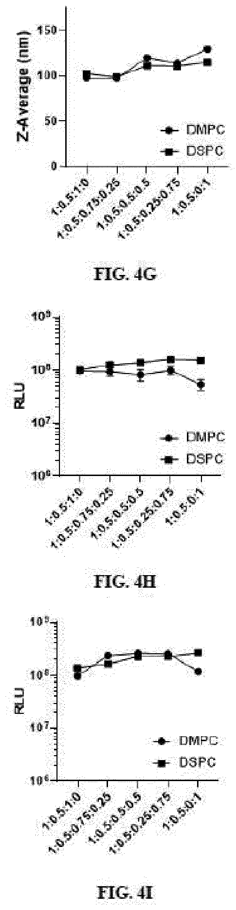

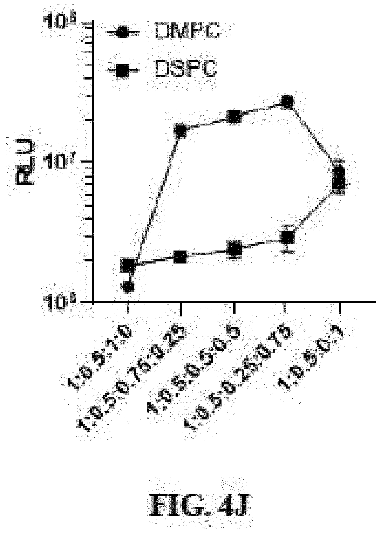

**FIG. 4J** is a plot illustrating expression of luciferase-encoding modRNA after 24 of transfection of RAW647.7 at 100ng of RNA/well of complexes formed with JLF99 + Tween20 + β-Sitosterol+ Helper Lipid at different molar ratios with either DMPC or DSPC.

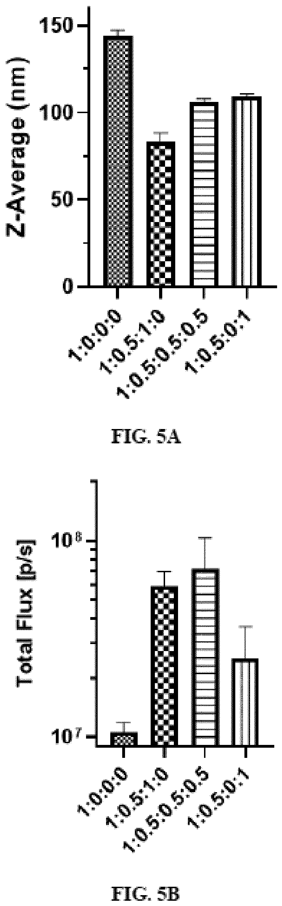

**FIG. 5A** is a bar graph illustrating hydrodynamic diameter of complexes formulated at different N/P6.
**FIG. 5B** is a bar graph illustrating quantified bioluminescence over the whole duration of the experiment represented as area under the curve.

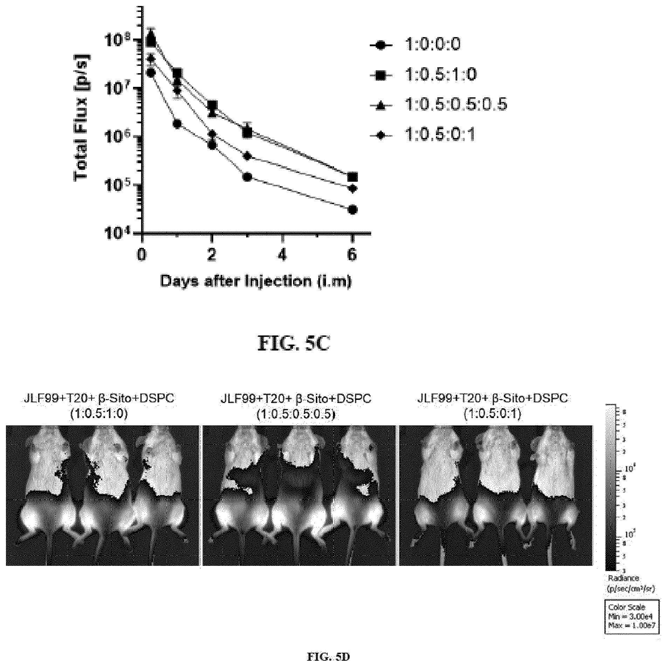

**FIG. 5C** is a plot illustrating quantified dorsal bioluminescence over the whole duration of the experiment.
**FIG. 5D** is an image of ventral bioluminescence measured after 6h of injection in mice.

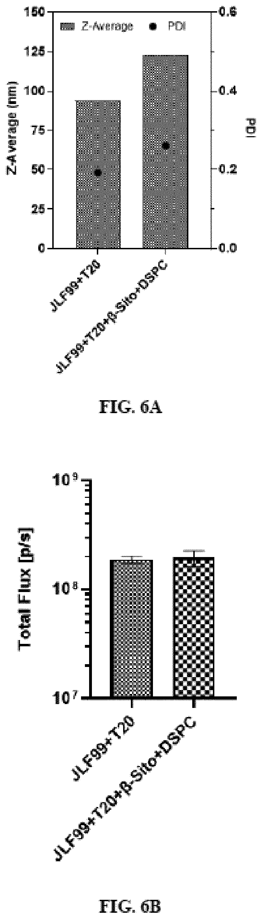

**FIG. 6A** is a bar graph illustrating hydrodynamic diameter of complexes formulated at different N/P6.
**FIG. 6B** is a bar graph illustrating quantified dorsal bioluminescence in the region of interest (muscle) 6h after injection.

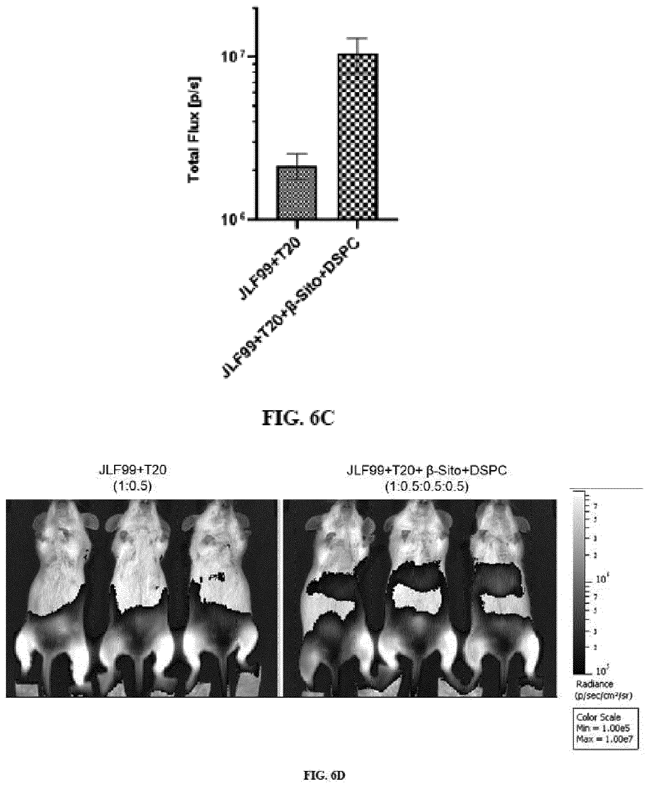

**FIG. 6C** is a bar graph illustrating quantified ventral bioluminescence in the region of interest (liver) 6h after injection.
**FIG. 6D** is an image of ventral bioluminescence measured 6h after injection in mice.

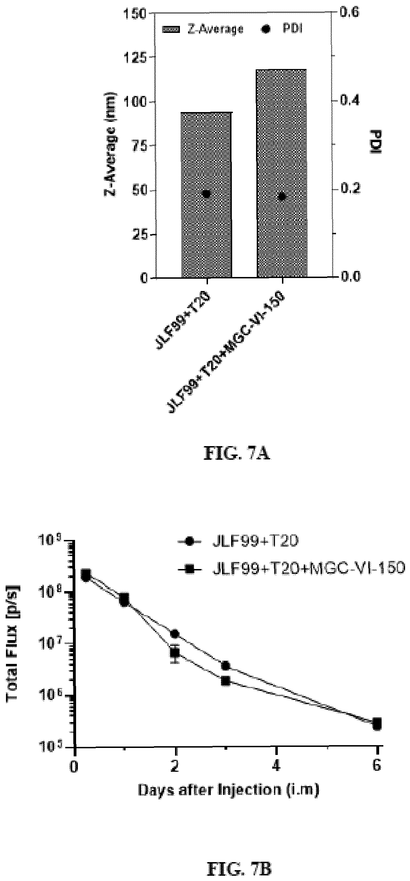

**FIG. 7A** is a bar graph illustrating hydrodynamic diameter of complexes formulated at different N/P6.

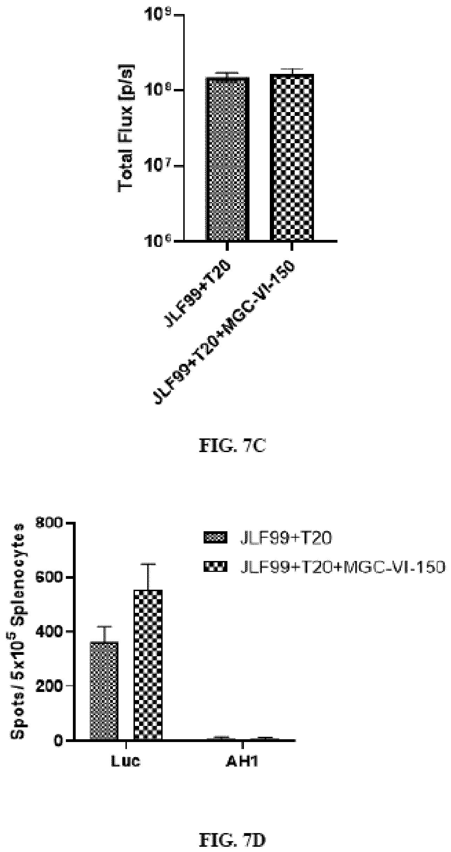

**FIG. 7B** and **FIG. 7C** are a plot and a bar graph, respectively, illustrating quantified dorsal bioluminescence in the region of interest (muscle) over the whole duration of the experiment as area under the curve.
**FIG. 7D** is a bar graph illustrating quantified spots count in an ELISpot read-out, reflecting the T-Cell responses against luciferase peptides or an irrelevant peptide (AH1).

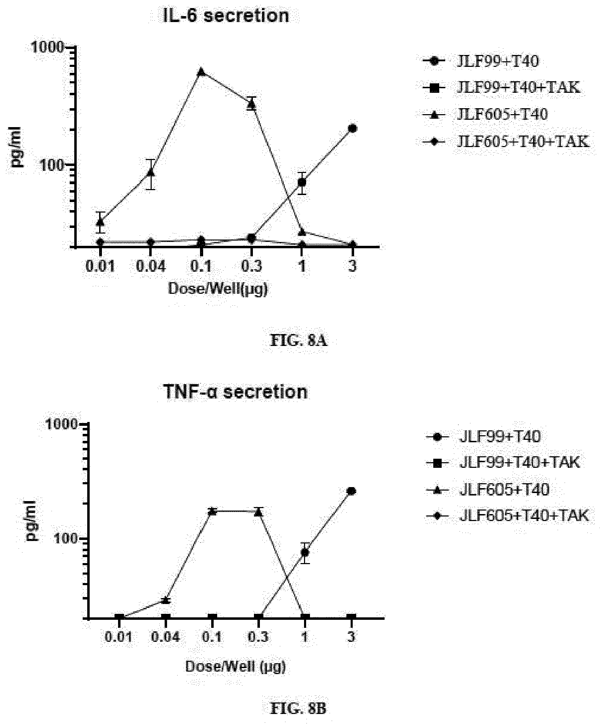

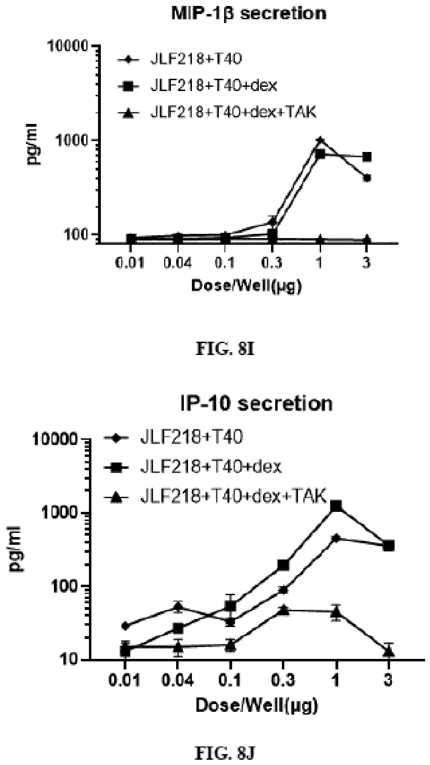

**FIG. 8A** is a scatter plot illustrating quantified IL-6 secretion in hPBMCs upon exposure to different doses of tested formulations. As used herein with reference to the present figures 8A-10I, "T40" refers to Tween40, "TAK" refers to TAK-242, and "dex" or "dexa" refers to dexamethasone.
**FIG.8B** is a scatter plot illustrating quantified TNF-α secretion in hPBMCs upon exposure to different doses of tested formulations.

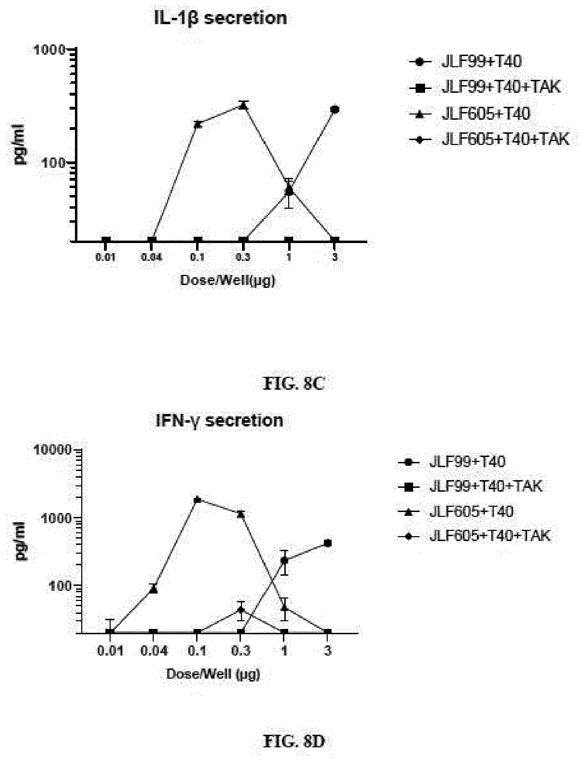

**FIG.8C** is a scatter plot illustrating quantified IL-1β secretion in hPBMCs upon exposure to different doses of tested formulations.
**FIG.8D** is a scatter plot illustrating quantified IFN-y secretion in hPBMCs upon exposure to different doses of tested formulations.

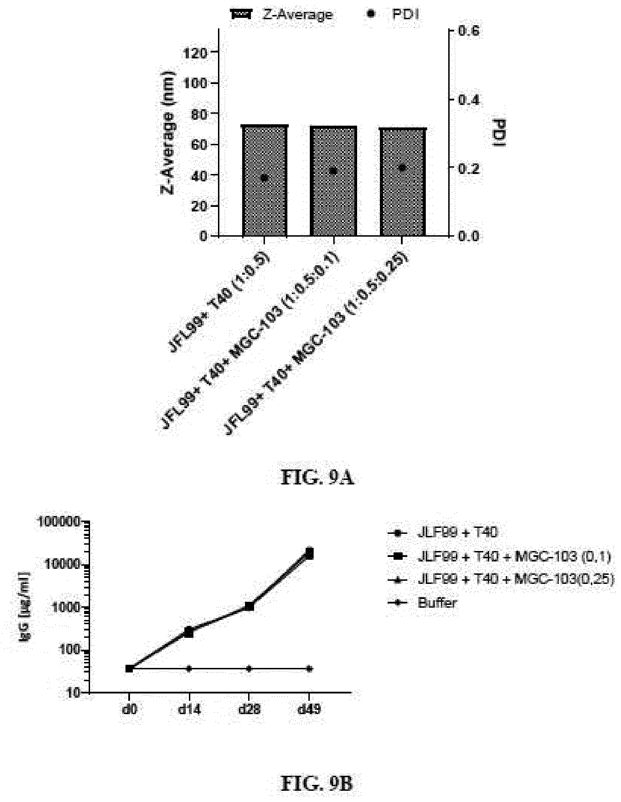

**FIG. 9A** is a bar graph illustrating hydrodynamic diameter of different complexes formulated at N/P6.
**FIG. 9B** is a scatter plot illustrating serological levels of specific IgG against HA at different time points over the course of the experiment (day 0, day 14, day 28, day 49).

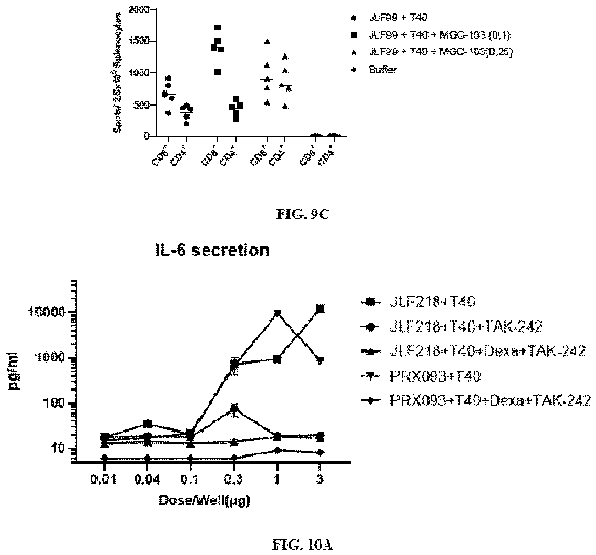

**FIG. 9C** is a plot illustrating quantified spots count in an IFN-ELISPOT read-out, reflecting CD4/CD8 T-Cell response against HA-peptide pools.
**FIG. 10A** is a scatter plot illustrating quantified IL-6 secretion in hPBMCs upon exposure to different doses of tested formulations.

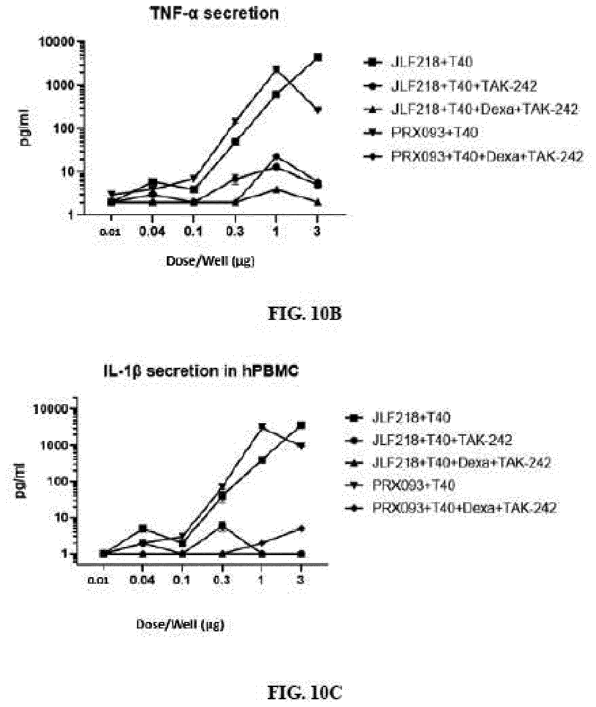

**FIG.10B** is a scatter plot illustrating quantified TNF-α secretion in hPBMCs upon exposure to different doses of tested formulations.
**FIG.10C** is a scatter plot illustrating quantified IL-1β secretion in hPBMCs upon exposure to different doses of tested formulations.

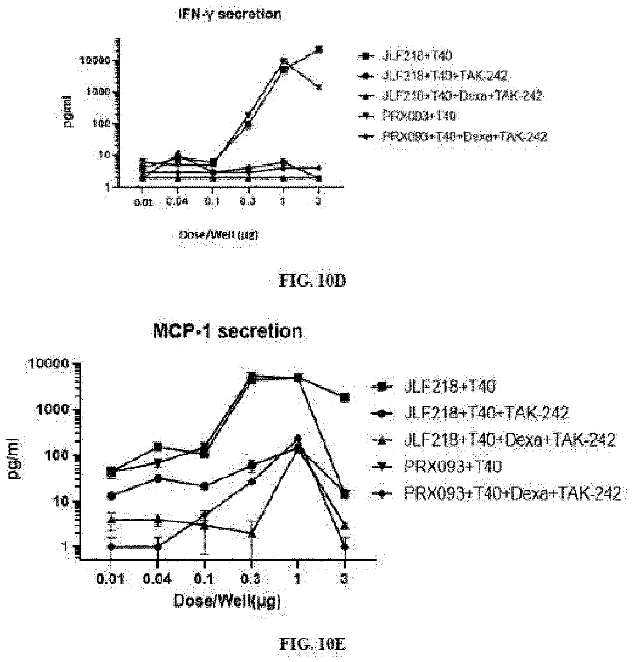

**FIG.10D** is a scatter plot illustrating quantified IFN-γ secretion in hPBMCs upon exposure to different doses of tested formulations.
**FIG.10E** is a scatter plot illustrating quantified MCP-1 secretion in hPBMCs upon exposure to different doses of tested formulations.

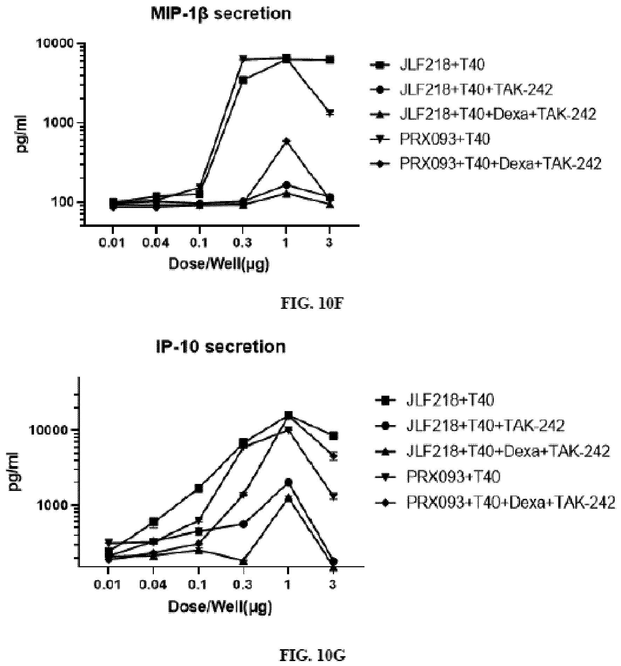

**FIG.10F** is a scatter plot illustrating quantified MIP-1β secretion in hPBMCs upon exposure to different doses of tested formulations.
**FIG.10G** is a scatter plot illustrating quantified IP-10 secretion in hPBMCs upon exposure to different doses of tested formulations.

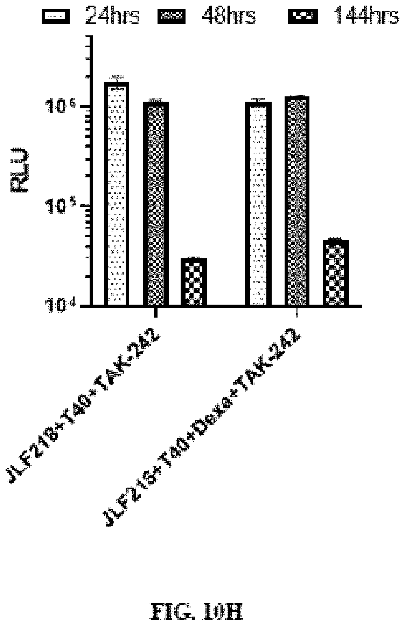

**FIG.10H** is a bar graph illustrating the cumulative luciferase expression in a dose range between 0.01-1µg for both tested groups over the course of 6 days after transfection.
**FIG.101** is a bar graph illustrating the cumulative luciferase expression in a dose range between 0.01-1µg for all tested groups at different time points over the course of the experiment (24 hours, 48 hours, 144 hours).

### Detailed Description of Certain Embodiments

The present disclosure, provides, among other things, a complex comprising a cationic oligosaccharide that efficiently delivers therapy (e.g., nucleic acid therapy) to a target tissue or cell in a patient. Complexes of the present disclosure avoid problems associated with other delivery systems by, for example, allowing for lower doses, and further, by avoiding particular adverse events (e.g., increased inflammatory activity manifesting as pain, swelling, and the like) that are associated with alternative and previous delivery systems. In some embodiments, the present disclosure provides among other things, a complex comprising i) a cationic oligosaccharide comprising one or more cationic moieties bonded to a trehalose, a sucrose, or a gluco-n-oligosaccharide moiety, where n is 2-6; ii) a surfactant; and iii) one or more additives selected from: a sterol, a helper lipid, an immunomodulator, and a targeting molecule.

### Definitions

Compounds of this disclosure include those described generally above and are further illustrated by the classes, subclasses, and species disclosed herein. As used herein, the following definitions shall apply unless otherwise indicated. For purposes of this disclosure, the chemical elements are identified in accordance with the Periodic Table of Elements, CAS version, Handbook of Chemistry and Physics, 75^{th} Ed. Additionally, general principles of organic chemistry are described in "Organic Chemistry", Thomas Sorrell, University Science Books, Sausalito: 1999, and "March's Advanced Organic Chemistry", 5th Ed., Ed.: Smith, M.B. and March, J., John Wiley & Sons, New York: 2001, the entire contents of which are hereby incorporated by reference.

Unless otherwise stated, structures depicted herein are meant to include all stereoisomeric (e.g., enantiomeric or diastereomeric) forms of the structure, as well as all geometric or conformational isomeric forms of the structure. For example, the R and S configurations of each stereocenter are contemplated as part of the disclosure. Therefore, single stereochemical isomers, as well as enantiomeric, diastereomeric, and geometric (or conformational) mixtures of provided compounds are within the scope of the disclosure. For example, in some cases, Tables 1 and 2 show one or more stereoisomers of a compound, and unless otherwise indicated, represents each stereoisomer alone and/or as a mixture. Unless otherwise stated, all tautomeric forms of provided compounds are within the scope of the disclosure.

Unless otherwise indicated, structures depicted herein are meant to include compounds that differ only in the presence of one or more isotopically enriched atoms. For example, compounds having the present structures including replacement of hydrogen by deuterium or tritium, or replacement of a carbon by ¹³C- or ¹⁴C-enriched carbon are within the scope of this disclosure.

***About or approximately:*** As used herein, the term "approximately" or "about," as applied to one or more values of interest, refers to a value that is similar to a stated reference value. In general, those skilled in the art, familiar within the context, will appreciate the relevant degree of variance encompassed by "about" or "approximately" in that context. For example, in some embodiments, the term "approximately" or "about" may encompass a range of values that are within (i.e., ±) 25%, 20%, 19%, 18%, 17%, 16%, 15%, 14%, 13%, 12%, 11%, 10%, 9%, 8%, 7%, 6%, 5%, 4%, 3%, 2%, 1%, or less of the referred value.

***Administering:*** As used herein, the term "administering" or "administration" typically refers to the administration of a composition to a subject to achieve delivery of an agent that is, or is included in, a composition to a target site or a site to be treated. Those of ordinary skill in the art will be aware of a variety of routes that may, in appropriate circumstances, be utilized for administration to a subject, for example a human. For example, in some embodiments, administration may be ocular, oral, parenteral, topical, *etc.* In some particular embodiments, administration may be bronchial *(e.g.,* by bronchial instillation), buccal, dermal (which may be or comprise, for example, one or more of topical to the dermis, intradermal, interdermal, transdermal, *etc.),* enteral, intra-arterial, intradermal, intragastric, intramedullary, intramuscular, intranasal, intraperitoneal, intrathecal, intravenous, intraventricular, within a specific organ (e.g., intrahepatic), mucosal, nasal, oral, rectal, subcutaneous, sublingual, topical, tracheal (e.g., by intratracheal instillation), vaginal, vitreal, *etc.* In some embodiments, administration may be parenteral. In some embodiments, administration may be oral. In some particular embodiments, administration may be intravenous. In some particular embodiments, administration may be subcutaneous. In some embodiments, administration may involve only a single dose. In some embodiments, administration may involve application of a fixed number of doses. In some embodiments, administration may involve dosing that is intermittent *(e.g.,* a plurality of doses separated in time) and/or periodic *(e.g.,* individual doses separated by a common period of time) dosing. In some embodiments, administration may involve continuous dosing (e.g., perfusion) for at least a selected period of time. In some embodiments, administration may comprise a prime-and-boost protocol. A prime-and-boost protocol can include administration of a first dose of a pharmaceutical composition (e.g., an immunogenic composition, e.g., a vaccine) followed by, after an interval of time, administration of a second or subsequent dose of a pharmaceutical composition (e.g., an immunogenic composition, e.g., a vaccine). In the case of an immunogenic composition, a prime-and-boost protocol can result in an increased immune response in a patient.

***Aliphatic:*** The term "aliphatic" refers to a straight-chain (i.e., unbranched) or branched, substituted or unsubstituted hydrocarbon chain that is completely saturated or that contains one or more units of unsaturation, or a monocyclic hydrocarbon or bicyclic hydrocarbon that is completely saturated or that contains one or more units of unsaturation, but which is not aromatic (also referred to herein as "cycloaliphatic"), that has a single point or more than one points of attachment to the rest of the molecule. Unless otherwise specified, aliphatic groups contain 1-12 aliphatic carbon atoms. As used herein, it is understood that an aliphatic group can also be bivalent (e.g., encompass a bivalent hydrocarbon chain that is saturated or contains one or more units of unsaturation, such as, for example, -CH₂-, -CH₂-CH₂-, -CH₂-CH₂-CH₂-, and so on). In some embodiments, aliphatic groups contain 1-6 aliphatic carbon atoms (e.g., C₁₋₆). In some embodiments, aliphatic groups contain 1-5 aliphatic carbon atoms (e.g., C₁₋₅). In other embodiments, aliphatic groups contain 1-4 aliphatic carbon atoms (e.g., C₁₋₄). In still other embodiments, aliphatic groups contain 1-3 aliphatic carbon atoms (e.g., C₁₋₃), and in yet other embodiments, aliphatic groups contain 1-2 aliphatic carbon atoms (e.g., C₁₋₂). In some embodiments, "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule. Suitable aliphatic groups include, but are not limited to, linear or branched, substituted or unsubstituted alkyl, alkenyl, or alkynyl groups and hybrids thereof. A preferred aliphatic group is C₁₋₆ alkyl.

***Alkyl:*** The term "alkyl", used alone or as part of a larger moiety, refers to a saturated, optionally substituted straight or branched chain hydrocarbon group having (unless otherwise specified) 1-12, 1-10, 1-8, 1-6, 1-4, 1-3, or 1-2 carbon atoms (e.g., C₁₋₁₂, C₁₋₁₀, C₁₋₈, C₁₋₆, C₁₋₄, C₁₋₃, or C₁₋₂). Exemplary alkyl groups include methyl, ethyl, propyl, butyl, pentyl, hexyl, and heptyl.

***Alkenyl:*** The term "alkenyl", used alone or as part of a larger moiety, refers to an optionally substituted straight or branched chain or cyclic hydrocarbon group having at least one double bond and having (unless otherwise specified) 2-12, 2-10, 2-8, 2-6, 2-4, or 2-3 carbon atoms (e.g., C₂₋₁₂, C₂₋₁₀, C₂₋₈, C₂₋₆, C₂₋₄, or C₂₋₃). Exemplary alkenyl groups include ethenyl, propenyl, butenyl, pentenyl, hexenyl, and heptenyl. The term "cycloalkenyl" refers to an optionally substituted non-aromatic monocyclic or multicyclic ring system containing at least one carbon-carbon double bond and having about 3 to about 10 carbon atoms. Exemplary monocyclic cycloalkenyl rings include cyclopentenyl, cyclohexenyl, and cycloheptenyl.

***Alkynyl:*** The term "alkynyl", used alone or as part of a larger moiety, refers to an optionally substituted straight or branched chain hydrocarbon group having at least one triple bond and having (unless otherwise specified) 2-12, 2-10, 2-8, 2-6, 2-4, or 2-3 carbon atoms (e.g., C₂₋₁₂, C₂₋₁₀, C₂₋₈, C₂₋₆, C₂₋₄, or C₂₋₃). Exemplary alkynyl groups include ethynyl, propynyl, butynyl, pentynyl, hexynyl, and heptynyl.

***Analog:*** As used herein, the term "analog" refers to a substance that shares one or more particular structural features, elements, components, or moieties with a reference substance. Typically, an "analog" shows significant structural similarity with the reference substance, for example sharing a core or consensus structure, but also differs in certain discrete ways. In some embodiments, an analog is a substance that can be generated from the reference substance, e.g., by chemical manipulation of the reference substance. In some embodiments, an analog is a substance that can be generated through performance of a synthetic process substantially similar to (e.g., sharing a plurality of steps with) one that generates the reference substance. In some embodiments, an analog is or can be generated through performance of a synthetic process different from that used to generate the reference substance.

***Aryl:*** The term "aryl" refers to monocyclic and bicyclic ring systems having a total of five to fourteen ring members (e.g., C₅-C₁₄), wherein at least one ring in the system is aromatic and wherein each ring in the system contains three to seven ring members. In some embodiments, an "aryl" group contains between six and twelve total ring members (e.g., C₆-C₁₂). The term "aryl" may be used interchangeably with the term "aryl ring". In certain embodiments of the present invention, "aryl" refers to an aromatic ring system which includes, but not limited to, phenyl, biphenyl, naphthyl, anthracyl and the like, which may bear one or more substituents. Unless otherwise specified, "aryl" groups are hydrocarbons. In some embodiments, an "aryl" ring system is an aromatic ring (e.g., phenyl) that is fused to a non-aromatic ring (e.g., cycloalkyl). Examples of aryl rings include that are fused include

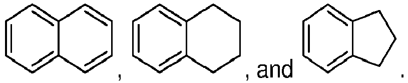

***Associated:*** Two events or entities are "associated" with one another, as that term is used herein, if the presence, level and/or form of one is correlated with that of the other. For example, a particular entity (e.g., polypeptide, genetic signature, metabolite, microbe, etc) is considered to be associated with a particular disease, disorder, or condition, if its presence, level and/or form correlates with incidence of and/or susceptibility to the disease, disorder, or condition (e.g., across a relevant population). In some embodiments, two or more entities are physically "associated" with one another if they interact, directly or indirectly, so that they are and/or remain in physical proximity with one another. In some embodiments, two or more entities that are physically associated with one another are covalently linked to one another; in some embodiments, two or more entities that are physically associated with one another are not covalently linked to one another but are non-covalently associated, for example by means of hydrogen bonds, van der Waals interaction, hydrophobic interactions, magnetism, and combinations thereof.

***Biological sample:*** As used herein, the term "biological sample" typically refers to a sample obtained or derived from a biological source (e.g., a tissue or organism or cell culture) of interest, as described herein. In some embodiments, a source of interest comprises an organism, such as an animal or human. In some embodiments, a biological sample is or comprises biological tissue or fluid. In some embodiments, a biological sample may be or comprise bone marrow; blood; blood cells; ascites; tissue or fine needle biopsy samples; cell-containing body fluids; free floating nucleic acids; sputum; saliva; urine; cerebrospinal fluid, peritoneal fluid; pleural fluid; feces; lymph; gynecological fluids; skin swabs; vaginal swabs; oral swabs; nasal swabs; washings or lavages such as a ductal lavages or broncheoalveolar lavages; aspirates; scrapings; bone marrow specimens; tissue biopsy specimens; surgical specimens; feces, other body fluids (e.g., sperm, sweat, tears), secretions, and/or excretions; and/or cells therefrom, etc. In some embodiments, a biological sample is or comprises cells obtained from an individual. In some embodiments, obtained cells are or include cells from an individual from whom the sample is obtained. In some embodiments, a sample is a "primary sample" obtained directly from a source of interest by any appropriate means. For example, in some embodiments, a primary biological sample is obtained by methods selected from the group consisting of biopsy (e.g., fine needle aspiration or tissue biopsy), surgery, collection of body fluid (e.g., blood, lymph, feces etc.), etc. In some embodiments, as will be clear from context, the term "sample" refers to a preparation that is obtained by processing (e.g., by removing one or more components of and/or by adding one or more agents to) a primary sample. For example, filtering using a semipermeable membrane. Such a "processed sample" may comprise, for example, nucleic acids or proteins extracted from a sample or obtained by subjecting a primary sample to techniques such as amplification or reverse transcription of mRNA, isolation and/or purification of certain components, etc.

***Carrier:*** As used herein, the term "carrier" refers to a diluent, adjuvant, excipient, or vehicle with which a composition is administered. In some exemplary embodiments, carriers can include sterile liquids, such as, for example, water and oils, including oils of petroleum, animal, vegetable or synthetic origin, such as, for example, peanut oil, soybean oil, mineral oil, sesame oil and the like. In some embodiments, carriers are or include one or more solid components.

***Combination therapy:*** As used herein, the term "combination therapy" refers to those situations in which a subject is simultaneously exposed to two or more therapeutic regimens (e.g., two or more therapeutic agents or modality(ies)). In some embodiments, the two or more regimens may be administered simultaneously; in some embodiments, such regimens may be administered sequentially (e.g., all "doses" of a first regimen are administered prior to administration of any doses of a second regimen); in some embodiments, such agents are administered in overlapping dosing regimens. In some embodiments, "administration" of combination therapy may involve administration of one or more agent(s) or modality(ies) to a subject receiving the other agent(s) or modality(ies) in the combination. For clarity, combination therapy does not require that individual agents be administered together in a single composition (or even necessarily at the same time), although in some embodiments, two or more agents, or active moieties thereof, may be administered together in a combination composition, or even in a combination compound (e.g., as part of a single chemical complex or covalent entity).

***Comparable:*** As used herein, the term "comparable" refers to two or more agents, entities, situations, sets of conditions, etc., that may not be identical to one another but that are sufficiently similar to permit comparison therebetween so that one skilled in the art will appreciate that conclusions may reasonably be drawn based on differences or similarities observed. In some embodiments, comparable sets of conditions, circumstances, individuals, or populations are characterized by a plurality of substantially identical features and one or a small number of varied features. Those of ordinary skill in the art will understand, in context, what degree of identity is required in any given circumstance for two or more such agents, entities, situations, sets of conditions, etc. to be considered comparable. For example, those of ordinary skill in the art will appreciate that sets of circumstances, individuals, or populations are comparable to one another when characterized by a sufficient number and type of substantially identical features to warrant a reasonable conclusion that differences in results obtained or phenomena observed under or with different sets of circumstances, individuals, or populations are caused by or indicative of the variation in those features that are varied.

***Composition:*** Those skilled in the art will appreciate that the term "composition" may be used to refer to a discrete physical entity that comprises one or more specified components. In general, unless otherwise specified, a composition may be of any form - e.g., gas, gel, liquid, solid, etc.

***Cycloaliphatic:*** As used herein, the term "cycloaliphatic" refers to a monocyclic C₃₋₈ hydrocarbon or a bicyclic C₇₋₁₀ hydrocarbon that is completely saturated or that contains one or more units of unsaturation, but which is not aromatic, that has a single point or more than one points of attachment to the rest of the molecule.

***Cycloalkyl:*** As used herein, the term "cycloalkyl" refers to an optionally substituted saturated ring monocyclic or polycyclic system of about 3 to about 10 ring carbon atoms. Exemplary monocyclic cycloalkyl rings include cyclopropyl, cyclobutyl, cyclopentyl, cyclohexyl, and cycloheptyl.

***Dosage form*** or ***unit dosage form:*** Those skilled in the art will appreciate that the term "dosage form" may be used to refer to a physically discrete unit of an active agent (e.g., a therapeutic or diagnostic agent) for administration to a subject. Typically, each such unit contains a predetermined quantity of active agent. In some embodiments, such quantity is a unit dosage amount (or a whole fraction thereof) appropriate for administration in accordance with a dosing regimen that has been determined to correlate with a desired or beneficial outcome when administered to a relevant population (*i.e.,* with a therapeutic dosing regimen). Those of ordinary skill in the art appreciate that the total amount of a therapeutic composition or agent administered to a particular subject is determined by one or more attending physicians and may involve administration of multiple dosage forms.

***Dosing regimen*** or ***therapeutic regimen:*** Those skilled in the art will appreciate that the terms "dosing regimen" and "therapeutic regimen" may be used to refer to a set of unit doses (typically more than one) that are administered individually to a subject, typically separated by periods of time. In some embodiments, a given therapeutic agent has a recommended dosing regimen, which may involve one or more doses. In some embodiments, a dosing regimen comprises a plurality of doses each of which is separated in time from other doses. In some embodiments, individual doses are separated from one another by a time period of the same length; in some embodiments, a dosing regimen comprises a plurality of doses and at least two different time periods separating individual doses. In some embodiments, all doses within a dosing regimen are of the same unit dose amount. In some embodiments, different doses within a dosing regimen are of different amounts. In some embodiments, a dosing regimen comprises a first dose in a first dose amount, followed by one or more additional doses in a second dose amount different from the first dose amount. In some embodiments, a dosing regimen comprises a first dose in a first dose amount, followed by one or more additional doses in a second dose amount same as the first dose amount. In some embodiments, a dosing regimen is correlated with a desired or beneficial outcome when administered across a relevant population (*i.e.,* is a therapeutic dosing regimen).

***Excipient:*** As used herein, the term "excipient" refers to a non-therapeutic agent that may be included in a pharmaceutical composition, for example, to provide or contribute to a desired consistency or stabilizing effect. Suitable pharmaceutical excipients include, for example, starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like.

***Heteroaliphatic:*** The term "heteroaliphatic" or "heteroaliphatic group", as used herein, denotes an optionally substituted hydrocarbon moiety having, in addition to carbon atoms, from one to five heteroatoms, that may be straight-chain (*i.e.,* unbranched), branched, or cyclic ("heterocyclic") and may be completely saturated or may contain one or more units of unsaturation, but which is not aromatic. The term "heteroatom" refers to nitrogen, oxygen, or sulfur, and includes any oxidized form of nitrogen or sulfur, and any quaternized form of a basic nitrogen. The term "nitrogen" also includes a substituted nitrogen. Unless otherwise specified, heteroaliphatic groups contain 1-10 carbon atoms wherein 1-3 carbon atoms are optionally and independently replaced with heteroatoms selected from oxygen, nitrogen, and sulfur. In some embodiments, heteroaliphatic groups contain 1-4 carbon atoms, wherein 1-2 carbon atoms are optionally and independently replaced with heteroatoms selected from oxygen, nitrogen, and sulfur. In yet other embodiments, heteroaliphatic groups contain 1-3 carbon atoms, wherein 1 carbon atom is optionally and independently replaced with a heteroatom selected from oxygen, nitrogen, and sulfur. Suitable heteroaliphatic groups include, but are not limited to, linear or branched, heteroalkyl, heteroalkenyl, and heteroalkynyl groups. For example, a 1- to 10 atom heteroaliphatic group includes the following exemplary groups: -O-CH₃, -CH₂-O-CH₃, -O-CH₂-CH₂-O-CH₂-CH₂-O-CH₃, and the like.

***Heteroaryl:*** The terms "heteroaryl" and "heteroar-", used alone or as part of a larger moiety, e.g., "heteroaralkyl", or "heteroaralkoxy", refer to monocyclic or bicyclic ring groups having 5 to 12 ring atoms (e.g., 5- to 6- membered monocyclic heteroaryl or 9- to 12-membered bicyclic heteroaryl); having 6, 10, or 14 π-electrons shared in a cyclic array; and having, in addition to carbon atoms, from one to five heteroatoms. The term "heteroatom" refers to nitrogen, oxygen, or sulfur, and includes any oxidized form of nitrogen or sulfur, and any quaternized form of a basic nitrogen. Heteroaryl groups include, without limitation, thienyl, furanyl, pyrrolyl, imidazolyl, pyrazolyl, triazolyl, tetrazolyl, oxazolyl, isoxazolyl, oxadiazolyl, thiazolyl, isothiazolyl, thiadiazolyl, pyridyl, pyridazinyl, pyrimidinyl, pyrazinyl, indolizinyl, purinyl, naphthyridinyl, pteridinyl, imidazo[1,2-a]pyrimidinyl, imidazo[1,2-a]pyridyl, imidazo[4,5-b]pyridyl, imidazo[4,5-c]pyridyl, pyrrolopyridyl, pyrrolopyrazinyl, thienopyrimidinyl, triazolopyridyl, and benzoisoxazolyl. The terms "heteroaryl" and "heteroar-", as used herein, also include groups in which a heteroaromatic ring is fused to one or more aryl, cycloaliphatic, or heterocyclyl rings, where the radical or point of attachment is on the heteroaromatic ring (i.e., a bicyclic heteroaryl ring having 1 to 3 heteroatoms). Nonlimiting examples include indolyl, isoindolyl, benzothienyl, benzofuranyl, dibenzofuranyl, indazolyl, benzimidazolyl, benzotriazolyl, benzothiazolyl, benzothiadiazolyl, benzoxazolyl, quinolyl, isoquinolyl, cinnolinyl, phthalazinyl, quinazolinyl, quinoxalinyl, 4H-quinolizinyl, carbazolyl, acridinyl, phenazinyl, phenothiazinyl, phenoxazinyl, tetrahydroquinolinyl, tetrahydroisoquinolinyl, pyrido[2,3-b]-1,4-oxazin-3(4H)-one, 4H-thieno[3,2-b]pyrrole, and benzoisoxazolyl. A heteroaryl group may be mono- or bicyclic. The term "heteroaryl" may be used interchangeably with the terms "heteroaryl ring", "heteroaryl group", or "heteroaromatic", any of which terms include rings that are optionally substituted. The term "heteroaralkyl" refers to an alkyl group substituted by a heteroaryl, wherein the alkyl and heteroaryl portions independently are optionally substituted.

***Heteroatom:*** The term "heteroatom" as used herein refers to nitrogen, oxygen, or sulfur, and includes any oxidized form of nitrogen or sulfur, and any quaternized form of a basic nitrogen.

***Heterocycle:*** As used herein, the terms "heterocycle", "heterocyclyl", "heterocyclic radical", and "heterocyclic ring" are used interchangeably and refer to a stable 3- to 8-membered monocyclic, a 7- to 12-membered bicyclic, or a 10- to 16-membered polycyclic heterocyclic moiety that is either saturated or partially unsaturated, and having, in addition to carbon atoms, one or more, such as one to four, heteroatoms, as defined above. When used in reference to a ring atom of a heterocycle, the term "nitrogen" includes a substituted nitrogen. As an example, in a saturated or partially unsaturated ring having 0-3 heteroatoms selected from oxygen, sulfur or nitrogen, the nitrogen may be N (as in 3,4-dihydro-2H-pyrrolyl), NH (as in pyrrolidinyl), or NR⁺ (as in N-substituted pyrrolidinyl). A heterocyclic ring can be attached to its pendant group at any heteroatom or carbon atom that results in a stable structure and any of the ring atoms can be optionally substituted. Examples of such saturated or partially unsaturated heterocyclic radicals include, without limitation, azetidinyl, oxetanyl, tetrahydrofuranyl, tetrahydrothienyl, pyrrolidinyl, piperidinyl, decahydroquinolinyl, oxazolidinyl, piperazinyl, dioxanyl, dioxolanyl, diazepinyl, oxazepinyl, thiazepinyl, morpholinyl, and thiamorpholinyl. A heterocyclyl group may be mono-, bi-, tri-, or polycyclic, preferably mono-, bi-, or tricyclic, more preferably mono- or bicyclic. The term "heterocyclylalkyl" refers to an alkyl group substituted by a heterocyclyl, wherein the alkyl and heterocyclyl portions independently are optionally substituted. A bicyclic heterocyclic ring also includes groups in which the heterocyclic ring is fused to one or more aryl rings. Exemplary bicyclic heterocyclic groups include indolinyl, isoindolinyl, benzodioxolyl, 1,3-dihydroisobenzofuranyl, 2,3-dihydrobenzofuranyl, tetrahydroquinolinyl, 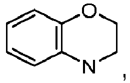 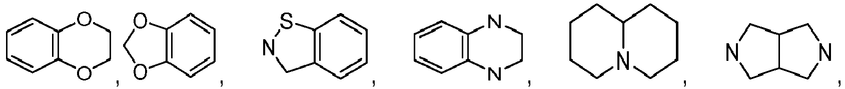 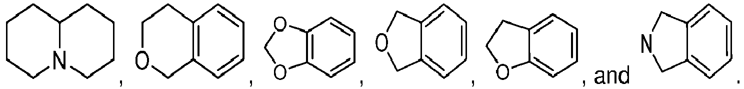 A bicyclic heterocyclic ring can also be a spirocyclic ring system (e.g., 7- to 11-membered spirocyclic fused heterocyclic ring having, in addition to carbon atoms, one or more heteroatoms as defined above (e.g., one, two, three or four heteroatoms)). A bicyclic heterocyclic ring can also be a bridged ring system (e.g., 7- to 11-membered bridged heterocyclic ring having one, two, or three bridging atoms.

***Immunomodulator:*** As used herein, the term "immunomodulator" refers to an agent (e.g., a small molecule, a protein, a nucleic acid, etc.) that acts as a immunostimulant or an immunosuppressant. An immunostimulant refers to an agent that stimulate the immune system by inducing activation or increasing activity of any of its components. An immunosuppressant refers to an agent that inhibit or prevent activity of the immune system.

***Nucleic acid*/ *Polynucleotide:*** As used herein, the term "nucleic acid" refers to a polymer of at least 10 nucleotides or more. In some embodiments, a nucleic acid is or comprises DNA. In some embodiments, a nucleic acid is or comprises RNA. In some embodiments, a nucleic acid is or comprises peptide nucleic acid (PNA). In some embodiments, a nucleic acid is or comprises a single stranded nucleic acid. In some embodiments, a nucleic acid is or comprises a double-stranded nucleic acid. In some embodiments, a nucleic acid comprises both single and double-stranded portions. In some embodiments, a nucleic acid comprises a backbone that comprises one or more phosphodiester linkages. In some embodiments, a nucleic acid comprises a backbone that comprises both phosphodiester and non-phosphodiester linkages. For example, in some embodiments, a nucleic acid may comprise a backbone that comprises one or more phosphorothioate or 5'-N-phosphoramidite linkages and/or one or more peptide bonds, *e.g.,* as in a "peptide nucleic acid". In some embodiments, a nucleic acid comprises one or more, or all, natural residues (*e.g.,* adenine, cytosine, deoxyadenosine, deoxycytidine, deoxyguanosine, deoxythymidine, guanine, thymine, uracil). In some embodiments, a nucleic acid comprises on or more, or all, non-natural residues. In some embodiments, a non-natural residue comprises a nucleoside analog (*e.g.,* 2-aminoadenosine, 2-thiothymidine, inosine, pyrrolo-pyrimidine, 3 -methyl adenosine, 5-methylcytidine, C-5 propynyl-cytidine, C-5 propynyl-uridine, 2-aminoadenosine, C5-bromouridine, C5-fluorouridine, C5-iodouridine, C5-propynyl-uridine, C5 -propynyl-cytidine, C5-methylcytidine, 2-aminoadenosine, 7-deazaadenosine, 7-deazaguanosine, 8-oxoadenosine, 8-oxoguanosine, 6-O-methylguanine, 2-thiocytidine, methylated bases, intercalated bases, and combinations thereof). In some embodiments, a non-natural residue comprises one or more modified sugars (*e.g.,* 2'-fluororibose, ribose, 2'-deoxyribose, arabinose, and hexose) as compared to those in natural residues. In some embodiments, a nucleic acid has a nucleotide sequence that encodes a functional gene product such as an RNA or polypeptide. In some embodiments, a nucleic acid has a nucleotide sequence that comprises one or more introns. In some embodiments, a nucleic acid may be prepared by isolation from a natural source, enzymatic synthesis *(e.g.,* by polymerization based on a complementary template, *e.g., in vivo* or *in vitro,* reproduction in a recombinant cell or system, or chemical synthesis. In some embodiments, a nucleic acid is at least 3, 4, 5, 6, 7, 8, 9, 10, 15, 20, 25, 30, 35, 40, 45, 50, 55, 60, 65, 70, 75, 80, 85, 90, 95, 100, 110, 120, 130, 140, 150, 160, 170, 180, 190, 20, 225, 250, 275, 300, 325, 350, 375, 400, 425, 450, 475, 500, 600, 700, 800, 900, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 15,500, 16,000, 16,500, 17,000, 17,500, 18,000, 18,500, 19,000, 19,500, or 20,000 or more residues or nucleotides long.

***Nucleic acid particle:*** A "nucleic acid particle" can be used to deliver nucleic acid to a target site of interest (e.g., cell, tissue, organ, and the like). A nucleic acid particle may be formed from at least one cationic or cationically ionizable lipid or lipid-like material, at least one cationic polymer such as protamine, or a mixture thereof and nucleic acid. Nucleic acid particles include lipid nanoparticle (LNP)-based and lipoplex (LPX)-based formulations.

***Nucleotide:*** As used herein, the term "nucleotide" refers to its art-recognized meaning. When a number of nucleotides is used as an indication of size, e.g., of a polynucleotide, a certain number of nucleotides refers to the number of nucleotides on a single strand, *e.g.,* of a polynucleotide.

***Oral:*** The phrases "oral administration" and "administered orally" as used herein have their art-understood meaning referring to administration by mouth of a compound or composition.

***Parenteral:*** The phrases "parenteral administration" and "administered parenterally" as used herein have their art-understood meaning referring to modes of administration other than enteral and topical administration, usually by injection, and include, without limitation, intravenous, intramuscular, intra-arterial, intrathecal, intracapsular, intraorbital, intracardiac, intradermal, intraperitoneal, transtracheal, subcutaneous, subcuticular, intraarticulare, subcapsular, subarachnoid, intraspinal, and intrasternal injection and infusion.

***Partially unsaturated:*** As used herein, the term "partially unsaturated" refers to a ring moiety that includes at least one double or triple bond between ring atoms. The term "partially unsaturated" is intended to encompass rings having multiple sites of unsaturation, but is not intended to include aromatic (e.g., aryl or heteroaryl) moieties, as herein defined.

***Patient*** or ***subject:*** As used herein, the term "patient" or "subject" refers to any organism to which a provided composition is or may be administered, e.g., for experimental, diagnostic, prophylactic, cosmetic, and/or therapeutic purposes. Typical patients or subjects include animals (e.g., mammals such as mice, rats, rabbits, non-human primates, and/or humans). In some embodiments, a patient is a human. In some embodiments, a patient or a subject is suffering from or susceptible to one or more disorders or conditions. In some embodiments, a patient or subject displays one or more symptoms of a disorder or condition. In some embodiments, a patient or subject has been diagnosed with one or more disorders or conditions. In some embodiments, a patient or a subject is receiving or has received certain therapy to diagnose and/or to treat a disease, disorder, or condition.

***Pharmaceutical composition:*** As used herein, the term "pharmaceutical composition" refers to an active agent, formulated together with one or more pharmaceutically acceptable carriers. In some embodiments, the active agent is present in unit dose amount appropriate for administration in a therapeutic or dosing regimen that shows a statistically significant probability of achieving a predetermined therapeutic effect when administered to a relevant population. In some embodiments, pharmaceutical compositions may be specially formulated for administration in solid or liquid form, including those adapted for the following: oral administration, for example, drenches (aqueous or non-aqueous solutions or suspensions), tablets, e.g., those targeted for buccal, sublingual, and systemic absorption, boluses, powders, granules, pastes for application to the tongue; parenteral administration, for example, by subcutaneous, intramuscular, intravenous or epidural injection as, for example, a sterile solution or suspension, or sustained-release formulation; topical application, for example, as a cream, ointment, or a controlled-release patch or spray applied to the skin, lungs, or oral cavity; intravaginally or intrarectally, for example, as a pessary, cream, or foam; sublingually; ocularly; transdermally; or nasally, pulmonary, and to other mucosal surfaces.

***Pharmaceutically acceptable:*** As used herein, the phrase "pharmaceutically acceptable" refers to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio.

***Pharmaceutically acceptable carrier:*** As used herein, the term "pharmaceutically acceptable carrier" means a pharmaceutically-acceptable material, composition or vehicle, such as a liquid or solid filler, diluent, excipient, or solvent encapsulating material, involved in carrying or transporting the subject compound from one organ, or portion of the body, to another organ, or portion of the body. Each carrier must be "acceptable" in the sense of being compatible with the other ingredients of the formulation and not injurious to the patient. Some examples of materials which can serve as pharmaceutically-acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; pH buffered solutions; polyesters, polycarbonates and/or polyanhydrides; and other non-toxic compatible substances employed in pharmaceutical formulations.

***Pharmaceutically acceptable salt:*** The term "pharmaceutically acceptable salt", as used herein, refers to salts of such compounds that are appropriate for use in pharmaceutical contexts, *i.e.,* salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, et al. describes pharmaceutically acceptable salts in detail in J. Pharmaceutical Sciences, 66: 1-19 (1977). In some embodiments, pharmaceutically acceptable salts include, but are not limited to, nontoxic acid addition salts, which are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. In some embodiments, pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, *p-*toluenesulfonate, undecanoate, valerate salts, and the like. Representative alkali or alkaline earth metal salts include sodium, lithium, potassium, calcium, magnesium, and the like. In some embodiments, pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

***Polycyclic:*** As used herein, the term "polycyclic" refers to a saturated or unsaturated ring system having two or more rings (for example, heterocyclyl rings, heteroaryl rings, cycloalkyl rings, or aryl rings), having between 7 and 20 atoms, in which one or more carbon atoms are common to two adjacent rings. For example, in some embodiments, a polycyclic ring system refers to a saturated or unsaturated ring system having three or more rings (for example, heterocyclyl rings, heteroaryl rings, cycloalkyl rings, or aryl rings), having between 14 and 20 atoms, in which one or more carbon atoms are common to two adjacent rings. The rings in a polycyclic ring system may be fused (i.e., bicyclic or tricyclic), spirocyclic, or a combination thereof. An example polycyclic ring is a steroid.

***Polypeptide:*** The term "polypeptide", as used herein, typically has its art-recognized meaning of a polymer of at least three amino acids or more. Those of ordinary skill in the art will appreciate that the term "polypeptide" is intended to be sufficiently general as to encompass not only polypeptides having a complete sequence recited herein, but also to encompass polypeptides that represent functional, biologically active, or characteristic fragments, portions or domains (e.g., fragments, portions, or domains retaining at least one activity) of such complete polypeptides. In some embodiments, polypeptides may contain L-amino acids, D-amino acids, or both and/or may contain any of a variety of amino acid modifications or analogs known in the art. Useful modifications include, e.g., terminal acetylation, amidation, methylation, *etc.* In some embodiments, polypeptides may comprise natural amino acids, non-natural amino acids, synthetic amino acids, and combinations thereof (e.g., may be or comprise peptidomimetics).

***Prevent*** or ***prevention:*** As used herein, the terms "prevent" or "prevention", when used in connection with the occurrence of a disease, disorder, and/or condition, refer to reducing the risk of developing the disease, disorder and/or condition and/or to delaying onset of one or more characteristics or symptoms of the disease, disorder or condition. Prevention may be considered complete when onset of a disease, disorder or condition has been delayed for a predefined period of time.

***Reference:*** As used herein describes a standard or control relative to which a comparison is performed. For example, in some embodiments, an agent, animal, individual, population, sample, sequence or value of interest is compared with a reference or control agent, animal, individual, population, sample, sequence or value. In some embodiments, a reference or control is tested and/or determined substantially simultaneously with the testing or determination of interest. In some embodiments, a reference or control is a historical reference or control, optionally embodied in a tangible medium. Typically, as would be understood by those skilled in the art, a reference or control is determined or characterized under comparable conditions or circumstances to those under assessment. Those skilled in the art will appreciate when sufficient similarities are present to justify reliance on and/or comparison to a particular possible reference or control.

***Ribonucleotide:*** As used herein, the term "ribonucleotide" encompasses unmodified ribonucleotides and modified ribonucleotides. For example, unmodified ribonucleotides include the purine bases adenine (A) and guanine (G), and the pyrimidine bases cytosine (C) and uracil (U). Modified ribonucleotides may include one or more modifications including, but not limited to, for example, (a) end modifications, *e.g.,* 5' end modifications *(e.g.,* phosphorylation, dephosphorylation, conjugation, inverted linkages, *etc.),* 3' end modifications *(e.g.,* conjugation, inverted linkages, *etc.),* (b) base modifications, *e.g.* , replacement with modified bases, stabilizing bases, destabilizing bases, or bases that base pair with an expanded repertoire of partners, or conjugated bases, (c) sugar modifications (*e.g.,* at the 2' position or 4' position) or replacement of the sugar, and (d) internucleoside linkage modifications, including modification or replacement of the phosphodiester linkages. The term "ribonucleotide" also encompasses ribonucleotide triphosphates including modified and non-modified ribonucleotide triphosphates. ***Ribonucleic acid (RNA):*** As used herein, the term "RNA" refers to a polymer of ribonucleotides. In some embodiments, an RNA is single stranded. In some embodiments, an RNA is double stranded. In some embodiments, an RNA comprises both single and double stranded portions. In some embodiments, an RNA can comprise a backbone structure as described in the definition of *"Nucleic acid* / *Polynucleotide"* above. An RNA can be a regulatory RNA *(e.g.,* siRNA, microRNA, *etc.),* or a messenger RNA (mRNA). In some embodiments where an RNA is a mRNA. In some embodiments where an RNA is a mRNA, a RNA typically comprises at its 3' end a poly(A) region. In some embodiments where an RNA is a mRNA, an RNA typically comprises at its 5' end an art-recognized cap structure, e.g., for recognizing and attachment of a mRNA to a ribosome to initiate translation. In some embodiments, a RNA is a synthetic RNA. Synthetic RNAs include RNAs that are synthesized *in vitro* (*e.g.,* by enzymatic synthesis methods and/or by chemical synthesis methods).

***Sample:*** As used herein, the term "sample" typically refers to an aliquot of material obtained or derived from a source of interest. In some embodiments, a source of interest is a biological or environmental source. In some embodiments, a source of interest may be or comprise a cell, tissue, or organism, such as a microbe, a plant, or an animal (e.g., a human). In some embodiments, a source of interest is or comprises biological tissue or fluid. In some embodiments, a source of interest may be or comprise a preparation generated in a production run. In some embodiments, a sample is a "primary sample" obtained directly from a source of interest by any appropriate means. In some embodiments, as will be clear from context, the term "sample" refers to a preparation that is obtained by processing (e.g., by removing one or more components of and/or by adding one or more agents to) a primary sample.

***Substituted*** or ***optionally substituted:*** As described herein, compounds of the invention may contain "optionally substituted" moieties. In general, the term "substituted," whether preceded by the term "optionally" or not, means that one or more hydrogens of the designated moiety are replaced with a suitable substituent. "Substituted" applies to one or more hydrogens that are either explicit or implicit from the structure (e.g., 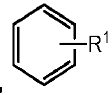 refers to at least 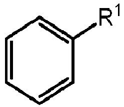 ; and 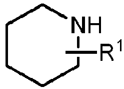 refers to at least 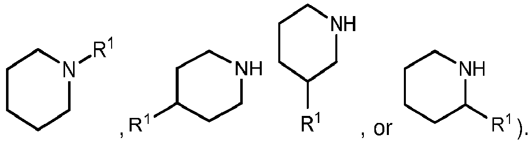 Unless otherwise indicated, an "optionally substituted" group may have a suitable substituent at each substitutable position of the group, and when more than one position in any given structure may be substituted with more than one substituent selected from a specified group, the substituent may be either the same or different at every position. Combinations of substituents envisioned by this invention are preferably those that result in the formation of stable or chemically feasible compounds. The term "stable," as used herein, refers to compounds that are not substantially altered when subjected to conditions to allow for their production, detection, and, in certain embodiments, their recovery, purification, and use for one or more of the purposes provided herein. Groups described as being "substituted" preferably have between 1 and 4 substituents, more preferably 1 or 2 substituents. Groups described as being "optionally substituted" may be unsubstituted or be "substituted" as described above.

Suitable monovalent substituents on a substitutable carbon atom of an "optionally substituted" group are independently halogen; -(CH₂)₀₋₄R°; -(CH₂)₀₋₄OR°; -O(CH₂)₀₋₄R°, -O-(CH₂)₀₋₄C(O)OR°; -(CH₂)₀₋₄CH(OR°)₂; -(CH₂)₀₋₄SR°; -(CH₂)₀₋₄Ph, which may be substituted with R°; -(CH₂)₀₋₄0(CH₂)₀₋₁Ph which may be substituted with R°; - CH=CHPh, which may be substituted with R°; -(CH₂)₀₋₄O(CH₂)₀₋₁-pyridyl which may be substituted with R°; -NO₂; -CN; -N₃; (CH₂)₀₋₄N(R°)₂; -(CH₂)₀₋₄N(R°)C(O)R°; - N(R°)C(S)R°; -(CH₂)₀₋₄N(R°)C(O)NR°2; N(R°)C(S)NR°2; -(CH₂)₀₋₄N(R°)C(O)OR°; - N(R°)N(R°)C(O)R°; N(R°)N(R°)C(O)NR°2; N(R°)N(R°)C(O)OR°; -(CH₂)₀₋₄C(O)R°; C(S)R°; -(CH₂)₀₋₄C(O)OR°; -(CH₂)₀₋₄C(O)SR°; (CH₂)₀₋₄C(O)OSiR°₃; -(CH₂)₀₋₄OC(O)R°; -OC(O)(CH₂)₀₋₄SR°; -(CH₂)₀₋₄SC(O)R°; -(CH₂)₀₋₄C(O)NR°₂; -C(S)NR°₂; -C(S)SR°; - SC(S)SR°, (CH₂)₀₋₄OC(O)NR°₂; C(O)N(OR°)R°; -C(O)C(O)R°; -C(O)CH₂C(O)R°; - C(NOR°)R°; (CH₂)₀₋₄SSR°; -(CH₂)₀₋₄S(O)₂R°; -(CH₂)₀₋₄S(O)₂OR°; -(CH₂)₀₋₄OS(O)₂R°; -S(O)₂NR°₂; (CH₂)₀₋₄S(O)R°; N(R°)S(O)₂NR°₂; -N(R°)S(O)₂R°; -N(OR°)R°; - C(NH)NR°₂; -P(O)₂R°; P(O)R°₂; OP(O)R°₂; -OP(O)(OR°)₂; SiR°₃; -(C₁₋₄ straight or branched alkylene)O-N(R°)₂; or -(C₁₋₄ straight or branched alkylene)C(O)O-N(R°)₂, wherein each R° may be substituted as defined below and is independently hydrogen, C₁₋₆ aliphatic, -CH₂Ph, -O(CH₂)₀₋₁Ph, -CH₂-(5- to 6-membered heteroaryl ring), or a 3-to 6-membered saturated, partially unsaturated, or aryl ring having 0-4 heteroatoms independently selected from nitrogen, oxygen, or sulfur, or, notwithstanding the definition above, two independent occurrences of R°, taken together with their intervening atom(s), form a 3- to 12-membered saturated, partially unsaturated, or aryl mono- or bicyclic ring having 0-4 heteroatoms independently selected from nitrogen, oxygen, or sulfur, which may be substituted as defined below.

Suitable monovalent substituents on R° (or the ring formed by taking two independent occurrences of R° together with their intervening atoms), are independently halogen, - (CH₂)₀₋₂R^{•}, -(haloR^{•}), -(CH₂)₀₋₂OH, -(CH₂)₀₋₂OR^{•}, -(CH₂)₀₋₂CH(OR^{•})₂, O(haloR^{•}), - CN, -N₃, -(CH₂)₀₋₂C(O)R^{•}, -(CH₂)₀₋₂C(O)OH, -(CH₂)₀₋₂C(O)OR^{•}, -(CH₂)₀₋₂SR^{•}, - (CH₂)₀₋₂SH, -(CH₂)₀₋₂NH₂, -(CH₂)₀₋₂NHR^{•}, -(CH₂)₀₋₂NR^{•}₂, -NO₂, -SiR^{•}₃, -OSiR^{•}₃, C(O)SR^{•}, -(C₁₋₄ straight or branched alkylene)C(O)OR^{•}, or -SSR^{•} wherein each R^{•} is unsubstituted or where preceded by "halo" is substituted only with one or more halogens, and is independently selected from C₁₋₄ aliphatic, -CH₂Ph, -O(CH₂)₀₋₁ Ph, or a 3- to 6-membered saturated, partially unsaturated, or aryl ring having 0-4 heteroatoms independently selected from nitrogen, oxygen, or sulfur. Suitable divalent substituents on a saturated carbon atom of R° include =O and =S.

Suitable divalent substituents on a saturated carbon atom of an "optionally substituted" group include the following: =O ("oxo"), =S, =NNR*₂, =NNHC(O)R*, =NNHC(O)OR*, =NNHS(O)₂R*, =NR*, =NOR*, -O(C(R*₂))₂₋₃O-, or -S(C(R*₂))₂₋₃S-, wherein each independent occurrence of R* is selected from hydrogen, C₁₋₆ aliphatic which may be substituted as defined below, or an unsubstituted 5- to 6-membered saturated, partially unsaturated, or aryl ring having 0-4 heteroatoms independently selected from nitrogen, oxygen, or sulfur. Suitable divalent substituents that are bound to vicinal substitutable carbons of an "optionally substituted" group include: -O(CR*₂)₂₋₃O-, wherein each independent occurrence of R* is selected from hydrogen, C₁₋₆ aliphatic which may be substituted as defined below, or an unsubstituted 5-6-membered saturated, partially unsaturated, or aryl ring having 0-4 heteroatoms independently selected from nitrogen, oxygen, or sulfur.

Suitable substituents on the aliphatic group of R* include halogen, -R^{•}, (haloR^{•}), OH, - OR^{•}, -O(haloR^{•}), -CN, -C(O)OH, -C(O)OR^{•}, -NH₂, -NHR^{•}, -NR*₂, or -NO₂, wherein each R^{•} is unsubstituted or where preceded by "halo" is substituted only with one or more halogens, and is independently C₁₋₄ aliphatic, -CH₂Ph, -O(CH₂)₀₋₁ Ph, or a 5- to 6-membered saturated, partially unsaturated, or aryl ring having 0-4 heteroatoms independently selected from nitrogen, oxygen, or sulfur.

Suitable substituents on a substitutable nitrogen of an "optionally substituted" group include -R^{†}, -NR^{†}₂, -C(O)R^{†}, -C(O)OR^{†}, -C(O)C(O)R^{†}, -C(O)CH₂C(O)R^{†}, S(O)₂R^{†}, S(O)₂NR^{†}₂, -C(S)NR^{†}₂, -C(NH)NR^{†}₂, or -N(R^{†})S(O)₂R^{†}; wherein each R^{†} is independently hydrogen, C₁₋₆ aliphatic which may be substituted as defined below, unsubstituted -OPh, or an unsubstituted 3- to 6-membered saturated, partially unsaturated, or aryl ring having 0-4 heteroatoms independently selected from nitrogen, oxygen, or sulfur, or, notwithstanding the definition above, two independent occurrences of R^{†}, taken together with their intervening atom(s) form an unsubstituted 3- to 12-membered saturated, partially unsaturated, or aryl mono- or bicyclic ring having 0-4 heteroatoms independently selected from nitrogen, oxygen, or sulfur.

Suitable substituents on the aliphatic group of R^{†} are independently halogen, -R^{•}, (haloR^{•}), -OH, -OR^{•}, -O(haloR^{•}), -CN, -C(O)OH, -C(O)OR^{•}, -NH₂, -NHR^{•}, -NR^{•}₂, or NO₂, wherein each R^{•} is unsubstituted or where preceded by "halo" is substituted only with one or more halogens, and is independently C₁₋₄ aliphatic, -CH₂Ph, -O(CH₂)₀₋₁ Ph, or a 3- to 6-membered saturated, partially unsaturated, or aryl ring having 0-4 heteroatoms independently selected from nitrogen, oxygen, or sulfur.

***Small molecule:*** As used herein, the term "small molecule" means a low molecular weight organic and/or inorganic compound. In general, a "small molecule" is a molecule that is less than about 5 kilodaltons (kD) in size. In some embodiments, a small molecule is less than about 4 kD, 3 kD, about 2 kD, or about 1 kD. In some embodiments, the small molecule is less than about 800 daltons (D), about 600 D, about 500 D, about 400 D, about 300 D, about 200 D, or about 100 D. In some embodiments, a small molecule is less than about 2000 g/mol, less than about 1500 g/mol, less than about 1000 g/mol, less than about 800 g/mol, or less than about 500 g/mol. In some embodiments, a small molecule is not a polymer.

Those of ordinary skill in the art, reading the present disclosure, will appreciate that certain small molecule compounds described herein, including, for example, oligosaccharide compounds described herein, may be provided and/or utilized in any of a variety of forms such as, for example, crystal forms (e.g., polymorphs, solvates, etc), salt forms, protected forms, pro-drug forms, ester forms, isomeric forms (e.g., optical and/or structural isomers), isotopic forms, etc.

Those of ordinary skill in the art will appreciate that certain small molecule compounds (e.g., oligosaccharide compounds described herein) have structures that can exist in one or more steroisomeric forms. In some embodiments, such a small molecule may be utilized in accordance with the present disclosure in the form of an individual enantiomer, diastereomer or geometric isomer, or may be in the form of a mixture of stereoisomers; in some embodiments, such a small molecule may be utilized in accordance with the present disclosure in a racemic mixture form.

Those of skill in the art will appreciate that certain small molecule compounds (e.g., oligosaccharide compounds described herein) have structures that can exist in one or more tautomeric forms. In some embodiments, such a small molecule may be utilized in accordance with the present disclosure in the form of an individual tautomer, or in a form that interconverts between tautomeric forms.

Those of skill in the art will appreciate that certain small molecule compounds (e.g., oligosaccharide compounds described herein) have structures that permit isotopic substitution (e.g., ²H or ³H for H; ¹¹C, ¹³C or ¹⁴C for ¹²C; ¹³N or ¹⁵N for ¹⁴N; ¹⁷O or ¹⁸O for ¹⁶O; ³⁶Cl for ³⁵Cl or ³⁷Cl; ¹⁸F for ¹⁹F; ¹³¹I for ¹²⁷I; etc.). In some embodiments, such a small molecule may be utilized in accordance with the present disclosure in one or more isotopically modified forms, or mixtures thereof.

In some embodiments, reference to a particular small molecule compound (e.g., oligosaccharide compounds described herein) may relate to a specific form of that compound. In some embodiments, a particular small molecule compound may be provided and/or utilized in a salt form (e.g., in an acid-addition or base-addition salt form, depending on the compound); in some such embodiments, the salt form may be a pharmaceutically acceptable salt form.

In some embodiments, where a small molecule compound is one that exists or is found in nature, that compound may be provided and/or utilized in accordance in the present disclosure in a form different from that in which it exists or is found in nature. Those of ordinary skill in the art will appreciate that, in some embodiments, a preparation of a particular small molecule compound (e.g., an oligosaccharide compound described herein) that contains an absolute or relative amount of the compound, or of a particular form thereof, that is different from the absolute or relative (with respect to another component of the preparation including, for example, another form of the compound) amount of the compound or form that is present in a reference preparation of interest (e.g., in a primary sample from a source of interest such as a biological or environmental source) is distinct from the compound as it exists in the reference preparation or source. Thus, in some embodiments, for example, a preparation of a single stereoisomer of a small molecule compound may be considered to be a different form of the compound than a racemic mixture of the compound; a particular salt of a small molecule compound may be considered to be a different form from another salt form of the compound; a preparation that contains only a form of the compound that contains one conformational isomer ((Z) or (E)) of a double bond may be considered to be a different form of the compound from one that contains the other conformational isomer ((E) or (Z)) of the double bond; a preparation in which one or more atoms is a different isotope than is present in a reference preparation may be considered to be a different form; etc. Those skilled in the art will further appreciate that, in small molecule structures, the symbol 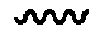 , as used herein, refers to a point of attachment between two atoms.

***Therapeutic agent:*** As used herein, the phrase "therapeutic agent" in general refers to any agent that elicits a desired pharmacological effect when administered to an organism. In some embodiments, an agent is considered to be a therapeutic agent if it demonstrates a statistically significant effect across an appropriate population. In some embodiments, the appropriate population may be a population of model organisms. In some embodiments, an appropriate population may be defined by various criteria, such as a certain age group, gender, genetic background, preexisting clinical conditions, etc. In some embodiments, a therapeutic agent is a substance that can be used to alleviate, ameliorate, relieve, inhibit, prevent, delay onset of, reduce severity of, and/or reduce incidence of one or more symptoms or features of a disease, disorder, and/or condition. In some embodiments, a "therapeutic agent" is an agent that has been or is required to be approved by a government agency before it can be marketed for administration to humans. In some embodiments, a "therapeutic agent" is an agent for which a medical prescription is required for administration to humans.

***Treat:*** As used herein, the terms "treat," "treatment," or "treating" refer to any method used to partially or completely alleviate, ameliorate, relieve, inhibit, prevent, delay onset of, reduce severity of, and/or reduce incidence of one or more symptoms or features of a disease, disorder, and/or condition. Treatment may be administered to a subject who does not exhibit signs of a disease, disorder, and/or condition. In some embodiments, treatment may be administered to a subject who exhibits only early signs of the disease, disorder, and/or condition, for example, for the purpose of decreasing the risk of developing pathology associated with the disease, disorder, and/or condition.

### Complexes and Compositions

The compositions described herein exhibit unexpectedly improved characteristics relative to previously described complexes comprising oligosaccharides or lipid-based complexes. Moreover, the present disclosure encompasses an insight that cationic and/or ionizable oligosaccharides are surprisingly useful for the delivery and targeted expression of particular therapeutic agents, e.g., nucleic acids, RNA. In some embodiments, the present disclosure provides a complex comprising i) a cationic oligosaccharide comprising one or more cationic moieties bonded to a trehalose, a sucrose, or a gluco-n-oligosaccharide moiety, where n is 2-6; ii) a surfactant; and iii) one or more additives selected from: a sterol, a helper lipid, an immunomodulator, and a targeting molecule.

The present disclosure further encompasses, among other things, an insight that particular combinations of cationic oligosaccharide, surfactant, and one or more additive, and in particular molar ratios, provides a complex that exhibits suitable properties for delivery of cargo (e.g., small molecule agents or nucleic acids).

### Polycationic Oligosaccharides

Complexes described herein comprise a cationic oligosaccharide comprising a plurality of cationic moieties bonded to a trehalose, a sucrose, or a gluco-n-oligosaccharide moiety, where n is 2-6. In some embodiments, a trehalose moiety is of structure: 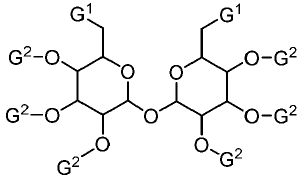 where each G¹ moiety is independently a cationic moiety optionally connected to the trehalose moiety via a linker group, and each G² is independently H or an C₁-C₆₀ aliphatic group.

In some embodiments, a sucrose moiety is of structure: 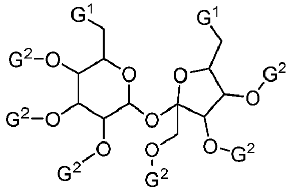 where each G¹ moiety is independently a cationic moiety optionally connected to the trehalose moiety via a linker group, and each G² is independently H or an C₁-C₆₀ aliphatic group.

In some embodiments, a gluco-n-oligosaccharide moiety is of structure: 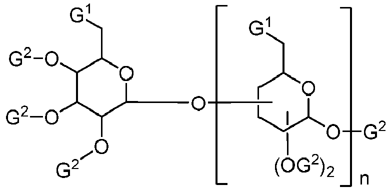 where each G¹ moiety is independently a cationic moiety optionally connected to the trehalose moiety via a linker group, each G² is independently H or an C₁-C₆₀ aliphatic group, and n is from 2-6 (e.g., 2, 3, 4, 5, or 6). In some embodiments, n is 2-5. In some embodiments, n is 3-6.

In some embodiments, each linker group is independently an optionally substituted C₁₋₃₀ aliphatic group wherein one or more carbons are optionally and independently replaced by -Cy-, -NR^{Y}-, -NR^{Y}C(O)-, -C(O)NR^{Y}-, -NR^{Y}C(O)O-, -OC(O)NR^{Y}-, -NR^{Y}C(O)NR^{Y}-, - NR^{Y}C(S)NR^{Y}-, -C(S)NR^{Y}- -C(O)NR^{Y}SO₂-, -SO₂NR^{Y}C(O)-,-OC(O)O-, -O-, -C(O)-, - OC(O)-, -C(O)O, SO, or SO₂; each R^{Y} is independently H or optionally substituted C₁-C₆ aliphatic; and each Cy is independently an optionally substituted C₃-C₁₄ cycloaliphatic, 5- to 14-membered heterocyclyl ring having 1-3 heteroatoms selected from N, O, S, 5-to 14-membered heteroaryl ring having 1-3 heteroatoms selected from N, O, S. It is understood that a cationic moiety refers to any moiety that carries a positive charge, or that is ionizable such that it will carry a positive charge in certain environments. Example cationic moieties include, but are not limited to, ammonium, guanidinium, isothiouronium, amidinium, piperazinium, piperidinium, morpholinium, pyrrolidinium, imidazolium, pyrazolium, oxazolium, thiazolium, triazolium and the like. A person of skill in the art will also appreciate that an oligosaccharide described herein comprises a plurality (i.e., one or more) cationic moieties. A person of skill in the art will also appreciate that cationic moieties described herein can also exist as a salt (e.g., a pharmaceutically acceptable salt) that comprises a cationic moiety and one or more suitable counterions. For example, in some embodiments, a cationic group described herein comprises ammonium chloride. Suitable counterions include halogens (e.g., Br⁻, Cl⁻, I⁻, F⁻), acetates (e.g., C(O)O⁻), and the like. For additional examples, see the definition for pharmaceutically acceptable salts described herein.

In some embodiments, a cationic oligosaccharide described herein is of formula I: 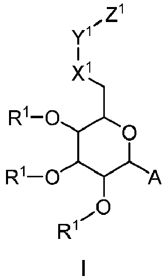 or a pharmaceutically acceptable salt thereof, wherein:
A is A¹, A², or A³: 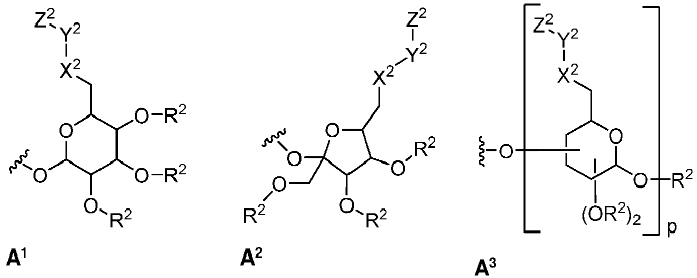
R¹ and R² are each independently selected from H, R^{a}, and -C(O)-R^{a} wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by Cy-, -NR^{Y}-, - NR^{Y}C(O)-, -C(O)NR^{Y}-, -NR^{Y}C(O)O-, -OC(O)NR^{Y}-, -NR^{Y}C(O)NR^{Y}-, -NR^{Y}C(S)NR^{Y}-, - C(S)NR^{Y}- -C(O)NR^{Y}SO₂-, -SO₂NR^{Y}C(O)-, -OC(O)O-, -O-, -C(O)-, -OC(O)-, -C(O)O-, - SO-, or -SO₂-;
each R^{Y} is independently H or optionally substituted C₁-C₆ aliphatic;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 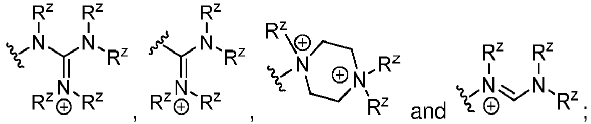
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5.

As described herein, A is A¹, A², or A³:

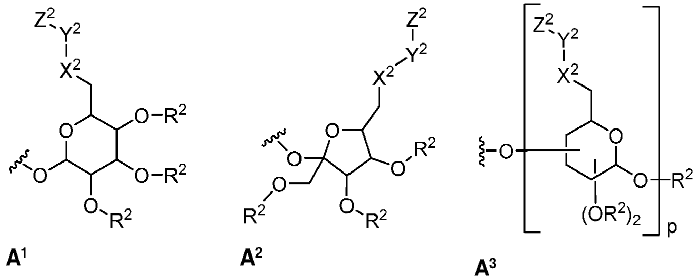

In some embodiments, A is A¹ or A². In some embodiments, A is A¹. In some embodiments, A is A². In some embodiments, A is A³. In some embodiments, A is A¹, and the oligosaccharide is of formula la: 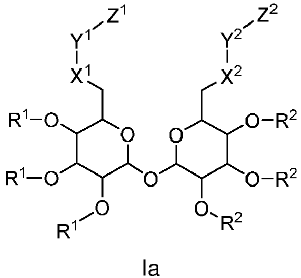 or a pharmaceutically acceptable salt thereof, wherein R¹, R², X¹, X², Y¹, Y², Z¹, and Z² are as described in classes and subclasses herein, both singly and in combination.

In some embodiments, A is A², and an oligosaccharide is of formula lb: 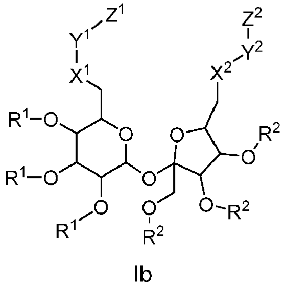 or a pharmaceutically acceptable salt thereof, wherein R¹, R², X¹, X², Y¹, Y², Z¹, and Z² are as described in classes and subclasses herein, both singly and in combination.

In some embodiments, A is A³, and an oligosaccharide is of formula Ic: 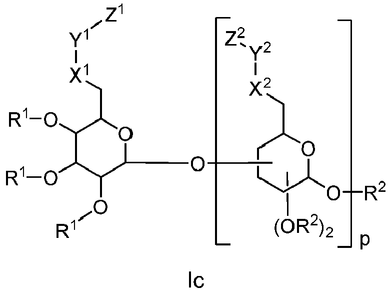 or a pharmaceutically acceptable salt thereof, wherein p, R¹, R², X¹, X², Y¹, Y², Z¹, and Z² are as described in classes and subclasses herein, both singly and in combination. In some embodiments, p is 1, and an oligosaccharide is of formula Ic-i: 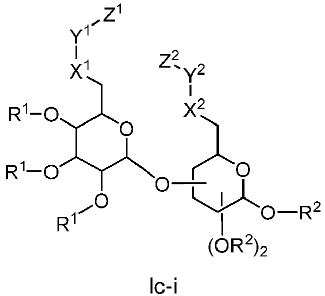 or a pharmaceutically acceptable salt thereof, wherein R¹, R², X¹, X², Y¹, Y², Z¹, and Z² are as described in classes and subclasses herein, both singly and in combination.

In some embodiments, p is 2, and an oligosaccharide is of formula Ic-ii: 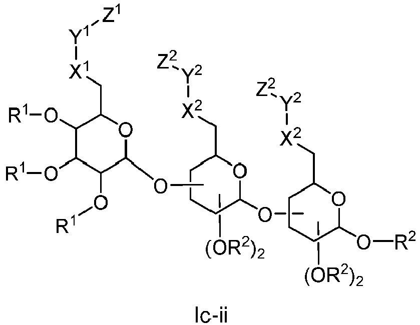 or a pharmaceutically acceptable salt thereof, wherein R¹, R², X¹, X², Y¹, Y², Z¹, and Z² are as described in classes and subclasses herein, both singly and in combination.

In some embodiments, p is 3, and an oligosaccharide is of formula Ic-iii: 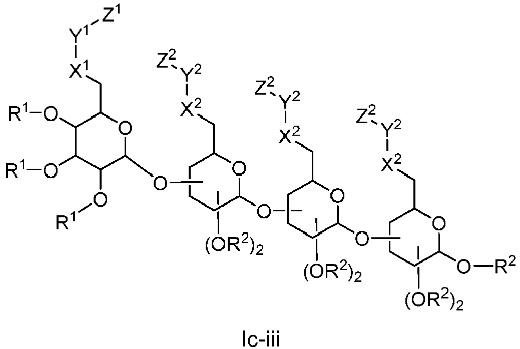 or a pharmaceutically acceptable salt thereof, wherein R¹, R², X¹, X², Y¹, Y², Z¹, and Z² are as described in classes and subclasses herein, both singly and in combination.

In some embodiments, p is 4, and an oligosaccharide is of formula Ic-iv: 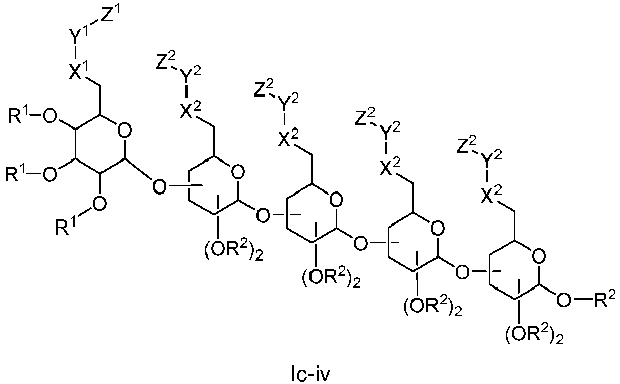 or a pharmaceutically acceptable salt thereof, wherein R¹, R², X¹, X², Y¹, Y², Z¹, and Z² are as described in classes and subclasses herein, both singly and in combination.

In some embodiments, p is 5, and an oligosaccharide is of formula Ic-v: 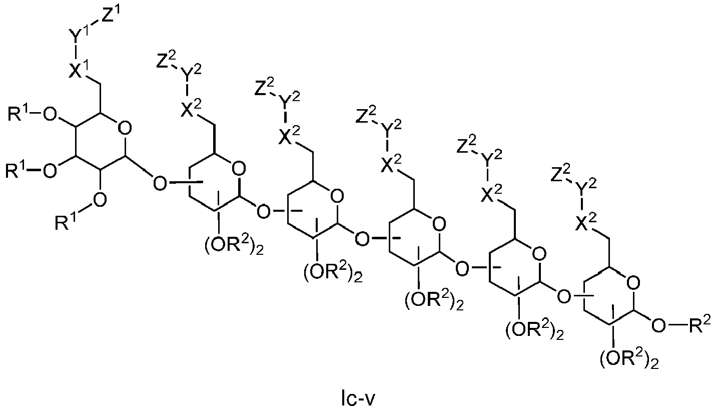 or a pharmaceutically acceptable salt thereof, wherein R¹, R², X¹, X², Y¹, Y², Z¹, and Z² are as described in classes and subclasses herein, both singly and in combination.

As described generally herein R¹ and R² are each independently selected from H, R^{a}, and -C(O)-R^{a}, wherein at least one instance of R¹ or R² is not H. In some embodiments, R¹ and R² are each independently selected from R^{a} and -C(O)-R^{a}. In some embodiments, R¹ and R² is R^{a}. In some embodiments, R¹ and R² is R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is R^{a}, and each R^{a} is independently selected from C₁-C₂₀ aliphatic optionally substituted with one or more R^{b}. In some embodiments, each of R¹ and R² is R^{a}, and each R^{a} is independently selected from C₁-C₁₄ aliphatic optionally substituted with one or more R^{b}. In some embodiments, each of R¹ and R² is R^{a}, and each R^{a} is independently selected from C₁-C₁₀ aliphatic optionally substituted with one or more R^{b}. In some embodiments, each of R¹ and R² is R^{a}, and each R^{a} is independently selected from C₅-C₁₀ aliphatic optionally substituted with one or more R^{b}. In some embodiments, each of R¹ and R² is R^{a}, and each R^{a} is independently selected from C₅-C₁₀ alkyl optionally substituted with one or more R^{b}. In some embodiments, each of R¹ and R² is R^{a}, and each R^{a} is independently selected from C₅-C₁₀ linear alkyl. In some embodiments, each of R¹ and R² is R^{a}, and each R^{a} is independently selected from methyl, ethyl, n-propyl, n-butyl, n-pentyl, n-hexyl, n-heptyl, n-octyl, and n-nonyl. In some embodiments, each R¹ and R² is R^{a}, and each R^{a} is independently selected from

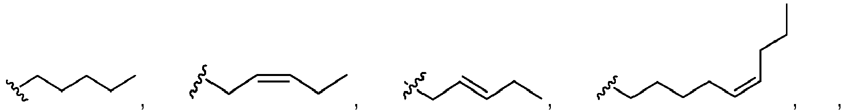

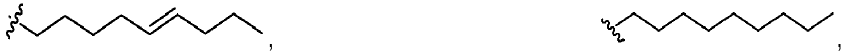

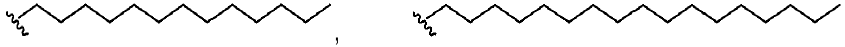

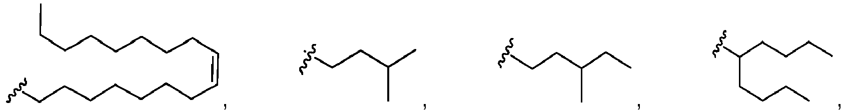

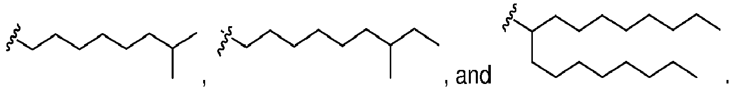

In some embodiments, each of R¹ and R² is R^{a}, and each R^{a} is independently selected from C₃-C₂₀ cycloaliphatic, optionally substituted with one or more R^{b}. In some embodiments, each of R¹ and R² is R^{a}, and each R^{a} is independently selected from C₃-C₆ cycloaliphatic optionally substituted with one or more R^{b}. In some embodiments, each of R¹ and R² is R^{a}, and each R^{a} is independently selected from cyclopropyl, cyclobutyl, cyclopentyl, and cyclohexyl.

In some embodiments, each of R¹ and R² is R^{a}, and each R^{a} is independently selected from C₅-C₆ aryl optionally substituted with one or more R^{b}. In some embodiments, each of R¹ and R² is R^{a}, and R^{a} is phenyl.

In some embodiments, each of R¹ and R² is R^{a}, and R^{a} is 3- to 12-membered heterocyclyl comprising 1 to 3 heteroatoms selected from N, O, and S optionally substituted with one or more R^{b}. In some embodiments, each of R¹ and R² is R^{a}, and R^{a} is 3- to 6-membered heterocyclyl comprising 1 to 3 heteroatoms selected from N, O, and S optionally substituted with one or more R^{b}.

In some embodiments, each of R¹ and R² is -C(O)-R^{a}, and each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b}.

In some embodiments, each of R¹ and R² is -C(O)-R^{a}. In some embodiments, each of R¹ and R² is -C(O)-R^{a}, and R^{a} is C₁-C₂₀ aliphatic optionally substituted with one or more R^{b}. In some embodiments, each of R¹ and R² is -C(O)-R^{a}, and R^{a} is C₁-C₁₄ aliphatic optionally substituted with one or more R^{b}. In some embodiments, each of R¹ and R² is -C(O)-R^{a}, and R^{a} is C₁-C₁₀ aliphatic optionally substituted with one or more R^{b}. In some embodiments, each of R¹ and R² is -C(O)-R^{a}, and R^{a} is C₅-C₁₀ aliphatic optionally substituted with one or more R^{b}. In some embodiments, each of R¹ and R² is -C(O)-R^{a}, and R^{a} is C₅-C₁₀ alkyl optionally substituted with one or more R^{b}. In some embodiments, each of R¹ and R² is -C(O)-R^{a}, and R^{a} is C₅-C₁₀ linear alkyl. In some embodiments, each of R¹ and R² is -C(O)-R^{a}, and R^{a} is methyl, ethyl, n-propyl, n-butyl, n-pentyl, n-hexyl, n-heptyl, n-octyl, or n-nonyl. In some embodiments, each of R¹ and R² is -C(O)-R^{a}, and each R^{a} is independently selected from 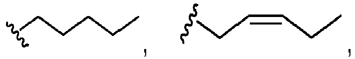 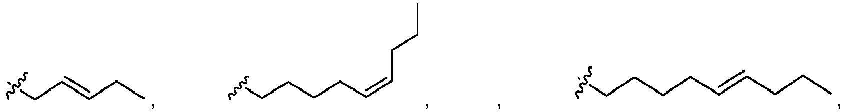 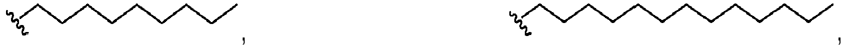 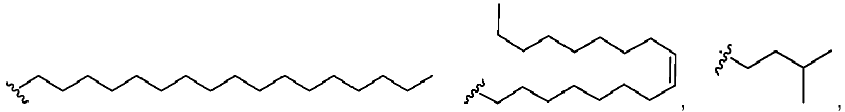 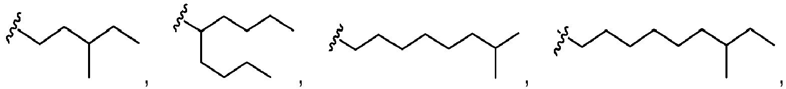 and

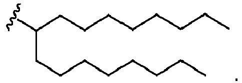

In some embodiments, each of R¹ and R² is -C(O)-R^{a}, and R^{a} is C₃-C₂₀ cycloaliphatic, optionally substituted with one or more R^{b}. In some embodiments, each of R¹ and R² is -C(O)-R^{a}, and R^{a} is C₃-C₆ cycloaliphatic optionally substituted with one or more R^{b}. In some embodiments, each of R¹ and R² is -C(O)-R^{a}, and each R^{a} is independently selected from cyclopropyl, cyclobutyl, cyclopentyl, or cyclohexyl. In some embodiments, each of R¹ and R² is -C(O)-R^{a}, and R^{a} is C₁₀-C₂₀ cycloaliphatic, optionally substituted with one or more R^{b}. In some embodiments, each of R¹ and R² is

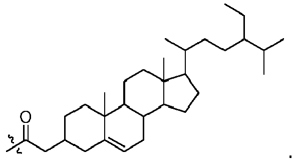

In some embodiments, each of R¹ and R² is -C(O)-R^{a}, and R^{a} is C₅-C₆ aryl optionally substituted with one or more R^{b}. In some embodiments, each of R¹ and R² is -C(O)-R^{a}, and R^{a} is phenyl.

In some embodiments, each of R¹ and R² is -C(O)-R^{a}, and R^{a} is 3- to 12-membered heterocyclyl comprising 1 to 3 heteroatoms selected from N, O, and S optionally substituted with one or more R^{b}. In some embodiments, each of R¹ and R² is -C(O)-R^{a}, and R^{a} is 3- to 6-membered heterocyclyl comprising 1 to 3 heteroatoms selected from N, O, and S optionally substituted with one or more R^{b}.

In some embodiments, each of R¹ and R² is independently selected from:

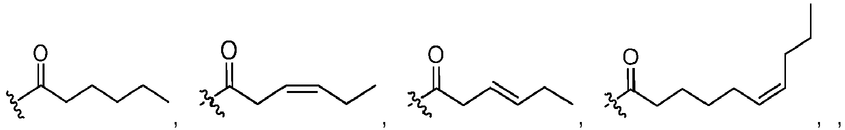

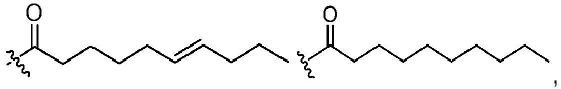

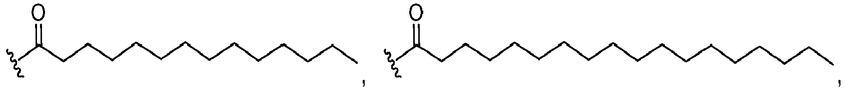

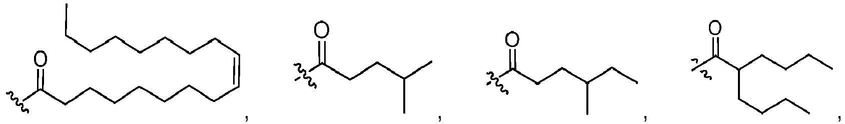

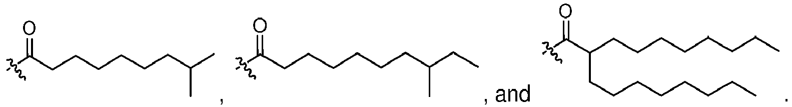

As described herein, each R^{b} is independently selected from halogen, -N₃, -R^{c}, -OR^{c}, - SR^{c}, -NHR^{c}, -C(O)-R^{c}, -OC(O)R^{c}, -NHC(O)R^{c}, -C(O)NHR^{c}, and -NHC(O)NHR^{c}. In some embodiments, R^{b} is halogen. In some embodiments, R^{b} is -N₃. In some embodiments, R^{b} is R^{c}. In some embodiments, R^{b} is -OR^{c}. In some embodiments, R^{b} is -SR^{c}. In some embodiments, R^{b} is -NHR^{c}. In some embodiments, R^{b} is -C(O)-R^{c}. In some embodiments, R^{b} is -OC(O)R^{c}. In some embodiments, R^{b} is -NHC(O)R^{c}. In some embodiments, R^{b} is -C(O)NHR^{c}. In some embodiments, R^{b} is -NHC(O)NHR^{c}.

As described herein, each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S. In some embodiments, R^{c} is optionally substituted C₁-C₂₀ aliphatic. In some embodiments, R^{c} is optionally substituted C₃-C₂₀ cycloaliphatic. In some embodiments, R^{c} is optionally substituted C₅-C₆ aryl. In some embodiments, R^{c} is optionally substituted 3- to 12-membered heterocyclyl comprising 1 to 3 heteroatoms selected from N, O, and S. In some embodiments, R^{c} is optionally substituted 4- to 12-membered heteroaryl comprising 1 to 3 heteroatoms selected from N, O, and S.

As described herein, X¹ and X² are each independently selected from -S-, -S-S-, and - NH-. In some embodiments, X¹ is -S-. In some embodiments, X¹ is -S-S-. In some embodiments, X¹ is -NH-. In some embodiments, X² is -S-. In some embodiments, X² is -S-S-. In some embodiments, X² is -NH-. In some embodiments, X¹ and X² are each -S-. In some embodiments, X¹ and X² are each -S-S-. In some embodiments, X¹ and X² are each -NH-. In some embodiments, X¹ is -S- and X² is -NH-. In some embodiments, X¹ is -NH- and X² is -S-.

As described herein, Y¹ and Y² are each independently an optionally substituted C₁₋₃₀ aliphatic group wherein one or more carbons of a Y¹ and/or Y² group are optionally and independently replaced by Cy-, -NR^{Y}-, -NR^{Y}C(O)-, -C(O)NR^{Y}-, -NR^{Y}C(O)O-, -OC(O)NR^{Y}-, -NR^{Y}C(O)NR^{Y}-, -NR^{Y}C(S)NR^{Y}-, -C(S)NR^{Y}- -C(O)NR^{Y}SO₂-, -SO₂NR^{Y}C(O)-,-OC(O)O-, - O-, -C(O)-, -OC(O)-, -C(O)O-, -SO-, or -SO₂-. In some embodiments, Y¹ and Y² are each independently an optionally substituted C₁₋₃₀ aliphatic group wherein one or more carbons of a Y¹ and/or Y² group are optionally and independently replaced by -Cy-, -NH-, -NHC(O)-, -C(O)NH-, -NHC(O)O-, -OC(O)NH-, -NHC(O)NH-, -NHC(S)NH-, -C(S)NH- - C(O)NHSO₂-, -SO₂NHC(O)-,-OC(O)O-, -O-, -C(O)-, -OC(O)-, -C(O)O-, -SO-, or -SO₂-. As described herein, it is understood that Y¹ and Y² are bivalent moieties, having one end bonded to a X¹ or X² group, and the other end bonded to a Z¹ or Z² group.

As described herein, each R^{Y} is independently selected from H and optionally substituted C₁-C₆ aliphatic. In some embodiments, each R^{Y} is independently selected from H and C₁-C₃ alkyl. In some embodiments, each R^{Y} is independently selected from H and CH₃. In some embodiments, Y¹ and Y² are each independently selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-NHC(O)-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, Y¹ and Y² are each C₁-C₁₀ aliphatic. In some embodiments, Y¹ and Y² are each methylene, ethylene, propylene, or butylene. In some embodiments, Y¹ and Y² are each -CH₂-CH₂-.

In some embodiments, Y¹ and Y² are each C₀-C₄-aliphatic-NHC(O)NH-C₀-C₄ aliphatic. In some embodiments, Y¹ and Y² are each C₁-C₄-aliphatic-NHC(O)NH-C₁-C₄ aliphatic. In some embodiments, Y¹ and Y² are each -CH₂-CH₂-NHC(O)NH-CH₂-CH₂-.

In some embodiments, Y¹ and Y² are each C₀-C₄-aliphatic-NHC(S)NH-C₀-C₄ aliphatic. In some embodiments, Y¹ and Y² are each C₁-C₄-aliphatic-NHC(S)NH-C₁-C₄ aliphatic. In some embodiments, Y¹ and Y² are each -CH₂-CH₂-NHC(S)NH-CH₂-CH₂-.

In some embodiments, Y¹ and Y² are each C₀-C₄-aliphatic-C(O)NH-C₀-C₄ aliphatic. In some embodiments, Y¹ and Y² are each C₁-C₄-aliphatic-C(O)NH-C₁-C₄ aliphatic. In some embodiments, Y¹ and Y² are each -CH₂-CH₂-C(O)NH-CH₂-CH₂-.

In some embodiments, Y¹ and Y² are each C₀-C₄-aliphatic-NHC(O)-C₀-C₄ aliphatic. In some embodiments, Y¹ and Y² are each C₁-C₄-aliphatic-NHC(O)-C₁-C₄ aliphatic. In some embodiments, Y¹ and Y² are each -CH₂-CH₂-NHC(O)-CH₂-CH₂-.

In some embodiments, Y¹ and Y² are each C₀-C₄-aliphatic-NHSO₂-C₀-C₄ aliphatic. In some embodiments, Y¹ and Y² are each C₁-C₄-aliphatic-NHSO₂-C₁-C₄ aliphatic. In some embodiments, Y¹ and Y² are each -CH₂-CH₂-NHSO₂-CH₂-CH₂-.

In some embodiments, Y¹ is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y¹ is C₁-C₁₀ aliphatic. In some embodiments, Y¹ is methylene, ethylene, propylene, or butylene. In some embodiments, Y¹ is -CH₂-CH₂-

In some embodiments, Y¹ is C₀-C₄-aliphatic-NHC(O)NH-C₀-C₄ aliphatic. In some embodiments, Y¹ is C₁-C₄-aliphatic-NHC(O)NH-C₁-C₄ aliphatic. In some embodiments, Y¹ is -CH₂-CH₂-NHC(O)NH-CH₂-CH₂-.

In some embodiments, Y¹ is C₀-C₄-aliphatic-NHC(S)NH-C₀-C₄ aliphatic. In some embodiments, Y¹ is C₁-C₄-aliphatic-NHC(S)NH-C₁-C₄ aliphatic. In some embodiments, Y¹ is -CH₂-CH₂-NHC(S)NH-CH₂-CH₂-.

In some embodiments, Y¹ is C₀-C₄-aliphatic-C(O)NH-C₀-C₄ aliphatic. In some embodiments, Y¹ is C₁-C₄-aliphatic-C(O)NH-C₁-C₄ aliphatic. In some embodiments, Y¹ is -CH₂-CH₂-C(O)NH-CH₂-CH₂-.

In some embodiments, Y¹ is C₀-C₄-aliphatic-NHC(O)-C₀-C₄ aliphatic. In some embodiments, Y¹ is C₁-C₄-aliphatic-NHC(O)-C₁-C₄ aliphatic. In some embodiments, Y¹ is -CH₂-CH₂-NHC(O)-CH₂-CH₂-.

In some embodiments, Y¹ is C₀-C₄-aliphatic-NHSO₂-C₀-C₄ aliphatic. In some embodiments, Y¹ is C₁-C₄-aliphatic-NHSO₂-C₁-C₄ aliphatic. In some embodiments, Y¹ is -CH₂-CH₂-NHSO₂-CH₂-CH₂-.

In some embodiments, Y² is selected from C₁-C₁₀ aliphatic, C₀-C₄-aliphatic-NHC(O)NH-C₀-C₄ aliphatic, C₀-C₄-aliphatic-NHC(S)NH-C₀-C₄ aliphatic, C₀-C₄-aliphatic-C(O)NH-C₀-C₄ aliphatic, C₀-C₄-aliphatic-C(S)NH-C₀-C₄ aliphatic, C₀-C₄-aliphatic-C(O)NH-C₀-C₄ aliphatic, C₀-C₄-aliphatic-NHSO₂-C₀-C₄ aliphatic, and C₀-C₄-aliphatic-C(O)-C₀-C₄ aliphatic.

In some embodiments, embodiments, Y² is C₁-C₁₀ aliphatic. In some embodiments, Y² is methylene, ethylene, propylene, or butylene. In some embodiments, Y² is -CH₂-CH₂-

In some embodiments, Y² is C₀-C₄-aliphatic-NHC(O)NH-C₀-C₄ aliphatic. In some embodiments, Y² is C₁-C₄-aliphatic-NHC(O)NH-C₁-C₄ aliphatic. In some embodiments, Y² is -CH₂-CH₂-NHC(O)NH-CH₂-CH₂-.

In some embodiments, Y² is C₀-C₄-aliphatic-NHC(S)NH-C₀-C₄ aliphatic. In some embodiments, Y² is C₁-C₄-aliphatic-NHC(S)NH-C₁-C₄ aliphatic. In some embodiments, Y² is -CH₂-CH₂-NHC(S)NH-CH₂-CH₂-.

In some embodiments, Y² is C₀-C₄-aliphatic-C(O)NH-C₀-C₄ aliphatic. In some embodiments, Y² is C₁-C₄-aliphatic-C(0)NH-C₁-C₄ aliphatic. In some embodiments, Y² is -CH₂-CH₂-C(O)NH-CH₂-CH₂-.

In some embodiments, Y² is C₀-C₄-aliphatic-NHC(O)-C₀-C₄ aliphatic. In some embodiments, Y² is C₁-C₄-aliphatic-NHC(0)-C₁-C₄ aliphatic. In some embodiments, Y² is -CH₂-CH₂-NHC(O)-CH₂-CH₂-.

In some embodiments, Y² is C₀-C₄-aliphatic-NHSO₂-C₀-C₄ aliphatic. In some embodiments, Y² is C₁-C₄-aliphatic-NHSO₂-C₁-C₄ aliphatic. In some embodiments, Y² is -CH₂-CH₂-NHSO₂-CH₂-CH₂-.

As described general herein, each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S.

In some embodiments, a moiety X¹-Y¹-Z¹ is:

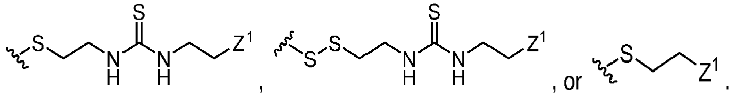

In some embodiments, a moiety X²-Y²-Z² is:

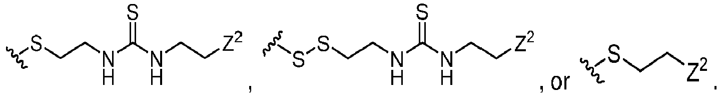

As described generally herein, Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃,

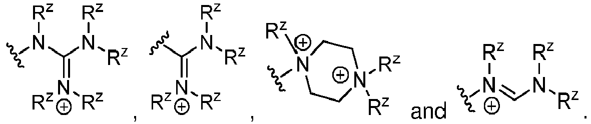

In some embodiments, Z¹ and Z² are each an optionally substituted 5- to 14-membered heterocyclyl ring having 1-3 heteroatoms selected from N, O, and S. In some embodiments, Z¹ and Z² are each an optionally substituted 5- to 6-membered heterocyclyl ring having 1-3 heteroatoms selected from N, O, and S. In some embodiments, Z¹ and Z² are each an optionally substituted 6-membered heterocyclyl ring having 1-3 heteroatoms selected from N, O, and S. In some embodiments, Z¹ and Z² are each selected from optionally substituted piperdinyl, piperazinyl, and morpholinyl.

In some embodiments, Z¹ and Z² are each independently selected from: 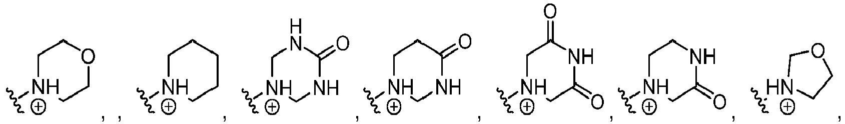 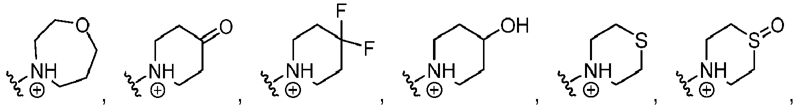 and

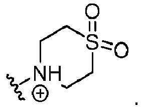

In some embodiments, Z¹ and Z² are each independently selected from:

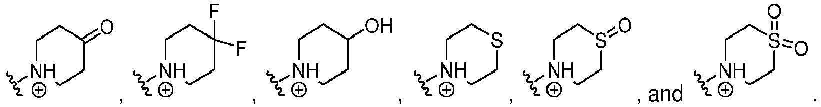

In some embodiments, Z¹ and Z² are each -N+(M)₃. In some embodiments, Z¹ and Z² are each -N⁺(C₀-C₆ aliphatic-R^{z})₃. In some embodiments, Z¹ and Z² are each -N⁺(R^{z})₃. In some embodiments, Z¹ and Z² are each -N⁺H₃. In some embodiments, Z¹ and Z² are each -N⁺H(R^{z})₂. In some embodiments, Z¹ and Z² are each -N⁺H(C₁-C₆aliphatic)₂. In some embodiments, Z¹ and Z² are each -N⁺(H)₂CH₃. In some embodiments, Z¹ and Z² are each -N+H(CH₃)₂. In some embodiments, Z¹ and Z² are each -N⁺(CH₃)₃.

In some embodiments, Z¹ and Z² are each -N⁺(R^{z})₂-C₁-C₆ aliphatic-N⁺(R^{z})₃. In some embodiments, Z¹ and Z² are each -N⁺(R^{z})(C₁-C₆ aliphatic-N⁺(R^{z})₃)₂. In some embodiments, Z¹ and Z² are each:

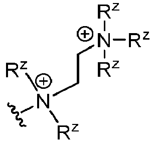

In some embodiments, Z¹ and Z² are each:

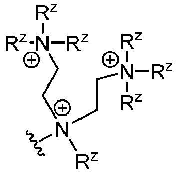

In some embodiments, Z¹ and Z² are each

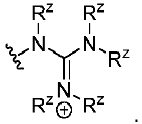

In some embodiments, Z¹ and Z² are each

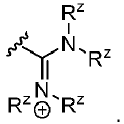

In some embodiments, Z¹ and Z² are each

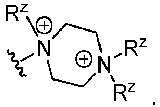

In some embodiments, Z¹ and Z² are each

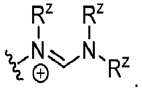

In some embodiments, Z¹ is optionally substituted 5- to 14-membered heterocyclyl ring having 1-3 heteroatoms selected from N, O, and S. In some embodiments, Z¹ is each an optionally substituted 5- to 6-membered heterocyclyl ring having 1-3 heteroatoms selected from N, O, and S. In some embodiments, Z¹ is an optionally substituted 6-membered heterocyclyl ring having 1-3 heteroatoms selected from N, O, and S. In some embodiments, Z¹ is selected from optionally substituted piperdinyl, piperazinyl, and morpholinyl.

In some embodiments, Z¹ is -N+(M)₃. In some embodiments, Z¹ is-N+(C₀-C₆ aliphatic-R^{z})₃. In some embodiments, Z¹ is-N⁺(R^{z})₃. In some embodiments, Z¹ is -N⁺H₃. In some embodiments, Z¹ is -N⁺H(R^{z})₂. In some embodiments, Z¹ is -N⁺H(C₁-C₆ aliphatic)₂. In some embodiments, Z¹ is -N⁺(H)₂CH₃. In some embodiments, Z¹ is -N+H(CH₃)₂. In some embodiments, Z¹ is -N+(CH₃)₃.

In some embodiments, Z¹ is -N⁺(R^{z})₂-C₁-C₆ aliphatic-N⁺(R^{z})₃. In some embodiments, Z¹ is -N⁺(R^{z})(C₁-C₆ aliphatic-N⁺(R^{z})₃)₂. In some embodiments, Z¹ is:

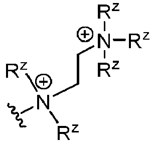

In some embodiments, Z¹ is:

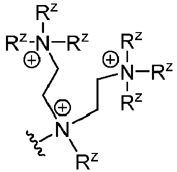

In some embodiments, Z¹ is:

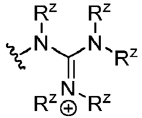

In some embodiments, Z¹ is:

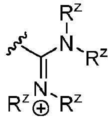

In some embodiments, Z¹ is

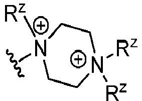

In some embodiments, Z¹ is

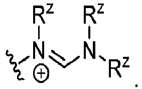

In some embodiments, Z² is optionally substituted 5- to 14-membered heterocyclyl ring having 1-3 heteroatoms selected from N, O, and S. In some embodiments, Z² is each an optionally substituted 5- to 6-membered heterocyclyl ring having 1-3 heteroatoms selected from N, O, and S. In some embodiments, Z² is an optionally substituted 6-membered heterocyclyl ring having 1-3 heteroatoms selected from N, O, and S. In some embodiments, Z² is selected from optionally substituted piperdinyl, piperazinyl, and morpholinyl.

In some embodiments, Z² is -N+(M)₃. In some embodiments, Z² is-N⁺(C₀-C₆ aliphatic-R^{z})₃. In some embodiments, Z² is-N⁺(R^{z})₃. In some embodiments, Z² is -N⁺H₃. In some embodiments, Z² is -N⁺H(R^{z})₂. In some embodiments, Z² is -N⁺H(C₁-C₆ aliphatic)₂. In some embodiments, Z² is -N⁺(H)₂CH₃. In some embodiments, Z² is -N+H(CH₃)₂. In some embodiments, Z² is -N+(CH₃)₃.

In some embodiments, Z² is -N⁺(R^{z})₂-C₁-C₆ aliphatic-N⁺(R^{z})₃. In some embodiments, Z² is -N⁺(R^{z})(C₁-C₆ aliphatic-N⁺(R^{z})₃)₂. In some embodiments, Z² is:

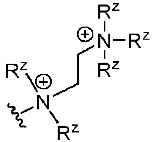

In some embodiments, Z² is:

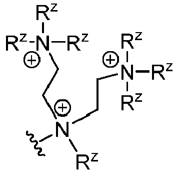

In some embodiments, Z² is:

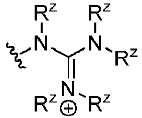

In some embodiments, Z² is:

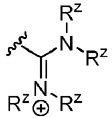

In some embodiments, Z² is

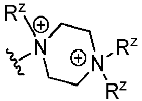

In some embodiments, Z² is

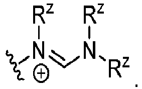

As described generally herein, each M is independently -C₀-C₆ aliphatic-R^{z} or -C₀-C₆ aliphatic-N⁺(R^{z})₃. In some embodiments, each M is C₀-C₆ aliphatic-R^{z}. In some embodiments, each M is -C₀-C₆ aliphatic-N⁺(R^{z})₃.

As described generally herein, each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S.

In some embodiments, each R^{z} is H. In some embodiments, each R^{z} is optionally substituted C₁-C₆ aliphatic. In some embodiments, each R^{z} is optionally substituted C₃-C₂₀ cycloaliphatic. In some embodiments, each R^{z} is optionally substituted C₅-C₆ aryl. In some embodiments, each R^{z} is optionally substituted 3- to 12-membered heterocyclyl comprising 1 to 3 heteroatoms selected from N, O, and S. In some embodiments, each R^{z} is optionally substituted 4- to 12-membered heteroaryl comprising 1 to 3 heteroatoms selected from N, O, and S.

In some embodiments, two or more R^{z} can come together with the atoms to which they are attached to form an optionally substituted 3- to 12-membered heterocyclyl comprising 1 to 3 heteroatoms selected from N, O, and S. In some embodiments, two or more R^{z} can come together with the atoms to which they are attached to form an optionally substituted 4- to 12-membered heteroaryl comprising 1 to 3 heteroatoms selected from N, O, and S.

In some embodiments, an R^{z} on a Z¹ moiety and an R^{z} on a Z² moiety can come together to form a 4- to 6-membered heterocyclic ring having 1 to 3 heteroatoms selected from N, O, and S.

As used herein, a "cationic" moiety is a group having a net positive charge. As used herein, an "ionizable" moiety is a group that may have a neutral charge at a certain pH, but may become charged (e.g., cationic) at a different pH. For example, in some embodiments, an ionizable moiety becomes cationic (i.e., positively charged) at physiological pH (e.g., a pH of about 7.4). Moreover, in some embodiments, a Z¹ and Z² moiety, which is a cationic or ionizable moiety described herein, further comprises a counterion (e.g., an anion for each cationic or ionizable moiety). For example, in some embodiments, a Z¹ and Z² moiety comprises a counterion that is a halide, hydroxide, carboxylate, sulfate, phosphate, nitrate, alkyl having from 1 to 6 carbon atoms, sulfonate and aryl sulfonate. In some embodiments, a Z¹ and Z² moiety comprises a counterion that is acetate (CH₃COO⁻), chloride (Cl⁻), iodide (I⁻), bromide (Br⁻), or fluoride (F-).

In some embodiments, Z¹ and Z² are each independently selected from:

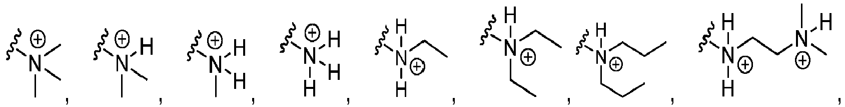

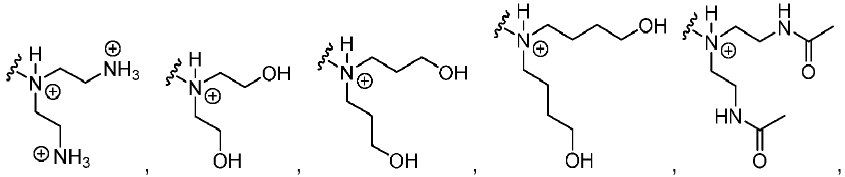

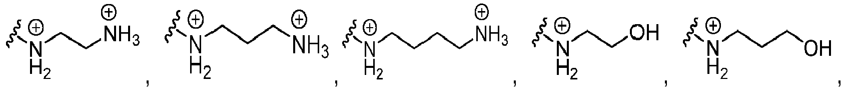

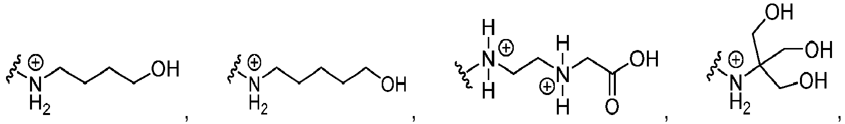

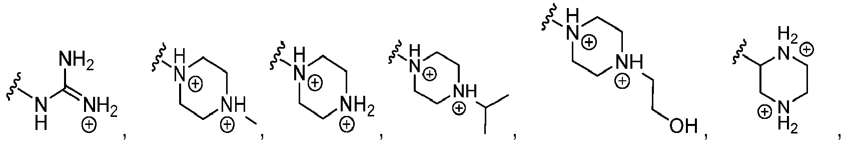

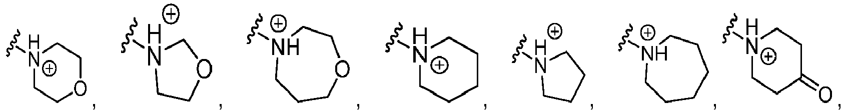

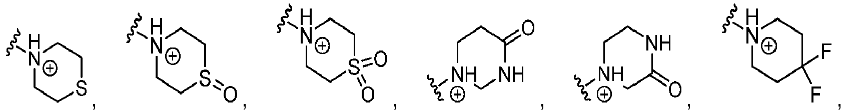

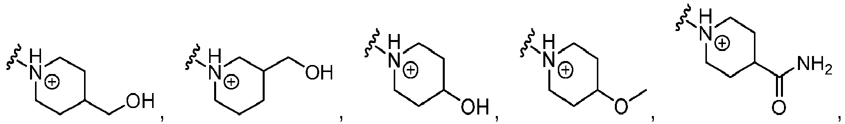

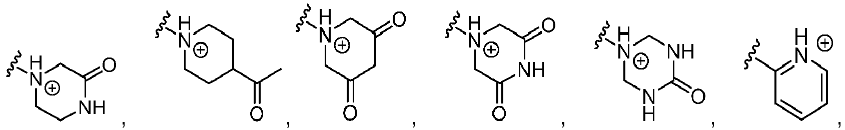

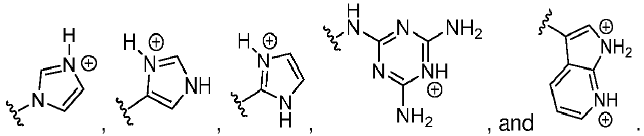

In some embodiments, a compound of formula I is a compound of formula Id: 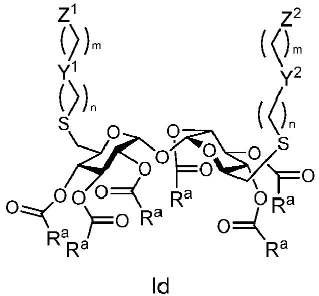 or a pharmaceutically acceptable salt thereof, wherein n and m are each independently selected from 0, 1, 2, 3, 4, 5, or 6, and R^{a}, Y¹, Y², Z¹, and Z² are as described in classes and subclasses herein, both singly and in combination.

In some embodiments, a compound of formula Id is a compound of formula Id-i: 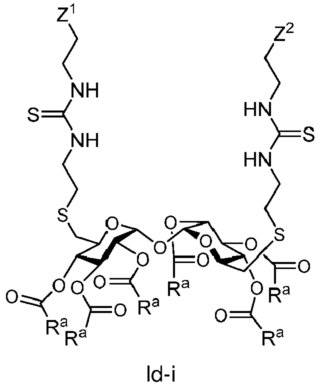 or a pharmaceutically acceptable salt thereof, wherein n and m are each independently selected from 0, 1, 2, 3, 4, 5, or 6, and R^{a}, Z¹, and Z² are as described in classes and subclasses herein, both singly and in combination.

In some embodiments, a compound of formula I is selected from Table 1.

**Table 1**

| **Name** | **Structure** |
|---|---|
| JLF19 | 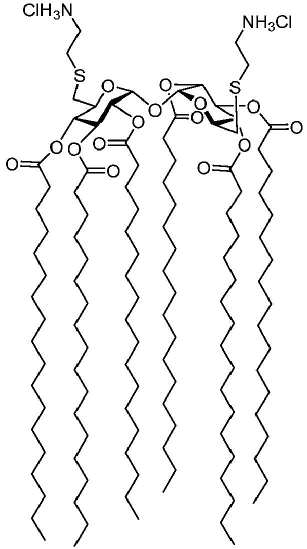 |
| JLF20 | 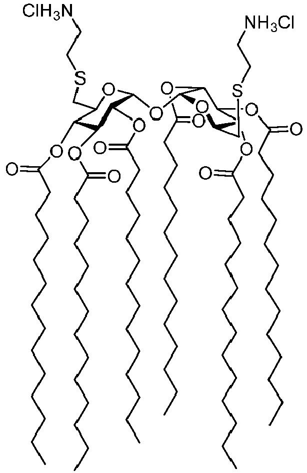 |
| JLF23 | 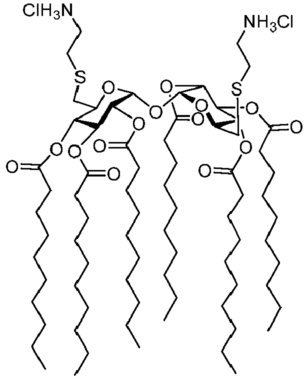 |
| JLF25 | 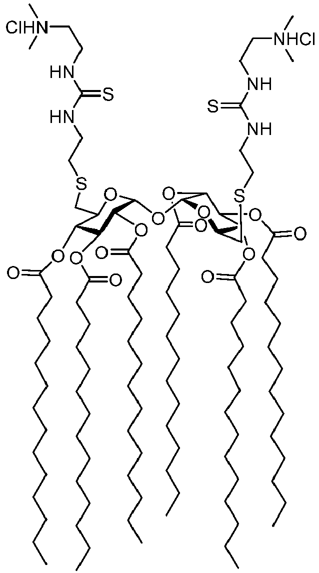 |
| JLF26 | 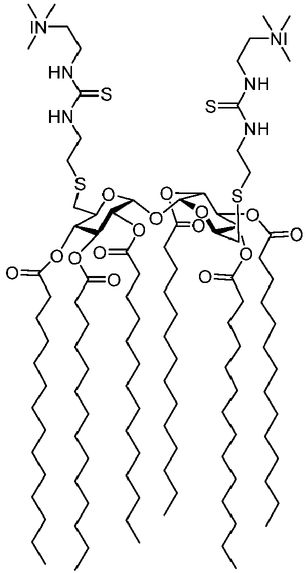 |
| JLF29 | 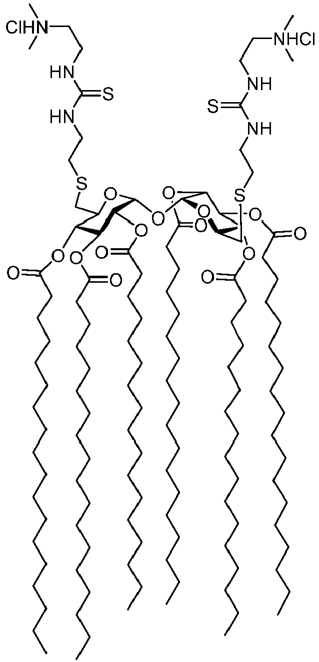 |
| JLF30 | 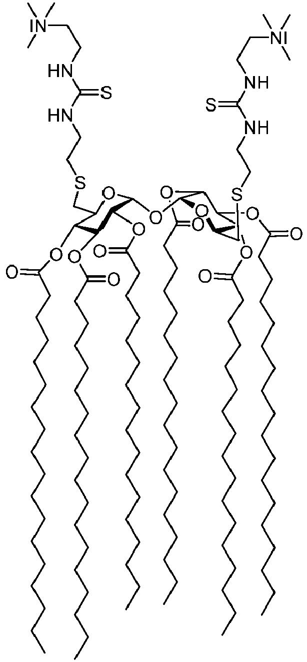 |
| JLF31 | 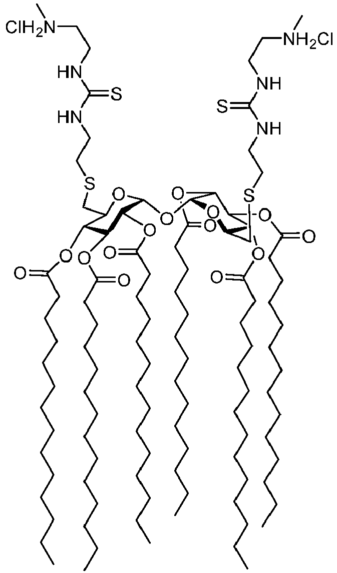 |
| JLF32 | 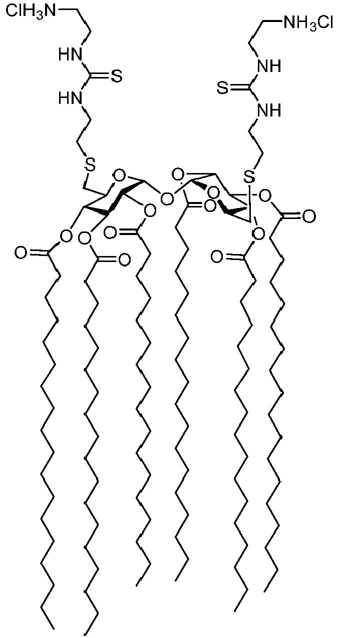 |
| JLF33 | 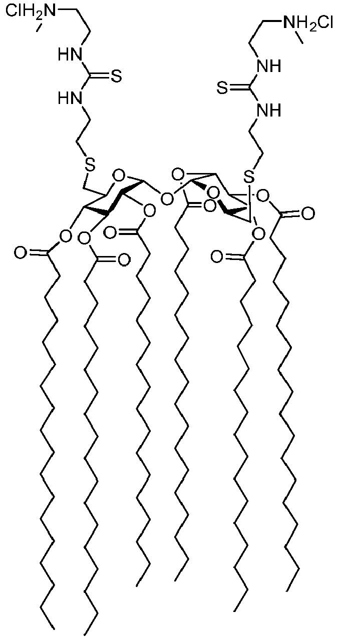 |
| JLF35 | 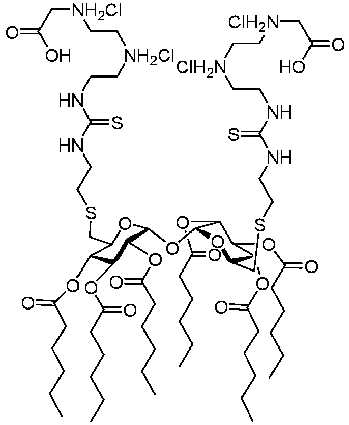 |
| JLF40 | 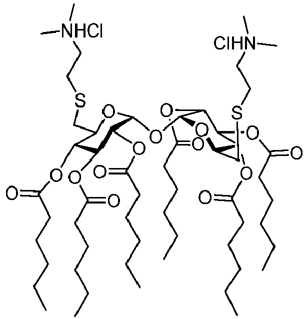 |
| JLF41 | 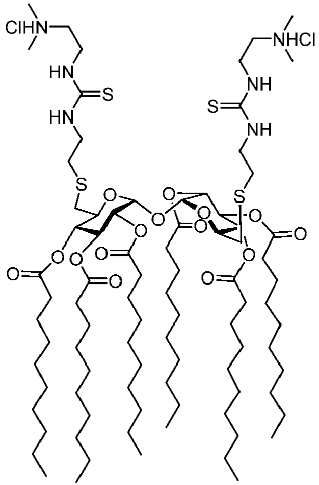 |
| JLF42 | 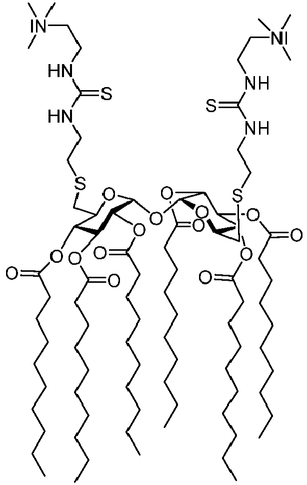 |
| JLF43 | 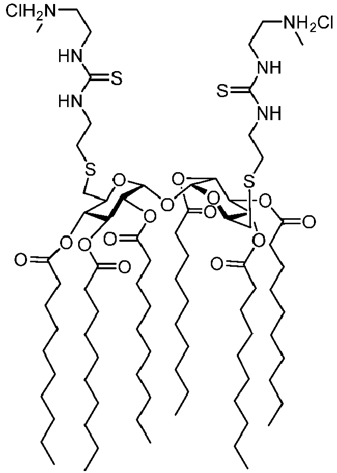 |
| JLF46 | 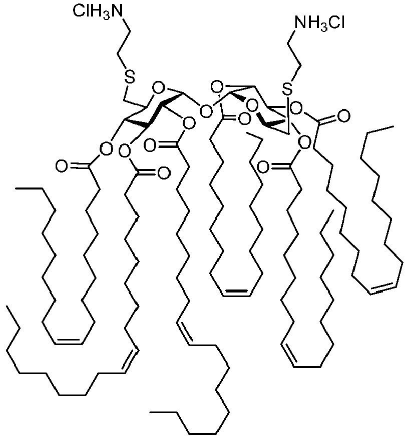 |
| JLF48 | 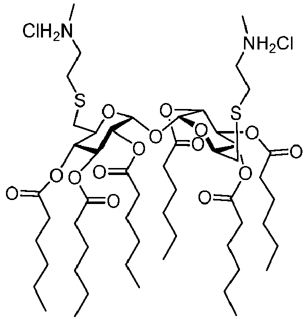 |
| JLF50 | 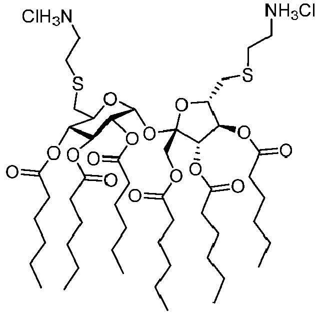 |
| JLF54 | 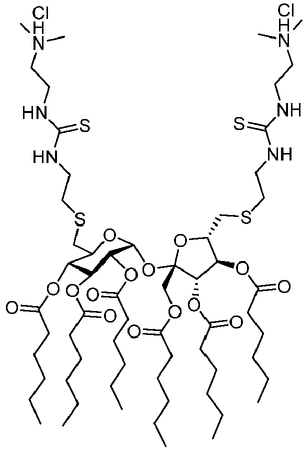 |
| JLF55 | 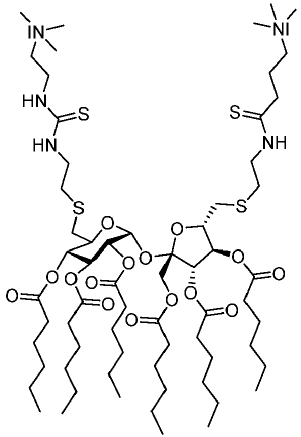 |
| JLF58 | 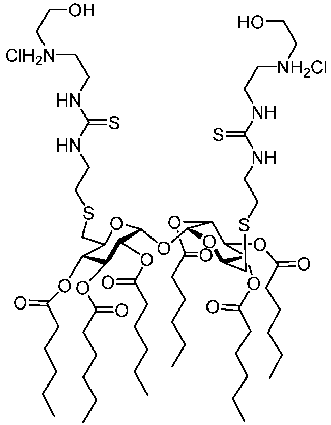 |
| JLF59 | 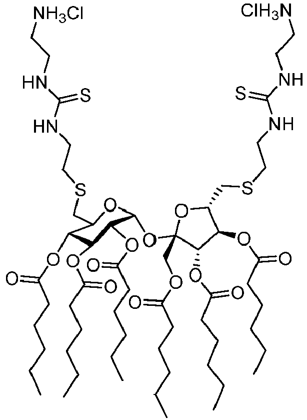 |
| JLF60 | 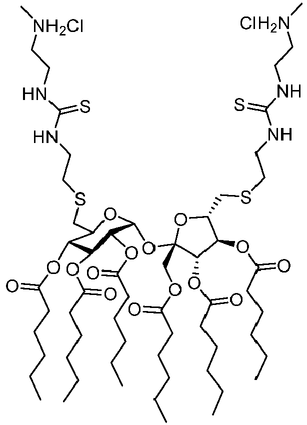 |
| JLF62 | 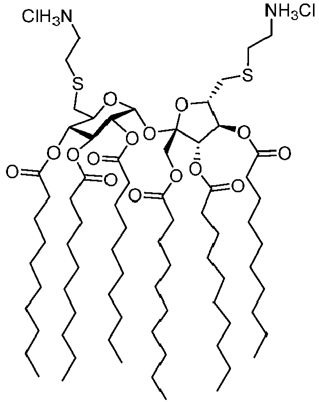 |
| JLF66 | 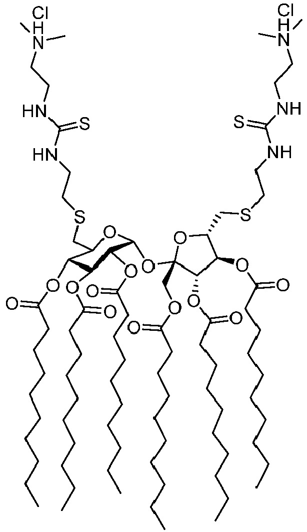 |
| JLF67 | 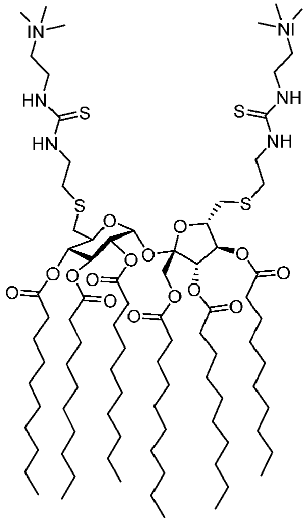 |
| JLF68 | 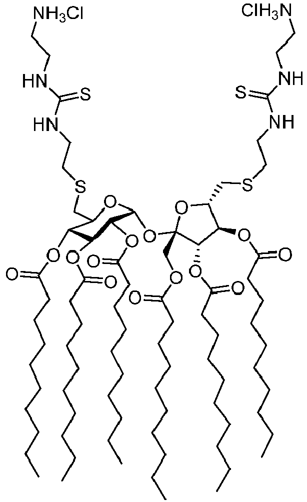 |
| JLF69 | 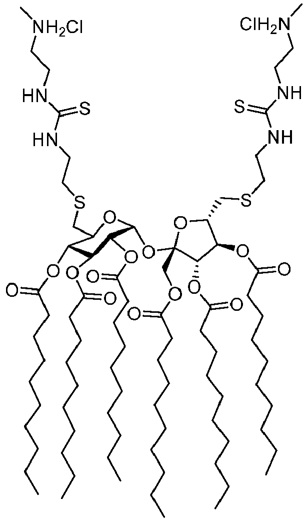 |
| JLF76 | 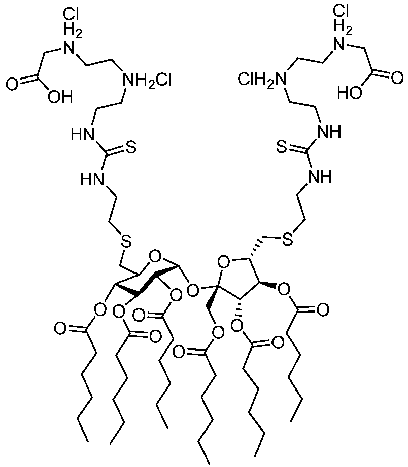 |
| JLF77 | 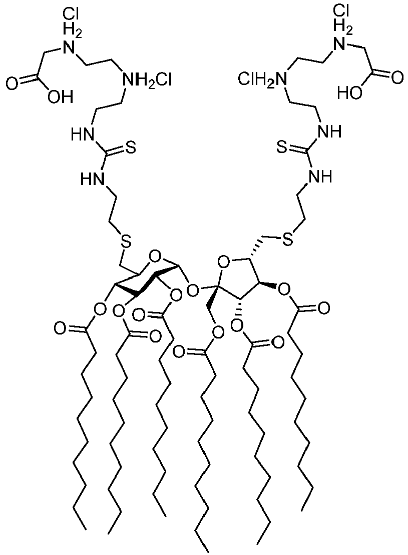 |
| JLF78 | 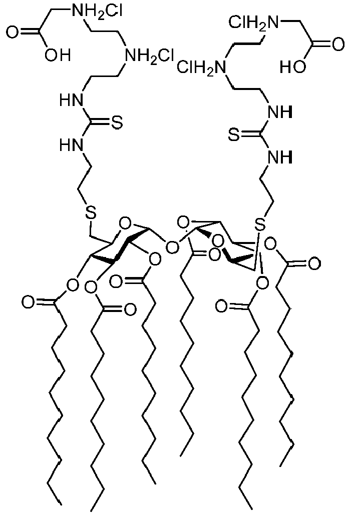 |
| JLF81 | 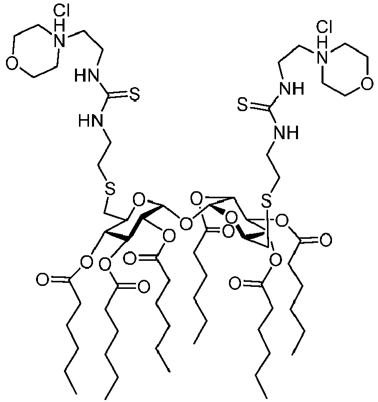 |
| JLF82 | 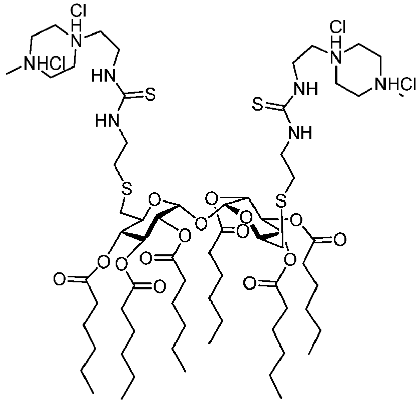 |
| JLF87 | 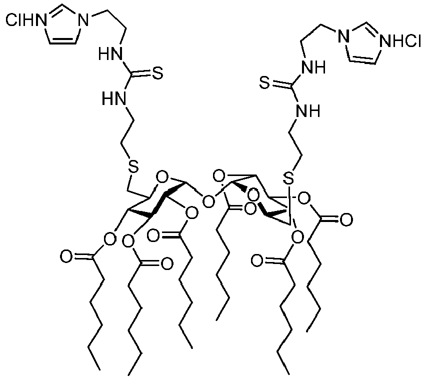 |
| JLF90 | 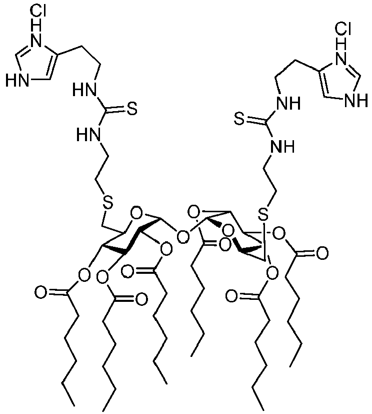 |
| JLF93 | 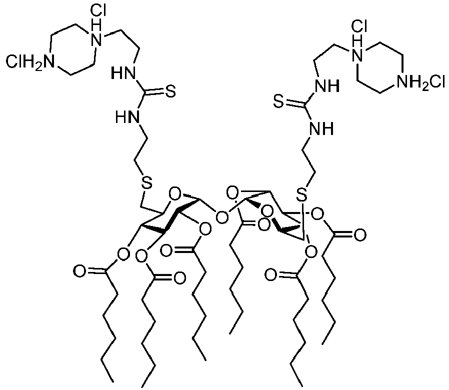 |
| JLF94 | 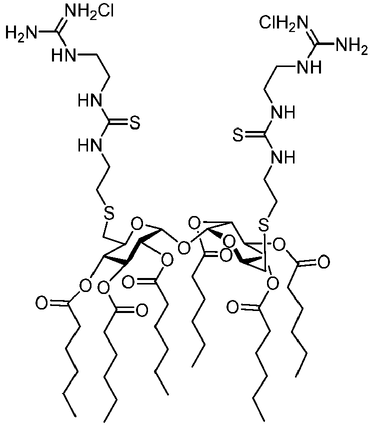 |
| JLF95 | 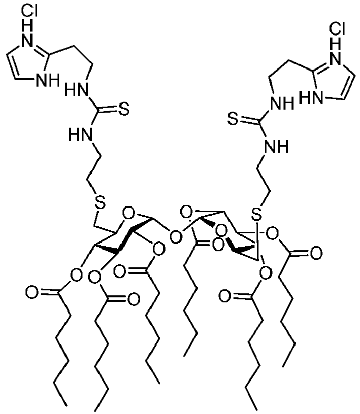 |
| JLF96 | 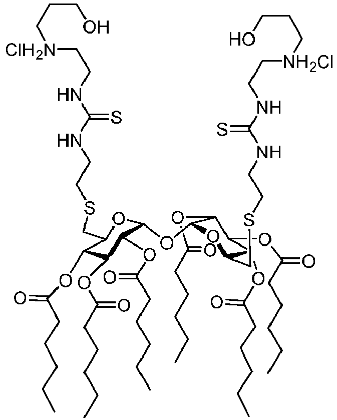 |
| JLF97 | 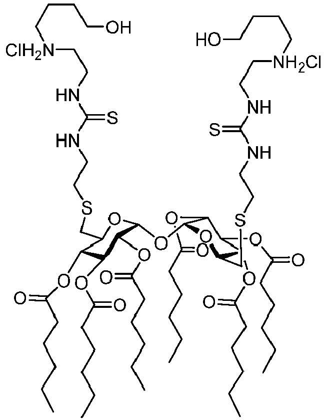 |
| JLF98 | 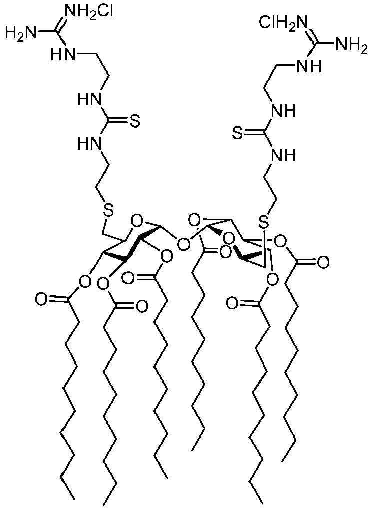 |
| JLF99 | 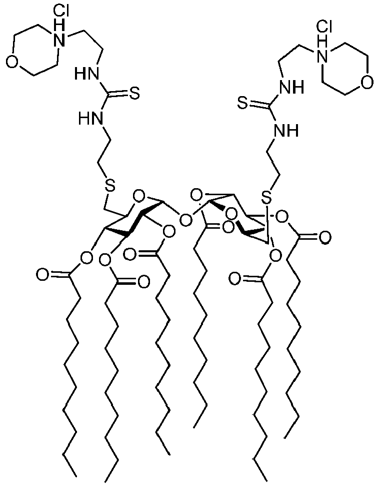 |
| JLF102 | 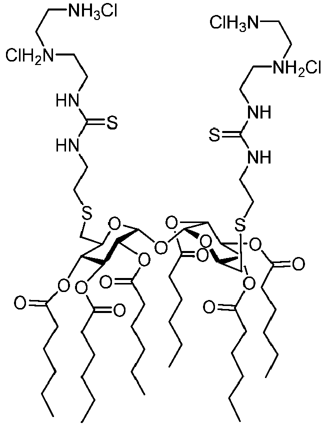 |
| JLF103 | 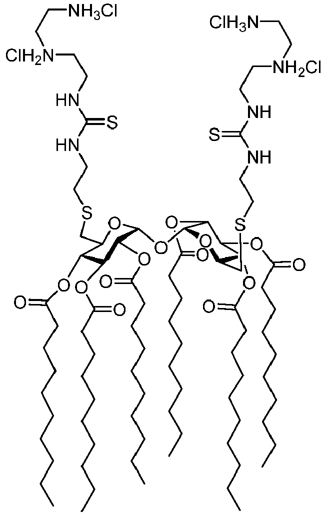 |
| JLF106 | 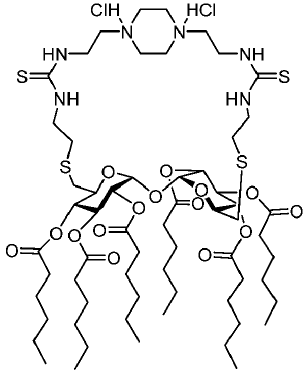 |
| JLF108 | 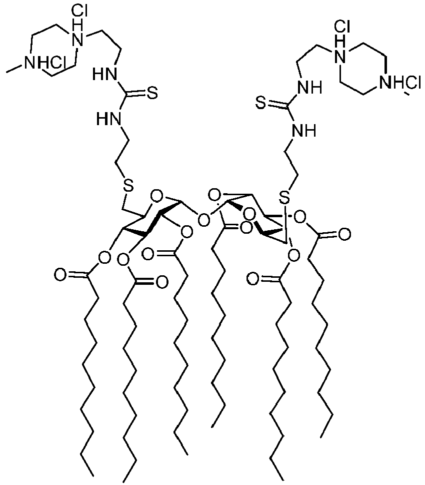 |
| JLF110 | 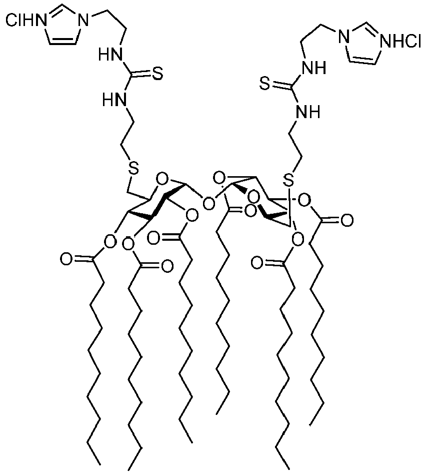 |
| JLF111 | 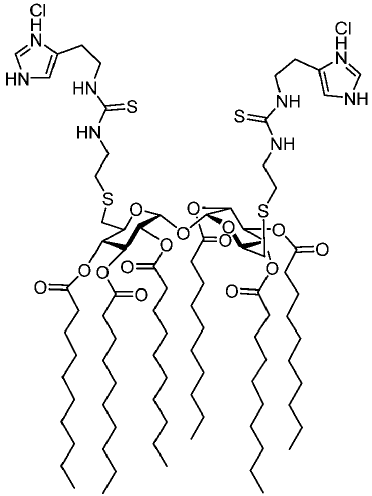 |
| JLF115 | 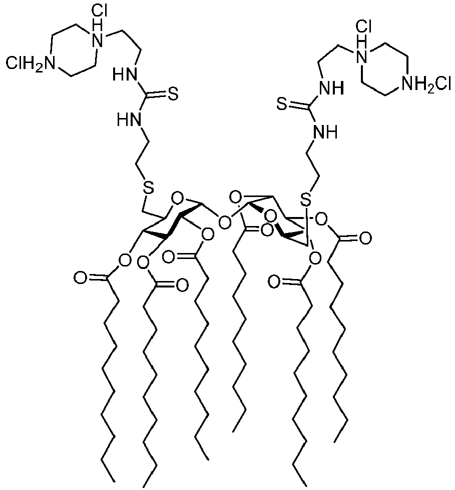 |
| JLF120 | 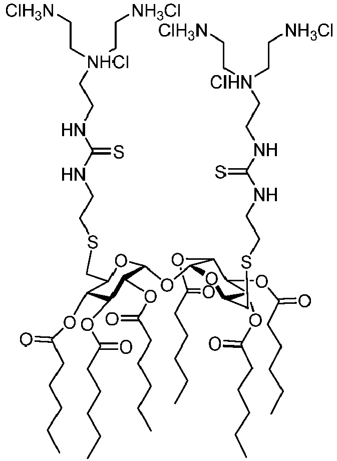 |
| JLF121 | 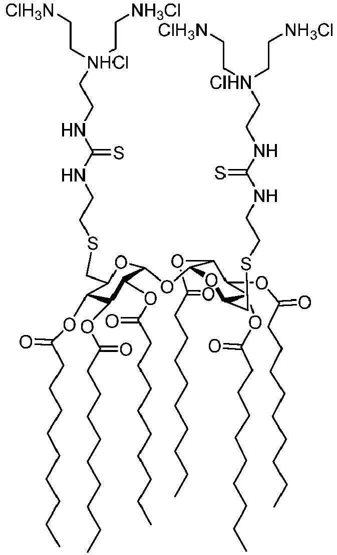 |
| JLF125 | 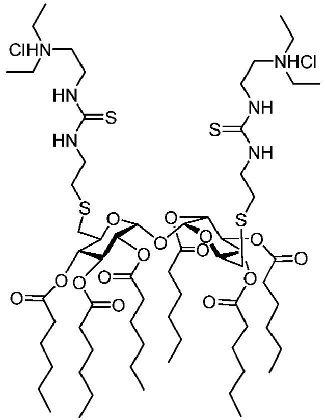 |
| JLF128 | 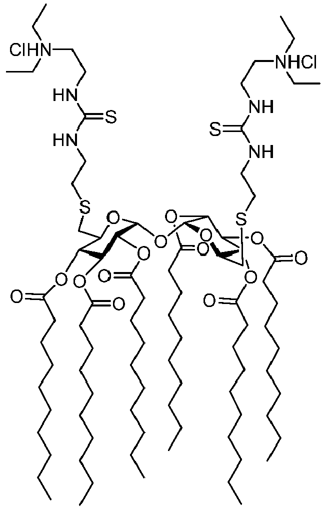 |
| JLF130 | 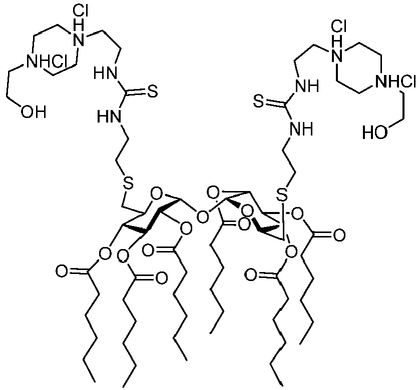 |
| JLF131 | 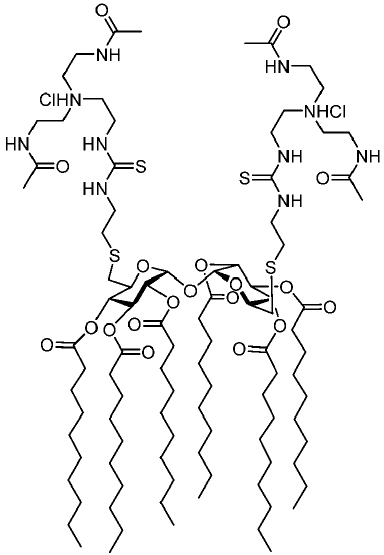 |
| JLF134 | 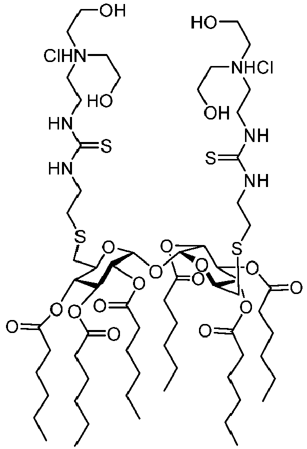 |
| JLF135 | 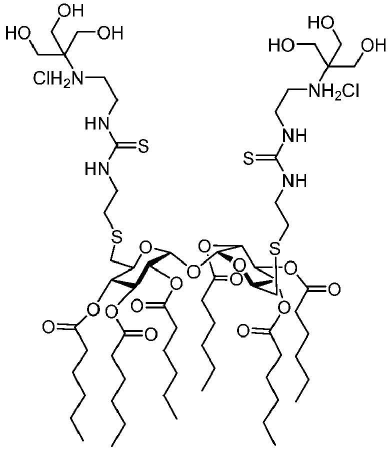 |
| JLF138 | 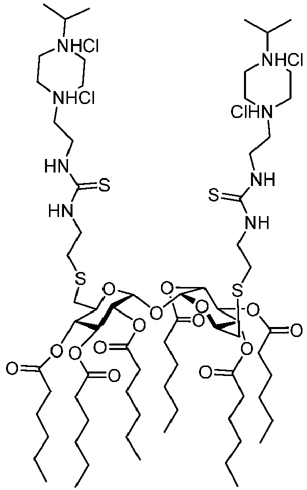 |
| JLF139 | 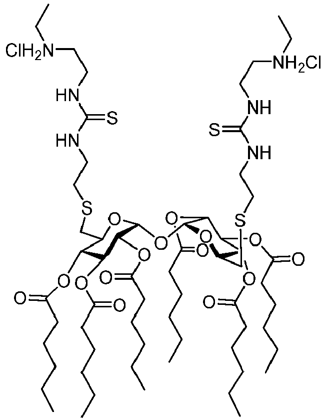 |
| JLF141 | 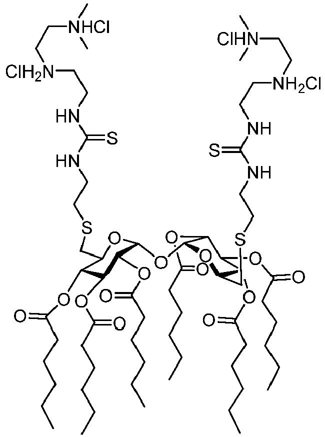 |
| JLF142 | 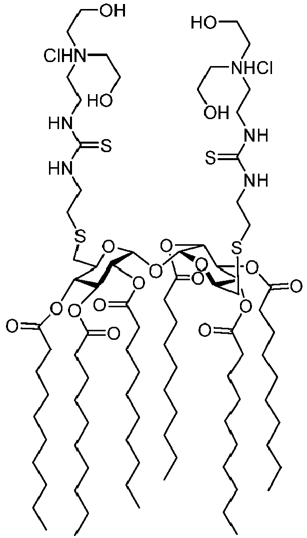 |
| JLF154 | 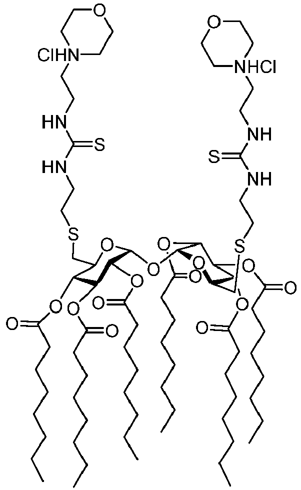 |
| JLF158 | 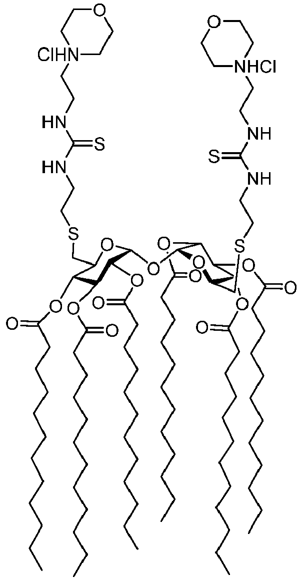 |
| JLF163 | 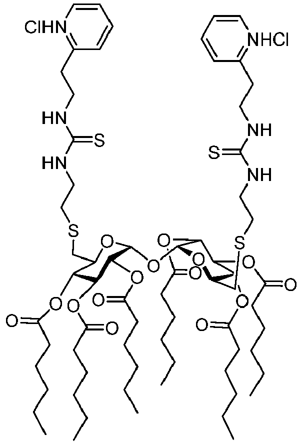 |
| JLF164 | 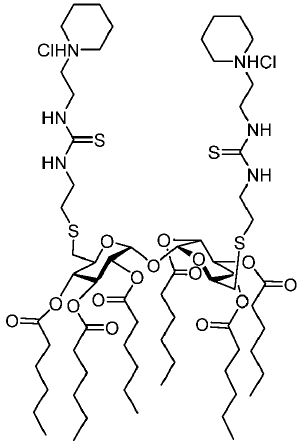 |
| JLF165 | 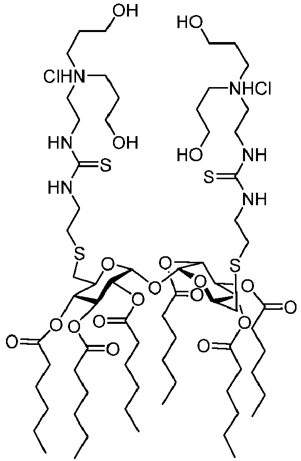 |
| JLF170 | 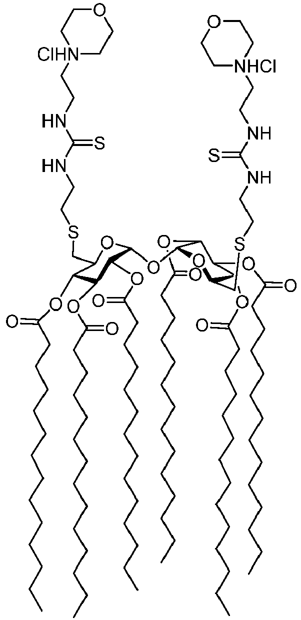 |
| JLF200 | 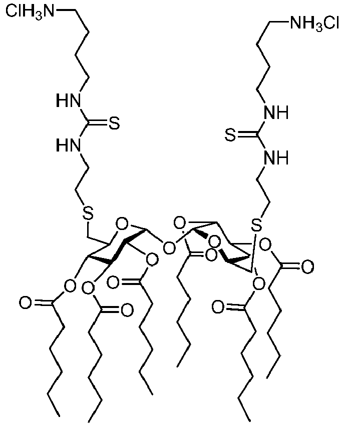 |
| JLF201 | 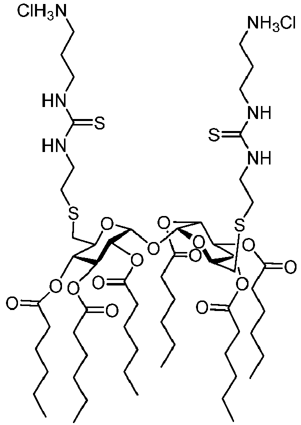 |
| JLF218 | 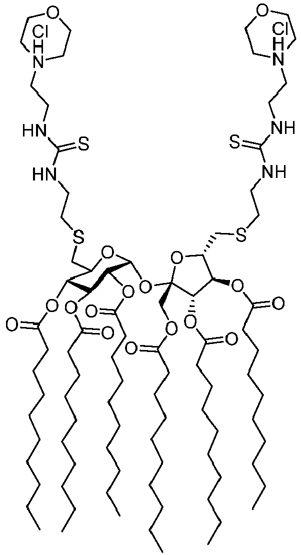 |
| JLF220 | 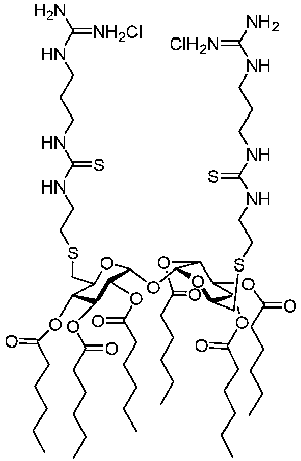 |
| JLF223 | 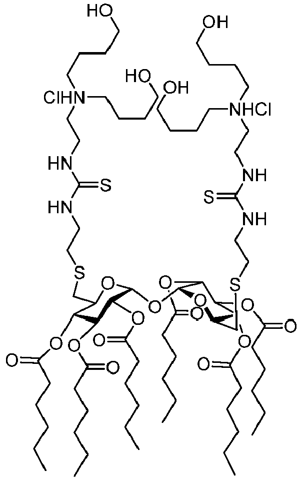 |
| JLF234 | 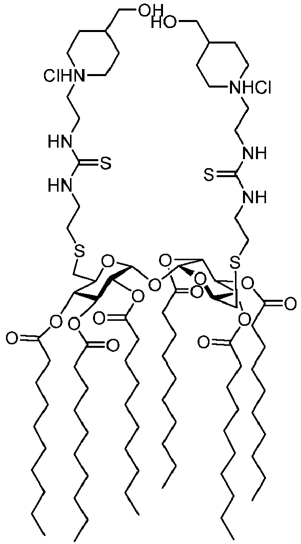 |
| JLF237 | 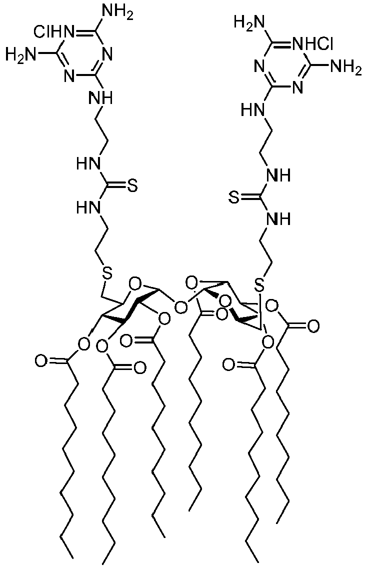 |
| JLF238 | 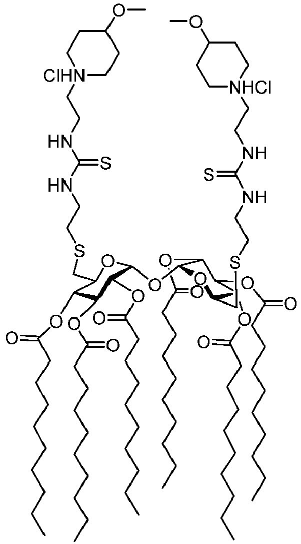 |
| JLF244 | 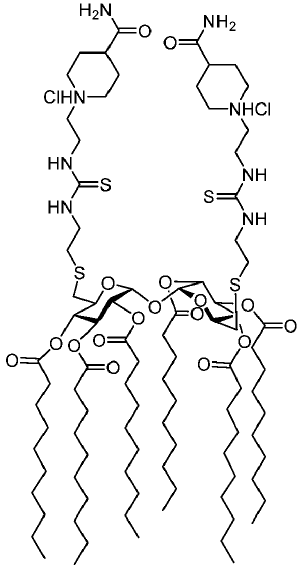 |
| JLF245 | 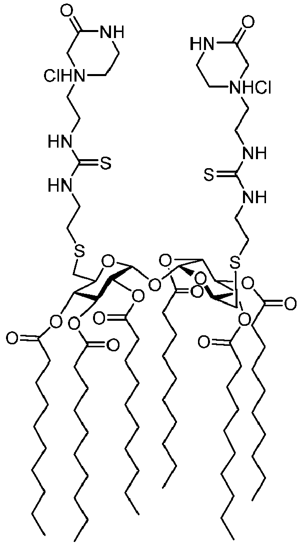 |
| JLF248 | 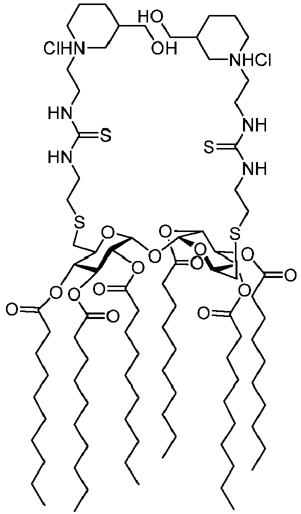 |
| JLF301 | 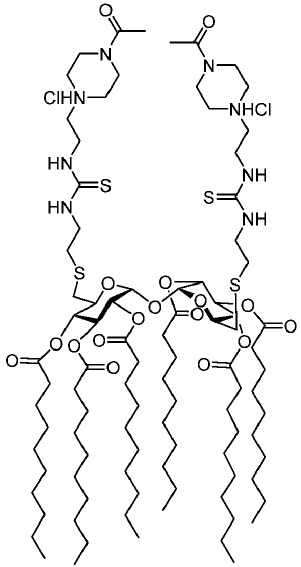 |
| JLJB604 | 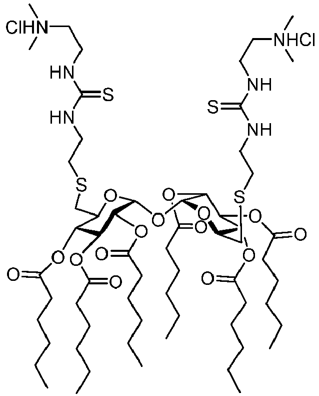 |
| JLJB605 | 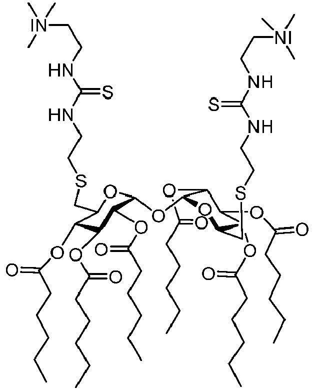 |
| JLJB617 | 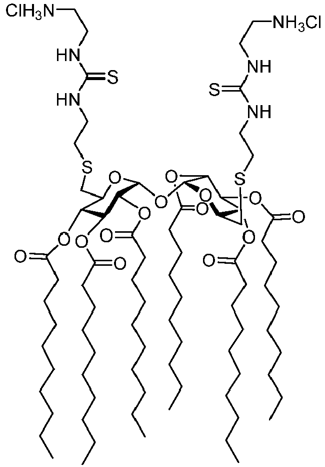 |
| JLJB618 | 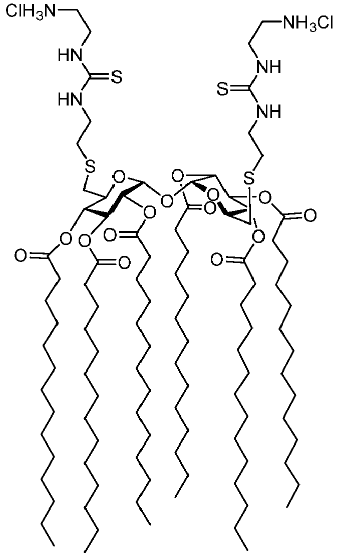 |
| JLJB619 | 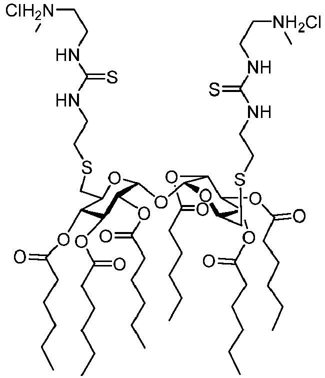 |
| PER20 | 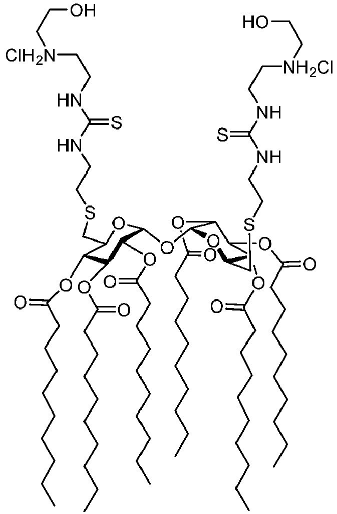 |
| JRL13 | 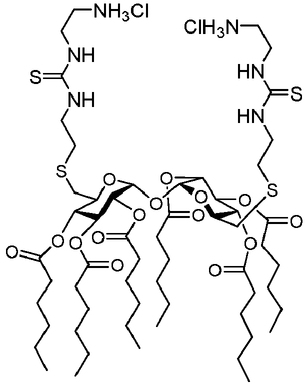 |
| JRL45 | 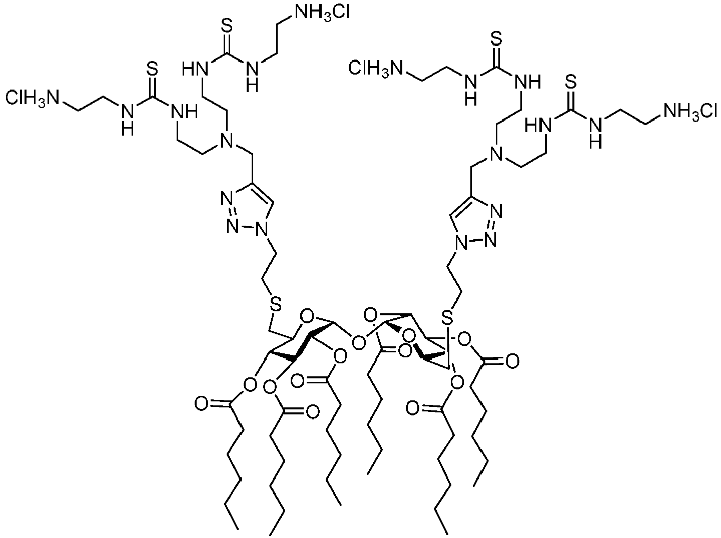 |
| JLF190 | 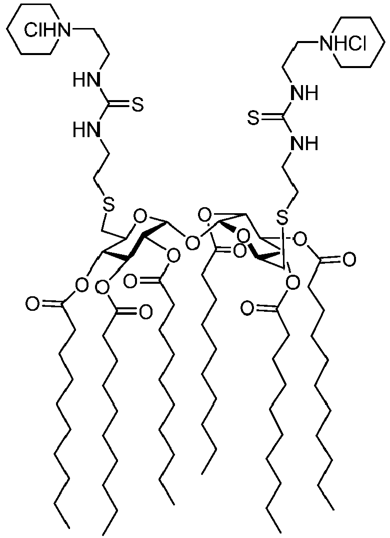 |
| JLF601 | 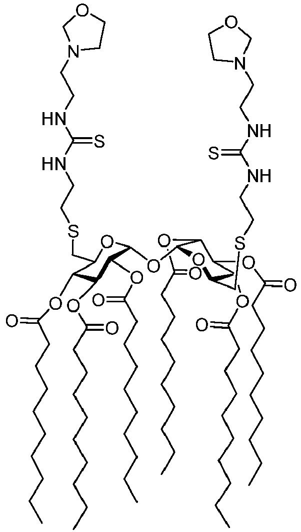 |
| JLF602 | 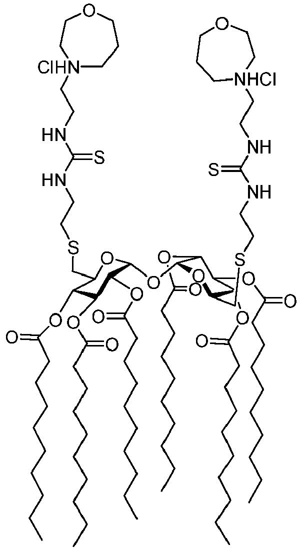 |
| JLF603 | 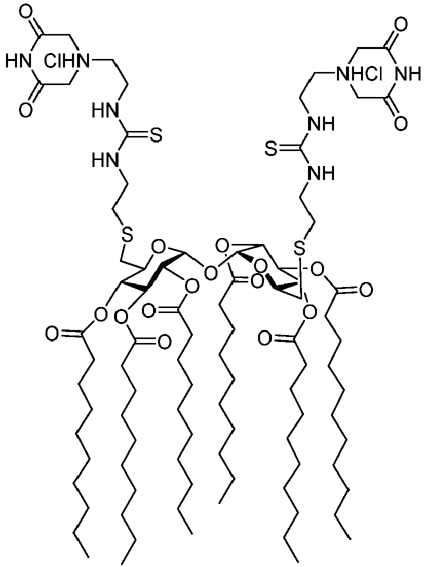 |
| JLF604 | 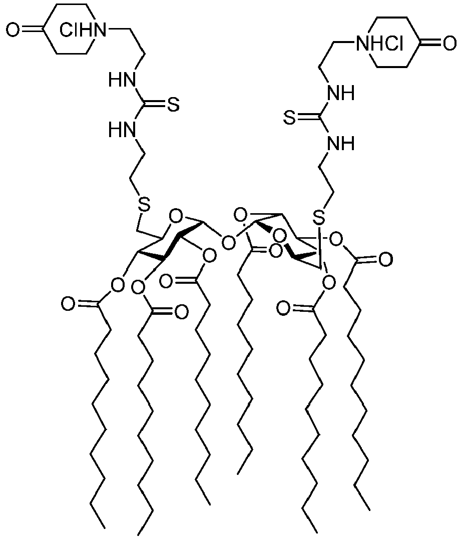 |
| JLF605 | 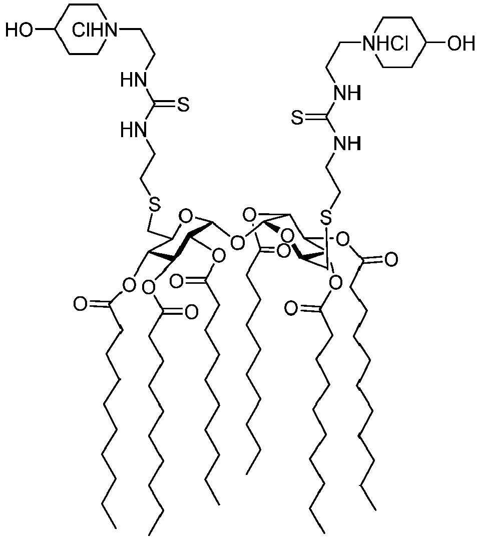 |
| JLF606 | 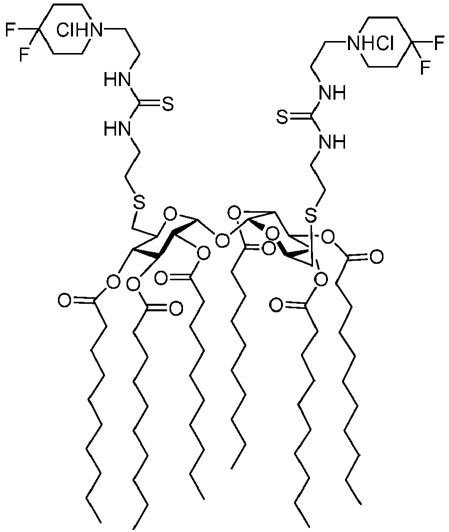 |
| JLF607 | 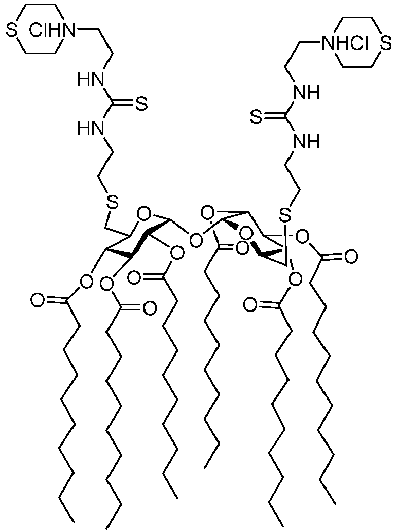 |
| JLF608 | 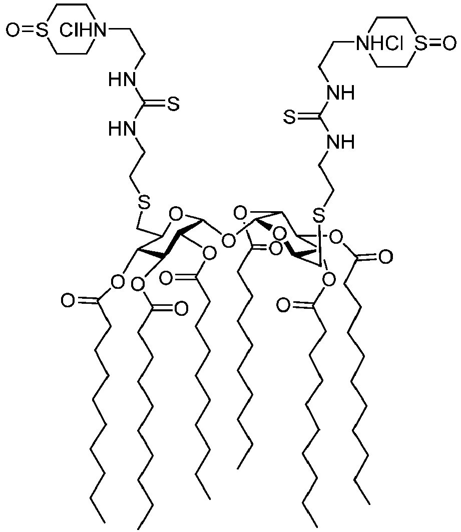 |
| JLF197 | 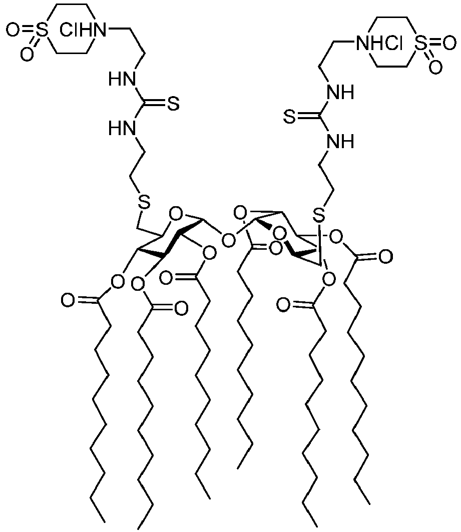 |
| JLF609 | 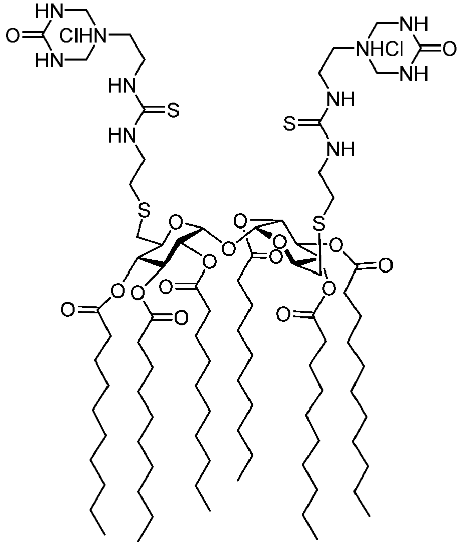 |
| JLF511 | 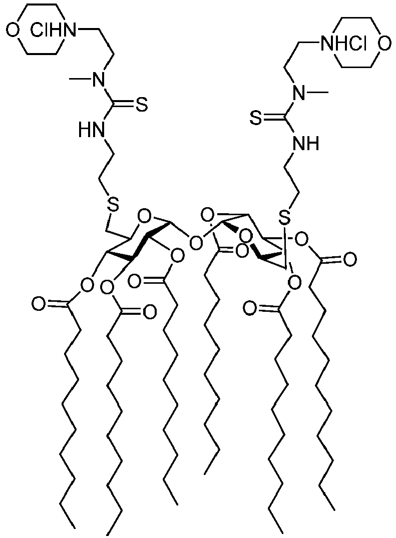 |
| JLF610 | 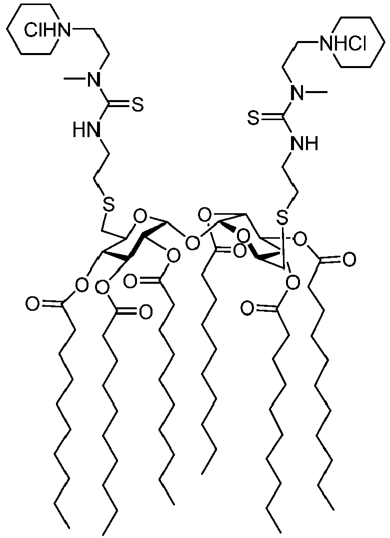 |
| JLF611 | 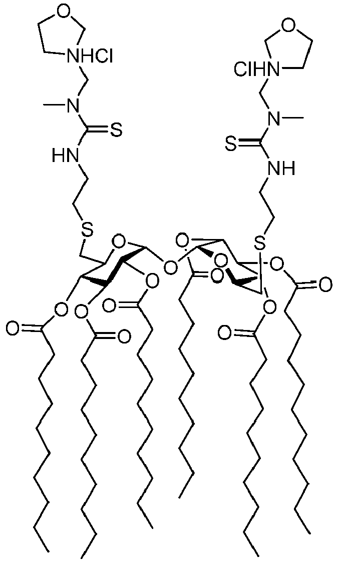 |
| JLF612 | 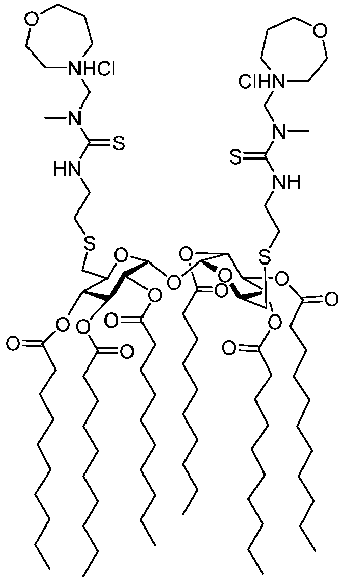 |
| JLF613 | 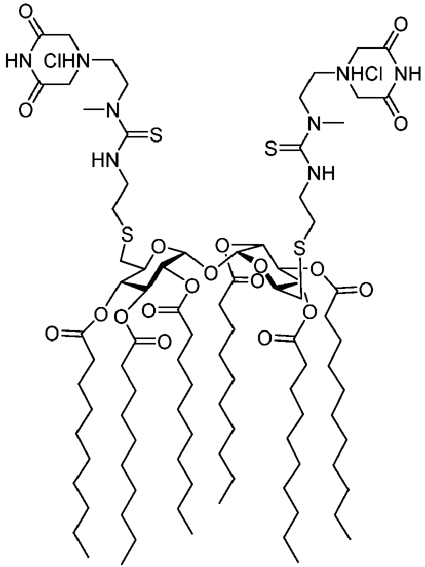 |
| JLF614 | 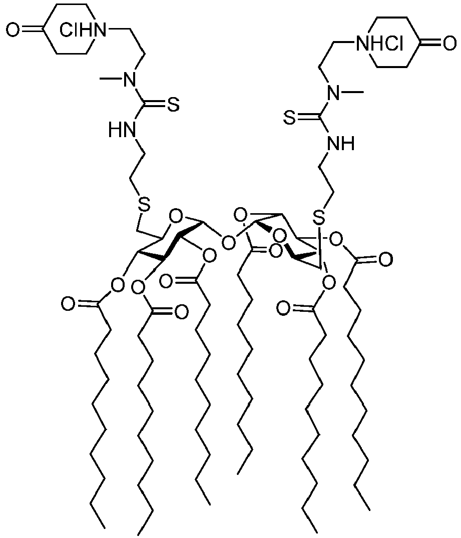 |
| JLF615 | 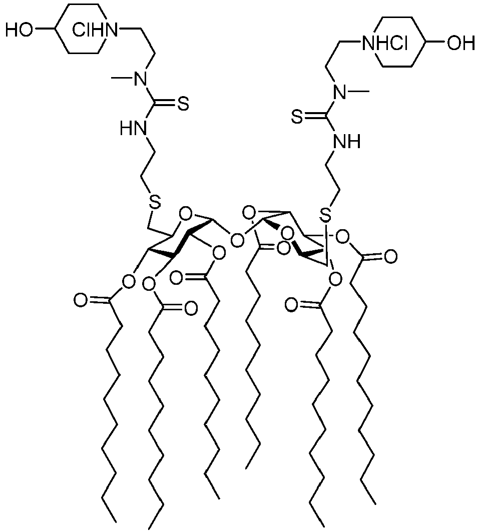 |
| JLF616 | 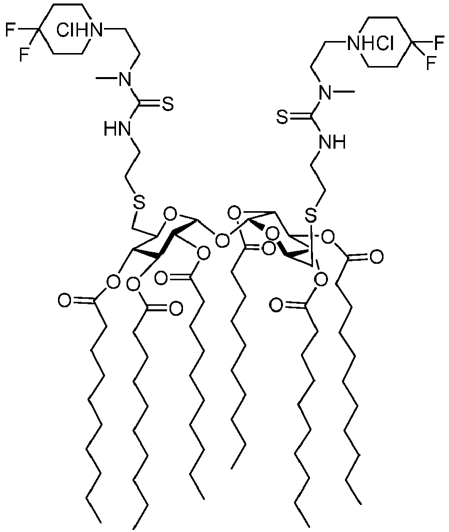 |
| JLF617 | 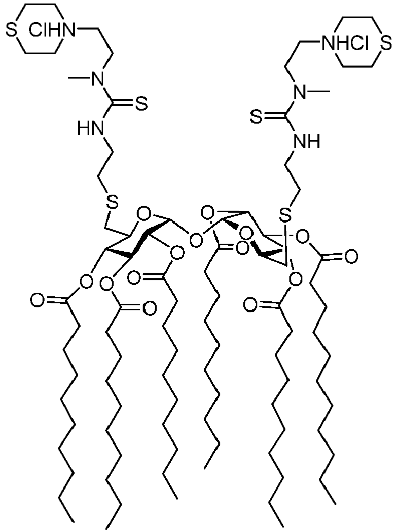 |
| JLF618 | 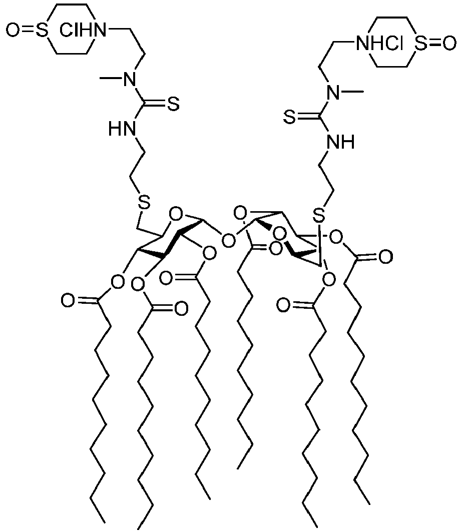 |
| JLF619 | 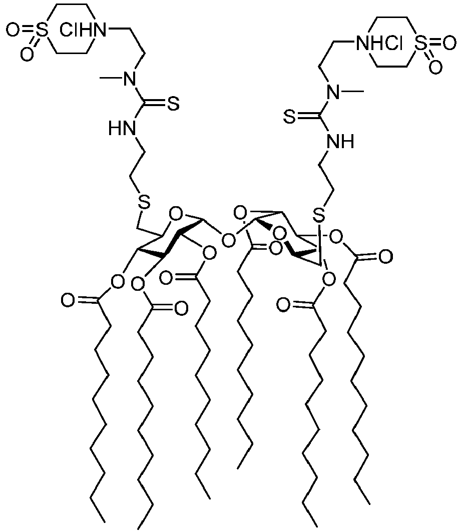 |
| JLF620 | 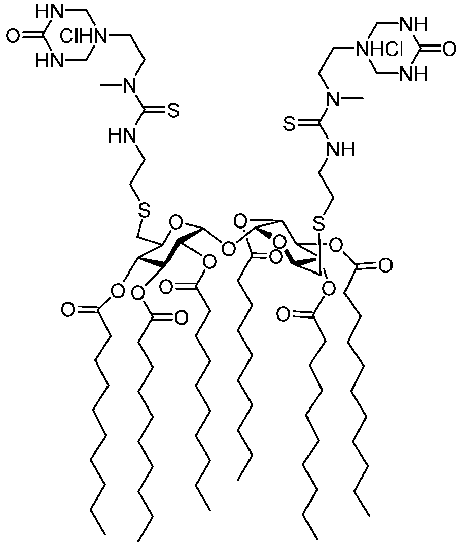 |
| PRX023 | 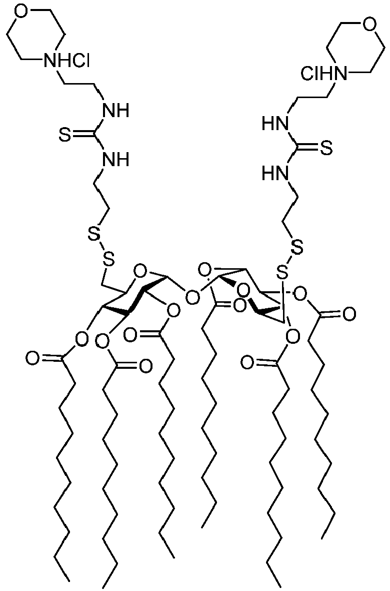 |
| JLF621 | 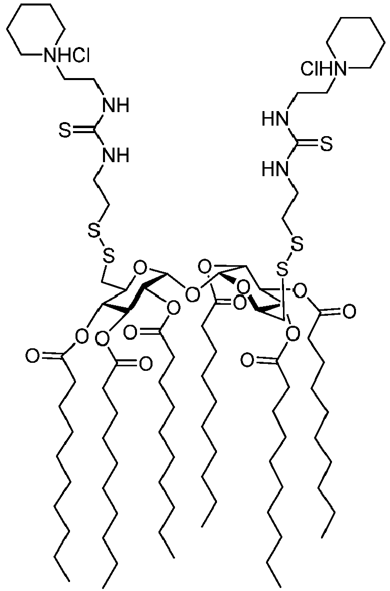 |
| JLF622 | 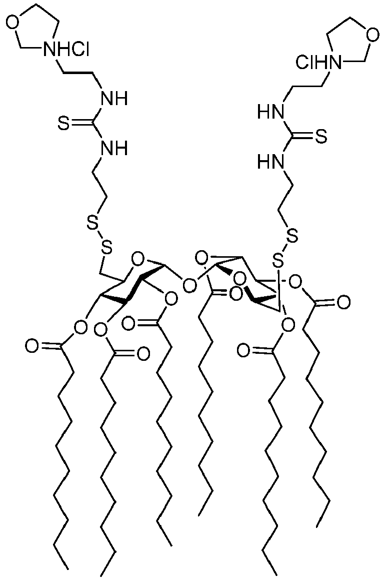 |
| JLF623 | 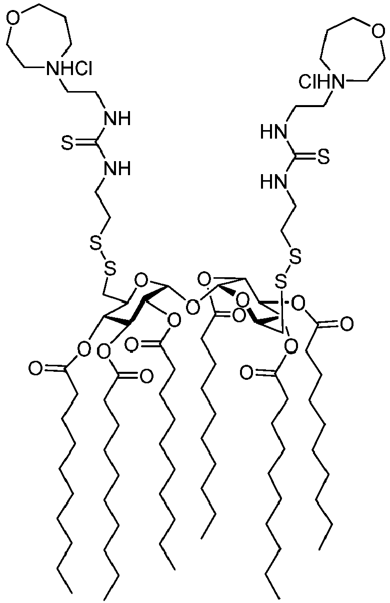 |
| JLF624 | 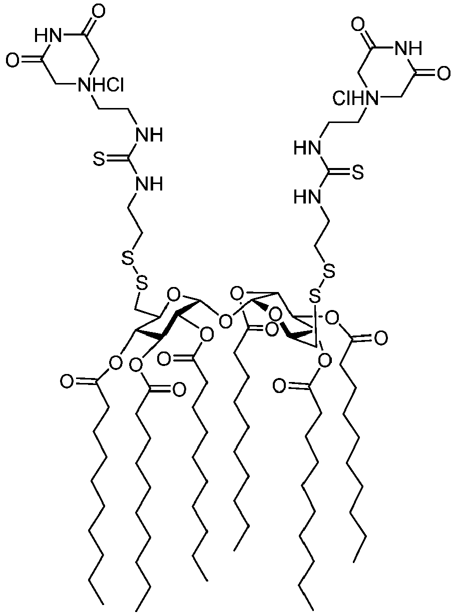 |
| JLF625 | 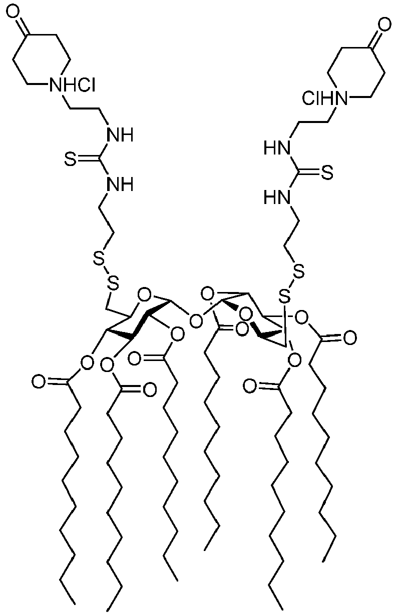 |
| JLF626 | 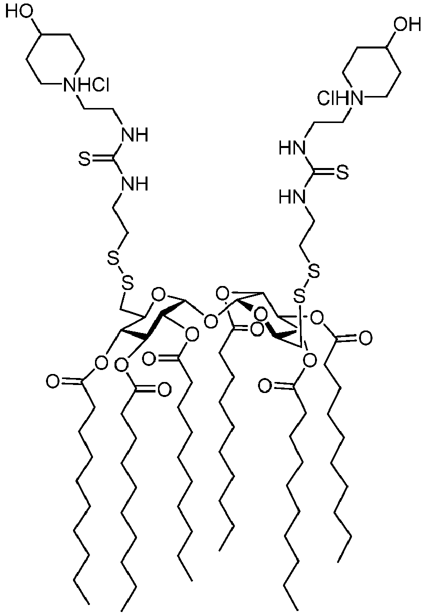 |
| JLF627 | 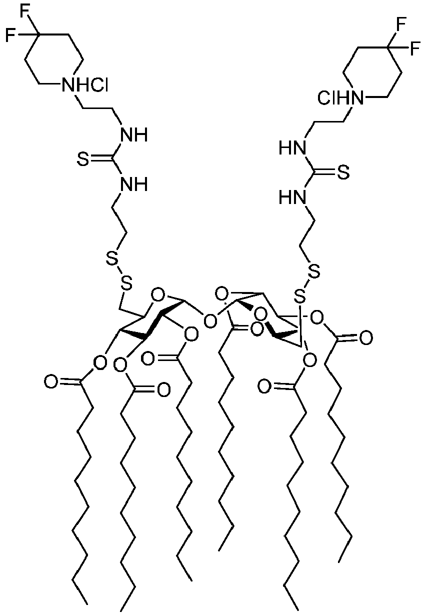 |
| JLF628 | 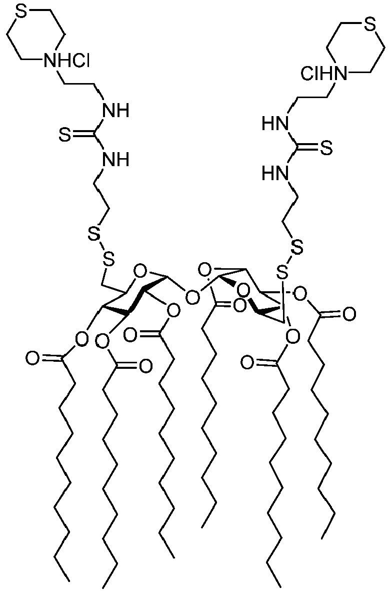 |
| JLF629 | 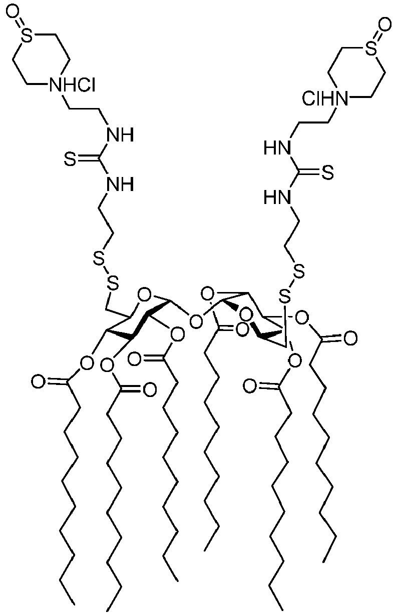 |
| JLF630 | 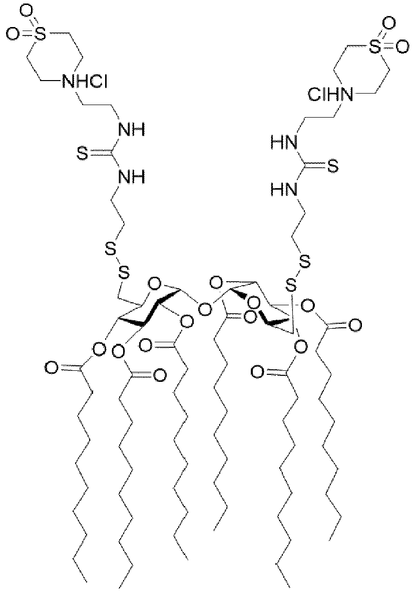 |
| JLF631 | 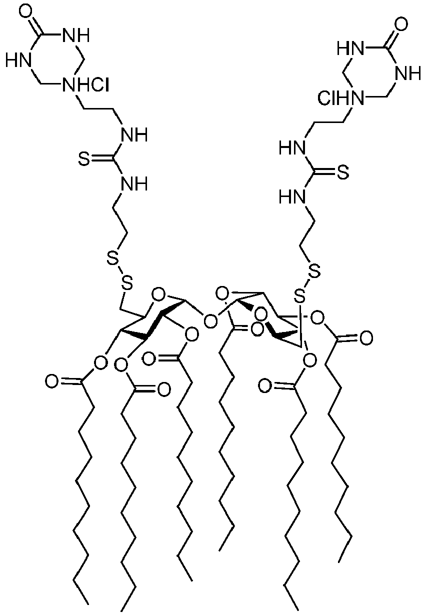 |
| JLF515 | 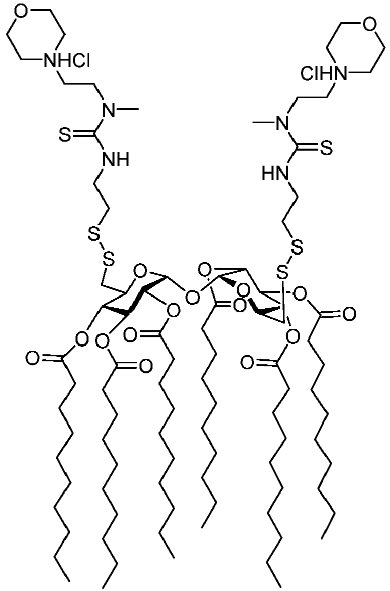 |
| JLF632 | 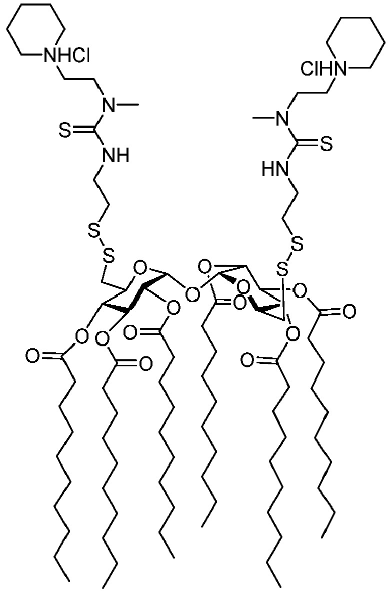 |
| JLF633 | 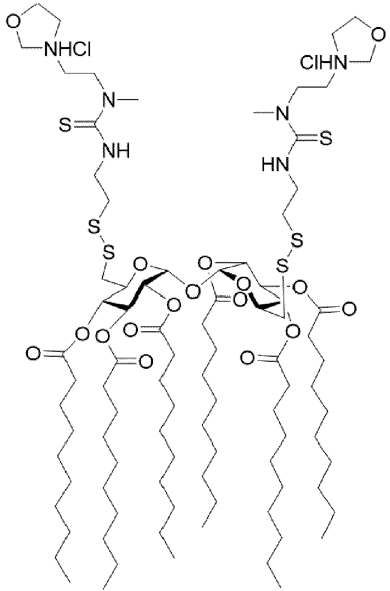 |
| JLF634 | 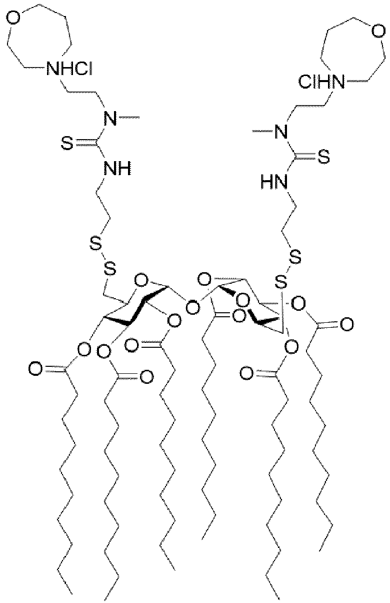 |
| JLF635 | 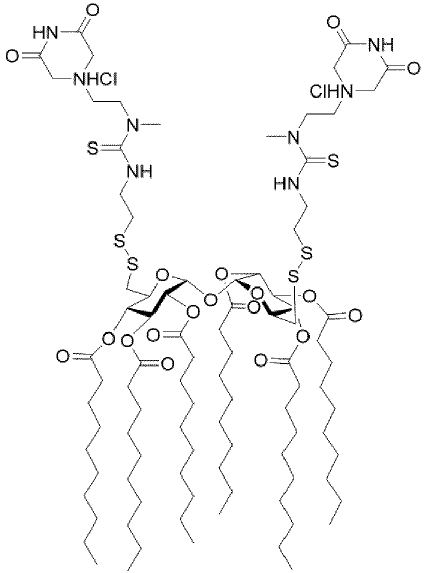 |
| JLF636 | 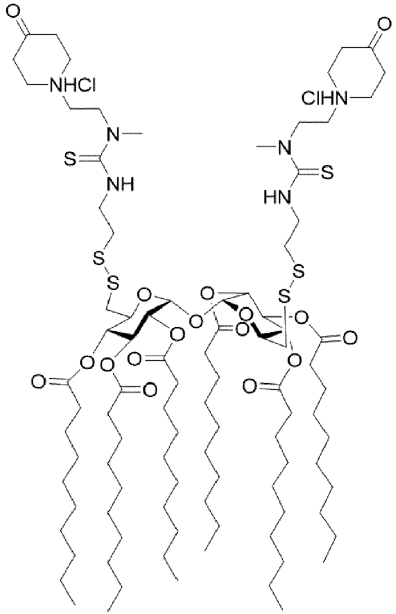 |
| JLF637 | 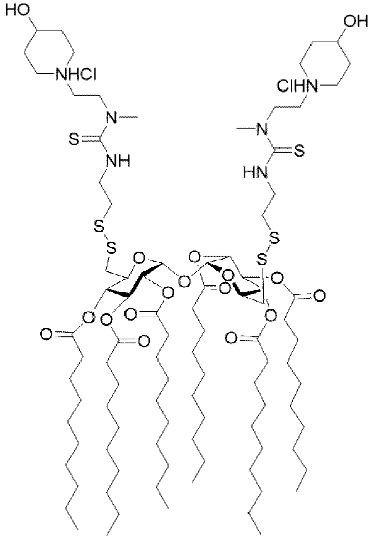 |
| JLF638 | 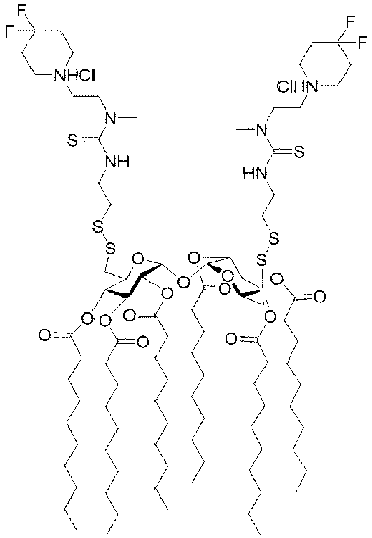 |
| JLF639 | 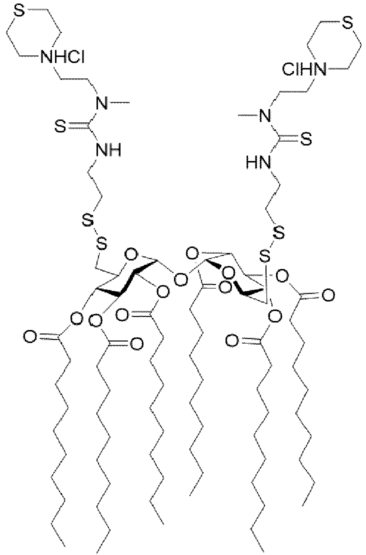 |
| JLF640 | 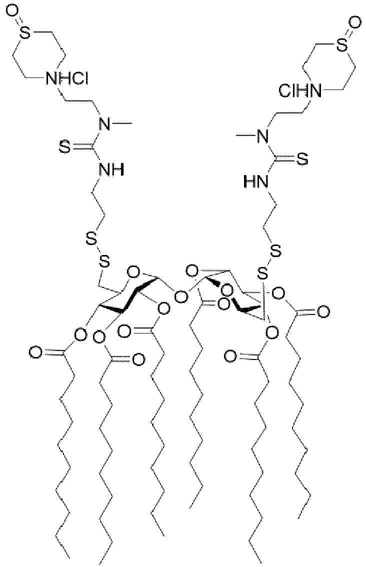 |
| JLF641 | 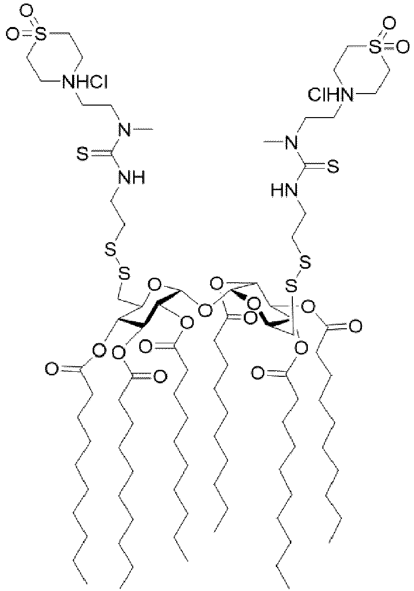 |
| JLF642 | 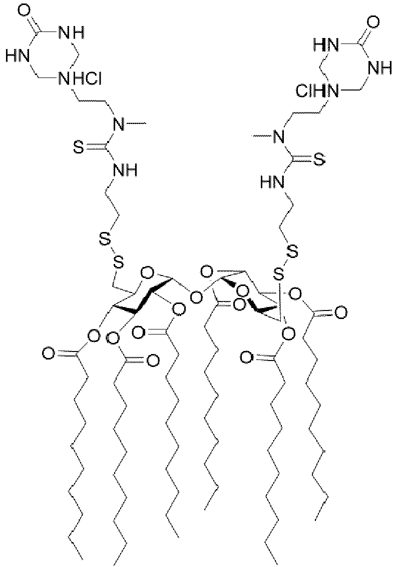 |
| PRX051 | 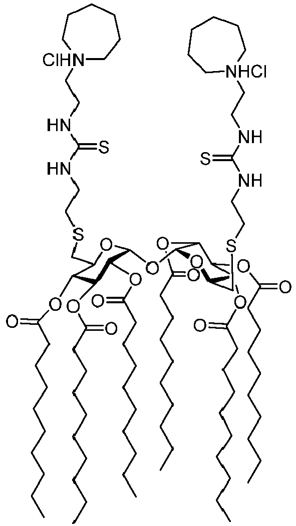 |
| PRX052 | 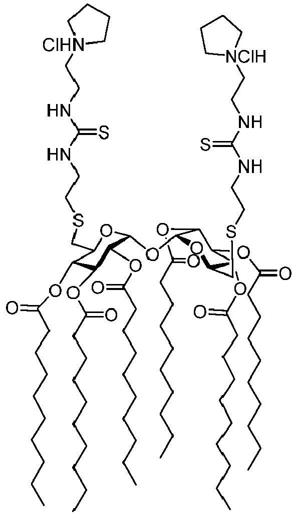 |
| PRX093 | 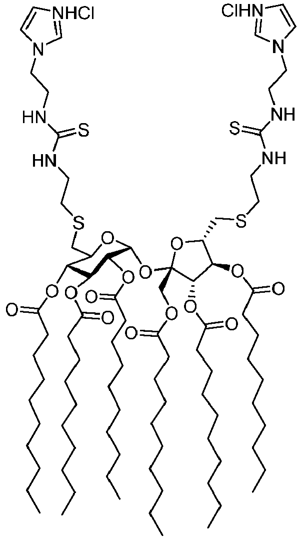 |
| PRX094 | 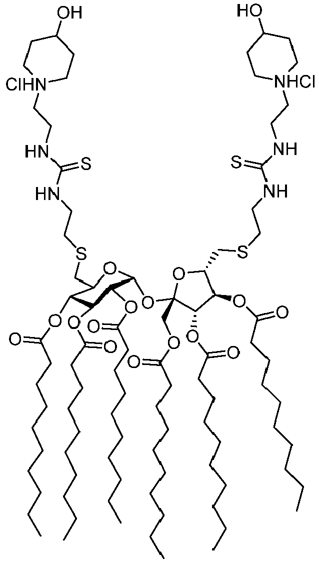 |
| PRX096 | 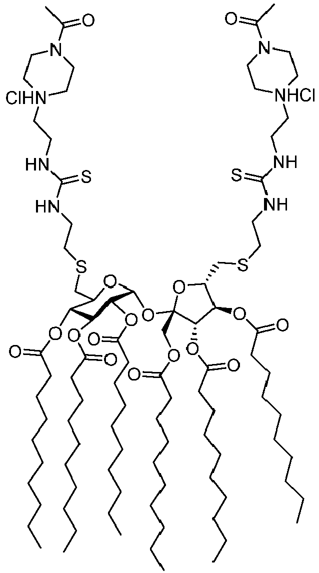 |

In some embodiments, provided compounds are provided and/or utilized in a salt form (e.g., a pharmaceutically acceptable salt form). Reference to a compound provided herein is understood to include reference to salts thereof, unless otherwise indicated. Moreover, reference to particular salts herein, e.g., in Table 1, is also intended to include reference to a free base form of said compound.

It is understood that while the exemplary compounds in Table 1 are represented as particular salts (e.g, chloride salts, iodide salts, and the like) that comprise a counterion (e.g., Cl⁻, I⁻, and the like), a person of skill in the art will appreciate that any suitable counterion can be used in conjunction with the cationic nitrogen groups in Table 1. Table 1, therefore, is intended to encompass any positively charged nitrogen group coupled with any chemically feasibly counterion. Accordingly, the specific counterions provided above are provided by way of example and are not intended to be limiting.

In some embodiments, a compound of formula I is selected from Table 2:

**Table 2**

| **Name** | **Structure** |
|---|---|
| JLF99 | 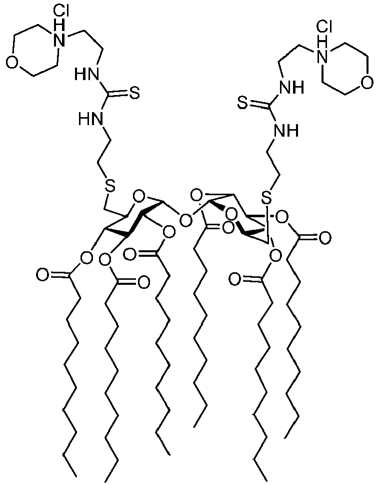 |
| JLF190 | 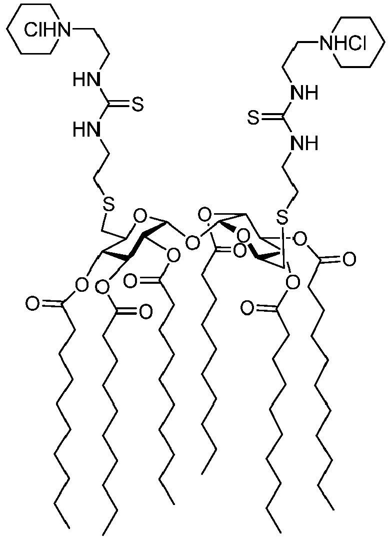 |
| JLF601 | 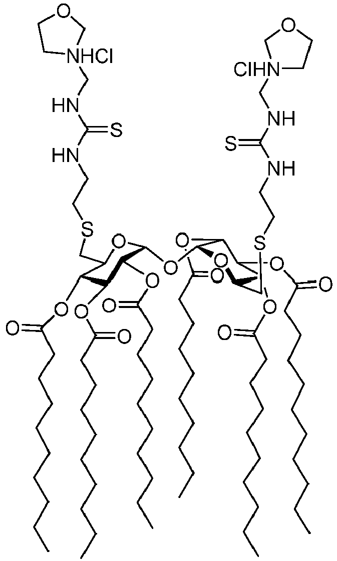 |
| JLF602 | 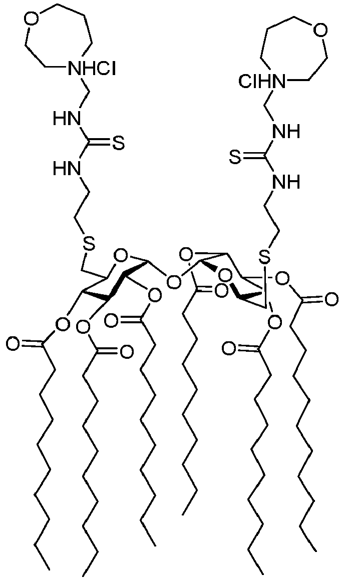 |
| JLF603 | 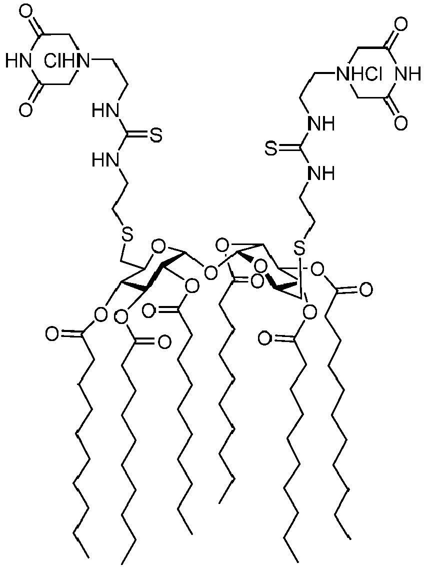 |
| JLF604 | 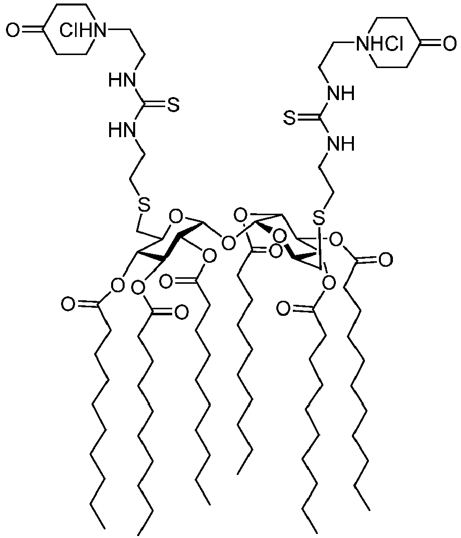 |
| JLF605 | 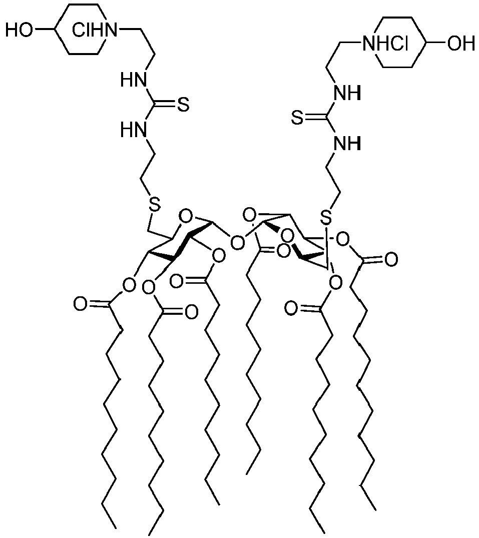 |
| JLF606 | 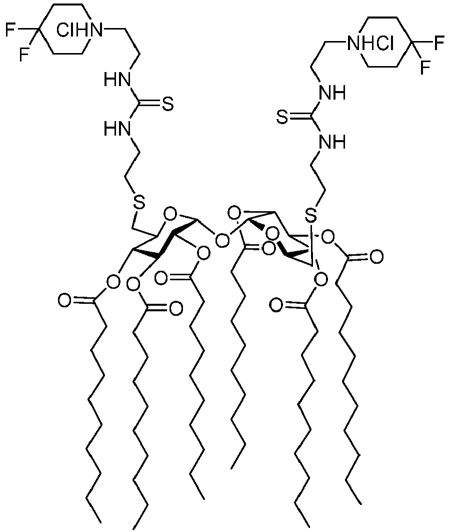 |
| JLF607 | 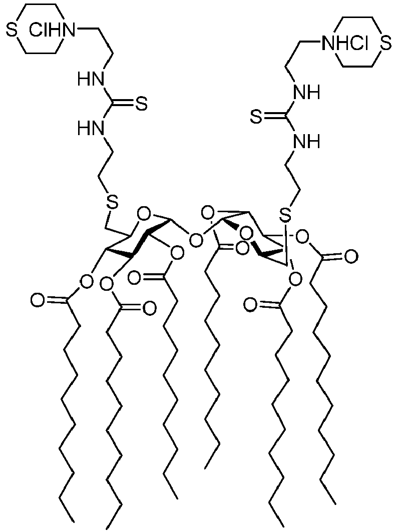 |
| JLF608 | 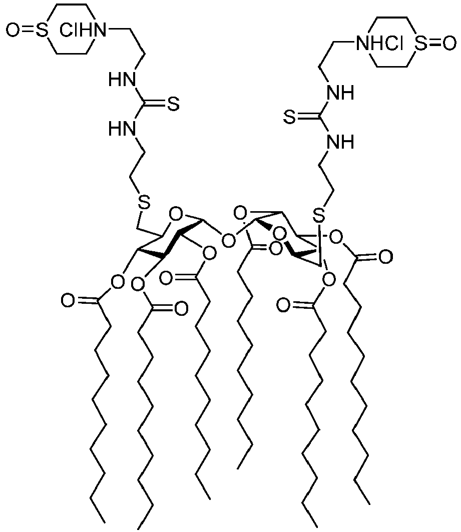 |
| JLF197 | 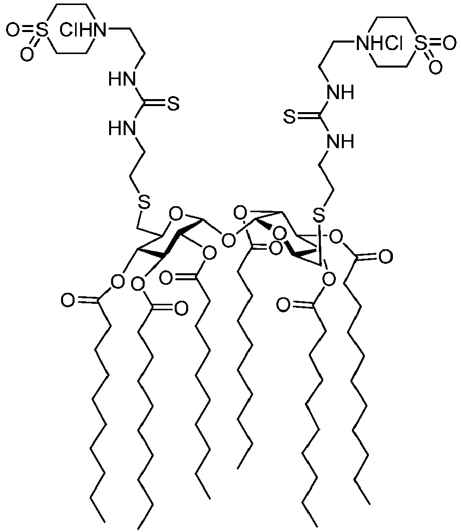 |
| JLF609 | 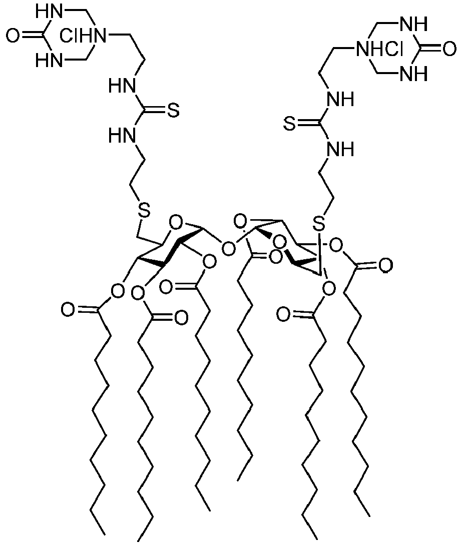 |
| JLF511 | 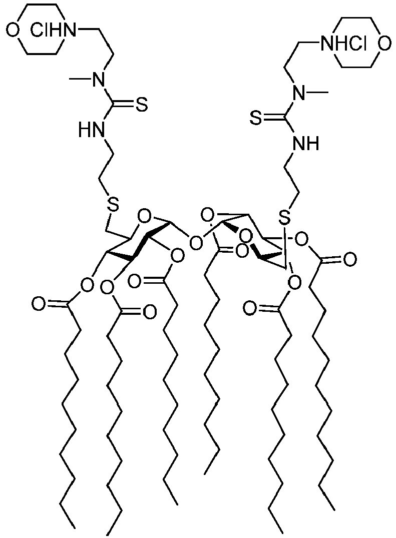 |
| JLF610 | 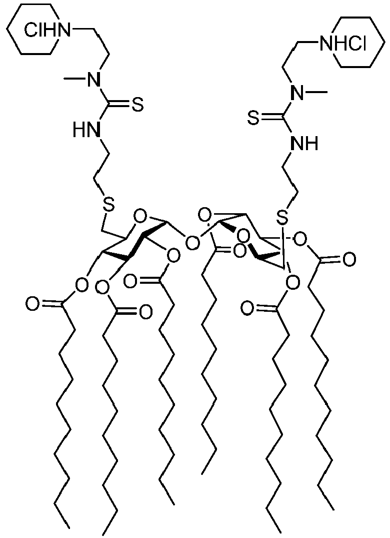 |
| JLF611 | 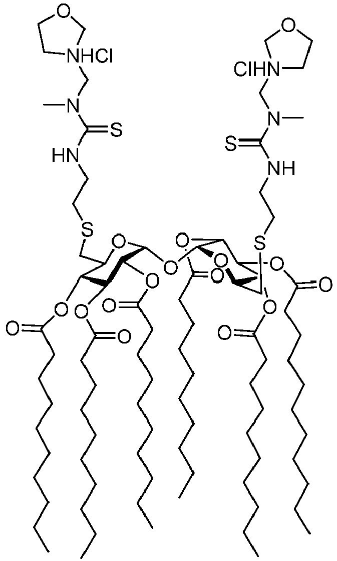 |
| JLF612 | 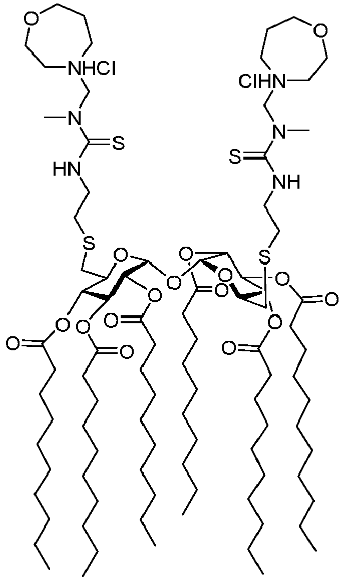 |
| JLF613 | 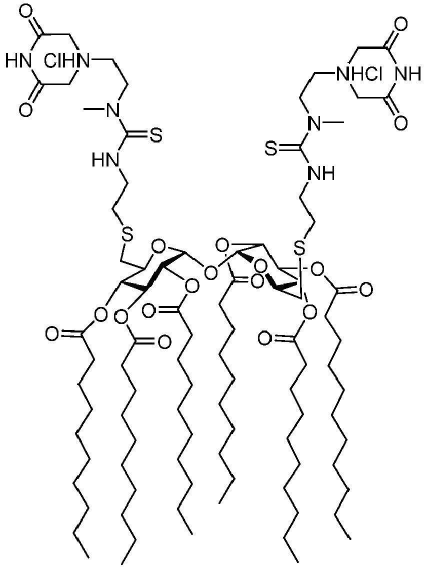 |
| JLF614 | 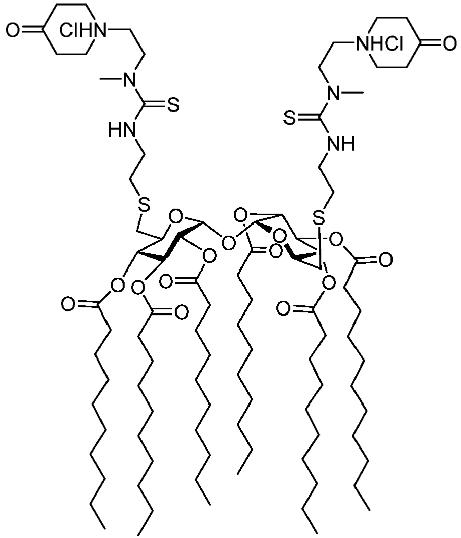 |
| JLF615 | 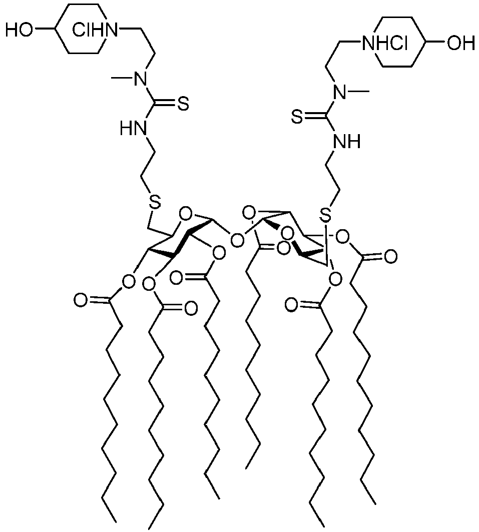 |
| JLF616 | 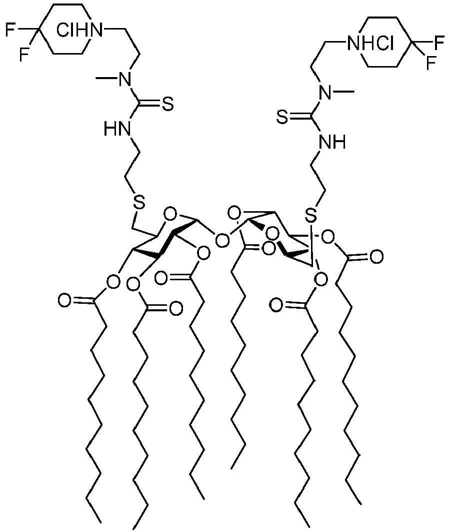 |
| JLF617 | 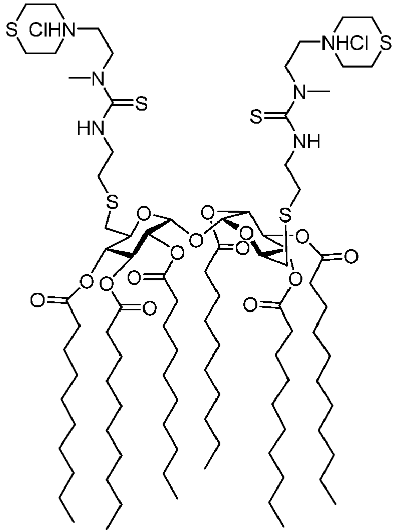 |
| JLF618 | 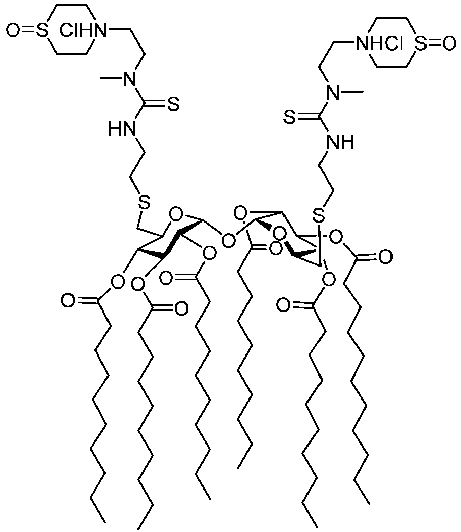 |
| JLF619 | 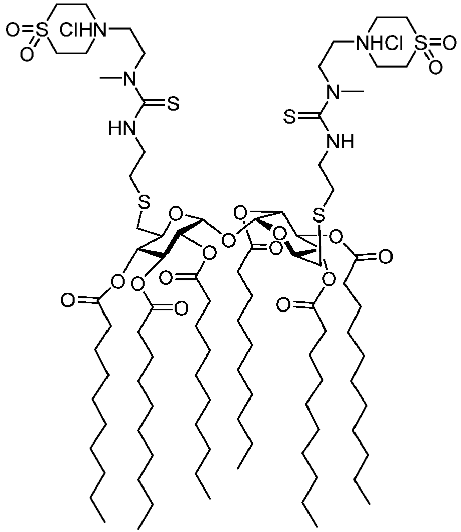 |
| JLF620 | 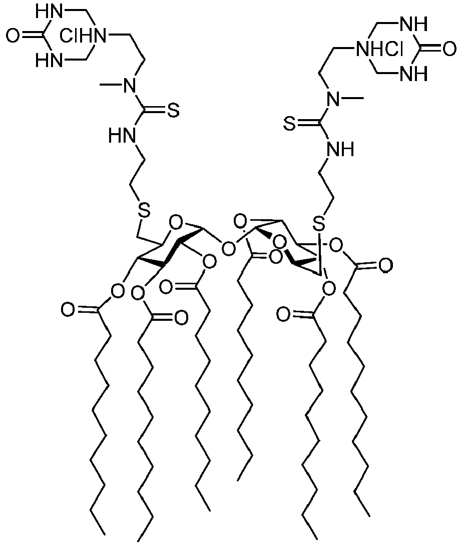 |
| PRX023 | 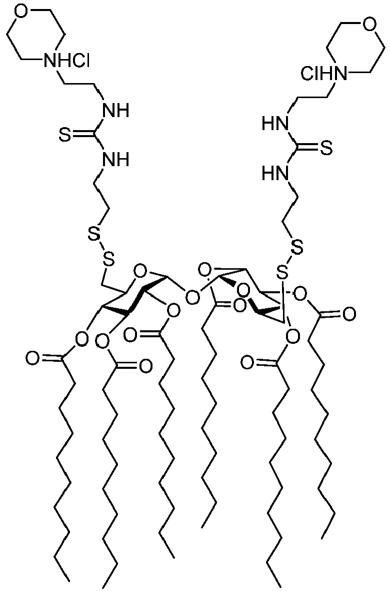 |
| JLF621 | 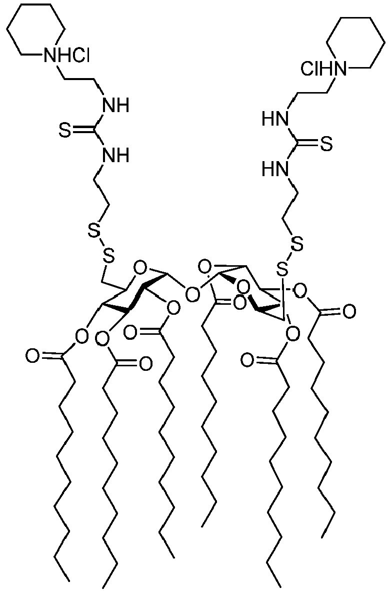 |
| JLF622 | 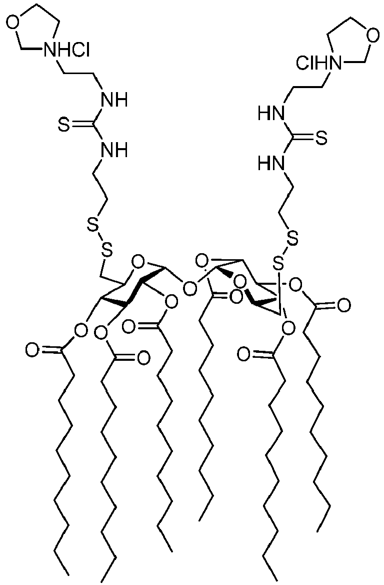 |
| JLF623 | 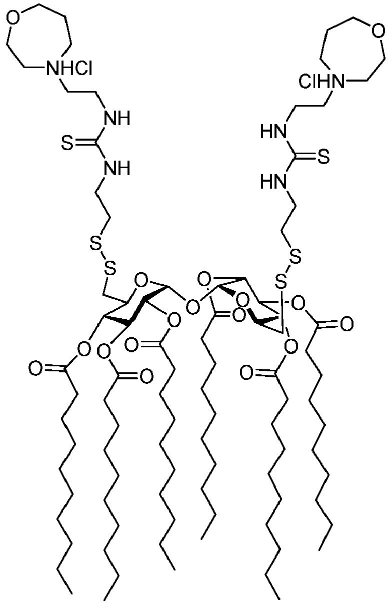 |
| JLF624 | 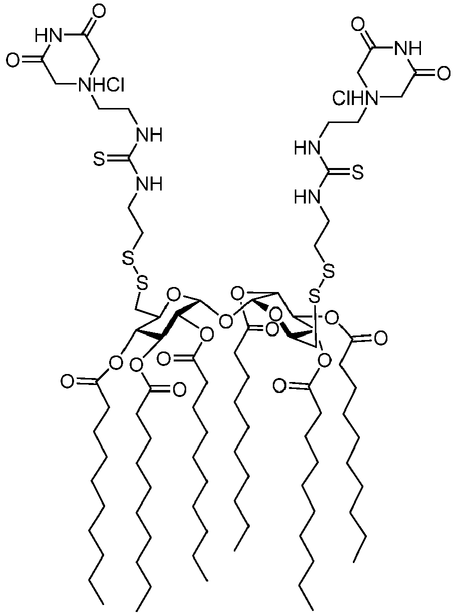 |
| JLF625 | 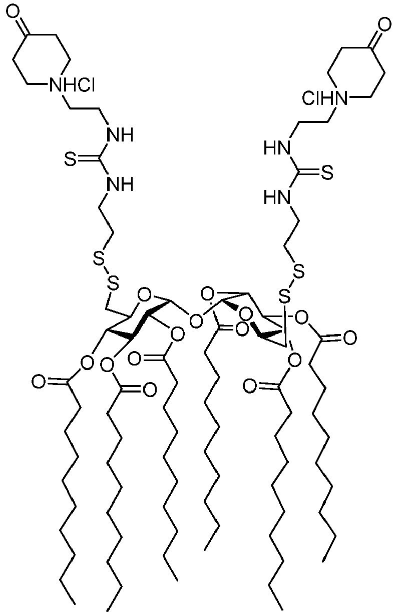 |
| JLF626 | 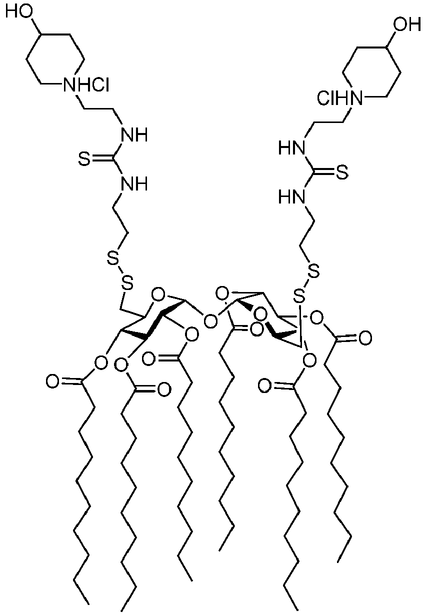 |
| JLF627 | 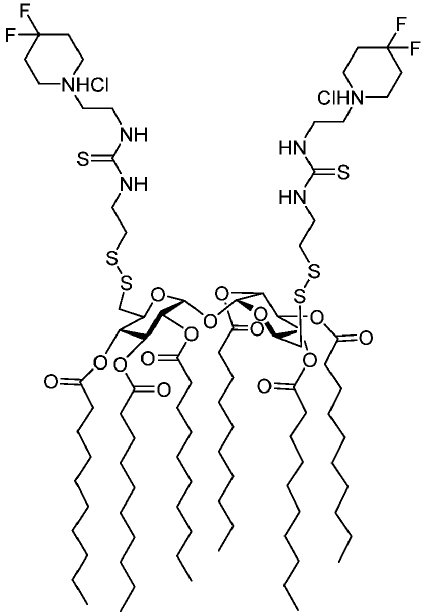 |
| JLF628 | 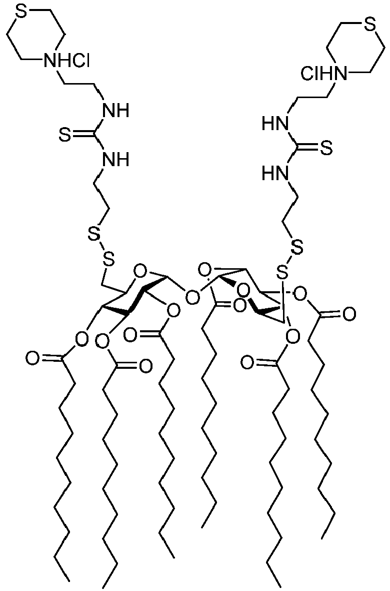 |
| JLF629 | 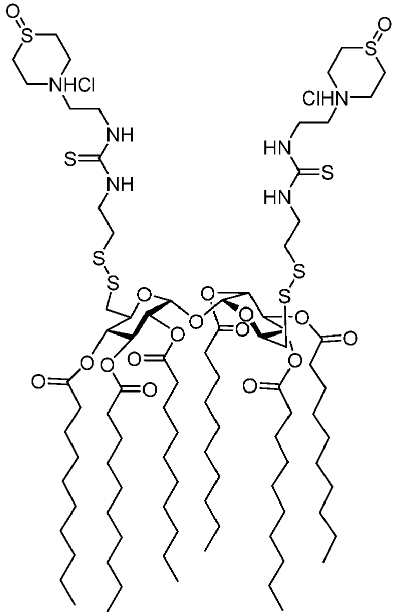 |
| JLF630 | 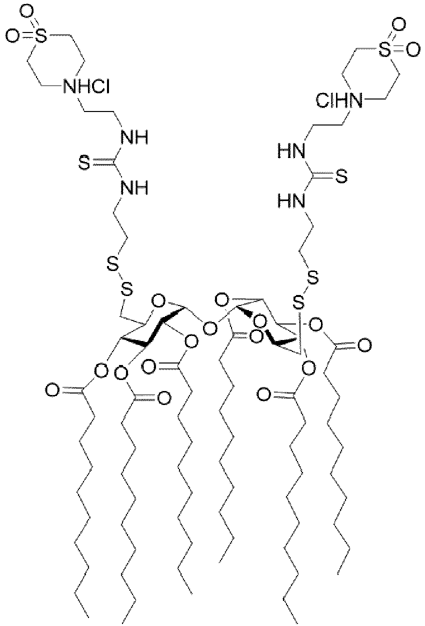 |
| JLF631 | 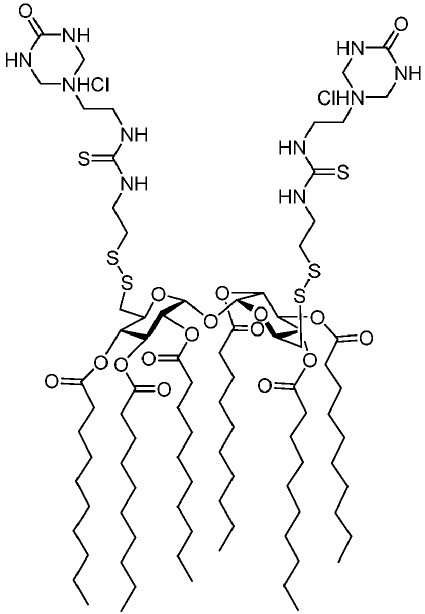 |
| JLF515 | 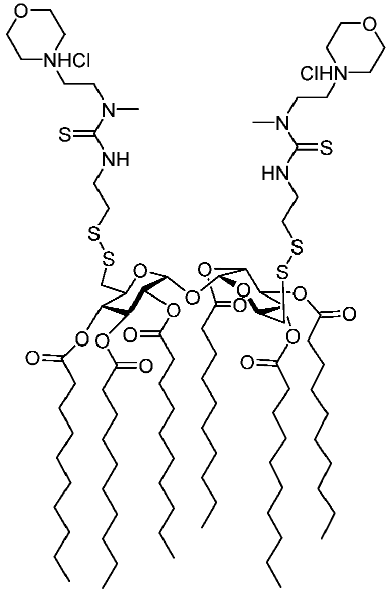 |
| JLF632 | 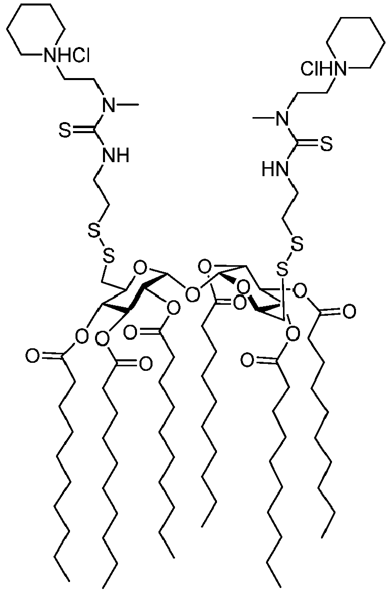 |
| JLF633 | 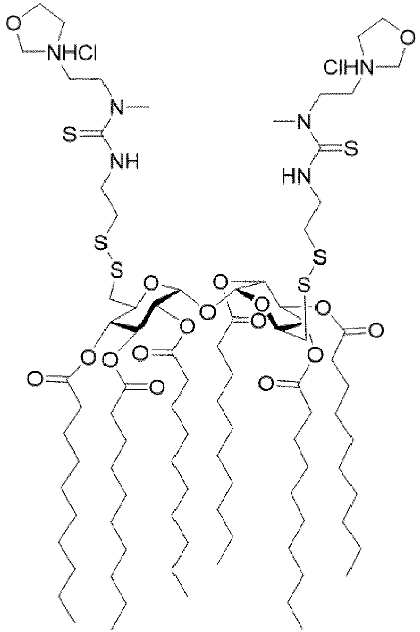 |
| JLF634 | 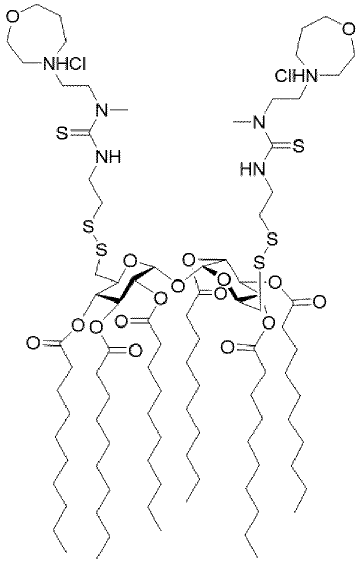 |
| JLF635 | 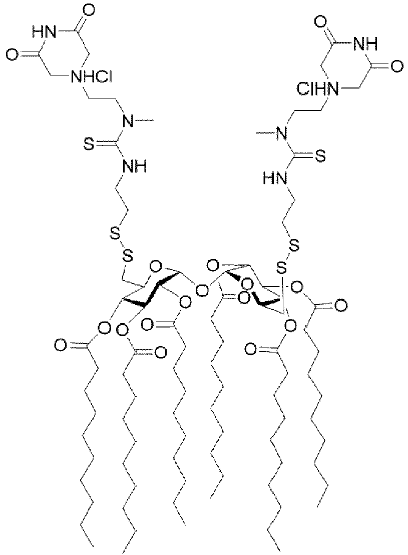 |
| JLF636 | 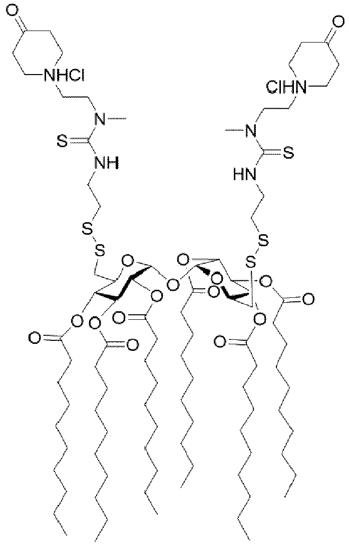 |
| JLF637 | 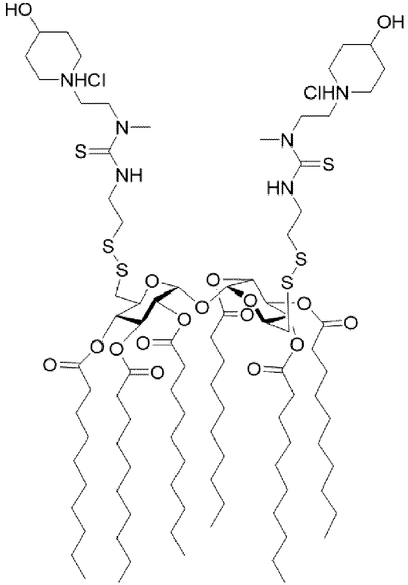 |
| JLF638 | 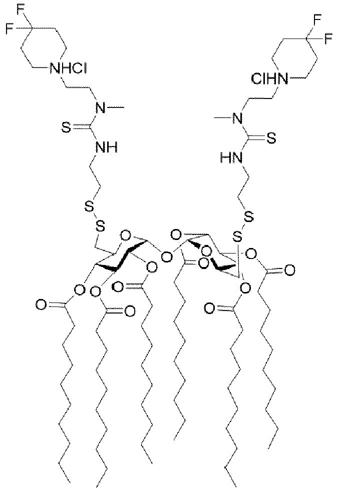 |
| JLF639 | 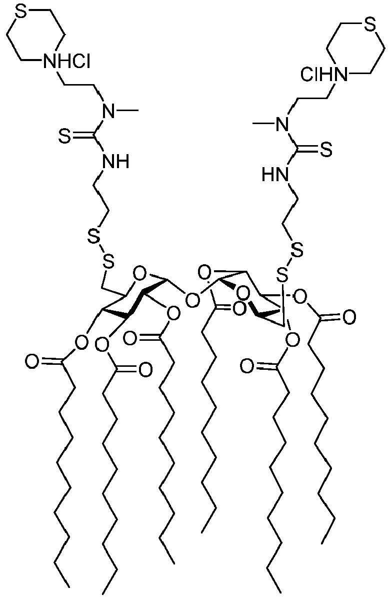 |
| JLF640 | 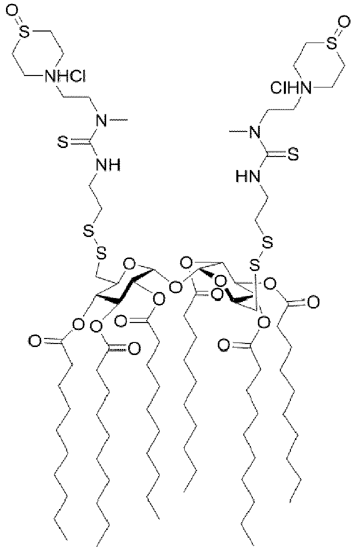 |
| JLF641 | 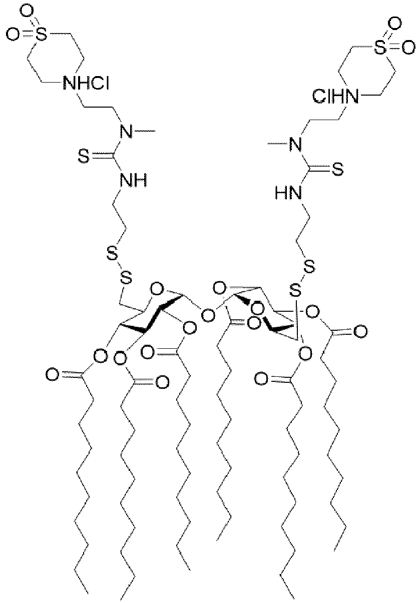 |
| JLF642 | 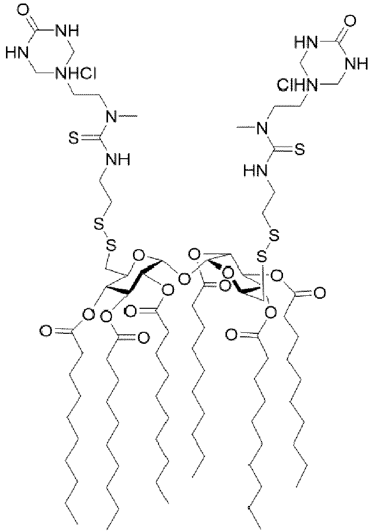 |

In some embodiments, provided compounds are provided and/or utilized in a salt form (e.g., a pharmaceutically acceptable salt form). Reference to a compound provided herein is understood to include reference to salts thereof, unless otherwise indicated. Moreover, reference to particular salts herein, e.g., in Table 2, is also intended to include reference to a free base form of said compound.

It is understood that while the exemplary compounds in Table 2 are represented as particular salts (e.g, chloride salts, iodide salts, and the like) that comprise a counterion (e.g., Cl⁻, I⁻, and the like), a person of skill in the art will appreciate that any suitable counterion can be used in conjunction with the cationic nitrogen groups in Table 2. Table 2, therefore, is intended to encompass any positively charged nitrogen group coupled with any chemically feasibly counterion. Accordingly, the specific counterions provided above are provided by way of example and are not intended to be limiting.. Polycationic oligosaccharaide complexes described herein can be prepared according to methods known to those of skill in the art. For example, provided oligosaccharide complexes can be prepared according to the methods provided in EP 21382958.3, which is incorporated herein by reference in its entirety.

In some embodiments, the present disclosure provides a complex comprising a compound selected from:

| **Name** | **Structure** |
|---|---|
| JRL13 | 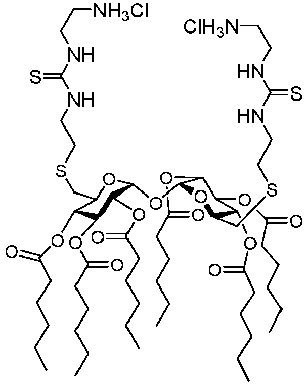 |
| JRL45 | 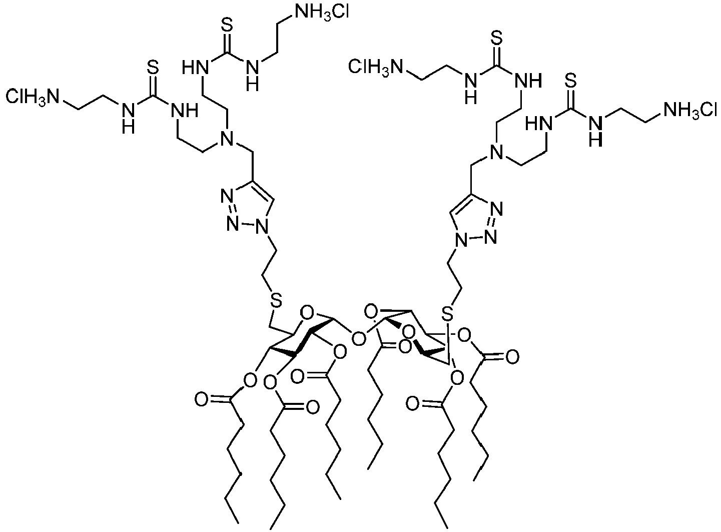 |

In some embodiments, a complex described herein does not comprise JRL13 or JRL45.

### Surfactants

In some embodiments, a complex described herein further comprises a surfactant. In some embodiments, a surfactant is selected from a polysorbate (e.g., polysorbate 20 (Tween20), polysorbate 40 (Tween40), polysorbate 60 (Tween60), and polysorbate 80 (Tween80)), poloxamer, and/or a compound comprising an amphiphilic moiety selected from polyalkylene glycols (e.g., polyethylene glycol), poly(2-oxazoline), poly(2-oxazine), polysarcosine, polyvinylpyrrolidone, and poly[N-(2-hydroxypropyl)methacrylamide, wherein the amphiphilic moiety is bonded to one or more C₁₂-C₂₀ aliphatic groups.

In some embodiments, a surfactant is a polysorbate. In some embodiments, a polysorbate is polysorbate 20 (Tween 20), polysorbate 40 (Tween 40), polysorbate 60 (Tween 60) or polysorbate 80 (Tween 80). In some embodiments, a polysorbate is polysorbate 20. In some embodiments, a polysorbate is polysorbate 40. In some embodiments, a polysorbate is polysorbate 60. In some embodiments, a polysorbate is polysorbate 80.

In some embodiments, a ratio of oligosaccharide to surfactant is about 1 :0.0075 to about 1:3. In some embodiments, a ratio of oligosaccharide to surfactant is about 1:0.0075 to about 1:1.5. In some embodiments, a ratio of oligosaccharide to surfactant is about 1:1.

In some embodiments, a ratio of oligosaccharide to surfactant about 1:0.0075, about 1:0.01, about 1:0.1, about 1:0.5, about 1:1, about 1:1.5, about 1:2, about 1:2.5, or about 1:3.

In some embodiments, a surfactant is a polysorbate. In some embodiments, a ratio of oligosaccharide to polysorbate is 1:0.0075 to 1:3. In some embodiments, a surfactant is a polysorbate. In some embodiments, a ratio of oligosaccharide to polysorbate is 1:0.0075 to 1:1.5. In some embodiments, a surfactant is a polysorbate. In some embodiments, a ratio of oligosaccharide to polysorbate is 1:0.0075 to 1:1. In some embodiments, a ratio of oligosaccharide to polysorbate is about 1:0.0075, about 1:0.01, about 1:0.1, about 1:0.5, about 1:1, about 1:1.5, about 1:2, about 1:2.5, or about 1:3.

In some embodiments, the present disclosure provides a complex comprising i) a cationic oligosaccharide comprising one or more cationic moieties bonded to a trehalose, a sucrose, or a gluco-n-oligosaccharide moiety, where n is 2-6; ii) a surfactant; and iii) one or more additives, wherein a molar ratio of a cationic oligosaccharide to a surfactant is about 1:0.0075 to about 1:3. In some embodiments, a molar ratio of cationic oligosaccharide to a surfactant is about 1:0.0075, about 1:0.01, about 1:0.2, about 1:0.3, about 1:0.4, about 1:0.5, about 1:0.6, about 1:0.7, about 1:0.8, about 1:0.9, about 1:1. In some embodiments, a molar ratio of a cationic oligosaccharide to a surfactant is about 1:0.5 to about 1:1 (e.g., about 1:0.5, about 1:0.75, or about 1:1). In some embodiments, a molar ratio of a cationic oligosaccharide to a surfactant is about 1:0.5.

### Additives

As described herein, the present disclosure provides a complex that further comprises one or more additives, wherein the one or more additives are selected from: a sterol, a helper lipid, an immunomodulator, and a targeting moiety. It is understood that a complex described herein comprises at least one of a sterol, a helper lipid, an immunomodulator, and a targeting moiety, alone or in any combination.

In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a helper lipid. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise an immunomodulator. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a targeting molecule.

In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or a helper lipid. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or an immunomodulator. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or a targeting molecule.

In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a helper lipid and/or an immunomodulator. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a helper lipid and/or a targeting molecule.

In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise an immunomodulator and/or a targeting molecule. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or a helper lipid and/or an immunomodulator. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or a helper lipid and/or a targeting molecule. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a helper lipid and/or an immunomodulator and/or a targeting molecule. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a sterol and/or a helper lipid and/or a targeting molecule. In some embodiments, a complex described herein comprises i) a cationic oligosaccharide comprising one or more cationic moieties bonded to a trehalose, a sucrose, or a gluco-n-oligosaccharide moiety, where n is 2-6; ii) a surfactant; and iii) one or more additives that are or comprise a helper lipid and/or an immunomodulator and/or a targeting molecule.

In some embodiments, a complex described herein comprises i) a cationic oligosaccharide comprising one or more cationic moieties bonded to a trehalose moiety; ii) a surfactant; and iii) one or more additives that are or comprise a sterol and/or a helper lipid and/or an immunomodulator. In some embodiments, a complex described herein comprises i) a cationic oligosaccharide comprising one or more cationic moieties bonded to a sucrose moiety; ii) a surfactant; and iii) one or more additives that are or comprise a sterol and/or a helper lipid and/or an immunomodulator.

### Sterols

In some embodiments, a sterol is or comprises β-sitosterol, stigmasterol, cholesterol, cholecalciferol, ergocalciferol, calcipotriol, botulin, lupeol, ursolic acid, oleanolic acid, cycloartenol, lanosterol, or α-tocopherol. In some embodiments, a sterol is or comprises an analogue of one or more of β-sitosterol, stigmasterol, cholesterol, cholecalciferol, ergocalciferol, calcipotriol, botulin, lupeol, ursolic acid, oleanolic acid, cycloartenol, lanosterol, or α-tocopherol, bonded to an optionally substituted C₁-C₃₀ aliphatic moiety. In some embodiments, a sterol is β-sitosterol. In some embodiments, a sterol is stigmasterol. In some embodiments, a sterol is cholesterol. In some embodiments, a sterol is cholecalciferol. In some embodiments, a sterol is ergocalciferol. In some embodiments, a sterol is calcipotriol. In some embodiments, a sterol is botulin. In some embodiments, a sterol is lupeol. In some embodiments, a sterol is ursolic acid. In some embodiments, a sterol is oleanolic acid. In some embodiments, a sterol is cycloartenol. In some embodiments, a sterol is lanosterol. In some embodiments, a sterol is α-tocopherol.

In some embodiments, a molar ratio of a cationic oligosaccharide to a sterol is from about 1:0.25 to about 1:1. In some embodiments, a molar ratio of cationic oligosaccharide to a sterol is about 1:0.25, about 1:0.5, or about 1:1. In some embodiments, a molar ratio of a cationic oligosaccharide to a sterol is about 1:0.5.

### Helper Lipids

In some embodiments, a helper lipid is or comprises a lipid selected from the group consisting of phosphatidylcholines, phosphatidylethanolamines, phosphatidylglycerols, phosphatidic acids, phosphatidylserines and sphingomyelins, more preferably selected from the group consisting of distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), and combinations thereof.

In some embodiments, a helper lipid is DSPC and/or DMPC. In some embodiments, a helper lipid is DSPC. In some embodiments, a helper lipid is DMPC.

In some embodiments, a molar ratio of a cationic oligosaccharide to a helper lipid is from about 1:0.25 to about 1:1. In some embodiments, a molar ratio of a cationic oligosaccharide to a helper lipid is about 1:0.25, about 1:0.5, or about 1:1. In some embodiments, a molar ratio of a cationic oligosaccharide to a helper lipid is about 1:0.5. In some embodiments, a complex described herein comprises a cationic oligosaccharide, a surfactant, a sterol, and a helper lipid, and a molar ratio of a cationic oligosaccharide to a sterol to a helper lipid (i.e., a cationic oligosaccharide : a sterol : a helper lipid) is from about 1:0.25:0.25 to about 1:1:1. In some embodiments, a molar ratio of a cationic oligosaccharide to a sterol to a helper lipid is from about 1:0.5:0.5 to about 1:1:1. In some embodiments, a molar ratio of a cationic oligosaccharide to a sterol to a helper lipid is about 1:0.25:025, about 1:0.5:0.25, about 1:0.75:0.25, about 1:1:0.25, about 1:0.25:0.5, about 1:0.25:0.75, about 1:0.25:1, about 1:0.5:0.5, about 1:0.75:0.5, about 1:1:0.5, about 1:0.5:0.75, about 1:0.5:1, about 1:0.75:0.75, about 1:1:0.75, about 1:0.75:1, or about 1:1:1. In some embodiments, a molar ratio of a cationic oligosaccharide to a sterol to a helper lipid is about 1:0.5:0.5.

In some embodiments, a molar ratio of a cationic oligosaccharide to a surfactant to a sterol to a helper lipid is about 1:0.25:0.25:0.25to about 1:1:1:1. In some embodiments, a molar ratio of a cationic oligosaccharide to a surfactant to a sterol to a helper lipid is about:

| Cationic Oligosaccharide Molar Ratio | Surfactant Molar Ratio | Sterol Molar Ratio | Helper Lipid Molar Ratio |
|---|---|---|---|
| 1 | 0.25 | 0.25 | 0.25 |
| 1 | 0.5 | 0.25 | 0.25 |
| 1 | 0.75 | 0.25 | 0.25 |
| 1 | 1 | 0.25 | 0.25 |
| 1 | 0.25 | 0.5 | 0.25 |
| 1 | 0.25 | 0.75 | 0.25 |
| 1 | 0.25 | 1 | 0.25 |
| 1 | 0.25 | 0.25 | 0.5 |
| 1 | 0.25 | 0.25 | 0.75 |
| 1 | 0.25 | 0.25 | 1 |
| 1 | 0.5 | 0.5 | 0.25 |
| 1 | 0.75 | 0.5 | 0.25 |
| 1 | 1 | 0.5 | 0.25 |
| 1 | 0.5 | 0.25 | 0.5 |
| 1 | 0.75 | 0.25 | 0.5 |
| 1 | 1 | 0.25 | 0.5 |
| 1 | 0.5 | 0.5 | 0.5 |
| 1 | 0.75 | 0.5 | 0.5 |
| 1 | 1 | 0.5 | 0.5 |
| 1 | 0.5 | 0.75 | 0.25 |
| 1 | 0.5 | 1 | 0.25 |
| 1 | 0.5 | 0.5 | 0.75 |
| 1 | 0.5 | 0.5 | 1 |
| 1 | 0.75 | 0.75 | 0.25 |
| 1 | 1 | 0.75 | 0.25 |
| 1 | 0.75 | 1 | 0.25 |
| 1 | 1 | 1 | 0.25 |
| 1 | 0.75 | 0.25 | 0.75 |
| 1 | 1 | 0.25 | 1 |
| 1 | 1 | 0.5 | 1 |
| 1 | 1 | 0.75 | 1 |
| 1 | 1 | 1 | 1 |

In some embodiments, a molar ratio of a cationic oligosaccharide to a surfactant to a sterol to a helper lipid is about 1:0.5:0.5:0.5. Immunomodulators

As described herein, a complex of the present disclosure comprises an immunomodulator. In some embodiments, a complex comprising an immunomodulator delivers a cargo to a target (e.g., a tissue or a cell) in a patient without invoking an inflammatory response, as seen in other delivery systems, such as in lipid nanoparticles. The present disclosure encompasses an insight that, among other things, a complex comprising an immunomodulator inhibits an immune response that is commonly seen in delivery of, for example, nucleic acid therapy. Such immune response is often associated with adverse effects such as pain, fever, swelling, and the like, and can inhibit or otherwise damped the therapeutics effect of nucleic acid therapy. The benefits of a complex comprising an immunomodulator is described in Examples 8, 9, and 10.

In some embodiments, an immunomodulator is an immunosuppressant. In some embodiments, an immunomodulator is an immunostimulant. In some embodiments, an immunomodulator is an agent (e.g., a small molecule) that is an agonist or antagonist of a toll-like receptor (e.g., TLR1, TLR2, TLR3, TLR4, TLR5, TLR6, TLR7, TLR8, TLR9, TLR10) or a pattern recognition receptor (PRR). In some embodiments, an immunomodulator is an sp²-iminosugar glycolipid. In some embodiments, an immunomodulator is selected from:

| **Compound Number** | **Structure** |
|---|---|
| IHG47 | 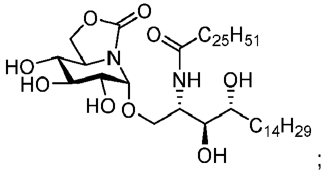 |
| MGC-VI-139 | 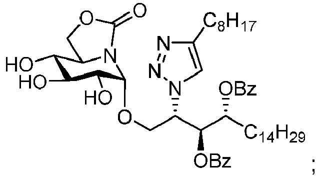 |
| IHG49 | 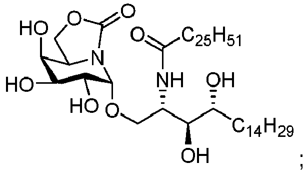 |
| MGC-VI-150 | 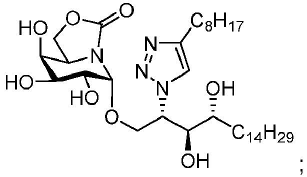 |
| MGC-VII-133 | 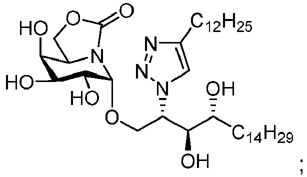 |
| MGC103 | 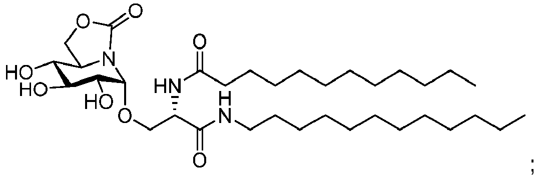 |
| MGC-VI-134 | 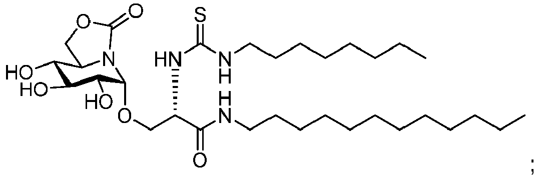 |
| MGC-VII-108 | 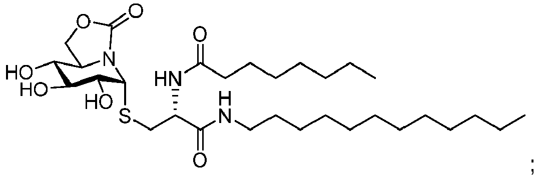 |
| MGC-VI-140 | 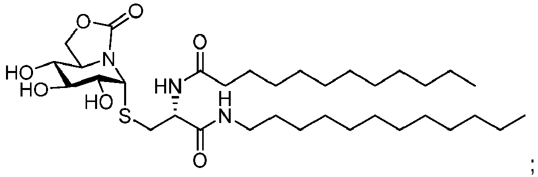 |
| MGC-VII-072 | 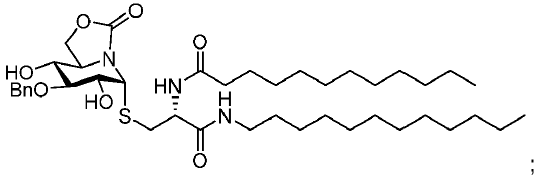 |
| MGC-VII-118 | 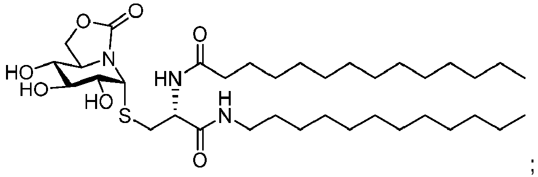 |
| MGC-VII-109 | 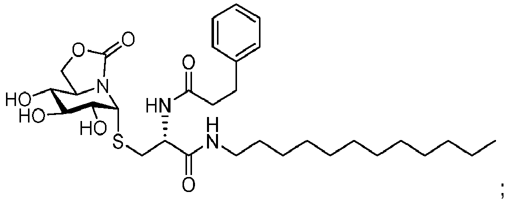 |
| MGC-VII-107 | 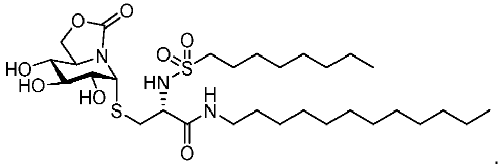 |
| MGC106 | 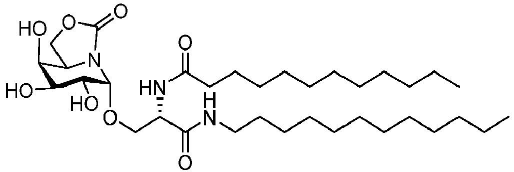 |
| MGC-VI-151 | 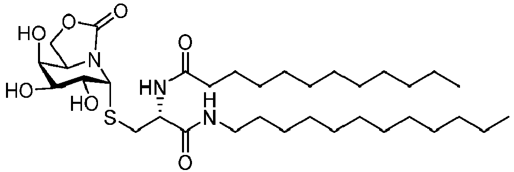 |
| MGCVII-098 | 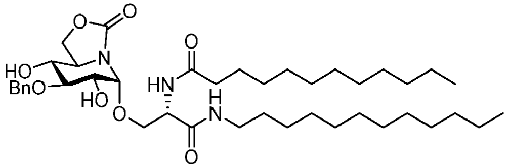 |
| MGC21 | 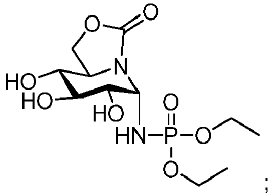 |
| MGC15 | 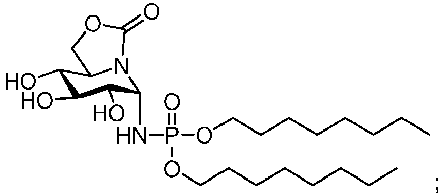 |
| MGC20 | 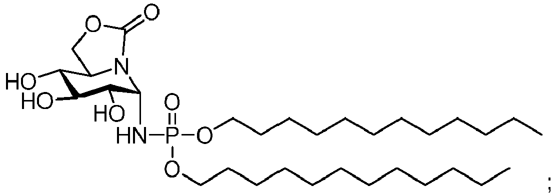 |
| MGC38 | 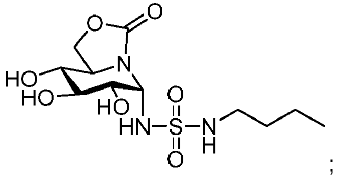 |
| MGC30 | 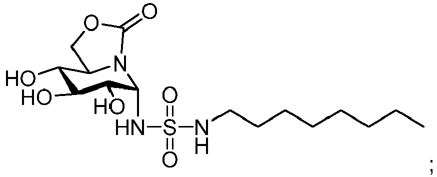 |
| MGC37 | 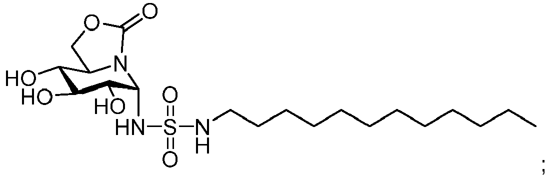 |
| MGC39 | 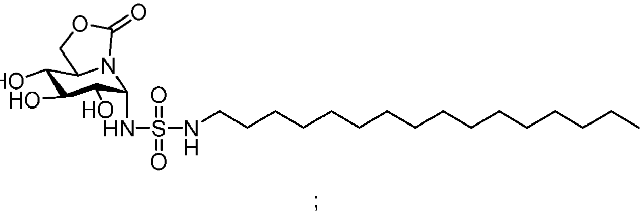 |
| MGC63 | 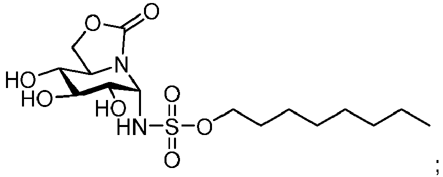 |
| MGC62 | 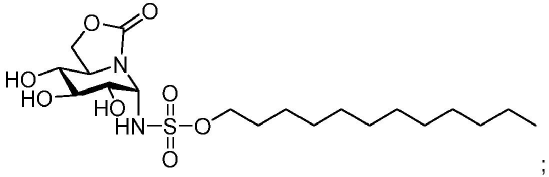 |
| MGC70 | 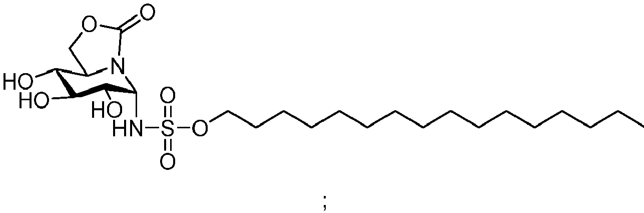 |
| MGC71 | 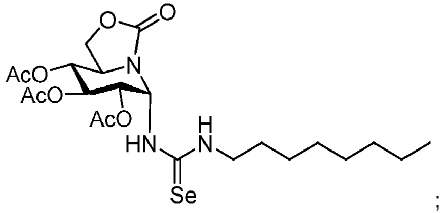 |
| ESF161 | 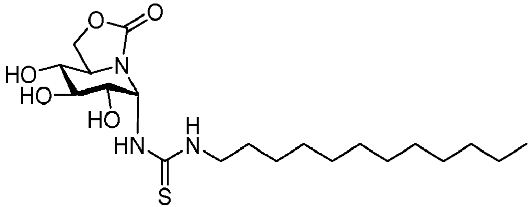 |
| ESF187 | 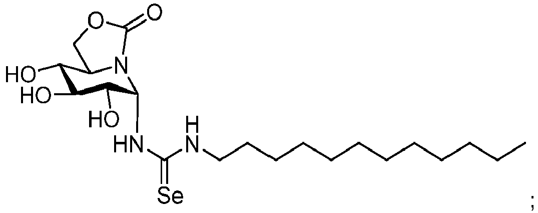 |
| ESF424 | 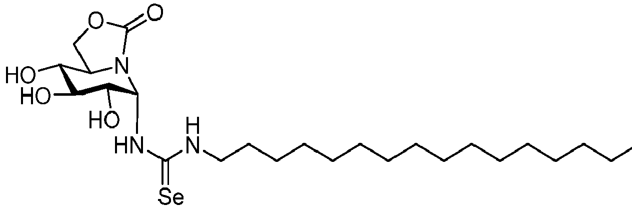 |
| MGC159 | 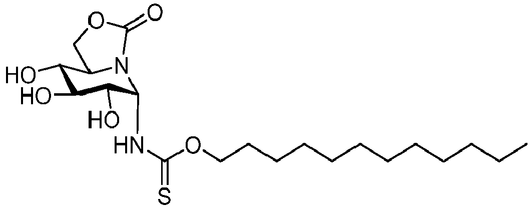 |
| E735 | 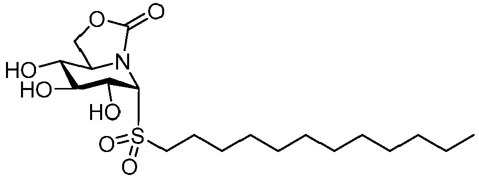 |
| MGC-103 | 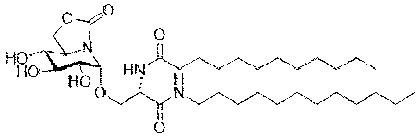 |

or a pharmaceutically acceptable salt thereof.

In some embodiments, an immunomodulator is a small molecule downstream inhibitor of NF-κβ. In some embodiments, an immunomodulator is an sp²-iminosugar glycolipid. In some embodiments, an immunomodulator is a TLR inhibitor. In some embodiments, a TLR inhibitor is an inhibitor of TLR2, TLR4, and/or TLR6. In some embodiments, a TLR inhibitor is an inhibitor of TLR2. In some embodiments, an immunomodulator is an inhibitor of TLR4. In some embodiments, an immunomodulator is an inhibitor of TLR6. In some embodiments, an inhibitor of TLR4 is TAK-242: 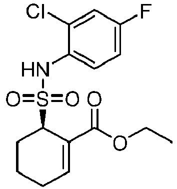 or a pharmaceutically acceptable salt thereof.

In some embodiments, an immunomodulator is a terpenoid. In some embodiments, a terpenoid is a triterpene. In some embodiments, a terpenoid is a triterpenoid. In some embodiments, a triterpenoid is a synthetic or natural derivative of amyrin, betulinic acid, oleanolic acid, sterols , squalene or ursolic acid.

In some embodiments, an immunomodulator is sterol, squalene, oleanolic acid, ursolic acid, betulinic acid, or amyrin.

In some embodiments, an immunomodulator is a corticosteroid. In some embodiments, a corticosteroid is a glucocorticoid. In some embodiments, a glucocorticoid is selected from: dexamethasone, prednisolone, fluticasone propionate, budesonide or a pharmaceutically acceptable salt thereof. In some embodiments, a glucocorticoid is dexamethasone, or a pharmaceutically acceptable salt thereof. In some embodiments, a glucocorticoid is prednisolone, or a pharmaceutically acceptable salt thereof. In some embodiments, a glucocorticoid is fluticasone, or a pharmaceutically acceptable salt thereof. In some embodiments, a glucocorticoid is propionate, or a pharmaceutically acceptable salt thereof. In some embodiments, a glucocorticoid is budesonide, or a pharmaceutically acceptable salt thereof.

In some embodiments, a complex described herein comprises more than one immunomodulator. In some embodiments, a complex comprises an immunomodulator and one or more additional immunomodulators. In some embodiments, a complex comprises a terpenoid (e.g., a corticosteroid, or a glucocorticoid such as dexamethasone, prednisolone, fluticasone propionate, or budesonide), and one or more additional immumomodulators (e.g., another of a corticosteroid, or a glucocorticoid such as dexamethasone, prednisolone, fluticasone propionate, or budesonide, or a TLR or PRR agonist or antagonist, as described herein). In some embodiments, a complex comprises an immunomodulator that is a terpenoid (including any subclasses described herein) and a small molecular agonist or antagonist of TLR or PRR (including any subclasses described herein, such as, e.g. ,TAK-242).

In some embodiments, a molar ratio of a cationic oligosaccharide to an immunomodulator is from about 1:0.05 to about 1:1. In some embodiments, a molar ratio of a cationic oligosaccharide to an immunomodulator is about 1 :0.05 to about 1:0.5. In some embodiments, a molar ratio of a cationic oligosaccharide to an immunomodulator is about 1:0.05, about 1:0.1, about 1:0.2, about 1:0.3, about 1:0.4, about 1:0.5, about 1:0.6, about 1:0.7, about 1:0.8, about 1:0.9, about 1:1.

### Targeting Molecule

In some embodiments, a targeting molecule is an agent having a strong binding affinity to certain cellular markers on a target cell, thereby enhancing targeting and internalization of the complex and/or the cargo into the target cell. In some embodiments, a targeting molecule is comprises one or more of a one or more of a small molecule, a peptide, an aptamer or other nucleic acid sequence, or a nanobody. In some embodiments, a small molecule targeting molecule is folic acid, phenylboronic acid, alendronate, telmisartan, glycyrrhetinic acid, adenosine, a vitamin, or a carbohydrate. In some embodiments, a vitamin is vitamin H (biotin) or vitamin B (flavin) mononucleotide. In some embodiments, a carbohydrate is a monosaccharide selected from glucose, mannose, galactose, fucose, sialic acid, mannose-6-phosphate, and lactobionic acid, or an oligosaccharide comprising one or more monosaccharides described herein. In some embodiments, a peptide is cysteine-arginine-glutamic acid-lysine-alanine (CREKA) peptide, K237 peptide, F3 peptide, A54 peptide, apamin, hexapeptide ligand AE, epidermal growth factor, octreotide, RGD (Arg-Gly-Asp) motif, iRGD, RGDF peptides, cRGD, U11 peptide, HIV trans-activating transcriptional activator (TAT) peptide, Lyp-1 peptide, Rabies virus glycoprotein (RVG), chlorotoxin (CITx), Aβ-binding peptides (KLVFF), TGNYKALHPHNG (TGN), QSHYRHISPAQV(QSH), Tet-1 peptide, T7 peptide, apolipoprotein, E Peptide(ApoE), Peptide motif B6, angiopep-2, cell-penetrating peptide (CPP), ferritin. In some embodiments, an aptamer is nucleotide-targeted ADN aptamer AS1411, anti-epidermal growth factor receptor aptamer, mucins-1 aptamer, prostate-specific membrane antigen (PSMA) aptamer A9g or PSMA A10. In some embodiments, the nanobody is anti-HER2 nanobody 2Rb17c or EGa1 nanobodies. In some embodiments, the targeting molecule is a multivalent dendron moiety comprising several identical or different targeting motifs.N/P Ratios and Complex Size

The presently reported complexes exhibit improved ratios of cationic moieties, or groups that are ionizable to cationic groups (on an oligosaccharide) to anionic moieties (on a nucleic acid). For example, as described herein, a complex comprises a ratio of cationic groups to anionic groups, which is referred to as an "N/P ratio." As described herein, an N/P ratio refers to a molar ratio of cationic groups (or ionizable groups that can become cationic, referred to as "N" of N/P) in a composition comprising cationic oligosaccharide compounds relative to anionic groups in a composition comprising mRNA (referred to as "P" of N/P). The presently reported compositions exhibit favorable ratios, thereby providing concentrated complexes for RNA delivery, relative to previously reported complexes with DNA. For example, the complexes of Carbajo-Gordillo requires an N/P of about 20:1 for effective targeting and delivery of pDNA. The present complexes, in contrast, comprise an N/P ratio of less than 20:1. An N/P ratio can also be expressed as a single digit, e.g., 5, where it is understood to refer to the indicated number as a ratio of X:1. For example, an N/P of 5, is intended to refer to an N/P of 5:1. Similarly, an N/P of 10, is intended to refer to an N/P of 10:1, etc.

For example, in some embodiments, an N/P ratio of a complex reported herein is less than 20:1. In some embodiments, an N/P ratio of a complex reported herein is less than or equal to 15:1. In some embodiments, an N/P ratio of a complex reported herein is less than or equal to 12:1. In some embodiments, an N/P ratio of a complex reported herein is less than or equal to 10:1. In some embodiments, an N/P ratio of a complex reported herein is less than or equal to 5:1. In some embodiments, an N/P ratio of a complex reported herein is less than or equal to 2:1. In some embodiments, an N/P ratio of a complex reported herein is less than or equal to 1:1. In some embodiments, an N/P ratio of a complex reported herein is from about 1:1 to about 15:1. In some embodiments, an N/P ratio of a complex reported herein is from about 1:1 to about 12:1. In some embodiments, an N/P ratio of a complex reported herein is from about 1:1 to about 10:1. In some embodiments, an N/P ratio of a complex reported herein is about 1:1, about 2:1, about 3:1, about 4:1, about 5:1, about 6:1, about 7:1, about 8:1, about 9:1, about 10:1, about 11:1, about 12:1, about 13:1, about 14:1, about 15:1, about 16:1, about 17:1, about 18:1, or about 19:1.

In some embodiments, a complex described herein has a diameter of about 30 nm to about 300 nm. In some embodiments a complex described herein has a diameter of about 50 nm to about 200 nm. In some embodiments, a complex described herein has a diameter that is about 50, 60, 70, 80, 90, 100, 110, 120, 130, 140, 150, 160, 170, 180, 190, or 200 nm. In some embodiments, a complex described herein has a diameter of about 30 nm to about 150 nm. In some embodiments, a complex described herein has a diameter of about 30 nm to about 100 nm. In some embodiments, a complex described herein has a diameter of less than 100 nm. In some embodiments, a complex described herein has a diameter of about 30, 35, 40, 45, 50, 55, 60, 65, 70, 75, 80, 85, 90, 95, or 100 nm.

In some embodiments, a complex comprising an oligosaccharide and a pharmaceutically acceptable surfactant has a diameter of about 30 nm to about 150 nm. In some embodiments, a complex described herein has a diameter of about 30 nm to about 100 nm. In some embodiments, a complex comprising an oligosaccharide and a pharmaceutically acceptable surfactant has a diameter of less than 100 nm. In some embodiments, a complex comprising an oligosaccharide and a pharmaceutically acceptable surfactant has a diameter of about 30, 35, 40, 45, 50, 55, 60, 65, 70, 75, 80, 85, 90, 95, or 100 nm.

### Cargo

In some embodiments, a complex described herein further comprises a cargo. In some embodiments, a cargo is a therapeutic agent, e.g., a small molecule, a peptide, a nucleic acid, or a combination thereof. In some embodiments, a cargo is a small molecule. In some embodiments, a cargo is a peptide. In some embodiments, a cargo is a nucleic acid.

### Nucleic Acids

In some embodiments, a complex described herein comprises a nucleic acid. In some embodiments, a nucleic acid is RNA.

In some embodiments, an RNA amenable to technologies described herein is a single-stranded RNA. In some embodiments, an RNA as disclosed herein is a linear RNA. In some embodiments, a single-stranded RNA is a non-coding RNA in that its nucleotide sequence does not include an open reading frame (or complement thereof). In some embodiments, a single-stranded RNA has a nucleotide sequence that encodes (or is the complement of a sequence that encodes) a polypeptide or a plurality of polypeptides (e.g., epitopes) of the present disclosure.

In some embodiments, an RNA is or comprises an siRNA, an miRNA, or other non-coding RNA.

In many embodiments, a relevant RNA includes at least one open reading frame (ORF) (*e.g.,* is an mRNA); in some embodiments, a relevant RNA includes a single ORF; in some embodiments, a relevant RNA includes more than one ORF.

In some embodiments, an RNA comprises an ORF, e.g., encoding a polypeptide of interest or encoding a plurality of polypeptides of interest. In some embodiments, an RNA produced in accordance with technologies provided herein comprises a plurality of ORFs (e.g., encoding a plurality of polypeptides). In some embodiments, an RNA produced in accordance with technologies herein comprises a single ORF that encodes a plurality of polypeptides. In some such embodiments, polypeptides are or comprise antigens or epitopes thereof (*e.g*., relevant antigens).

In some embodiments, an ORF for use in accordance with the present disclosure encodes a polypeptide that includes a signal sequence, *e.g.,* that is functional in mammalian cells, such as an intrinsic signal sequence or a heterologous signal sequence. In some embodiments, a signal sequence directs secretion of an encoded polypeptide, in some embodiments, a signal sequence directs transport of an encoded polypeptide into a defined cellular compartment, preferably the cell surface, the endoplasmic reticulum (ER) or the endosomal-lysosomal compartment.

In some embodiments, an ORF encodes a polypeptide that includes a multimerization element (*e.g.,* an intrinsic or heterologous multimerization element). In some embodiments, an ORF that encodes a surface polypeptide (*e.g.,* that includes a signal sequence directing surface localization) includes a multimerization element.

In some embodiments, an ORF encodes a polypeptide that includes a transmembrane element or domain.

In some embodiments, an ORF is codon-optimized for expression in a cells of a particular host, *e.g.,* a mammalian host, *e.g.,* a human.

In some embodiments, an RNA includes unmodified uridine residues; an RNA that includes only unmodified uridine residues may be referred to as a "uRNA". In some embodiments, an RNA includes one or more modified uridine residues; in some embodiments, such an RNA (*e.g.,* an RNA including entirely modified uridine residues) is referred to as a "modRNA". In some embodiments, an RNA may be a self-amplifying RNA (saRNA). In some embodiments, an RNA may be a trans-amplifying RNA (taRNA) (*see,* for example, WO2017/162461).

In some embodiments, a relevant RNA includes a polypeptide-encoding portion or a plurality of polypeptide-encoding portions. In some particular embodiments, such a portion or portions may encode a polypeptide or polypeptides that is or comprises a biologically active polypeptide or portion thereof (*e.g.,* an enzyme or cytokine or therapeutic protein such as a replacement protein or antibody or portion thereof). In some particular embodiments, such a portion or portions may encode a polypeptide or polypeptides that is or comprises an antigen (or an epitope thereof), a cytokine, an enzyme, etc. In some embodiments, an encoded polypeptide or polypeptides may be or include one or more neoantigens or neoepitopes associated with a tumor. In some embodiments, an encoded polypeptide or polypeptides may be or include one or more antigens (or epitopes thereof) of an infectious agent (*e.g.,* a bacterium, fungus, virus, etc.). In certain embodiments, an encoded polypeptide may be a variant of a wild type polypeptide.

In some embodiments, a single-stranded RNA (*e.g.,* mRNA) may comprise a secretion signal-encoding region (*e.g.,* a secretion signal-encoding region that allows an encoded target entity or entities to be secreted upon translation by cells). In some embodiments, such a secretion signal-encoding region may be or comprise a non-human secretion signal. In some embodiments, such a secretion signal-encoding region may be or comprise a human secretion signal.

In some embodiments, a single-stranded RNA (*e.g.,* mRNA) may comprise at least one non-coding element (*e.g.,* to enhance RNA stability and/or translation efficiency). Examples of non-coding elements include but are not limited to a 3' untranslated region (UTR), a 5' UTR, a cap structure (*e.g.,* in some embodiments, an enzymatically-added cap; in some embodiments, a co-transcriptional cap), a poly adenine (polyA) tail (*e.g.,* that, in some embodiments, may be or comprise 100 A residues or more, and/or in some embodiments may include one or more "interrupting" [*i.e.,* non-A] sequence elements), and any combinations thereof. Exemplary embodiments of such non-coding elements may be found, for example, in WO2011015347, WO2017053297, US 10519189, US 10494399, WO2007024708, WO2007036366, WO2017060314, WO2016005324, WO2005038030, WO2017036889, WO2017162266, and WO2017162461, each of which is incorporated herein by referenced in its entirety.

At least four formats useful for RNA pharmaceutical compositions (e.g., immunogenic compositions or vaccines) have been developed, namely non-modified uridine containing mRNA (uRNA), nucleosidemodified mRNA (modRNA), self-amplifying mRNA (saRNA), and trans-amplifying RNAs.

Features of a non-modified uridine platform may include, for example, one or more of intrinsic adjuvant effect, good tolerability and safety, and strong antibody and T cell responses.

Features of modified uridine (e.g., pseudouridine) platform may include reduced adjuvant effect, blunted immune innate immune sensor activating capacity and thus augmented antigen expression, good tolerability and safety, and strong antibody and CD4-T cell responses. As noted herein, the present disclosure provides an insight that such strong antibody and CD4 T cell responses may be particularly useful for vaccination. Features of self-amplifying platform may include, for example, long duration of polypeptide (*e.g.,* protein) expression, good tolerability and safety, higher likelihood for efficacy with very low vaccine dose.

In some embodiments, a self-amplifying platform (*e.g.,* RNA) comprises two nucleic acid molecules, wherein one nucleic acid molecule encodes a replicase (*e.g.,* a viral replicase) and the other nucleic acid molecule is capable of being replicated (*e.g.,* a replicon) by said replicase *in trans* (*trans*-replication system). In some embodiments, a self-amplifying platform (*e.g.,* RNA) comprises a plurality of nucleic acid molecules, wherein said nucleic acids encode a plurality of replicases and/or replicons.

In some embodiments, a *trans*-replication system comprises the presence of both nucleic acid molecules in a single host cell.

In some such embodiments, a nucleic acid encoding a replicase (*e.g.,* a viral replicase) is not capable of self-replication in a target cell and/or target organism. In some such embodiments, a nucleic acid encoding a replicase (*e.g.,* a viral replicase) lacks at least one conserved sequence element important for (-) strand synthesis based on a (+) strand template and/or for (+) strand synthesis based on a (-) strand template.

In some embodiments, a self-amplifying RNA comprises a 5'-cap; in some *trans-*replication systems, at least an RNA encoding a replicase is capped. Without wishing to be bound by any one theory, it has been found that a 5'-cap can be important for high level expression of a gene of interest *in trans.*

In some embodiments, a self-amplifying platform does not require propagation of virus particles (*e.g.,* is not associated with undesired virus-particle formation). In some embodiments, a self-amplifying platform is not capable of forming virus particles.

In some embodiments, an RNA may comprise an Internal Ribosomal Entry Site (IRES) element. In some embodiments, an RNA does not comprise an IRES site; in particular, in some embodiments, an saRNA does not comprise an IRES site. In some such embodiments, translation of a gene of interest and/or replicase is not driven by an IRES element. In some embodiments, an IRES element is substituted by a 5'-cap. In some such embodiments, substitution by a 5'-cap does not affect the sequence of a polypeptide encoded by an RNA.

In some embodiments, a complex described herein comprises modRNA, saRNA, taRNA, or uRNA. In some embodiments, a complex comprises modRNA. In some embodiments, a complex comprises saRNA. In some embodiments, a complex comprises taRNA. In some embodiments, a complex comprises uRNA.

### Methods of Use

Complexes described herein are useful in the treatment and prophylaxis in a subject of diseases, disorders, and conditions described herein. In some embodiments, the present disclosure provides a method of treating a disease, disorder, or condition in a subject comprising administering to the subject a complex described herein. In some embodiments, the present disclosure provides use of a complex described herein for the treatment of a disease, disorder, or condition. In some embodiments, a disease, disorder, or condition is an infectious disease, cancer, an autoimmune disease, or a rare disease.

In some embodiments, the present disclosure provides a complex as described herein for use as a medicament.

In some embodiments, the present disclosure provides a complex as described herein, for use in the treatment and/or prevention of a disease or disorder, wherein the disease or disorder is an infectious disease, cancer, a genetic disorder, an autoimmune disease, or a rare disease.

In some embodiments, an infectious disease is caused by or associated with a viral pathogen. In some embodiments, a viral pathogen is of a family selected from poxviridae, rhabdoviridae, filoviridae, paramyxoviridae, hepadnaviridae, coronaviridae, caliciviridae, picornaviridae, reoviridae, retroviridae, and orthomyxoviridae. In some embodiments, an infectious disease is a virus selected from SARS-CoV-2, influenza, Crimean-Congo Hemorhhagic Fever (CCHF), Ebola virus, Lassa virus, Marburg virus, HIV, Nipah virus, and MERS-CoV.

In some embodiments, an infectious disease is caused by or associated with a bacterial pathogen. In some embodiments, a bacterial pathogen is of a species selected from Actinomyces israelii, bacillus antracis, Bacteroides fragilis, Bordetella pertussis, Borrelia burgdorferi, Borrelia garinii, Borrelia afzelii, Borrelia recurrentis, Brucella abortus, Brucella canis, Brucella melitensis, Brucella suis, Campolobacter jejuni, Chlamydia pneumoniae, Chlamydia trachomatis, Chlamydophila psittaci, Clostridium botulinum, Clostridium difficile, Clostridium perfringens, Clostridium tetani, Corynebacterium idphteriae, Ehrlichia canis, Ehrlichia chaffeensis, Enterococcus faecalis, Enterococcus faecium, Escherichia coli, Francisella tularensis, Haemophilus influenzae, Helicobacter pylori, Klebsiella pneumoniae, Legionella pneumophila, Leptospira, Listeria monocytogenes, Mycobacterium leprae, Mycobacterium tuberculosis, Mycoplasma pneumoniae, Neisseria gonorrhoeae, Neisseria meningitidis, Pseudomonas aeruginosa, Nocardia asteroids, Rickettsia ricektssii, Salmonella typhi, Salmonella typhimurium, Shigella sonnei, Shigella dysenteriae, Staphylococcus aureus, Staphylococcus epidermidis, Staphylococcus saprophyticus, Streptococcus agalactiae, Streptococcus pneumoniae, Streptococcus pyogenes, Streptococcus viridans, Treponema pallidum, Vibrio cholerae, and Yersinia pestis.

In some embodiments, an infectious disease is caused by or associated with a parasite. In some embodiments, a parasite is of a family selected from Plasmodium, Leishmania, Cryptosporidium, Entamoeba, Trypanosomas, Schistosomes, Ascaris, Echinococcus and Taeniidae.

In some embodiments, a disease, disorder, or condition is a cancer. In some embodiments, a cancer is selected from bladder cancer, breast cancer, colorectal cancer, kidney cancer, lung cancer, lymphoma, melanoma, oral/oropharyngeal cancer, pancreatic cancer, prostate cancer, thyroid cancer, and uterine cancer.

In some embodiments, a disease, disorder, or condition is a genetic disorder. In some embodiments, a genetic disorder is associated with a gain-of-function mutation or a loss-of-function mutation.

In some embodiments, a disease, disorder, or condition is an autoimmune disease. In some embodiments, an autoimmune disease is selected from addison disease, celiac disease, rheumatoid arthritis, lupus, inflammatory bowel disease, dermatomyositis, multiple sclerosis, diabetes, guillain-barre syndrome, chronic inflammatory demyelinating polyneuropathy, psoriasis, pernicious anemia, Graves' disease, Hashimoto's thyroiditis, myasthenia gravis, and vasculitis sjörgen syndrome.

In some embodiments, a disease, disorder, or condition is a rare disease. As described herein, a rare disease refers to a life-threatening or chronically debilitating diseases which are of such low prevalence (e.g., fewer than 1/2000 people) that special combined efforts are needed to address them.

In some embodiments, the present disclosure provides complexes that can selectively target particular systems within a body. As used herein, reference to "targeting" a particular system refers to causing increased expression of RNA derived from cargo in the complex in the desired system. For example, in some embodiments, complexes described herein can selectively target the lungs, liver, spleen, heart, brain, lymph nodes, bladder, kidneys, and pancreas. As described herein, a complex "selectively targets" an organ when a single target expresses mRNA in an amount that is 65% or greater than expression in other organs post administration (e.g., 65% or more of mRNA throughout the body is expressed from a single organ, with the remaining 35% distributed between one or more different organs). In some embodiments, a complex described herein selectively targets the lungs. In some embodiments, a complex described herein selectively targets the liver. In some embodiments, a complex described herein selectively targets the spleen. In some embodiments, a complex described herein selectively targets the heart.

In some embodiments, the present disclosure provides a method of increasing or causing increased expression of RNA in a target in a subject comprising administering to the subject a complex described herein. In some embodiments, a target is selected from the lungs, liver, spleen, heart, brain, lymph nodes, bladder, kidneys, and pancreas.

### Methods of Delivery

The present disclosure provides, among other things, a complex (e.g., a pharmaceutical composition or a pharmaceutical formulation, as referred to herein) to be administered to a subject. For example, in some embodiments, a complex is administered as a monotherapy. In some embodiments, a complex is administered as part of a combination therapy. In some embodiments, a concentration of total RNA (e.g., a total concentration of all of the one or more RNA molecules) in a pharmaceutical composition described herein is of about 0.01 mg/mL to about 0.5 mg/mL, or about 0.05 mg/mL to about 0.1 mg/mL.

Pharmaceutical compositions may additionally comprise a pharmaceutically acceptable excipient, which, as used herein, includes any and all solvents, dispersion media, diluents, or other liquid vehicles, dispersion or suspension aids, surface active agents, isotonic agents, thickening or emulsifying agents, preservatives, solid binders, lubricants and the like, as suited to the particular dosage form desired. Remington's The Science and Practice of Pharmacy, 21st Edition, A. R. Gennaro (Lippincott, Williams & Wilkins, Baltimore, MD, 2006; incorporated herein by reference in its entirety) discloses various excipients used in formulating pharmaceutical compositions and known techniques for the preparation thereof. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

In some embodiments, an excipient is approved for use in humans and for veterinary use. In some embodiments, an excipient is approved by the United States Food and Drug Administration. In some embodiments, an excipient is pharmaceutical grade. In some embodiments, an excipient meets the standards of the United States Pharmacopoeia (USP), the European Pharmacopoeia (EP), the British Pharmacopoeia, and/or the International Pharmacopoeia.

Pharmaceutically acceptable excipients used in the manufacture of pharmaceutical compositions include, but are not limited to, inert diluents, dispersing and/or granulating agents, surface active agents and/or emulsifiers, disintegrating agents, binding agents, preservatives, buffering agents, lubricating agents, and/or oils. Such excipients may optionally be included in pharmaceutical formulations. Excipients such as cocoa butter and suppository waxes, coloring agents, coating agents, sweetening, flavoring, and/or perfuming agents can be present in the composition, according to the judgment of the formulator.

General considerations in the formulation and/or manufacture of pharmaceutical agents may be found, for example, in Remington: The Science and Practice of Pharmacy 21st ed., Lippincott Williams & Wilkins, 2005 (incorporated herein by reference in its entirety). In some embodiments, pharmaceutical compositions provided herein may be formulated with one or more pharmaceutically acceptable carriers or diluents as well as any other known adjuvants and excipients in accordance with conventional techniques such as those disclosed in Remington: The Science and Practice of Pharmacy 21st ed., Lippincott Williams & Wilkins, 2005 (incorporated herein by reference in its entirety). Pharmaceutical complexes and compositions described herein can be administered by appropriate methods known in the art. As will be appreciated by a skilled artisan, the route and/or mode of administration may depend on a number of factors, including, e.g., but not limited to stability and/or pharmacokinetics and/or pharmacodynamics of pharmaceutical compositions described herein.

In some embodiments, pharmaceutical compositions described herein are formulated for parenteral administration, which includes modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. In some embodiments, pharmaceutical compositions described herein are formulated for intravenous administration. In some embodiments, pharmaceutically acceptable carriers that may be useful for intravenous administration include sterile aqueous solutions or dispersions and sterile powders for preparation of sterile injectable solutions or dispersions.

In some particular embodiments, pharmaceutical compositions described herein are formulated for subcutaneous (s.c) administration. In some particular embodiments, pharmaceutical compositions described herein are formulated for intramuscular (i.m) administration.

Therapeutic compositions typically must be sterile and stable under the conditions of manufacture and storage. The composition can be formulated as a solution, dispersion, powder (e.g., lyophilized powder), microemulsion, lipid nanoparticles, or other ordered structure suitable to high drug concentration. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the required particle size in the case of dispersion and by the use of surfactants. In many cases, it will be preferable to include isotonic agents, for example, sugars, polyalcohols such as mannitol, sorbitol, or sodium chloride in the composition. In some embodiments, prolonged absorption of the injectable compositions can be brought about by including in the composition an agent that delays absorption, for example, monostearate salts and gelatin.

Sterile injectable solutions can be prepared by incorporating the active compound in the required amount in an appropriate solvent with one or a combination of ingredients enumerated above, as required, followed by sterilization microfiltration.

In some embodiments, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying (lyophilization) that yield a powder of the active ingredient plus any additional desired ingredient from a previously sterile-filtered solution thereof.

Examples of suitable aqueous and nonaqueous carriers which may be employed in the pharmaceutical compositions described herein include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the presence of microorganisms may be ensured both by sterilization procedures, and by the inclusion of various antibacterial and antifungal agents, for example, paraben, chlorobutanol, phenol sorbic acid, and the like. It may also be desirable to include isotonic agents, such as sugars, sodium chloride, and the like into pharmaceutical compositions described herein. In addition, prolonged absorption of the injectable pharmaceutical form may be brought about by the inclusion of agents which delay absorption such as aluminum monostearate and gelatin.

Formulations of pharmaceutical compositions described herein may be prepared by any method known or hereafter developed in the art of pharmacology. In general, such preparatory methods include the step of bringing active ingredient(s) into association with a diluent or another excipient and/or one or more other accessory ingredients, and then, if necessary and/or desirable, shaping and/or packaging the product into a desired single- or multi-dose unit.

A pharmaceutical composition in accordance with the present disclosure may be prepared, packaged, and/or sold in bulk, as a single unit dose, and/or as a plurality of single unit doses. As used herein, a "unit dose" is discrete amount of the pharmaceutical composition comprising a predetermined amount of at least one RNA product produced using a system and/or method described herein.

In some embodiments, an active agent that may be included in a pharmaceutical composition described herein is or comprises a therapeutic agent administered in a combination therapy described herein. Pharmaceutical compositions described herein can be administered in combination therapy, i.e., combined with other agents. In some embodiments, such therapeutic agents may include agents leading to depletion or functional inactivation of regulatory T cells. For example, in some embodiments, a combination therapy can include a provided pharmaceutical composition with at least one immune checkpoint inhibitor.

In some embodiments, pharmaceutical composition described herein may be administered in conjunction with radiotherapy and/or autologous peripheral stem cell or bone marrow transplantation.

In some embodiments, a pharmaceutical composition described herein can be frozen to allow long-term storage.

Although the descriptions of pharmaceutical compositions provided herein are principally directed to pharmaceutical compositions that are suitable for administration to humans, it will be understood by the skilled artisan that such compositions are generally suitable for administration to animals of all sorts. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation.

### Exemplary Embodiments

Embodiment 1. A complex comprising:
i) a cationic oligosaccharide comprising one or more cationic moieties bonded to a trehalose, a sucrose, or a gluco-n-oligosaccharide moiety, where n is 2-6;
ii) a surfactant; and
iii) one or more additives selected from: a sterol, a helper lipid, an immunomodulator, and a targeting molecule.

Embodiment 2. The complex of Embodiment 1, wherein the surfactant is or comprises a polysorbate, a poloxamer, and/or a compound comprising an amphiphilic moiety selected from polyalkylene glycols (e.g., polyethylene glycol), poly(2-oxazoline), poly(2-oxazine), polysarcosine, polyvinylpyrrolidone, and poly[N-(2-hydroxypropyl)methacrylamide, wherein the amphiphilic moiety is bonded to one or more C₁₂-C₂₀ aliphatic groups.

Embodiment 3. The complex of Embodiments 1 or 2, wherein the surfactant is or comprises a polysorbate selected from polysorbate 20, polysorbate 40, polysorbate 60, polysorbate 80, and combinations thereof.

Embodiment 4. The complex of any one of Embodiments 1-3, wherein a molar ratio of the cationic oligosaccharide to the surfactant is about 1:0.0075 to about 1:3.

Embodiment 5. The complex of any one of Embodiments 1-4, wherein a molar ratio of the cationic oligosaccharide to the surfactant is about 1:0.5 to about 1:1.

Embodiment 6. The complex of any one of Embodiments 1-5, wherein a molar ratio of the cationic oligosaccharide to the surfactant is about 1:0.5.

Embodiment 7. The complex of any one of Embodiments 1-6, further comprising a cargo.

Embodiment 8. The complex of Embodiment 7, wherein the cargo is a nucleic acid.

Embodiment 9. The complex of Embodiment 8, wherein the nucleic acid is RNA.

Embodiment 10. The complex of Embodiment 9, wherein the RNA is modRNA, saRNA, taRNA, or uRNA.

Embodiment 11. The complex of any one of Embodiments 1-10, wherein the complex has an N/P ratio of less than 20:1.

Embodiment 12. The complex of any one of Embodiments 1-11, wherein the complex has an N/P ratio less than or equal to 12:1.

Embodiment 13. The complex of any one of Embodiments 1-12, wherein the complex has an N/P ratio that is about 6:1.

Embodiment 14. The complex of any one of Embodiments 1-13, wherein the complex has a diameter of about 50 nm to about 150 nm.

Embodiment 15. The complex of any one of Embodiments 1-14, wherein the one or more additives are or comprise a sterol and/or a helper lipid and/or an immunomodulator.

Embodiment 16. The complex of any one of Embodiments 1-15, wherein the one or more additives are or comprise a sterol.

Embodiment 17. The complex of Embodiment 16, wherein the sterol is selected from β-sitosterol, stigmasterol, cholesterol, cholecalciferol, ergocalciferol, calcipotriol, botulin, lupeol, ursolic acid, oleanolic acid, cycloartenol, lanosterol, or α-tocopherol.

Embodiment 18. The complex of any one of Embodiments 1-17, wherein a molar ratio of the cationic oligosaccharide to the sterol is from about 1:0.25 to about 1:1.

Embodiment 19. The complex of Embodiment 18, wherein a ratio of the cationic oligosaccharide to the sterol is about 1:0.5.

Embodiment 20. The complex of any one of Embodiments 1-19, wherein the one or more additives are or comprise a helper lipid.

Embodiment 21. The complex of any one of Embodiments 1- 20, wherein the helper lipid is selected from the group consisting of phosphatidylcholines, phosphatidylethanolamines, phosphatidylglycerols, phosphatidic acids, phosphatidylserines and sphingomyelins, more preferably selected from the group consisting of distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), and combinations thereof.

Embodiment 22. The complex of any one of Embodiments 1-21, wherein the helper lipid is selected from DSPC and DMPC.

Embodiment 23. The complex of any one of Embodiments 1-21, wherein a molar ratio of the cationic oligosaccharide to the helper lipid is from about 1:0.25 to about 1:1.

Embodiment 24. The complex of any one of Embodiments 1-23, wherein a molar ratio of the cationic oligosaccharide to the helper lipid is about 1:0.5.

Embodiment 25. The complex of any one of Embodiments 1-24, wherein the one or more additives are or comprise a sterol and a helper lipid.

Embodiment 26. The complex of any one of Embodiments 1-25, wherein a molar ratio of the cationic oligosaccharide : the sterol: the helper lipid is from about 1 :0.25:0.25 to about 1:1:1.

Embodiment 27. The complex of Embodiment 26, wherein a molar ratio of the cationic oligosaccharide : the sterol: the helper lipid is about 1:0.5:0.5.

Embodiment 28. The complex of any one of Embodiments 1-27, wherein a molar ratio of the cationic oligosaccharide : the surfactant : the sterol : the helper lipid is about 1:0.5:0.5:0.5.

Embodiment 29. The complex of any one of Embodiments 1-28, wherein the one or more additives are or comprise an immunomodulator.

Embodiment 30. The complex of any one of Embodiments 1-29, wherein the immunomodulator is an immunostimulatant or an immunosuppressor.

Embodiment 31. The complex of any one of Embodiments 1-30, wherein the immunomodulator is a small molecule agonist or antagonist of TLR or PRR receptors.

Embodiment 32. The complex of any one of Embodiments 1-31, wherein the immunomodulator is a small molecule downstream inhibitor of NF-κβ.

Embodiment 33. The complex of any one of Embodiments 1-32, wherein the immunomodulator is an sp²-iminosugar glycolipid.

Embodiment 34. The complex of any one of Embodiment 1-33, wherein the immunomodulator is a TLR inhibitor.

Embodiment 35. The complex of Embodiment 34, wherein the TLR inhibitor is an inhibitor of TLR2, TLR4, and/or TLR6.

Embodiment 36. The complex of Embodiment 35, wherein the TLR inhibitor is an inhibitor of TLR4.

Embodiment 37. The complex of Embodiment 13, wherein the inhibitor of TLR4 is TAK-242.

Embodiment 38. The complex of any one of Embodiments 1-30, wherein the immunomodulator is a terpenoid.

Embodiment 39. The complex of Embodiment 38, wherein the terpenoid is a triterpene.

Embodiment 40. The complex of Embodiment 39, wherein the triterpene is a triterpenoid.

Embodiment 41. The complex of Embodiment 39, wherein the triterpenoid is a synthetic or natural derivate of amyrin, betulinic acid, oleanolic acid, sterols , squalene, or ursolic acid.

Embodiment 42. The complex of any one of Embodiments 38-41, wherein the immunomodulator is a sterol, squalene, oleanolic acid, ursolic acid, betulinic acid, or amyrin.

Embodiment 43. The complex of any one of Embodiments 38-42, wherein the immunomodulator is a glucocorticoid.

Embodiment 44. The complex of Embodiment 43, wherein the glucocorticoid is dexamethasone, prednisolone, fluticasone propionate, budesonide or a pharmaceutically acceptable salt thereof.

Embodiment 45. The complex of Embodiment 44, wherein the glucocorticoid is dexamethasone or a pharmaceutically acceptable salt thereof.

Embodiment 46. The complex of any one of Embodiments 38-45, wherein the complex further comprises one or more additional immunomodulators.

Embodiment 47. The complex of Embodiment 46, where the one or more additional immunomodulators are or comprise a small molecule agonist or antagonist of TLR or PRR receptors.

Embodiment 48. The complex of Embodiment 47, wherein the one or more additional immunomodulators are or comprise TAK-242.

Embodiment 49. The complex of any one of Embodiments 1-48, wherein the one or more additives are or comprise a sterol, a helper lipid, and an immunomodulator.

Embodiment 50. The complex of any one of Embodiments 1-49, wherein a molar ratio of the cationic oligosaccharide to the immunomodulator is from about 1:0.05 to about 1:0.5.

Embodiment 51. The complex of Embodiment 50, wherein a molar ratio of the cationic oligosaccharide to the immunomodulator is about 1:0.5.

Embodiment 52. The complex of any one of Embodiments 1-51, wherein the one or more additives are or comprise a targeting molecule.

Embodiment 53. The complex of any one of Embodiments 1-52, wherein the cationic oligosaccharide is a compound of formula I: 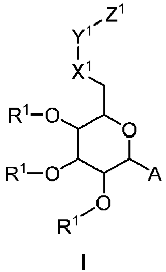 or a pharmaceutically acceptable salt thereof, wherein:
A is A¹, A², or A³: 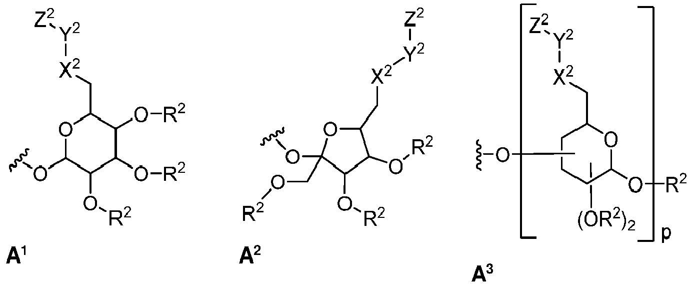
R¹ and R² are each independently selected from H, R^{a}, and -C(O)-R^{a}, wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by -Cy-, -NR^{Y}-, - NR^{Y}C(O)-, -C(O)NR^{Y}-, -NR^{Y}C(O)O-, -OC(O)NR^{Y}-, -NR^{Y}C(O)NR^{Y}-, -NR^{Y}C(S)NR^{Y}-, - C(S)NR^{Y}- -C(O)NR^{Y}SO₂-, -SO₂NR^{Y}C(O)-,-OC(O)O-, -O-, -C(O)-, -OC(O)-, -C(O)O, SO, or SO₂;
each R^{Y} is independently H or optionally substituted C₁-C₆ aliphatic;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 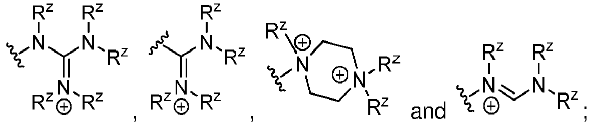
each M is independently -C₀-C₆ aliphatic-R^{z} or -C₀-C₆ aliphatic-N⁺(R^{z})₃;
each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and
p is an integer selected from 1, 2, 3, 4, or 5.

Embodiment 54. The complex of Embodiment 53, wherein the cationic oligosaccharide is of formula la: 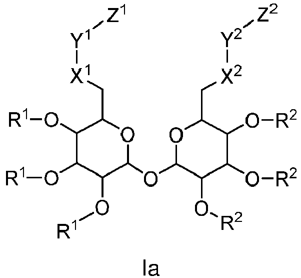 or a pharmaceutically acceptable salt thereof.

Embodiment 55. The complex of Embodiment 53, wherein the oligosaccharide is of formula Ib: 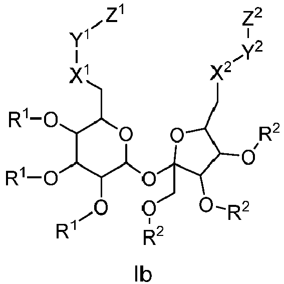 or a pharmaceutically acceptable salt thereof.

Embodiment 56. The complex of Embodiment 53, wherein the oligosaccharide is of formula Ic: 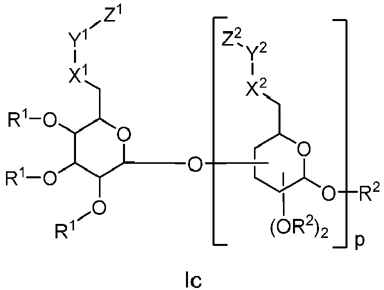 or a pharmaceutically acceptable salt thereof.

Embodiment 57. The complex of any one of Embodiments 53-56, wherein R¹ and R² are each independently selected from R^{a} and -C(O)-R^{a}, and R^{a} is C₁-C₂₀ aliphatic.

Embodiment 58. The complex of any one of Embodiments 53-57, wherein R¹ and R² are each -C(O)-R^{a}, and R^{a} is C₁-C₁₄ aliphatic.

Embodiment 59. The complex of any one of Embodiments 53-58, wherein each R^{a} is C₅-C₁₀ linear alkyl.

Embodiment 60. The complex of any one of Embodiments 53-59, wherein R¹ and R² are each -C(O)-R^{a}, and each R^{a} is independently selected from:

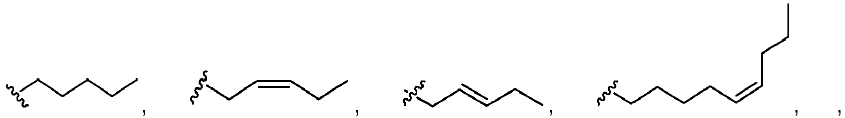

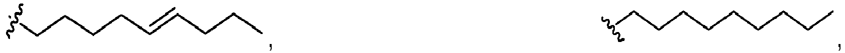

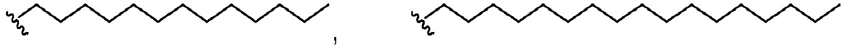

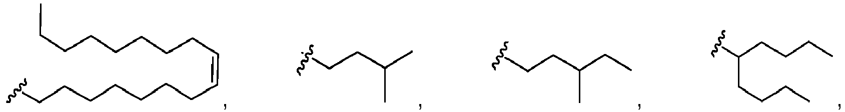

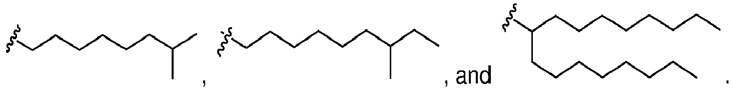

Embodiment 61. The complex of any one of Embodiments 53-60, wherein X¹ and X² are each -S-.

Embodiment 62. The complex of any one of Embodiments 53-61, wherein Y¹ and Y² are each independently an optionally substituted C₁₋₃₀ aliphatic group wherein one or more carbons are independently replaced by -NR^{Y}C(O)NR^{Y}- or -NR^{Y}C(S)NR^{Y}-.

Embodiment 63. The complex of Embodiment 62, wherein R^{Y} is H.

Embodiment 64. The complex of any one of Embodiments 53-63, wherein Y¹ and Y² are each C₁-C₄ aliphatic-NHC(S)NH-C₁-C₄ aliphatic.

Embodiment 65. The complex of Embodiment 64, wherein Y¹ and Y² are each - CH₂-CH₂-NHC(S)NH-CH₂-CH₂-.

Embodiment 66. The complex of any one of Embodiments 53-65, wherein Z¹ and Z² are each independently a cationic or ionizable group comprising an optionally substituted 5- to 14-membered heterocyclyl ring having 1-3 heteroatoms selected from N, O, and S.

Embodiment 67. The complex of any one of Embodiments 53-66, wherein Z¹ and Z² are each independently selected from: 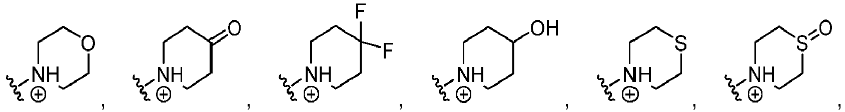 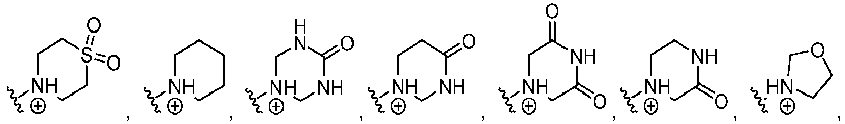 and

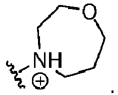

Embodiment 68. The composition of Embodiment 67, wherein Z¹ and Z² are each independently selected from: 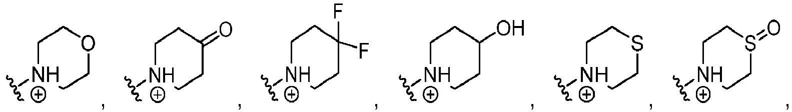 and

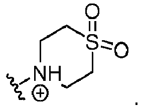

Embodiment 69. The complex of any one of Embodiments 53-68, wherein the cationic oligosaccharide further comprising one or more suitable counterions.

Embodiment 70. The complex of any one of Embodiments 1-53, wherein the cationic oligosaccharide is selected from Table 1, or a pharmaceutically acceptable salt thereof.

Embodiment 71. The complex of any one of Embodiments 1-53, wherein the cationic oligosaccharide is selected from Table 2, or a pharmaceutically acceptable salt thereof.

Embodiment 72. A method of increasing or causing increased expression of RNA in a target in a subject comprising administering to the subject the complex of any one of Embodiments 1-71.

Embodiment 73. The method of Embodiment 72, wherein the target is selected from the lungs, liver, spleen, heart, brain, lymph nodes, bladder, kidneys, and pancreas.

Embodiment 74. A method of treating a disease, disorder, or condition in a subject comprising administering to the subject a complex of any one of Embodiments 1-71.

Embodiment 75. The method of Embodiment 74, wherein the disease, disorder, or condition is an infectious disease, cancer, a genetic disorder, an autoimmune disease, or a rare disease.

Embodiment 76. The method of any one of Embodiments 72-75, wherein the complex is administered intramuscularly.

Embodiment 77. The method of any one of Embodiments 72-75, wherein the complex is administered subcutaneously.

Embodiment 78. A complex of any one of Embodiments 1-71, for use as a medicament.

Embodiment 79. A complex of any one of Embodiments 1-71, for use in the treatment and/or prevention of a disease or disorder, wherein the disease or disorder is an infectious disease, cancer, a genetic disorder, an autoimmune disease, or a rare disease.

Embodiment 80. A composition comprising an oligosaccharide of formula I: 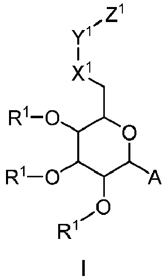 or a pharmaceutically acceptable salt thereof, wherein:
A is A¹, A², or A³: 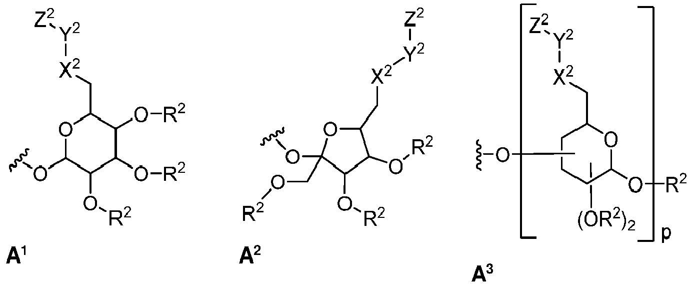
R¹ and R² are each independently selected from H, R^{a}, and -C(O)-R^{a}, wherein at least one instance of R¹ or R² is not H; each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by -NR^{Y}C(O)NR^{Y}- or NR^{Y}C(S)NR^{Y}-
each R^{Y} is independently selected from H and optionally substituted C₁-C₆ aliphatic; Z¹ and Z² are each independently selected from: 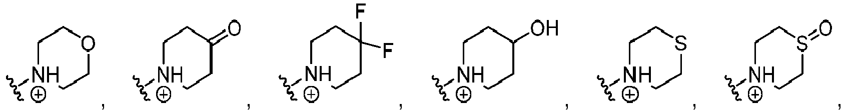 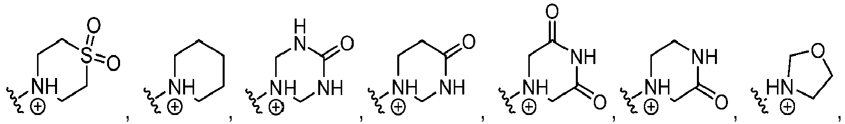 and 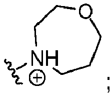 and
p is an integer selected from 1, 2, 3, 4 or 5.

Embodiment 81. The composition of Embodiment 80, wherein Z¹ and Z² are each independently selected from: 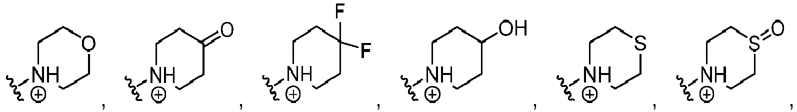 and

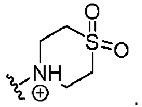

Embodiment 82. The composition of Embodiments 80 or 81, wherein the cationic oligosaccharide is of formula la: 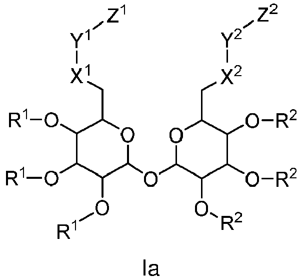 or a pharmaceutically acceptable salt thereof.

Embodiment 83. The composition of Embodiments 80 or 81, wherein the oligosaccharide is of formula Ib: 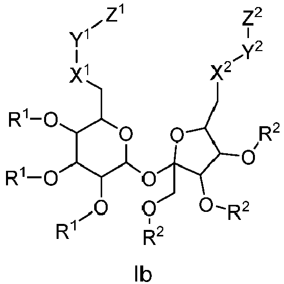 or a pharmaceutically acceptable salt thereof.

Embodiment 84. The composition of Embodiments 80 or 81, wherein the oligosaccharide is of formula Ic: 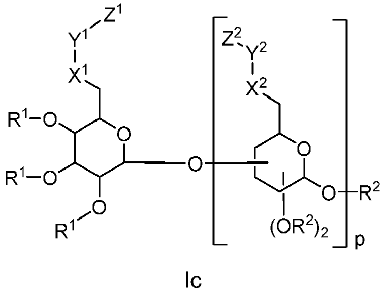 or a pharmaceutically acceptable salt thereof.

Embodiment 85. The composition of any one of Embodiments 80-84, wherein R¹ and R² are each independently selected from R^{a} and -C(O)-R^{a}, and R^{a} is C₁-C₂₀ aliphatic.

Embodiment 86. The composition of any one of Embodiments 80-85, wherein R¹ and R² are each -C(O)-R^{a}, and R^{a} is C₁-C₁₄ aliphatic.

Embodiment 87. The composition of any one of Embodiments 80-86, wherein each R^{a} is C₅-C₁₀ linear alkyl.

Embodiment 88. The composition of any one of Embodiments 80-86, wherein R¹ and R² are each -C(O)-R^{a}, and each R^{a} is independently selected from:

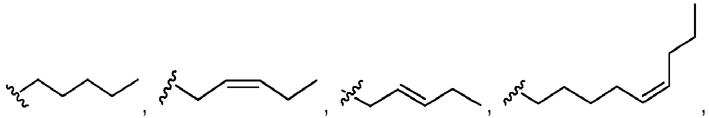

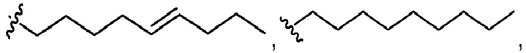

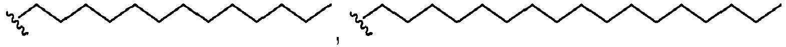

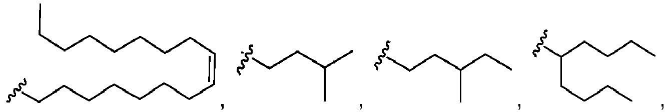

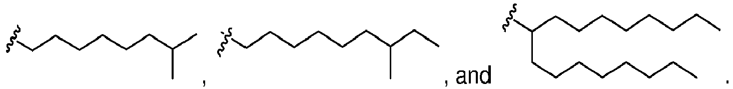

Embodiment 89. The composition of any one of Embodiments 80-88, wherein X¹ and X² are each -S-.

Embodiment 90. The composition of any one of Embodiments 80-89, wherein Y¹ and Y² are each C₁-C₄ aliphatic-NHC(S)NH-C₁-C₄ aliphatic.

Embodiment 91. The composition of Embodiment 90, wherein Y¹ and Y² are each -CH₂-CH₂-NHC(S)NH-CH₂-CH₂-.

Embodiment 92. The composition of any one of Embodiments 80-91, wherein the cationic oligosaccharide further comprising one or more suitable counterions.

Embodiment 93. The composition of Embodiment 80, wherein the cationic oligosaccharide is selected from Table 2.

Embodiment 94. Use of a complex of any one of Embodiments 1-71, for the treatment of a disease, disorder, or condition.

Embodiment 95. The use of Embodiment 94, wherein the disease, disorder, or condition is an infectious disease, cancer, a genetic disorder, an autoimmune disease, or a rare disease.

The foregoing has been a description of certain non-limiting embodiments of the subject matter described within. Accordingly, it is to be understood that the embodiments described in this specification are merely illustrative of the subject matter reported within. Reference to details of the illustrated embodiments is not intended to limit the scope of the claims, which themselves recite those features regarded as essential.

It is contemplated that systems and methods of the claimed subject matter encompass variations and adaptations developed using information from the embodiments described within. Adaptation, modification, or both, of the systems and methods described within may be performed by those of ordinary skill in the relevant art. Throughout the description, where systems are described as having, including, or comprising specific components, or where methods are described as having, including, or comprising specific steps, it is contemplated that, additionally, there are systems encompassed by the present subject matter that consist essentially of, or consist of, the recited components, and that there are methods encompassed by the present subject matter that consist essentially of, or consist of, the recited processing steps.

It should be understood that the order of steps or order for performing certain action is immaterial so long as any embodiment of the subject matter described within remains operable. Moreover, two or more steps or actions may be conducted simultaneously

### Acknowledgements

The Applicant wishes to thank Consejo Superior de Investigaciones Cientificas and FIUS/UNIVERSITY OF SEVILLE/ for their support and contribution to the development of this work.

### EXAMPLES

The present teachings include descriptions provided in the Examples that are not intended to limit the scope of any claim. Unless specifically presented in the past tense, inclusion in the Examples is not intended to imply that the experiments were actually performed. The following non-limiting examples are provided to further illustrate the present teachings. Those of skill in the art, in light of the present application, will appreciate that many changes can be made in the specific embodiments that are provided herein and still obtain a like or similar result without departing from the spirit and scope of the present teachings

As described in the Examples below, in certain exemplary embodiments, compounds are prepared according to procedures in EP21382958.3, which is incorporated herein by reference in its entirety. It will be appreciated that, although the general methods depict the synthesis of certain compounds of the present disclosure, the following general methods and other methods known to one of ordinary skill in the art can be applied to all compounds and subclasses and species of each of these compounds, as described herein.

The following abbreviations may be used in the Examples below: aq. (aqueous); ACN (acetonitrile); CSA (camphorsulfonic acid); d (day or days); DCM (dichloromethane); DEA (diethylamine); Dexa (dexamethasone); DHP (dihydropyran); DMF (N,N-dimethylformamide); DIPEA (N,N-diisopropylethylamine); DMAP (4-dimethylaminopyridine); DMSO (dimethyl sulfoxide); EA (ethyl acetate); ee (enantiomeric excess); equiv. (equivalent); ethanol (EtOH); h (hour or hours); Hex (hexanes); HPLC (high-performance liquid chromatography); IPA (isopropyl alcohol); KHMDS (potassium bis(trimethylsilyl)amide); LAH (lithium aluminum hydride); LCMS (liquid chromatography-mass spectrometry); LDA (lithium diisopropylamide); LiHMDS (lithium bis(trimethylsilyl)amide); MeOH (methanol); min (minute or minutes); NMR (nuclear magnetic resonance); Pd/C (palladium on carbon); PPhₛO (triphenylphosphine oxide); Pt/C (platinum on carbon); rb (round-bottomed); Rf (retention factor); rt or RT (room temperature); SM (starting material); TEA (triethylamine); THF (tetrahydrofuran); THP (tetrahydropyran); TLC (thin layer chromatography); TsOH (p-toluenesulfonic acid or tosylic acid); T20/T40/T60 (Tween20/Tween40/Tween60); and UV (ultraviolet).

### Example 1 - Delivery of modRNA Using Cationic Oligosaccharides

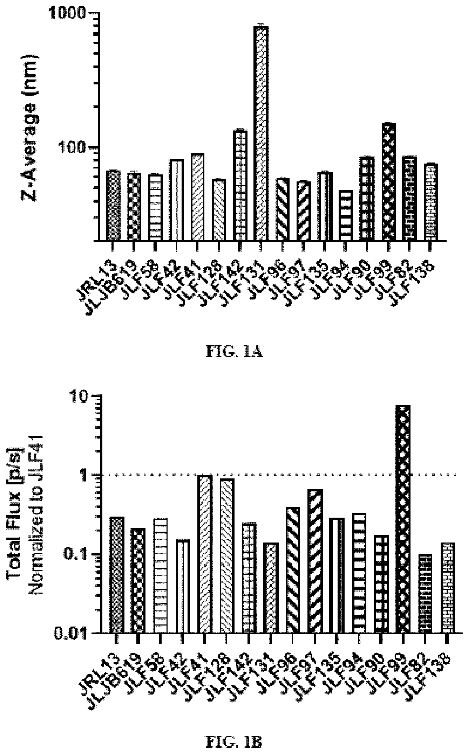

Forty-eight female BALB/c mice are divided into sixteen study groups. All groups received the same amount of formulated luciferase encoding saRNA (2µg), applied intramuscularly (i.m.) to each leg. At six time points (6 hours, day 1, day 2, day 3, day 6) in-vivo luciferase expression in applied tissue was measured. All groups received modRNA formulated at N/P6. Each group received modRNA formulated with one of the following cationic oligosaccharides: JRL13, JLJB619, JLF42, JLF41, JLF128, JLF142, JLF131, JLF58, JLF96, JLF97, JLF135, JLF94, JLF90, JLF99, JLF82, JLF138. Formulations were complexed in MBG Buffer (final concentration 5% w/v glucose, 10mM MES, pH 6.1), at RNA final concentration of 0.1 mg/ml and the mixing ratio of the RNA containing solution and the cationic oligosaccharide containing solution was 9:1 (v:v) for all the cases. Additives, such as Tween20/40/60, when present, are mixed with the oligosaccharide in DMSO at the recited molar ratio prior to complexing with the RNA. Formulations were characterized prior to injection to confirm hydrodynamic diameter. **FIG. 1A** is a bar graph indicating hydrodynamic diameter of complexes formulated at N/P6 ratios with modRNA and different cationic oligosaccharides. **FIG. 1B** is a bar graph indicating quantified bioluminescence over the duration of the experiment and normalized to the JLF41-modRNA group.

### Example 2 - Complexes Comprising a Surfactant

The present example considers different surfactants of various structure and chemical composition. All candidates possess a surfactant that is based either on polyethylene glycol, poly(2-oxazoline), or polysarcosine. All candidates comprise aliphatic chains of a length between 6 - 18 carbons.

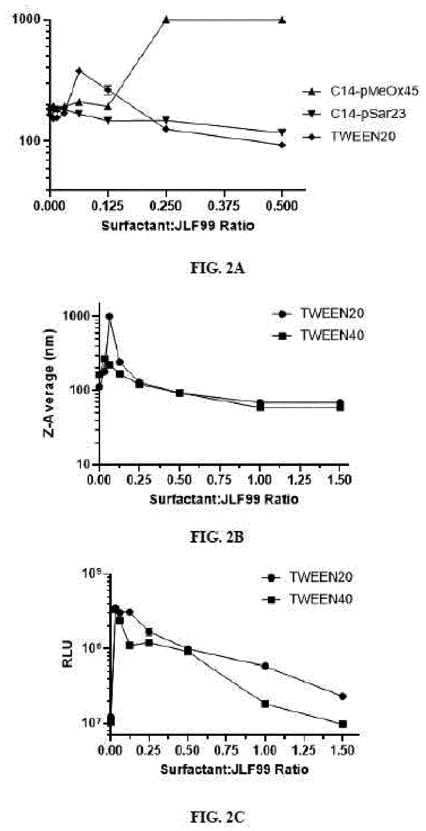

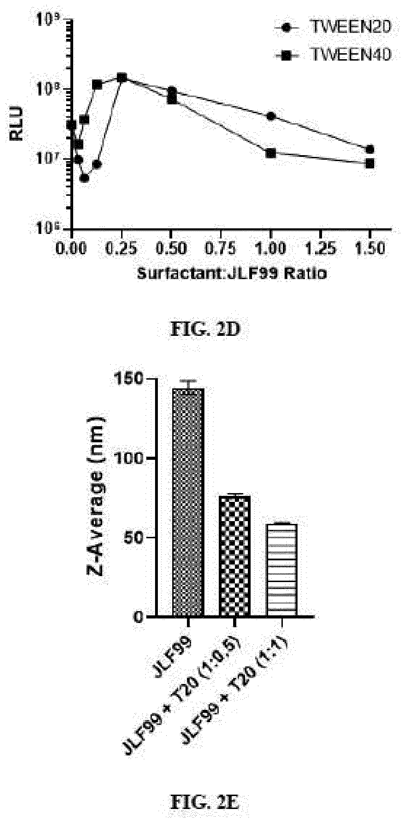

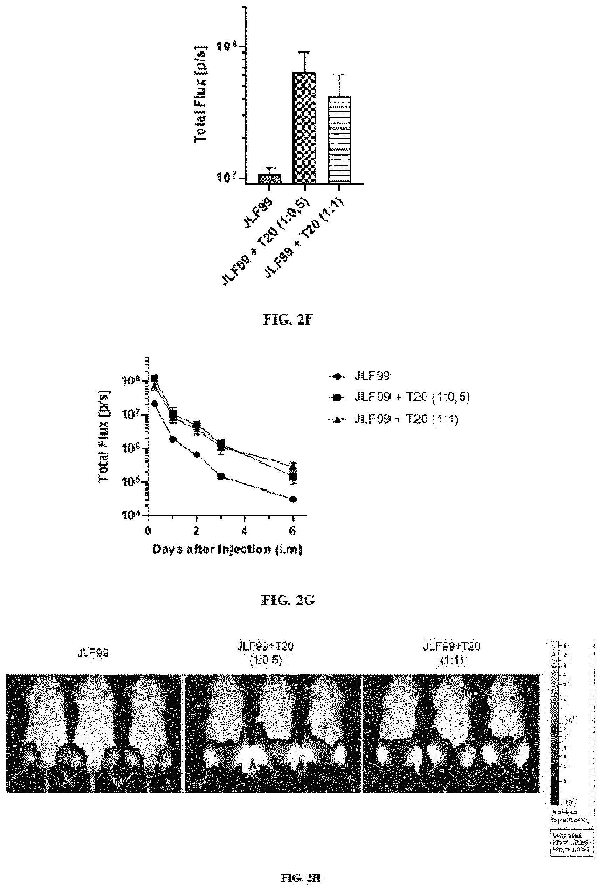

Nine female BALB/c mice were divided into 3 study groups. All groups received the same amount of formulated luciferase encoding modRNA (2µg), applied i.m to each leg. At five time points (6 hours, day 1, day 2, day 3, day 6) in-vivo luciferase expression in applied tissue was measured. The first group received modRNA formulated exclusively with JLF99, the second group received modRNA formulated with a mixture of JLF99+Tween20 (1:0.5 molar ratio) and the third group received modRNA formulated with a mixture of JLF99+ Tween20 (1:1 molar ratio). All groups received modRNA formulated at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharide + Tween20 containing solution was 9:1 (v:v) for all cases. Formulations were characterized prior to injection to confirm hydrodynamic diameter. **FIG. 2A** is a plot indicating hydrodynamic diameter of complexes formulated at N/P6 but with different surfactants and within a surfactant:JLF99 ratio between about 0:1 to about 0.5:1. C14-pMeOx45 is monoalkyl (C₁₄) having 45 repeating units of poly(2-methyl-2-oxazoline); C14-pSar23 is monoalkyl (C₁₄) having 23 repeating units of polysarcosine. **FIG. 2B** is a plot indicating hydrodynamic diameter of complexes formulated at N/P6 with either Tween20 or Tween40 and within a surfactant:JLF99 ratio between about 0:1 to about 0.5:1. **FIG. 2C** is a plot indicating expression of luciferase-encoding modRNA after 24 hours of transfection of C2C12 cells, N/P6 formulated modRNA with either Tween20 or Tween40 at different ratios of JLF, at 100ng of RNA/well. Data here represents 3 independent experiments (n=3) with technical triplicates. **FIG. 2D** is a plot illustrating expression of luciferase-encoding modRNA after 24 hours of transfection of HepG2 cells, N/P6 formulated modRNA with either Tween20 or Tween40 at different surfactant to JLF99 ratios, at 100ng of RNA/well. Data here represents 3 independent experiments (n=3) with technical triplicates. **FIG. 2E** is a bar graph illustrating hydrodynamic diameter of complexes formulated at different ratios of JLF99 to surfactant. **FIG. 2F** is a bar graph illustrating quantified bioluminescence over the whole duration of the experiment represented as area under the curve. **FIG. 2G** is a plot illustrating dorsal quantified bioluminescence over about 6 days. **FIG. 2H** is an image illustrating bioluminescence on the ventral axis 6 hours after injection of modRNA into each group.

### Example 3 - Physical Properties of Example Complexes

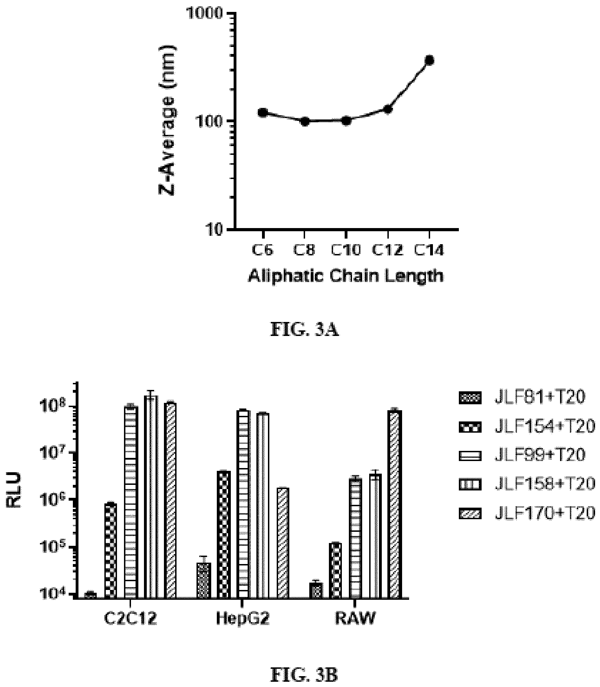

In the present example, six different cationic oligosaccharides having different length carbon chains were tested in combination with modRNA: JLF81 (C₆ aliphatic), JLF154 (C₈ aliphatic), JLF99 (C₁₀ aliphatic), JLF158 (C₁₂ aliphatic), and JLF170 (C₁₄ aliphatic). Each of the cationic oligosaccharides and modRNA complexes were formed in combination with Tween20. While the cationic oligosaccharides systematically varied in each formulation, the molar ratio of cationic oligosaccharides to Tween20 was constant (1:0.5) in all formulations. All formulations were prepared at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharides + Tween20 containing solution was 9:1 (v:v) for all the cases. **FIG. 3A** is a plot illustrating hydrodynamic diameter of example complexes. **FIG. 3B** is a bar graph illustrating expression of luciferase-encoding modRNA after 24 hours of transfection of either C2C12, HepG2 or RAW264.7 cells, at 100ng of RNA/well. Data here represents 2 independent experiments (n=2) with technical triplicates.

Sizes of complexes described in the present examples were analyzed by DynaPro PlateReader II, using dynamic light scattering (DLS) for calculating the hydrodynamic size of complexes. The formulations are measured by diluting the sample to 0.01 mg/mL RNA in 120µ1 of MGB (10 mM MES, pH 6.1, 5% D-Glucose) 5% in wells of a 96-well plate. Ten data points are recorded per well, each lasting 10 seconds.

### Example 4 - Complexes Comprising a Helper Lipid and/or a Sterol

The present example examines a complex JLF99 and Tween20 and certain helper lipids and/or sterols. The combination of JL99+Tween20 was used at a constant molar ratio of 1:0.5, whereas different helper lipids or sterol are used in different molar ratios to JLF99. All the formulations here were prepared with luciferase encoding-modRNA at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharides and Tween/sterol/helper lipid containing solution was 9:1 (v:v) for all the cases.

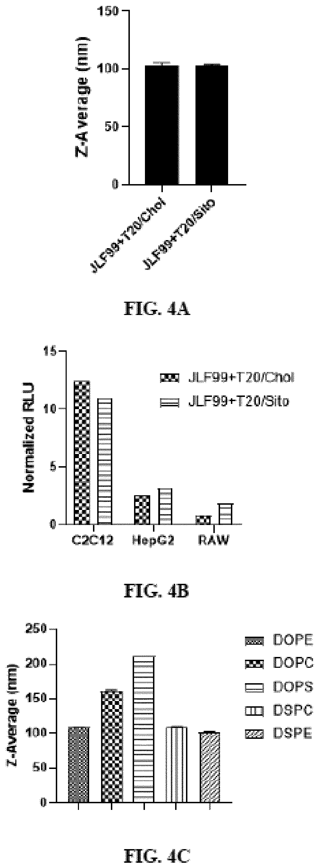

The formulations of the present example provide (1) a combination of JLF99 + Tween20 + Cholesterol at a molar ratio of 1:0.5:1 (JLF99:Tween20:Cholesterol) and (2) JLF99 + Tween20 + β-Sitosterol at a molar ratio of 1:0.5:1 (JLF99:Tween20: β-Sitosterol). Five formulations comprise JLF99 + Tween20 + Helper Lipid + β-Sitosterol, where the helper lipid in the formulation is DOPE, DOPC, DOPS, DSPC, or DSPE and in all cases the molar ratio of JLF99:Tween20:Helper Lipid:β-Sitosterol is 1:0.5:0.5:0.5. Three of the formulations illustrate compositions of JLF99 + Tween20 + DSPC + Sterol, where the sterol is β-Sitosterol, Stigmasterol or Acetate-α-Tocopherol and in all cases the molar ratio of JLF99:Tween20:DSPC:Sterol is 1:0.5:0.5:0.5. Ten formulations illustrate a combination of JLF99 + Tween20 + Helper Lipid+ β-Sitosterol, where said helper lipid is either DSPC or DMPC in five different molar ratios of JLF99:Tween20:Helper Lipid:β-Sitosterol that are 1 :0.5:1 :0,1:0.5:0.25:0.75, 1:0.5:0.5:0.5, 1:0.5:0.75:0.25 and 1 :0.5:0:1. **FIG. 4A** is a bar graph illustrating hydrodynamic diameter of complexes formed with either a combination of JLF99+Tween20+Cholesterol or JLF99+Tween20+β-Sitosterol.

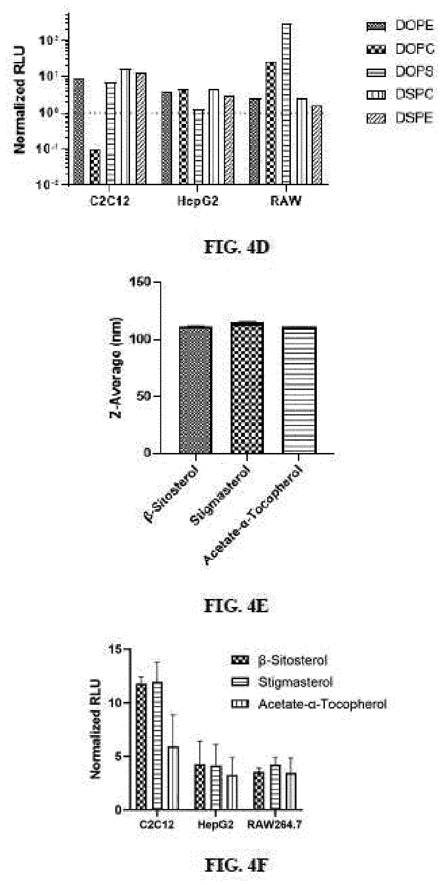

**FIG. 4B** is a bar graph illustrating normalized expression of luciferase-encoding modRNA to JLF99 benchmark after 24 hours after of either C2C12, HepG2 or RAW264.7 cells, at 100ng of RNA/well complexes formed with either a combination of JLF99+Tween20+Cholesterol or JLF99+Tween20+ β-Sitosterol. **FIG. 4C** is a bar graph illustrating hydrodynamic diameter of complexes formed of the combination of JLF99 + Tween20 + β-Sitosterol + Helper Lipid at a molar ratio of 1:0.5:0.5:0.5 with either DOPE, DOPC, DOPS, DSPC, or DSPE. **FIG. 4D** is a bar graph illustrating normalized expression of luciferase-encoding modRNA to JLF99 benchmark after 24 of transfection of either C2C12, HepG2 or RAW264.7 cells, at 100ng of RNA/well of complexes formed with JLF99 + Tween20 + β-Sitosterol + Helper Lipid at a molar ratio of 1:0.5:0.5:0.5 with either DOPE, DOPC, DOPS, DSPC, or DSPE. Data here represents 2 independent experiments (n=2) with technical triplicates. **FIG. 4E** is a bar graph illustrating hydrodynamic diameter of complexes formed of the combination of JLF99 + Tween20 + Sterol + DSPC at a molar ratio of 1:0.5:0.5:0.5 with either β-Sitosterol, Stigmasterol, or Acetate-α-Tocopherol. **FIG. 4F** is a bar graph illustrating the normalized expression of luciferase-encoding modRNA to JLF99 benchmark 24 hours after of transfection of either C2C12, HepG2 or RAW264.7 cells, at 100ng of RNA/well of complexes formed with JLF99 + Tween20 + Sterol + DSPC at a molar ratio of 1:0.5:0.5:0.5 wherein said sterol is either β-Sitosterol, Stigmasterol, or Acetate-α-Tocopherol. Data here represents 2 independent experiments (n=2) with technical triplicates. **FIG. 4G** is a scatter plot illustrating hydrodynamic diameter of complexes formed of the combination of JLF99 + Tween20 + β-Sitosterol + Helper Lipid at different molar ratios with either DMPC or DSPC. **FIG. 4H** is a scatter plot illustrating the expression of luciferase-encoding modRNA after 24 hours of transfection of C2C12 at 100ng of RNA/well of complexes formed with JLF99 + Tween20 + β-Sitosterol + Helper Lipid at different molar ratios with either DMPC or DSPC. Data here represents 2 independent experiments (n=2) with technical triplicates. **FIG 41** is a plot illustrating expression of luciferase-encoding modRNA 24 hours after transfection of HepG2 at 100ng of RNA/well of complexes formed with JLF99 + Tween20 + β-Sitosterol + Helper Lipid at different molar ratios with either DMPC or DSPC. Data here represents 2 independent experiments (n=2) with technical triplicates. **FIG. 4J** is a plot illustrating expression of luciferase-encoding modRNA after 24 hours of transfection of RAW647.7 at 100ng of RNA/well of complexes formed with JLF99 + Tween20 + β-Sitosterol+ Helper Lipid at different molar ratios with either DMPC or DSPC. Data here represents 2 independent experiments (n=2) with technical triplicates.

### Example 5 - Complexes Comprising Example Additives

The present example examines incorporation of β -sitosterol and DSPC into the formulation at different molar ratios to JLF99, while the molar ratio of JLF99:Tween20 was kept constant. Twelve female BALB/c mice are divided into 4 study groups. All the groups received the same amount of formulated luciferase-encoding modRNA (2µg), applied i.m. to each leg. At five time points (6 hours, day 1, day 2, day 3, day 6) in-vivo luciferase expression in applied tissue was measured. While the first group received modRNA formulated exclusively with JLF99, the second group received modRNA formulated with a mixture of JLF99 + Tween20 + β -sitosterol + DSPC (1:0.5:1:0 molar ratio), the third group received modRNA formulated with a mixture of JLF99 + Tween20 + β -sitosterol + DSPC (1:0.5:0.5:0.5 molar ratio) and the fourth group received modRNA formulated with a mixture of JLF99 + Tween20 + β -sitosterol + DSPC (1:0.5:0:1 molar ratio). All groups received modRNA formulated at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharide (+ Tween20 + β -sitosterol + DSPC) containing solution was 9:1 (v:v) for all the cases. Formulations were characterized prior to injection to confirm hydrodynamic diameter. **FIG. 5A** is a bar graph illustrating hydrodynamic diameter of different complexes formulated at N/P 6. **FIG. 5B** is a bar graph illustrating quantified bioluminescence over the whole duration of the experiment represented as area under the curve. **FIG. 5C** is a plot illustrating quantified dorsal bioluminescence over the whole duration of the experiment. **FIG. 5D** is an image of ventral bioluminescence measured after 6 hours of injection.

### Example 6 - Analysis of Complexes Comprising Certain Additives

Six female BALB/c mice are divided into 2 study groups. All groups received the same amount of formulated luciferase encoding modRNA (2µg), applied i.m to each leg. Six hours post injection, in-vivo luciferase expression in applied tissue was measured. The first group received modRNA formulated exclusively with JLF99 + Tween20 (1:0.5 molar ratio), the second group received modRNA formulated with a mixture of JLF99 + Tween20 + β-sitosterol + DSPC (1:0.5:0.5:0.5 molar ratio). All groups received modRNA formulated at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharide + Tween20 + β-sitosterol + DSPC containing solution was 9:1 (v:v) for all the cases. Formulations were characterized prior to injection to confirm hydrodynamic diameter. **FIG. 6A** is a bar graph illustrating hydrodynamic diameter of different complexes formulated at N/P6. **FIG. 6B** is a bar graph illustrating quantified dorsal bioluminescence in the region of interest (muscle) 6 hours after injection. **FIG. 6C** is a bar graph illustrating quantified ventral bioluminescence in the region of interest (liver) 6 hours after injection. **FIG. 6D** is an image of ventral bioluminescence measured 6 hours after injection.

### Example 7 - Complexes Comprising an Immunomodulator

The present example provides a complex comprising a third component which can enable a specific immune response (e.g., a Th1, Th2, or Th1/Th2 response) or, in the alternative, inhibit an immune response towards the expressed protein from delivered RNA, thereby avoiding pro-inflammatory signals that can hamper the therapeutic effect of RNA-therapy. In the present experiment, an immunostimulator molecule, MGC-VI-150, was used in combination with JLF99 + Tween20 to formulate modRNA encoding for luciferase. Six female BALB/c mice were divided into 2 study groups. All the groups received the same amount of formulated luciferase encoding modRNA (2µg), applied i.m. to each leg. At five time points (6 hours, day 1, day 2, day 3, day 6) in-vivo luciferase expression in applied tissue was measured. At day 9 post injection, mice were sacrificed and the spleen extracted in order to quantify the amount of T-cell response against luciferase peptides. The first group received modRNA formulated exclusively with JLF99 + Tween20 (1:0.5 molar ratio), the second group received modRNA formulated with a mixture of JLF99 + Tween20 + MGC-VI-150 (1:0.5:0.1 molar ratio). All groups received modRNA formulated at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharide + Tween20 + MGC-VI-150 containing solution was 9:1 (v:v) for all the cases. Formulations were characterized prior to injection to confirm hydrodynamic diameter. **FIG. 7A** is a bar graph illustrating hydrodynamic diameter of different complexes formulated at N/P6. **FIG. 7B** and **FIG. 7C** are a plot and a bar graph, respectively, illustrating quantified dorsal bioluminescence in the region of interest (muscle) over the whole duration of the experiment as area under the curve. **FIG. 7D** is a bar graph illustrating quantified spots count in an ELISpot read-out, reflecting the T-Cell responses against luciferase peptides or an irrelevant peptide (AH1).

### Example 8 - Complexes Comprising an Immunomodulator

The present example provides a complex comprising an immunomodulator which can inhibit the immune response against an expressed protein from RNA delivery, thereby avoiding pro-inflammatory signals that can hamper the therapeutic effect of RNA therapy. In the present experiment, two different immunoinhibitory molecules, TAK-242 (a TLR4 inhibitor) and dexamethasone (a wide spectrum immunosuppressor), were used in combination with cationic oligosaccharides + Tween40 to formulate modRNA encoding for luciferase.

### Example 8a - Analysis of Pro-Inflammatory Cytokines Using Formulations Comprising Cationic Oligosaccharides and a TLR4 Inhibitor

In the first part of the present example, secretion of pro-inflammatory cytokines in human peripheral blood mononuclear cell (hPBMCs) was evaluated for two different cationic oligosaccharides: JLF99 and JLF605. In total, 4 formulations were tested: one formulation comprising JLF99 and Tween40 (1:0.5 molar ratio), a second formulation comprising JLF99, Tween40 and TAK-242 (1:0.5:0.5 molar ratio), a third formulation comprising JLF605 and Tween40 (1:0.5 molar ratio) and fourth formulation comprising JLF605, Tween40 and TAK-242 (1:0.5:0.5 molar ratio)). All groups received modRNA (coding for firefly luciferase) formulated at N/P6. Formulations were complexed in MBG Buffer (final concentration 5% w/v Glucose, 10mM MES, pH 6.1), at RNA final concentration of 0.15 mg/ml and the mixing ratio of the RNA containing solution and the cationic oligosaccharides + Tween40 (+ TAK-242 optionally) containing solution was 9:1 (v:v) for all the cases. IL-6, TNF-α, iL-1β and IFN-γ secretion after 24 hours of transfection of 5 x 10⁵ hPMBCs was evaluated in the supernatant using MSD Kit Standard Protocol.
**FIG. 8A** is a scatter plot illustrating quantified IL-6 secretion in hPBMCs upon exposure to different doses of tested formulations. **FIG.8B** is a scatter plot illustrating quantified TNF-α secretion in hPBMCs upon exposure to different doses of tested formulations. **FIG.8C** is a scatter plot illustrating quantified IL-1β secretion in hPBMCs upon exposure to different doses of tested formulations. **FIG.8D** is a scatter plot illustrating quantified IFN-γ secretion in hPBMCs upon exposure to different doses of tested formulations.

### Example 8b - Analysis of Pro-Inflammatory Cytokines and Chemokines Using Formulations Comprising Cationic Oligosaccharides and a TLR4 Inhibitor, Optionally with Dexamethasone

In the second part of the experiment, secretion of both pro-inflammatory cytokines and chemokines in hPBMCs as well as luciferase expression in human primary hepatocytes, was evaluated in the presence of combination of dexamethasone and TAK-242 in order to further understand the requirements for a non-inflammatory formulation. In total, 3 formulations were tested: first formulation comprising JLF218 and Tween40 (1:0.5 molar ratio), a second formulation comprising JLF218, Tween40 and Dexamethasone (1:0.5:0.5 molar ratio) and a third formulation comprising JLF218, Tween40, Dexamethasone and TAK-242 (1:0.5:0.5:0.5 molar ratio). All groups received modRNA (encoding for firefly luciferase) formulated at N/P6. Formulations were complexed in MBG Buffer (final concentration 5% w/v Glucose, 10mM MES, pH 6.1), at RNA final concentration of 0.15mg/ml and the mixing ratio of the RNA containing solution and the cationic oligosaccharides + Tween40 (+ Dexamethasone + TAK-242, optionally) containing solution was 9:1 (v:v) in all cases. IL-6, TNF-α, iL-1β, IFN-γ, MIP-1β, IP-10 and MCP-1 secretion after 24 hours of transfection of 5 x10⁵ hPMBCs was evaluated in the supernatant using MSD Kit Standard Protocol. Primary human hepatocytes were transfected with 0.3µg of modRNA and luciferase expression was evaluated over the course of 6 days.

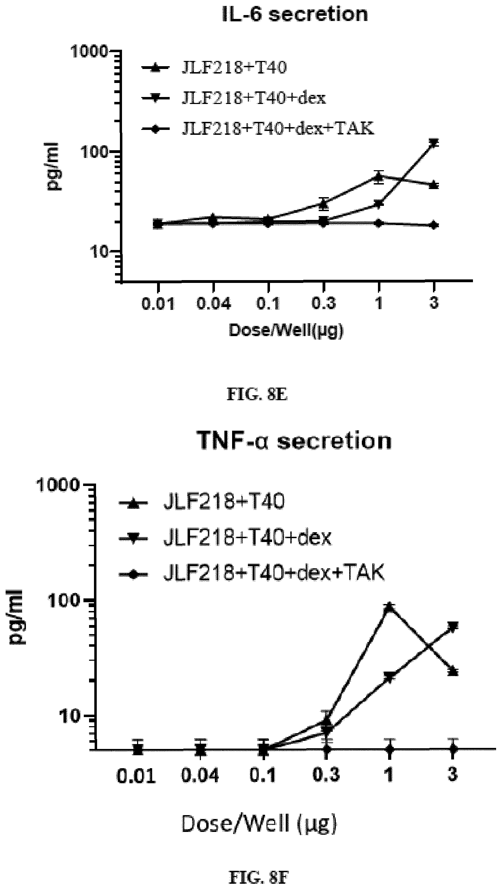

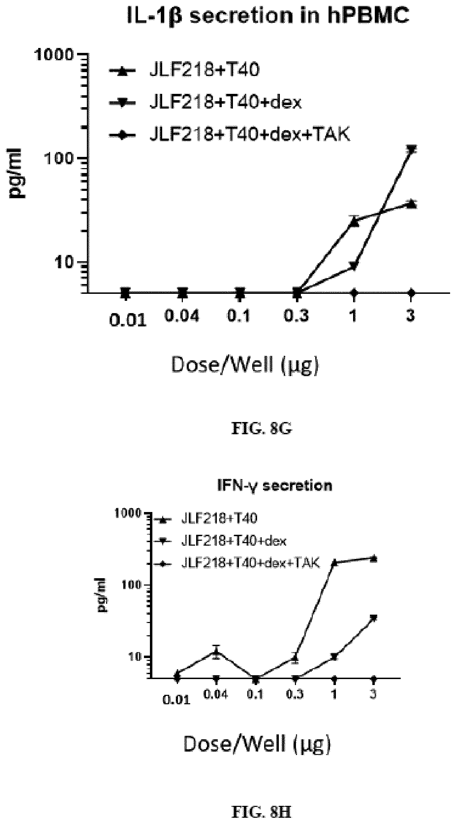

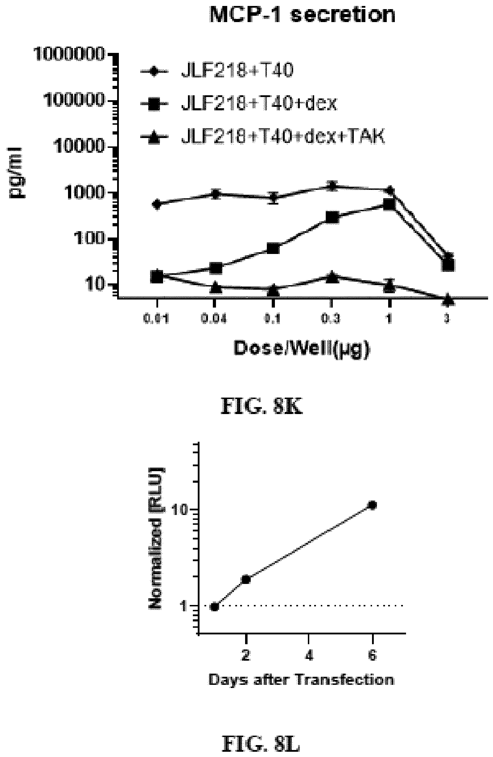

**FIG.8E** is a scatter plot illustrating quantified IL-6 secretion in hPBMCs upon exposure to different doses of tested formulations. **FIG.8F** is a scatter plot illustrating quantified TNF-α secretion in hPBMCs upon exposure to different doses of tested formulations. **FIG.8G** is a scatter plot illustrating quantified IL-1β secretion in hPBMCs upon exposure to different doses of tested formulations. **FIG.8H** is a scatter plot illustrating quantified IFN-γ secretion in hPBMCs upon exposure to different doses of tested formulations. **FIG.81** is a scatter plot illustrating quantified MIP-1β secretion in hPBMCs upon exposure to different doses of tested formulations. **Figure 8J** is a scatter plot illustrating quantified IP-10 secretion in hPBMCs upon exposure to different doses of tested formulations. **FIG.8K** is a scatter plot illustrating quantified MCP-1 secretion in hPBMCs upon exposure to different doses of tested formulations. **FIG.8L** is a plot illustrating the normalized expression of luciferase-encoding modRNA at different time points (6 hours, day 2, day 6) after transfection of primary human hepatocytes with 0.3µg of modRNA complexed with JLF218 + Tween40 + Dexamethasone + TAK-242 over JLF218 + Tween40 + Dexamethasone.

### Example 9 - Modulation of T-Cell Response

The present example provides a complex comprising a third component which can boost cellular immune response, with no significant effect on humoral response. In the present example, an immunostimulator molecule, MGC-103, was used in combination with JLF99 + Tween40 to formulate modRNA encoding for luciferase. Twenty female BALB/c mice were divided into four study groups. One group received only buffer as a negative control and three groups received 1µg of formulated modRNA codig for Hemagglutinin (HA) protein of the influenza strain California/7/2009 applied i.m in one of the legs in a prime/boost schedule (day 0/day 28). While the first group received modRNA formulated exclusively with a mixture of JLF99 + Tween40 (1:0.5 molar ratio), the second group modRNA was formulated with a mixture of JLF99 + Tween40 + MGC-103 (1:0.5:0.1 molar ratio) and in the third group modRNA was formulated with a mixture of JLF99 + Tween40 + MGC-103 (1:0.5:0.25 molar ratio). All groups received modRNA formulated at N/P6. Formulations were complexed in MBG Buffer (final concentration 5% w/v Glucose, 10mM MES, pH 6.1), at RNA final concentration of 0.1mg/ml and the mixing ratio of the RNA containing solution and the cationic oligosaccharides + Tween40 (and optionally MGC-103) containing solution was 9:1 (v:v) in all cases. Formulations were characterized prior to injection to confirm hydrodynamic diameter. The animals were subjected to non-invasive serological surveillance over 49 days at different time points (day 0, day 14, day 21, day 28, day 49) and spleen was extracted at the end of experiment for quantification of CD4/CD8 T-Cell response to Influenza HA peptides. AntiInfluenza HA antibodies in serum were quantified by enzyme-linked immunosorbent assay. CD4/CD8 T-Cell response was quantified by IFN -ELISPOT of splenocytes. **FIG. 9A** is a bar graph illustrating hydrodynamic diameter of different complexes formulated at N/P6. **FIG. 9B** is a scatter plot illustrating serological levels of specific IgG against HA at different time points over the course of the experiment (day 0, day 14, day 28, day 49). **FIG. 9C** is a plot illustrating quantified spots count in an IFN-ELISPOT read-out, reflecting CD4/CD8 T-Cell response against HA-peptide pools.

### Example 10 - Complexes Comprising a TLR4 Inhibitor in Combination with Dexamethasone

The present example provides complexes comprising a third component which can inhibit the immune response against the expressed protein from the delivered RNA, avoiding pro-inflammatory signals that can hamper the therapeutic effect of RNA-therapy. In the present experiment, a TLR4 inhibitor molecule, TAK-242, was used alone or together with a wide spectrum immunosuppressor, dexamethasone, in combination with cationic oligosaccharides described herein + Tween40 to formulate modRNA encoding for luciferase.

In the first part of the experiment, secretion of pro-inflammatory cytokines in hPBMCs was evaluated for two different cationic oligosaccharides: JLF218 and PRX093, whereas different combinations including TAK-242 and/or Dexamethasone are tested. In total, 5 formulations are tested: one formulation comprising JLF218 and Tween40 (1:0.5 molar ratio), a second formulation comprising JLF218, Tween40 and TAK-242 (1:0.5:0.5 molar ratio), a third formulation comprising JLF218, Tween40, TAK-242 and Dexamethasone (1:0.5:0.5:0.5 molar ratio), a fourth formulation comprising PRX093 and Tween40 (1:0.5 molar ratio) and a fifth formulation comprising PRX093, Tween40, TAK-242 and Dexamethasone (1:0.5:0.5:0.5 molar ratio). All groups received modRNA (coding for firefly luciferase) formulated at N/P6. Formulations were complexed in MBG Buffer (final concentration 5% w/v Glucose, 10mM MES, pH 6.1), at RNA final concentration of 0.15mg/ml and the mixing ratio of the RNA containing solution and the cationic oligosaccharides + Tween40 (+ TAK-242 + Dexamethasone, optionally) containing solution was 9:1 (v:v) for all the cases. IL-6, TNF-α, IL-1β, IFN-γ, MIP-β, IP-10 and MCP-1 secretion after 24 hours of transfection of 5x 10⁵ hPMBCs was evaluated in the supernatants using MSD Kit Standard Protocol. Primary human hepatocytes were transfected with a dose variation between 0.01-1µg of modRNA and luciferase expression is evaluated over the course of 6 days.

**FIG. 10A** is a scatter plot illustrating quantified IL-6 secretion in hPBMCs upon exposure to different doses of tested formulations. **FIG.10B** is a scatter plot illustrating quantified TNF-α secretion in hPBMCs upon exposure to different doses of tested formulations. **FIG.10C** is a scatter plot illustrating quantified IL-1β secretion in hPBMCs upon exposure to different doses of tested formulations. **FIG.10D** is a scatter plot illustrating quantified IFN-γ secretion in hPBMCs upon exposure to different doses of tested formulations.. **FIG.10E** is a scatter plot illustrating quantified MCP-1 secretion in hPBMCs upon exposure to different doses of tested formulations. **FIG.10F** is a scatter plot illustrating quantified MIP-1β secretion in hPBMCs upon exposure to different doses of tested formulations. **FIG.10G** is a scatter plot illustrating quantified IP-10 secretion in hPBMCs upon exposure to different doses of tested formulations. **FIG.10H** is a bar graph illustrating the cumulative luciferase expression in a dose range between 0.01-1µg for both tested groups over the course of 6 days after transfection.

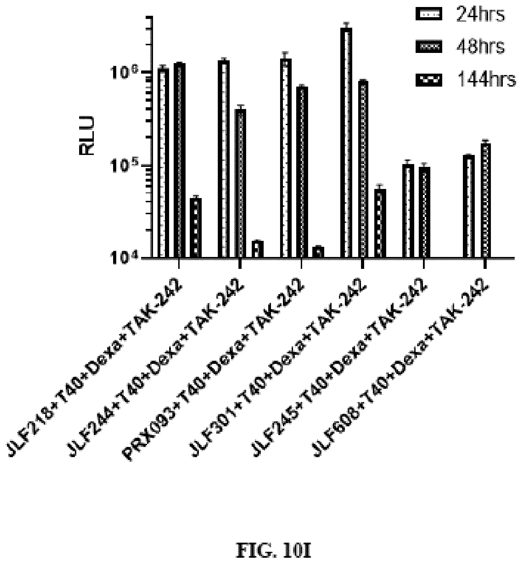

In the second part of the experiment, expression of luciferase-encoding modRNA was evaluated in combination with a variety of cationic oligonucleotides in the presence of Dexamethasone and TAK-242. In total, six formulations are tested, each comprising a combination of one of JLF218, JLF244, PRX093, JLF301, JLF245 or JLF608 with Tween40, Dexamethasone and TAK-242 in a molar ratio of 1:0.5:0.5:0.5. All groups received modRNA (coding for firefly luciferase) formulated at N/P6. Formulations were complexed in MBG Buffer (final concentration 5% w/v Glucose, 10mM MES, pH 6.1), at RNA final concentration of 0.15mg/ml and the mixing ratio of the RNA containing solution and the cationic oligonucleotides + Tween40 (+ Dexamethasone + TAK-242, optionally) containing solution was 9:1 (v:v) for all the cases. Primary human hepatocytes were transfected with a dose variation between 0.01-1µg of modRNA and luciferase expression was evaluated over the course of 6 days. **FIG.10I** is a bar graph illustrating the cumulative luciferase expression in a dose range between 0.01-1µg for all tested groups at different time points over the course of the experiment (24 hours, 48 hours, 144 hours).

The present example illustrates that the incorporation of TAK-242 leads to reduction of pro-inflammatory cytokines (FIGs. 10A to 10D), whereas the incorporation of dexamethasone had only a marginal effect in TNFα secretion (FIG. 10B). The addition of dexamethasone has an improved effect on the secretion of pro-inflammatory chemokines (FIGs. 10E to 10G) relative to TAK-242 alone.

### Example 11 - Synthetic Examples

The present example provides certain example methods of synthesis for cationic oligosaccharides described herein. Exemplary methods of preparing cationic oligosaccharides of the present disclosure are provided in EP 21382958.3, which is incorporated herein by reference in its entirety.

All reagents and solvents used in the preparations disclosed in the following examples were purchased from commercial sources and used without further purification, unless otherwise specified. Thin-layer chromatography (TLC) was carried out on aluminum sheets coated with Silica gel 60 F₂₅₄ Merck with visualization by UV light (λ 254 nm) and by charring with 10% ethanolic H₂SO₄, 0.1% ethanolic ninhydrin and heating at 100 °C. Column chromatography was carried out on Silice 60 A.C.C. Chromagel (SDS 70-200 and 35-70 µm). The structure of all compounds described herein, including synthetic intermediates and precursors, was confirmed by ¹H and ¹³C nuclear magnetic resonance (NMR) and mass spectrometry (MS). NMR experiments were performed at 300 (75.5), and 500 (125.7, 202) MHz with Bruker 300 ADVANCE and 500 DRX. 1D TOCSY, 2D COSY, HMQC and HSQC experiments were used to assist on NMR assignments. Mass spectra (High-resolution mass spectra) were carried out on a Bruker Daltonics Esquire6000^{™} (LTQ-Orbitrap XL ETD). The samples were introduced via solid probe heated from 30 to 280 °C. ESI as ionization source (Electrospray Ionization) was used to which methanol was used as solvent. The samples were introduced via direct injection using a Cole-Parmer syringe at a flow rate of 2 µl/min. Ions were scanned between 300 and 3000 Da with a scan speed of 13000 Da/s at unit resolution using resonance ejection at the multipole resonance of one-third of the radio frequency (Ω = 781.25 kHz).

### Methods

Scheme 1 depicts synthesis of a compound of formula I wherein A is A¹, R¹ and R² are both -C(O)-R^{a}, X¹ and X² are both -S-, Y¹ and Y² are both -(CH₂)₂- and Z¹ and Z² are NH₃+ (compounds **4T** in Scheme 1). Scheme 1 also shows the general routes (routes A1, A2, B1 and B2) implemented for the syntheses of a compound of formula I wherein A is A¹, R¹ and R² are both -C(O)-R^{a}, X¹ and X² are both -S-, Y¹ and Y² are both -(CH₂)₂-NHC(S)NH-(CH₂)₂- and Z¹ and Z² are identical (compounds **6T** in Scheme 1):

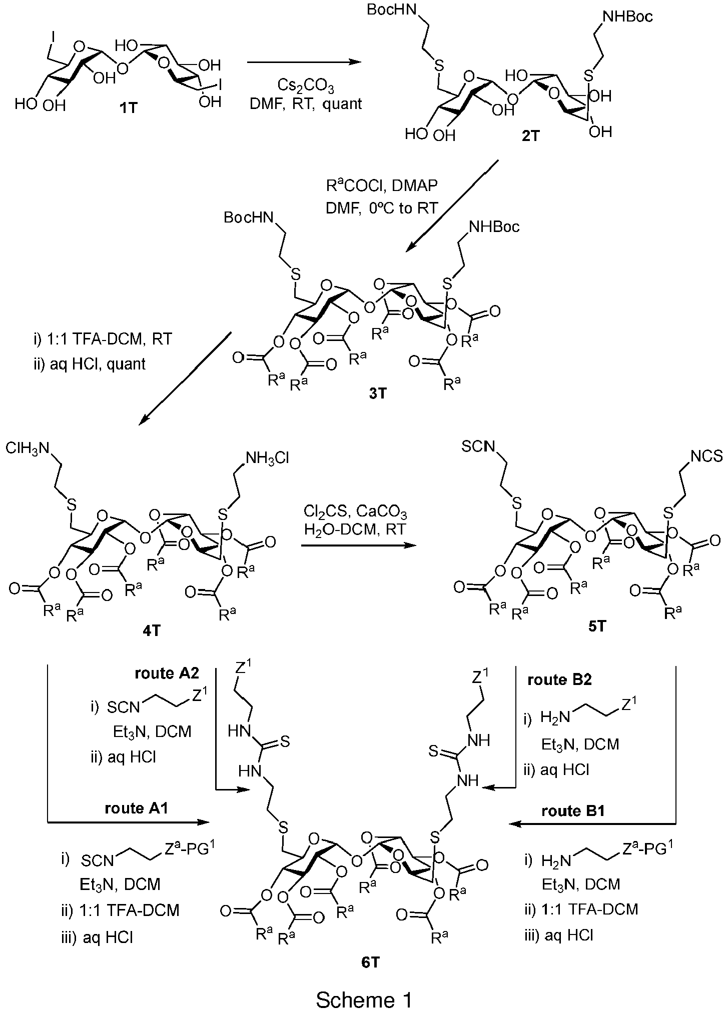

Scheme 2 depicts synthesis of a compound of formula I wherein A is A², R¹ and R² are both -C(O)--R^{a}, X¹ and X² are both -S-, Y¹ and Y² are both -(CH₂)₂- and Z¹ and Z² are NH₃⁺ (compounds **4S** in Scheme 2). Scheme 2 also shows the general routes (routes A1 and A2) implemented for the syntheses of a compound of formula I wherein A is A², R¹ and R² are both -(C=O)R^{a}, X¹ and X² are both -S- and Y¹ and Y² are both -(CH₂)₂-NHC(S)NH-(CH₂)₂- and Z¹ and Z² are identical:

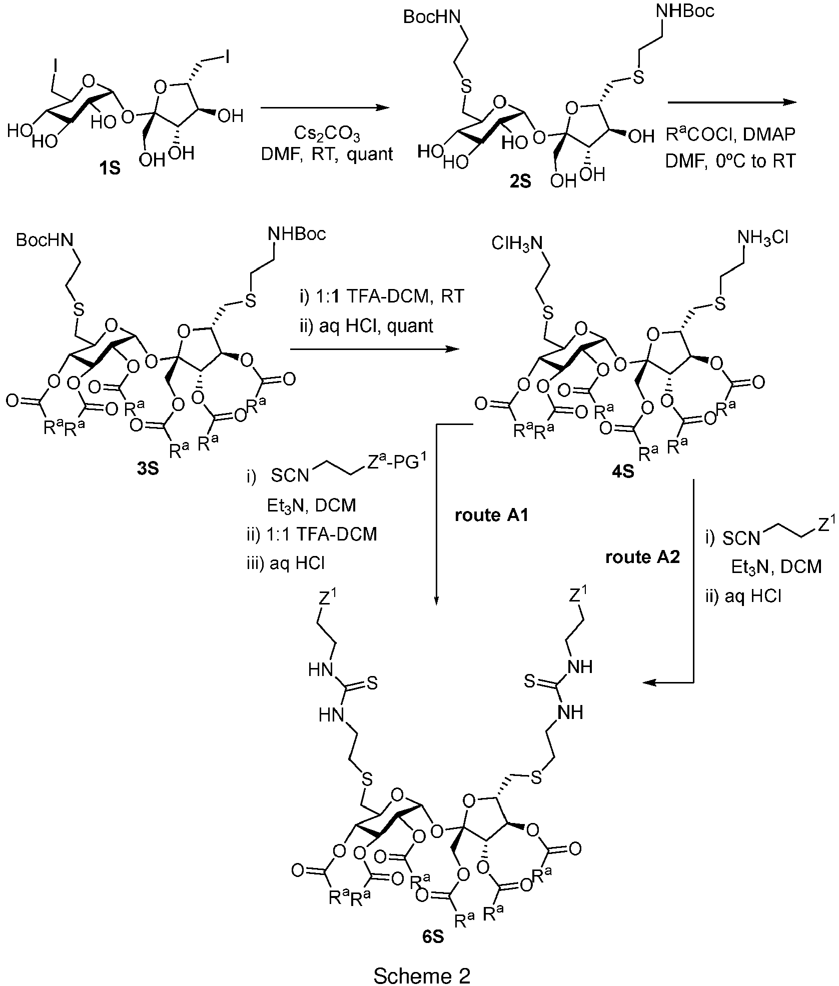

Scheme 3 depicts the synthesis of a compound of formula I wherein A is A¹, R¹ and R² are both -C(O)R^{a}, X¹ and X² are both -S-, Y¹ and Y² are both -(CH₂)₂- and Z¹ and Z² are -N(CH₃)H₂+ (compounds **8T** in Scheme 3) or -N(CH₃)₂H+ (compounds **9T** in Scheme 3):

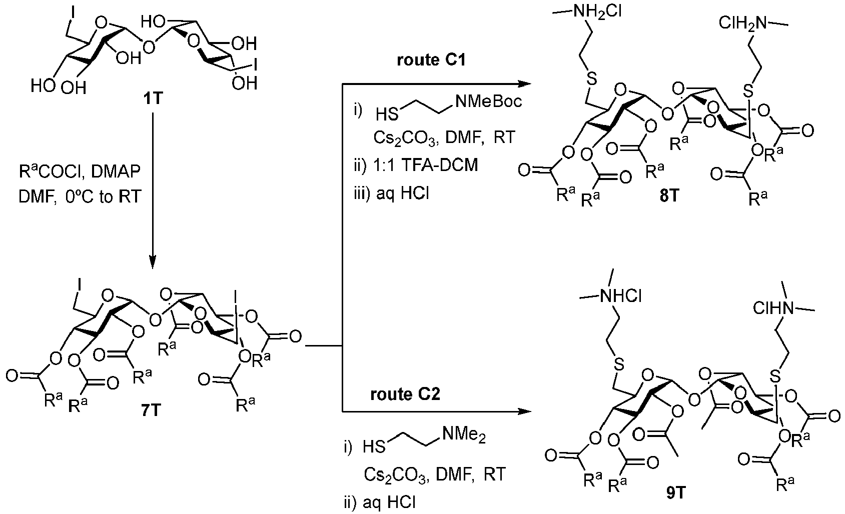

In some embodiments, synthesis of a compound of formula I wherein R¹ and R² are both -C(O)-R^{a} comprises the following lipid reagents:

| Reagent | **R^{a}** | code |
|---|---|---|
| 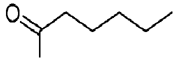 | 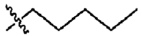 | L1 |
| 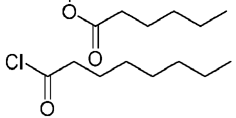 | 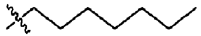 | L2 |
| 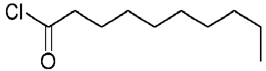 | 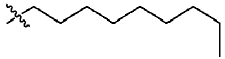 | L3 |
| 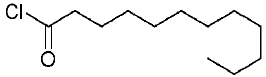 | 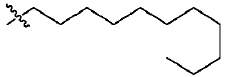 | L4 |
| 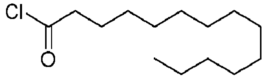 | 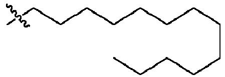 | L5 |
| 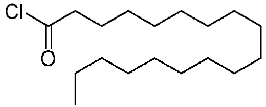 | 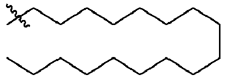 | L7 |
| 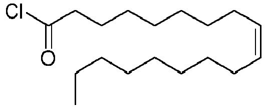 | 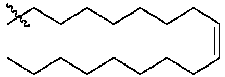 | L7.1 |

In exemplary embodiments, the synthesis of the compounds of formula I according to Schemes 1 or 2 involved the following isothiocyanate or amine reagents in the reaction step leading to the installation of the cationic heads Z¹ and Z²:

**Exemplary Cationic Moieties**

| reagent | **Z1 and Z²** | | code |
|---|---|---|---|
| 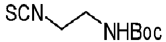 | 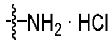 | | C1 |
| 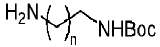 | | n = 2 | C51 |
| | | n = 3 | C52 |
| 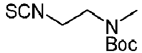 | 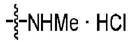 | | C2 |
| 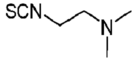 | 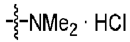 | | C3 |
| 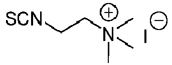 | 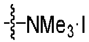 | | C4 |
| 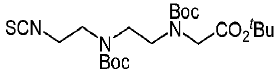 | 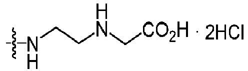 | | C5 |
| 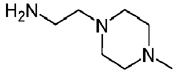 | 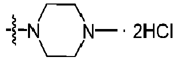 | | C7 |
| 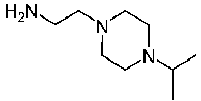 | 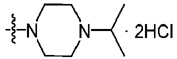 | | C7.1 |
| 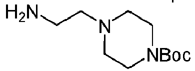 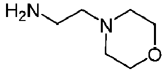 | 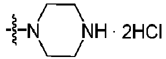 | | C8 |
| 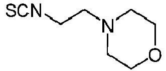 | 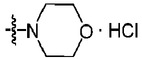 | | C9 |
| 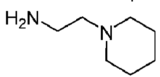 | 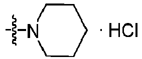 | | C9.1 |
| 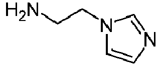 | 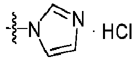 | | C10 |
| 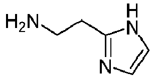 | 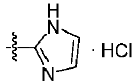 | | C10.1 |
| 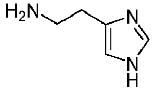 | 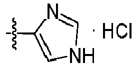 | | C10.2 |
| 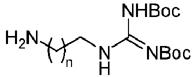 | 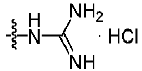 | n = 1 | C11 |
| | | n = 2 | C65 |
| 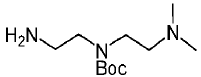 | 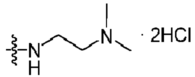 | | C12 |
| 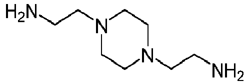 | 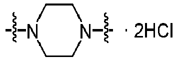 | | C14 |
| 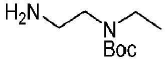 | 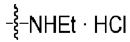 | | C15 |
| 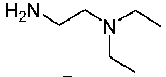 | 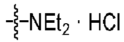 | | C16 |
| 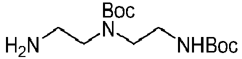 | 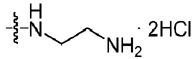 | | C18 |
| 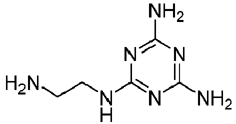 | 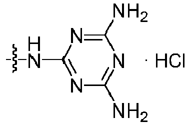 | | C26 |
| 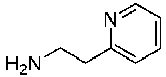 | 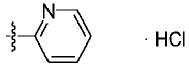 | | C30 |
| 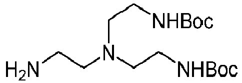 | 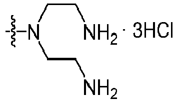 | | C43 |
| 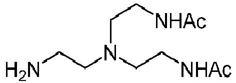 | 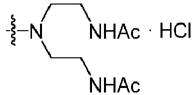 | | C45 |
| 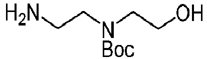 | 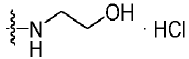 | | OH1 |
| 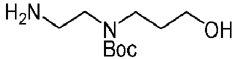 | 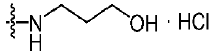 | | OH2 |
| 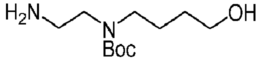 | 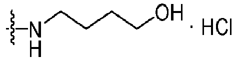 | | OH3 |
| 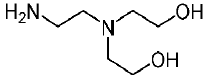 | 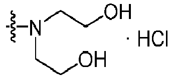 | | OH5 |
| 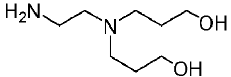 | 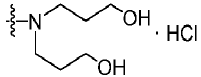 | | OH6 |
| 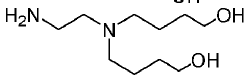 | 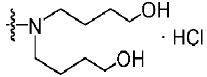 | | OH7 |
| 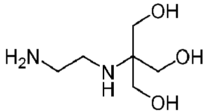 | 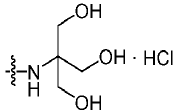 | | OH10 |
| 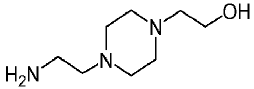 | 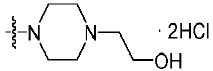 | | OH12 |
| 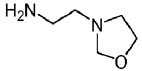 | 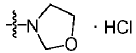 | | C47 |
| 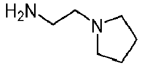 | 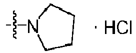 | | C47.1 |
| 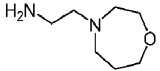 | 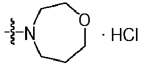 | | C48 |
| 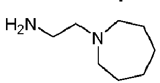 | 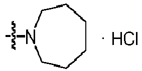 | | C48.1 |
| 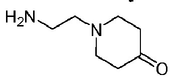 | 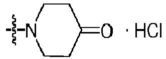 | | C54 |
| 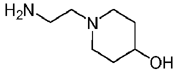 | 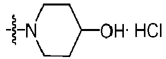 | | C59 |
| 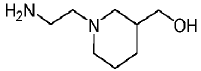 | 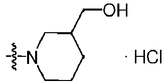 | | C59.1 |
| 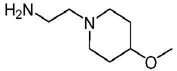 | 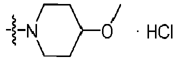 | | C59.2 |
| 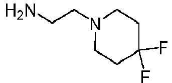 | 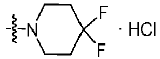 | | C60 |
| 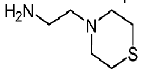 | 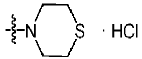 | | C61 |
| 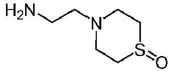 | 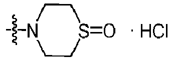 | | C62 |
| 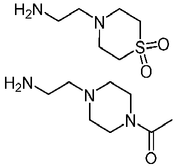 | 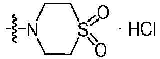 | | C63 |
| | 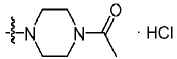 | | C66 |
| 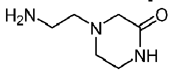 | 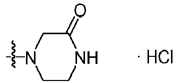 | | C68 |
| 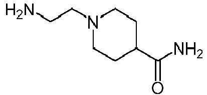 | 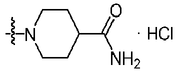 | | C69 |
| 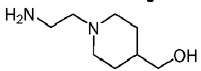 | 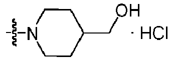 | | C70 |

### Materials

In some exemplary embodiments, the following precursors used in the synthesis of specific compounds described herein were prepared according to known methods: 6,6'-Diiodo-6,6'-dideoxy-a,a'-trehalose (1T) was prepared following the procedure described in the literature. J. M. Garcia Fernández, C. Ortiz Mellet, J. L. Jiménez Blanco, J. Fuentes Mota, A. Gadelle, A. Coste-Sarguet, J. Defaye, Carbohydr. Res. 1995, 268, 57-71. 6,6'-Diiodo-6,6'-dideoxy-sucrose **(1S)** was prepared according to the procedure described in the literature. *See* J. M. Garcia Fernandez, A. Gadelle, J. Defaye, Carbohydr. Res. 1994, 265, 249-269.

As used herein, an arrow followed by a code between parentheses, e.g., "(→ **code)"** indicates the lipophilic tails (R¹ and R² substituents in a compound of formula I) or cationic heads (Z¹ and Z² substituents in a compound of formula I) that the preceding reagent provided during the synthesis. E.g., "hexanoic anhydride (→ **L1)"** is intended to refer to moiety L1, as described in the tables above, was bonded to a compound through use of hexanoic anhydride.

Hexanoic anhydride (CAS# 2051-49-2, → **L1**) and octanoyl (CAS# 111-64-8, → **L2),** decanoyl (CAS# 112-13-0, → **L3),** lauroyl (CAS# 112-16-3, → **L4),** myristoyl (CAS# 112-64-1, → **L5),** stearoyl (CAS# 112-76-5, → **L7)** and oleoyl (CAS# 112-77-6, → **L7.1)** chlorides were purchased from commercial sources. 2-(*N*-*tert*-Butoxycarbonylamino)ethanethiol (CAS# 67385-09-5, **C1SH),** 2-(*N,N-*dimethylamino)ethylisothiocyanate (CAS# 7097-89-4, → **C3),** 2-(4-methylpiperazin-1-yl)ethanamine (CAS# 934-98-5, → **C7),** 2-(4-isopropylpiperazin-1-yl)ethanamine (CAS# 132740-59-1, → **C7.1),** *tert*-butyl 4-(2-aminoethyl)piperazine-1-carboxylate (CAS# 192130-34-0, → **C8),** 2-morpholinoethanamine (CAS# 2038-03-1, → **C9),** 2-morpholinoethylisothiocyanate (CAS# 2038-03-1, → **C9),** 1-(2-aminoethyl)piperidine (CAS# 63224-35-1, → **C9.1),** 2-(1 H-imidazol-1-yl)ethanamine (CAS# 5739-10-6, → **C10),** 2-(1 H-imidazol-2-yl)ethanamine (CAS# 19225-96-8, → **C10.1),** 2-(1 H-imidazol-4-yl)ethan-1-amine (CAS# 51-45-6, → **C10.2),** 2,2'-(piperazine-1,4-diyl)diethanamine (CAS# 6531-38-0, → **C14),** *tert*-butyl (2-aminoethyl)(ethyl)carbamate (CAS# 105628-63-5, → **C15),** *N*,*N*-diethylethylenediamine (CAS# 100-36-7, → **C16),** 1,4-bis-Boc-1,4,7-triazaheptane (CAS# 120131-72-8, → **C18),** 1-(2-aminoethyl)pyrrolidine (CAS# 7154-73-6, → **C47.1),** *N*-(2-aminoethyl)-1,4-oxazepan (CAS# 878155-50-1, → **C48),** 1-(2-aminoethyl)homopiperidine (CAS# 51388-00-2, → **C48.1),** *tert*-butyl *N*-(2-aminoethyl)-*N*-(2-hydroxyethyl)carbamate (CAS# 364056-56-4, → **OH1),** *N*,*N*-bis(2-hydroxyethyl)ethylenediamine (CAS# 3197-06-6, → **OH5),** 3,3'-((2-aminoetil)azanediil)bis(propan-1-ol) (CAS# 50331-68-5, → **OH6),** 4,4'-((2-aminoetil)azanedil)bis(butan-1-ol) (CAS# 197966-97-5, → **OH7),** 2-((2-aminoethyl)amino)-2-(hydroxymethyl)propane-1,3-diol (CAS# 1211477-35-8, → **OH10**), 2-(4-(2-aminoethyl)piperazin-1-yl)ethanol (CAS# 20542-08-9, → **OH12),** 1-(2-aminoethyl)piperidin-4-one (CAS# 1196887-97-4, → **C54),** *N*-(2-aminoethyl)-4-piperidinol (CAS# 129999-60-6, → **C59),** [1-(2-amino-ethyl)-piperidin-3-yl]-methanol (CAS# 857637-03-7, → **C59.1),** 2-(4-methoxy-piperidin-1-yl)-ethylamine (CAS# 911300-69-1, → **C59.2),** 2-(4,4-difluoropiperidin-1-yl)ethanamine (CAS# 605659-03-8, → **C60),** 4-(2-aminoethyl)-thiomorpholine (CAS# 53515-36-9, → **C61),** 4-(2-aminoethyl)thiomorpholine-1,1-dioxide (CAS# 89937-52-0, → **C63),** 1-[4-(2-aminoethyl)piperazin-1-yl]ethenone (CAS# 148716-35-2, → **C66),** 4-(2-aminoethyl)piperazin-2-one (CAS# 145625-71-4, → **C68),** 1-(2-aminoethyl)piperidine-4-carboxamide (CAS# 443897-95-8, → **C69),** [1-(2-aminoethyl)piperidin-4-yl]methanol dihydrochloride (CAS# 1311315-83-9, → **C70),** 2-[*N-*methyl-*N-*(*tert-*butoxycarbonyl)amino]ethanethiol (CAS# 134464-53-2, **C2SH),** and 2-(*N,N-*dimethylamino)ethanethiol (CAS# 108-02-1, **C3SH)** were purchased from commercial sources . 2-(*N-tert*-Butoxycarbonylamino)ethylisothiocyanate (→ **C1**), D. M. Kneeland, K. Ariga, V. M. Lynch, C. Y. Huang, E. V. Anslyn, J. Am. Chem. Soc. 1993, 115,10042-10055. 2-(*N*-methyl-*N*-*tert*-butoxycarbonylamino)ethylisothiocyanate (→ **C2),** T. Kim, Y.-J. Kim, I.-H. Han, D. Lee, J. Ham, K. S. Kan, J. W. Lee, Bioorg. Med. Chem. Lett. 2015, 25, 62-66. 2-(*N*,*N*,*N*-trimethylamino)ethylisothiocyanate (→ **C4),** Y.-J. Ghang, J. J. Lloyd, M. P. Moehlig, J. K. Arguelles, M. Mettry, X. Zhang, R. R. Julian, Q. Cheng, R. J. Hooley, Langmuir 2014, 30, 10160-10166. 2-(2-aminoethyl)-1,3-di-(tert-butoxycarbonyl)guanidine (→ **C11),** S. M. Hickey, T. D. Ashton, J. M. White, J. Li, R. L. Nation, H. Y. Yu, A. G. Elliott, M. S. Butler, J. X. Huang, M. A. Cooper, F. M. Pfeffer, RSC Adv. 2015, 5, 28582-28596. *N*,*N*-bis[2-(*tert*-butoxycarbonylamino)ethyl]ethylenediamine (→ **C43),** Y. Wu, A. Kaur, E. Fowler, M. M. Wiedmann, R. Young, W. R. J. D. Galloway, L. Olsen, H. F. Sore, A. Chattopadhyay, T. T.-L. Kwan, W. Xu, S. J. Walsh, P. de Andrade, M. Janecek, S. Arumugam, L. S. Itzhaki, Y. Heng Lau, D. R. Spring, ACS Chem. Biol. 2019, 14, 526-533. And *N*,*N*-bis(2-acetamidoethyl)ethylenediamine (→ **C45)** were prepared according to the described procedures. L. Liu, M. Rozenman, R. Breslow, J. Am. Chem. Soc.2002, 124, 12660-12661. *N'*,*N*'-dimethyl-*N"*-*tert*-butoxycarbonyl-diethylenetriamine (→ **C12),** *N*-(3-hydroxypropyl)-*N*-(*tert*-butoxycarbonyl)ethylenediamine (→ **OH2)** and *N*-(4-hydroxybutyl)-*N*-(*tert-*butoxycarbonyl)ethylenediamine (→ **OH3)** were synthesized from commercially available *N'*,*N'*-dimethyl-diethylenetriamine, *N*-(3-hydroxypropyl)ethylenediamine and N-(4-hydroxybutyl)ethylenediamine, respectively, by sequential primary amine trifluoroacetylation with ethyl trifluoroacetate, carbamoylation of the remaining amino groups with Boc₂O and base-promoted trifluoroacetamide hydrolysis. *N*-(2-Aminoethyl)-1,3-oxazolidine (CAS# 67626-78-2, → **C47)** was synthesised from commercially available *N*-(2-hydroxyethyl)-1,3-oxazolidine (CAS# 20073-50-1) by sequential hydroxyl mesylation using mesyl chloride, substitution by sodium azide and final catalytic hydrogenation using H₂-Pd/C. 4-(2-Aminoethyl)-thiomorpholine-1-oxide (→ **C62)** was synthesized by sequential primary amine carbamoylation with Boc₂O, thioether oxidation with m-chloroperbenzoic acid and final acid promoted carbamate cleavage. *N'*-(*tert*-Butoxycarbonylmethyl)-*N"-*(2-isothiocyanatoethyl)-ethylenediamine (→ **C5)** was synthesized according to the following scheme (Scheme 4) from commercially available diethylenetriamine.

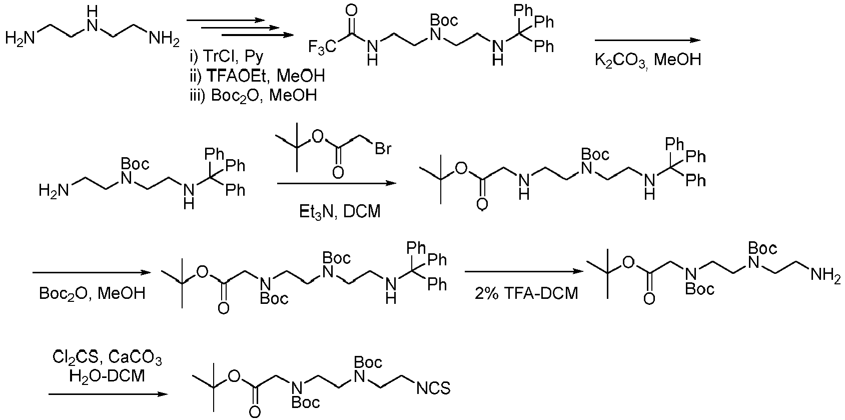

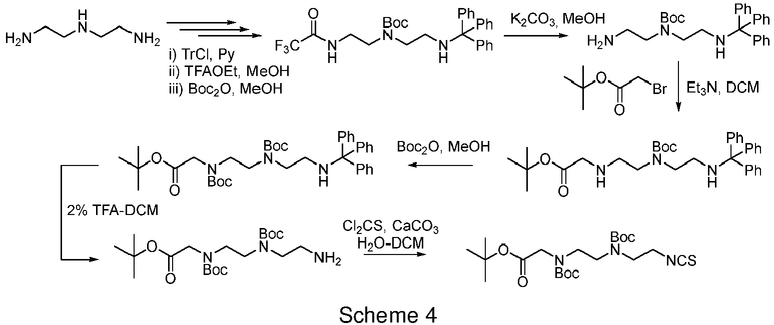

The synthetic intermediates **2T**, **2S, 3T** and **3S** in the reaction sequences depicted in Schemes 1 and 2 were prepared as described below:
**6,6'-Bis[2-(*tert*-butoxycarbonylamino)ethylthio)-α,α'-trehalose (2T).** To a mixture of diiodo-trehalose **1T** (14.3 g, 25.44 mmol) and Cs₂CO₃ (23.45 g, 71.23 mmol) in anhydrous DMF (150 mL) under N₂ atmosphere, a solution of 2-(*N-tert-*butoxycarbonylamino)ethanethiol **(C1SH,** 13.6 mL, 76.32 mmol) in anhydrous DMF (100 mL) under N₂ was added. The reaction mixture was stirred at RT overnight and the solid remaining in suspension was decanted and filtrated off. The solvent was then removed under reduced pressure and the resulting residue was triturated with Et₂O (500 mL),
upon which a sticky solid evolved. The residue was filtrated and triturated with EtOH (250 mL) and the remaining solid again filtrated off. The solvents were finally evaporated to dryness to furnish analytically pure compound **2T** in virtually quantitative yield (16.8 g).
Chemical Formula: C₂₆H₄₈N₂O₁₃S₂. Molecular Weight: 660.26. ESI-MS *(m*/*z)* 705.51([M + HCO₂]⁻).
6,6'-Bis[2-(tert-butoxycarbonylamino)ethylthio)sucrose (2S). To a mixture of diiodo-sucrose 1S (1.07 g, 1.9 mmol) and Cs2CO3 (1.75 g, 5.33 mmol) in anhydrous DMF (30 mL) under N2 atmosphere, 2-(tert-butoxycarbonylamino)ethylmercaptane (1.0 mL, 5.7 mmol) was added. The reaction mixture was stirred at RT overnight. The solvent was then removed under reduced pressure and the resulting residue was triturated with Et2O (100 mL) upon which a sticky solid evolved. The residue was filtrated and triturated with EtOH (50 mL) and the remaining solid again filtrated off. The solvents were finally evaporated to dryness to furnish analytically pure compound 2S in virtually quantitative yield (1.25 g). Chemical Formula: C26H48N2O13S2. Molecular Weight: 660.26. ESI-MS (m/z) 705.51([M + HCO2]-).

**General procedure for exhaustive hydroxyl acylation of 2T and 2S to give reaction intermediates 3T and 3S.** To a solution of the corresponding acylating agent (→ **L1** to **L7.1,** 46 mmol) in anhydrous DMF (20 mL) under N₂ inert atmosphere at 0 ºC, a solution of **2T** or **2S** (3.37 g, 5.1 mmol) and DMAP (11.3 g, 92 mmol) in anhydrous DMF (100 mL) was dropwise added over a period of 10 min. The reaction mixture was allowed to warm to RT over 2 h and further stirred overnight. The reaction was then diluted in a 1:1 water-DCM mixture (400 mL). The organic layer was decanted and further washed with water (100 mL), 2 N H₂SO₄ (2 × 100 mL), saturated NaHCO₃ (100 mL) and water (100 mL), dried over MgSO₄ or Na₂SO₄, filtrated and evaporated under reduced pressure. The resulting syrupy residue was purified by column chromatography using the eluent indicated in each case to give the corresponding **3T** or **3S** reaction intermediate with R^{a} substituents as defined by the codes **L1** to **L7.1** in the above lipid reagent list.

| | | |
|---|---|---|
| Compound **3T-L1** | eluent 1:4 EtOAc-petroleum ether | yield 82% |
| Compound **3T-L2** | eluent 1:4→2:3 EtOAc-petroleum ether | yield 37% |
| Compound **3T-L3** | eluent 1:10→1:3 EtOAc-petroleum ether | yield 54% |
| Compound **3T-L4** | eluent 1:10→1:5 EtOAc-petroleum ether | yield 27% |
| Compound **3T-L5** | eluent 1:10→1:5 EtOAc-petroleum ether | yield 63% |
| Compound **3T-L7** | eluent 1:10→1:5 EtOAc-petroleum ether | yield 34% |
| Compound **3T-L7.1** | eluent 1:10→1:5 EtOAc-petroleum ether | yield 39% |
| Compound **3S-L1** | eluent 1:4 EtOAc-petroleum ether | yield 72% |
| Compound **3S-L3** | eluent 1:10→1:3 EtOAc-petroleum ether | yield 17% |

**General procedure for *tert-*butoxycarbonyl group hydrolysis in reaction intermediates 3T** (→ **4T) and 3S** (→ **4S).** To a solution of **3T** or **3S** (1 mmol) in DCM (10 mL), at RT, trifluoroacetic acid (TFA, 10 mL) was added. The reaction mixture was stirred at RT for 1 h, then the solvents were evaporated under reduced pressure and the traces of acid were eliminated by repeated coevaporation with water. The resulting residue was lyophilized from 10-mM HCI to furnish the corresponding diamines **4T** and **4S,** as hydrochloride salts, in quantitative yield.

In some preferred embodiments, the following compounds of formula I featuring structure **4T** were synthesized according to Scheme 1 following the above procedure: JLF19, JLF150, JLF20, JLF155, JLF23, JLF46..

In some preferred embodiments, the following compounds of formula I featuring structure **4S** were synthesized according to Scheme 1 following the above procedure: JLF50, JLF62.

**General procedure for the synthesis of diisothiocyanate reaction intermediates 5T in Scheme 1.** To a solution of **4T** (1 mmol) in a heterogeneous mixture of water and DCM (1:1, mL), CaCO₃ (0.48 g, 4.8 mmol) and Cl₂CS (0.22 ml, 2.4 mmol) were sequentially added. The mixture was vigorously stirred at RT for 1 h and then transferred to a decantation funnel and diluted with water and DCM (1:1, 100 mL). The organic layer was decanted and washed with water (50 mL) and saturated aq NaHCO₃ (50 mL), dried over MgSO₄ or Na₂SO₄, filtrated and evaporated under reduced pressure to furnish the target diisothiocyanate reaction intermediates **5T,** with R^{a} substituents as defined by the codes **L1** or **L3** in the **Lipid reagent list:**
Compound **5T-L1:** Yield 99%. Chemical Formula: C₅₄H₈₈N₂O₁₅S₄. MW: 1133.54. ESI-MS *(m*/*z)* 1155.58 ([M + Na]⁺).
Compound **5T-L3):** Yield 90%. Chemical Formula: C₇₈H₁₃₆N₂O₁₅S₄. MW: 1470.19. ESI-MS *(m*/*z)* 1514.87 ([M + HCO₂]⁻).
Compound **5S-L1:** Yield 99%. Chemical Formula: C₅₄H₈₈N₂O₁₅S₄. MW: 1133.54. ESI-MS (m/z) 1155.58 ([M + Na]⁺).
Compound **5S-L3:** Yield 90%. Chemical Formula: C₇₈H₁₃₆N₂O₁₅S₄. MW: 1470.19. ESI-MS (m/z) 1514.87 ([M + HCO₂]⁻).

### General procedure for the synthesis of a compound of formula I according to Schemes 1 and 2, routes A1, A2, B1 and B2.

**Route A1 (schemes 1 or 2):** To a solution of the corresponding diamine dihydrochloride **(4T** or **4S,** 0.1 mmol) in DCM (10 mL) and triethylamine (Et₃N, 0.3 mmol, 41 µL), a solution of the corresponding isothiocyanate reagent SCN-(CH₂)₂-Z^{a}-PG¹ (0.3 mmol) in DCM (5 mL) were sequentially added. The reaction mixture was stirred at RT for 16 h while monitoring pH to ensure it remains basic (In case pH is found below neutrality, it should be readjusted to slightly basic (pH ca. 8) by addition of aliquots of Et₃N). Upon reaction completion (TLC revealed the complete disappearance of the starting material **4T** or **4S** and the formation of a novel spot at a higher *R*_{f}), the reaction mixture was concentrated to dryness and the crude material purified by column chromatography using the eluent indicated in each case. The resulting intermediate with Boc-protected amino groups was subsequently treated with a 1:1 DCM-TFA mixture (10 mL) at RT for 1 h followed by evaporation of the solvents under reduced pressure. Traces of TFA were eliminated by coevaporation with toluene (3 × 10 mL). Lyophilization from 10 mM HCI furnished the target products **6T** or **6S** in the yield indicated in each case.

In some embodiments, the following compounds of formula I featuring structure **6T** were synthesized according to Scheme 1, route A1: JLF20, JLF32, JLF33, JLF35, JLF43, JLF58, JLF78, JLJB617, JLJB618, JLJB619.

In some embodiments, the following compounds of formula I featuring structure **6S** were synthesized according to Scheme 2, route A1: JLF59, JLF60, JLF68, JLF69, JLF76, JLF77

**Route** A**2 (schemes 1 or 2):** Similarly, to route A1, route A2 implies the coupling reaction of a compound of structure **4T** or **4S** with an isothiocyanate reagent SCN-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification was conducted in these cases. The resulting product was lyophilized from 10 mM HCI to furnish the target product **6T** or **6S** in the yield indicated in each case.

In some embodiments, the following compounds of formula I featuring structure **6T** were synthesized according to Scheme 1, route A2: JLF25, JLF26, JLF29, JLF30, JLF41, JLF42, JLF154, JLF158, JLF170, JLJB604, JLJB605.

In some embodiments, the following compounds of formula I featuring structure **6S** were synthesized according to Scheme 2, route A2: JLF54, JLF55, JLF66, JLF67, **Route B1 (scheme 1):** To a solution of the corresponding diisothiocyanate reaction intermediate **5T** (1 mmol) in DCM (10 mL) a solution the corresponding amine reagent H₂N-(CH₂)₂-Z^{a}-PG¹ (0.3 mmol) in DCM (5 mL) was added. The reaction mixture was stirred at RT for 16 h while monitoring pH to ensure it remains basic. Upon reaction completion (TLC revealed the complete disappearance of the starting material **5T** and the formation of a novel spot at a lower *R*_{f}), the reaction mixture was concentrated to dryness and the crude material purified by column chromatography using the indicated eluent. The resulting intermediate was further treated with a 1:1 DCM-TFA mixture (10 mL) at RT for 1 h followed by evaporation of the solvents under reduced pressure, coevaporation of the acidic traces with toluene (3 × 10 mL) and lyophilization from 10 mM HCI to furnish the target products **6T** in the yield indicated in each case.

In some embodiments, the following compounds of formula I featuring structure **6T** were synthesized according to Scheme 1, route B1: JLF93, JLF94, JLF96, JLF97, JLF98, JLF102, JLF103, JLF111, JLF115, JLF120, JLF121, JLF139, JLF141, JLF200, JLF201, JLF220, PER20.

**Route B2:** Similarly, to route B1, route B2 implies the coupling reaction of a compound of structure **5T** with an amine reagent H₂N-(CH₂)₂-Z¹, using an analogous protocol. Upon reaction completion, the reaction mixture was transferred to a decantation funnel and partitioned between DCM and 0.1 N aq HCI (1:1, 20 mL). The organic layer was decanted, dried over MgSO₄ or Na₂SO₄, filtrated and evaporated under reduced pressure. Column chromatography purification is unnecessary in these cases. The resulting product was lyophilized from 10 mM HCI to furnish the target products **6T** in the indicated yields.

In some embodiments, the following compounds of formula I featuring structure 6T were synthesized according to Scheme 1, route B2: JLF81, JLF82, JLF87, JLF90, JLF95, JLF99, JLF106, JLF108, JLF110, JLF111, JLF125, JLF128, JLF130, JLF131, JLF134, JLF135, JLF138, JLF142, JLF163, JLF164, JLF165, JLF190, JLF197, JLF223, JLF234, JLF237, JLF238, JLF244, JLF245, JLF248, JLF301, JLF601, JLF602, JLF604, JLF605, JLF606, JLF607, JLF608, PRX051.

General procedure for the synthesis of a compound of formula I according to Scheme 3, routes C1 and C2.

The diiodo trehalose derivative **1T** was per-*O*-acylated following a procedure analogous to the **General procedure for exhaustive hydroxyl acylation of 2T and 2S to give reaction intermediates 3T and 3S** described above. The resulting crude product was subjected to column chromatography purification (1:10 EtOAc-petroleum ether) to furnish reaction intermediate **7T** in 67% yield.

In a preferred embodiment, the diiodo reaction intermediate **7T** wherein the R^{a} substituents are defined by the code **L1** (**7T-L1**), as indicated in the **Lipid reagent list,** was prepared according to this procedure.

**Route C1:** To a mixture of **7T** (0.1 mmol) and Cs₂CO₃ (91 mg, 0.28 mmol) in anhydrous DMF (3 mL) under N₂ atmosphere, 2-[*N*-methyl-*N*-(*tert-*butoxycarbonyl)amino]ethanethiol **(C2SH,** 52 µL, 0.3 mmol) was added and the reaction mixture was stirred at RT overnight. The solvent was then removed under reduced pressure and the resulting residue was purified by column chromatography (1:6 EtOAc-petroleum ether). The resulting intermediate was treated with a 1:1 DCM-TFA mixture (4 mL) at RT for 1 h. The solvents were evaporated under reduced pressure, the traces of acid were repeatedly coevaporated with water and the residue was lyophilized from 10-mM HCI to furnish **8T** in 60% yield.

In a preferred embodiment, the following compound of formula I featuring structure **8T** was synthesized according to Scheme 3, route C1: JLF48.

**Route C2:** Similarly to route C1, route C2 implies the coupling reaction between **7T** and 2-(*N*,*N*-dimethylamino)ethanethiol **(C3SH),** following an analogous protocol. Upon reaction completion, the reaction mixture was transferred to a decantation funnel and partitioned between DCM and 0.1N aq HCI (1:1, 20 mL). The organic layer was decanted, dried over MgSO₄ or Na₂SO₄, filtrated and evaporated under reduced pressure. Column chromatography purification is unnecessary in this case. The resulting product was lyophilized from 10 mM HCI to furnish compound **8T** in 82% yield.

In a preferred embodiment, the following compound of formula I featuring structure **8T** was synthesized according to Scheme 3, route C1: JLF40.

Compound JLF99 was synthesized from the diisothiocyanate precursor **5T-L3** and the corresponding amine mine reagent (code **C9)** according to Scheme 1, Route B2 **(5T** to **6T** step). Yield 45%. Chemical Formula: C₉₀H₁₆₆Cl₂N₆O₁₇S₄. Molecular Weight: 1803.48 ESI-MS *(m*/*z)* 1731.03 ([M - HCI - Cl]⁺), 866.19 ([M - 2Cl]²⁺).

The embodiments of the disclosure described above are intended to be merely exemplary, numerous variations and modifications will be apparent to those skilled in the art. All such variations and modifications are intended to be within the scope of the present invention as defined in any appended claims.

## Claims

1. A complex comprising:
i) a cationic oligosaccharide comprising one or more cationic moieties bonded to a trehalose, a sucrose, or a gluco-n-oligosaccharide moiety, where n is 2-6;
ii) a surfactant; and
iii) one or more additives selected from: a sterol, a helper lipid, an immunomodulator, and a targeting molecule.

2. The complex of claim 1, wherein the surfactant is or comprises a polysorbate, a poloxamer, and/or a compound comprising an amphiphilic moiety selected from polyalkylene glycols (e.g., polyethylene glycol), poly(2-oxazoline), poly(2-oxazine), polysarcosine, polyvinylpyrrolidone, and poly[N-(2-hydroxypropyl)methacrylamide, wherein the amphiphilic moiety is bonded to one or more C₁₂-C₂₀ aliphatic groups.

3. The complex of claims 1 or 2, wherein the surfactant is or comprises a polysorbate selected from polysorbate 20, polysorbate 40, polysorbate 60, polysorbate 80, and combinations thereof.

4. The complex of any one of claims 1-3, wherein a molar ratio of the cationic oligosaccharide to the surfactant is about 1:0.0075 to about 1:3.

5. The complex of any one of claims 1-4, wherein a molar ratio of the cationic oligosaccharide to the surfactant is about 1:0.5 to about 1:1.

6. The complex of any one of claims 1-5, wherein a molar ratio of the cationic oligosaccharide to the surfactant is about 1:0.5.

7. The complex of any one of claims 1-6, further comprising a cargo.

8. The complex of claim 7, wherein the cargo is a nucleic acid.

9. The complex of claim 8, wherein the nucleic acid is RNA.

10. The complex of claim 9, wherein the RNA is modRNA, saRNA, taRNA, or uRNA.

11. The complex of any one of claims 1-10, wherein the complex has an N/P ratio of less than 20:1.

12. The complex of any one of claims 1-11, wherein the complex has an N/P ratio less than or equal to 12:1.

13. The complex of any one of claims 1-12, wherein the complex has an N/P ratio that is about 6:1.

14. The complex of any one of claims 1-13, wherein the complex has a diameter of about 50 nm to about 150 nm.

15. The complex of any one of claims 1-14, wherein the one or more additives are or comprise a sterol and/or a helper lipid and/or an immunomodulator.

16. The complex of any one of claims 1-15, wherein the one or more additives are or comprise a sterol.

17. The complex of claim 16, wherein the sterol is selected from β-sitosterol, stigmasterol, cholesterol, cholecalciferol, ergocalciferol, calcipotriol, botulin, lupeol, ursolic acid, oleanolic acid, cycloartenol, lanosterol, or α-tocopherol.

18. The complex of any one of claims 1-17, wherein a molar ratio of the cationic oligosaccharide to the sterol is from about 1:0.25 to about 1:1.

19. The complex of claim 18, wherein a ratio of the cationic oligosaccharide to the sterol is about 1:0.5.

20. The complex of any one of claims 1-19, wherein the one or more additives are or comprise a helper lipid.

21. The complex of any one of claims 1- 20, wherein the helper lipid is selected from the group consisting of phosphatidylcholines, phosphatidylethanolamines, phosphatidylglycerols, phosphatidic acids, phosphatidylserines and sphingomyelins, more preferably selected from the group consisting of distearoylphosphatidylcholine (DSPC), dioleoylphosphatidylcholine (DOPC), dimyristoylphosphatidylcholine (DMPC), dipentadecanoylphosphatidylcholine, dilauroylphosphatidylcholine, dipalmitoylphosphatidylcholine (DPPC), diarachidoylphosphatidylcholine (DAPC), dibehenoylphosphatidylcholine (DBPC), ditricosanoylphosphatidylcholine (DTPC), dilignoceroylphatidylcholine (DLPC), palmitoyloleoyl-phosphatidylcholine (POPC), 1,2-di-O-octadecenyl-sn-glycero-3-phosphocholine (18:0 Diether PC), 1-oleoyl-2-cholesterylhemisuccinoyl-sn-glycero-3-phosphocholine (OChemsPC), 1-hexadecyl-sn-glycero-3-phosphocholine (C16 Lyso PC), dioleoylphosphatidylethanolamine (DOPE), distearoyl-phosphatidylethanolamine (DSPE), dipalmitoyl-phosphatidylethanolamine (DPPE), dimyristoyl-phosphatidylethanolamine (DMPE), dilauroyl-phosphatidylethanolamine (DLPE), diphytanoyl-phosphatidylethanolamine (DPyPE), and combinations thereof.

22. The complex of any one of claims 1-21, wherein the helper lipid is selected from DSPC and DMPC.

23. The complex of any one of claims 1-21, wherein a molar ratio of the cationic oligosaccharide to the helper lipid is from about 1:0.25 to about 1:1.

24. The complex of any one of claims 1-23, wherein a molar ratio of the cationic oligosaccharide to the helper lipid is about 1:0.5.

25. The complex of any one of claims 1-24, wherein the one or more additives are or comprise a sterol and a helper lipid.

26. The complex of any one of claims 1-25, wherein a molar ratio of the cationic oligosaccharide : the sterol : the helper lipid is from about 1:0.25:0.25 to about 1:1:1.

27. The complex of claim 26, wherein a molar ratio of the cationic oligosaccharide : the sterol : the helper lipid is about 1:0.5:0.5.

28. The complex of any one of claims 1-27, wherein a molar ratio of the cationic oligosaccharide : the surfactant : the sterol: the helper lipid is about 1:0.5:0.5:0.5.

29. The complex of any one of claims 1-28, wherein the one or more additives are or comprise an immunomodulator.

30. The complex of any one of claims 1-29, wherein the immunomodulator is an immunostimulatant or an immunosuppressor.

31. The complex of any one of claims 1-30, wherein the immunomodulator is a small molecule agonist or antagonist of TLR or PRR receptors.

32. The complex of any one of claims 1-31, wherein the immunomodulator is a small molecule downstream inhibitor of NF-κβ.

33. The complex of any one of claims 1-32, wherein the immunomodulator is an sp²-iminosugar glycolipid.

34. The complex of any one of claim 1-33, wherein the immunomodulator is a TLR inhibitor.

35. The complex of claim 34, wherein the TLR inhibitor is an inhibitor of TLR2, TLR4, and/or TLR6.

36. The complex of claim 35, wherein the TLR inhibitor is an inhibitor of TLR4.

37. The complex of claim 13, wherein the inhibitor of TLR4 is TAK-242.

38. The complex of any one of claims 1-30, wherein the immunomodulator is a terpenoid.

39. The complex of claim 38, wherein the terpenoid is a triterpene.

40. The complex of claim 39, wherein the triterpene is a triterpenoid.

41. The complex of claim 39, wherein the triterpenoid is a synthetic or natural derivate of amyrin, betulinic acid, oleanolic acid, sterols , squalene, or ursolic acid.

42. The complex of any one of claims 38-41, wherein the immunomodulator is a sterol, squalene, oleanolic acid, ursolic acid, betulinic acid, or amyrin.

43. The complex of any one of claims 38-42, wherein the immunomodulator is a glucocorticoid.

44. The complex of claim 43, wherein the glucocorticoid is dexamethasone, prednisolone, fluticasone propionate, budesonide or a pharmaceutically acceptable salt thereof.

45. The complex of claim 44, wherein the glucocorticoid is dexamethasone or a pharmaceutically acceptable salt thereof.

46. The complex of any one of claims 38-45, wherein the complex further comprises one or more additional immunomodulators.

47. The complex of claim 46, where the one or more additional immunomodulators are or comprise a small molecule agonist or antagonist of TLR or PRR receptors.

48. The complex of claim 47, wherein the one or more additional immunomodulators are or comprise TAK-242.

49. The complex of any one of claims 1-48, wherein the one or more additives are or comprise a sterol, a helper lipid, and an immunomodulator.

50. The complex of any one of claims 1-49, wherein a molar ratio of the cationic oligosaccharide to the immunomodulator is from about 1:0.05 to about 1:0.5.

51. The complex of claim 50, wherein a molar ratio of the cationic oligosaccharide to the immunomodulator is about 1:0.5.

52. The complex of any one of claims 1-51, wherein the one or more additives are or comprise a targeting molecule.

53. The complex of any one of claims 1-52, wherein the cationic oligosaccharide is a compound of formula I: 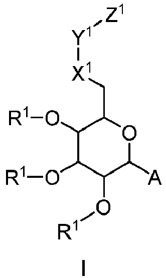 or a pharmaceutically acceptable salt thereof, wherein:
A is A¹, A², or A³: 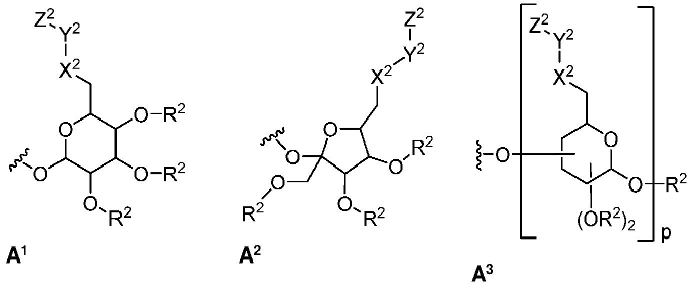
R¹ and R² are each independently selected from H, R^{a}, and -C(O)-R^{a}, wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by -Cy-, -NR^{Y}-, - NR^{Y}C(O)-, -C(O)NR^{Y}-, -NR^{Y}C(O)O-, -OC(O)NR^{Y}-, -NR^{Y}C(O)NR^{Y}-, -NR^{Y}C(S)NR^{Y}-, - C(S)NR^{Y}- -C(O)NR^{Y}SO₂-, -SO₂NR^{Y}C(O)-,-OC(O)O-, -O-, -C(O)-, -OC(O)-, -C(O)O, SO,
or SO₂;
each R^{Y} is independently H or optionally substituted C₁-C₆ aliphatic;
each Cy is independently an optionally substituted C₃-C₁₄ cycloaliphatic, 5- to 14-membered heterocyclyl ring having 1-3 heteroatoms selected from N, O, S, 5- to 14-membered heteroaryl ring having 1-3 heteroatoms selected from N, O, S;
Z¹ and Z² are each independently a cationic or ionizable group selected from optionally substituted 5- to 14-membered heterocyclyl ring having 1-3 heteroatoms selected from N, O, and S, 5- to 14-membered heteroaryl ring having 1-3 heteroatoms selected from N, O, and S, -N⁺(M)₃, 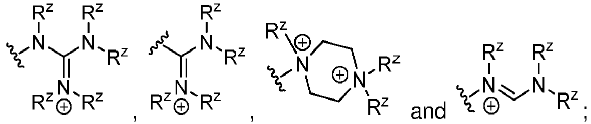
each M is independently -C₀-C₆ aliphatic-R^{z} or -C₀-C₆ aliphatic-N⁺(R^{z})₃; each R^{z} is independently selected from H, optionally substituted C₁-C₆ aliphatic, optionally substituted C₃-C₂₀ cycloaliphatic, optionally substituted C₅-C₆ aryl, optionally substituted 3- to 12-membered heterocyclyl comprising 1 to 3 heteroatoms selected from N, O, and S, and optionally substituted 4- to 12-membered heteroaryl comprising 1 to 3 heteroatoms selected from N, O, and S; or
two or more R^{z} can come together with the atoms to which they are attached to form an optionally substituted 3- to 12-membered heterocyclyl comprising 1 to 3 heteroatoms selected from N, O, and S, or an optionally substituted 4- to 12-membered heteroaryl comprising 1 to 3 heteroatoms selected from N, O, and S; and p is an integer selected from 1, 2, 3, 4, or 5.

54. The complex of claim 53, wherein the cationic oligosaccharide is of formula la: 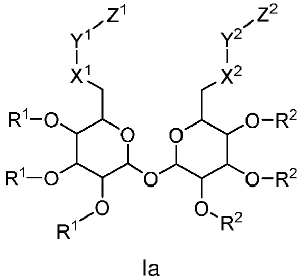 or a pharmaceutically acceptable salt thereof.

55. The complex of claim 53, wherein the oligosaccharide is of formula Ib: 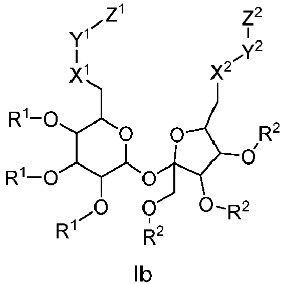 or a pharmaceutically acceptable salt thereof.

56. The complex of claim 53, wherein the oligosaccharide is of formula Ic: 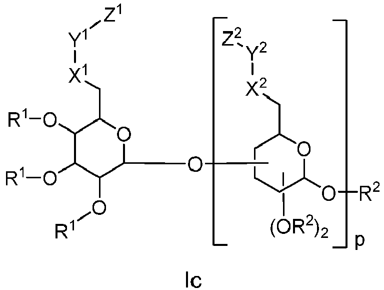 or a pharmaceutically acceptable salt thereof.

57. The complex of any one of claims 53-56, wherein R¹ and R² are each independently selected from R^{a} and -C(O)-R^{a}, and R^{a} is C₁-C₂₀ aliphatic.

58. The complex of any one of claims 53-57, wherein R¹ and R² are each -C(O)-R^{a}, and R^{a} is C₁-C₁₄ aliphatic.

59. The complex of any one of claims 53-58, wherein each R^{a} is C₅-C₁₀ linear alkyl.

60. The complex of any one of claims 53-59, wherein R¹ and R² are each -C(O)-R^{a}, and each R^{a} is independently selected from:

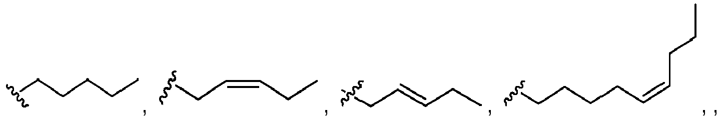

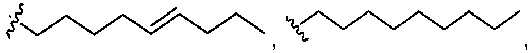

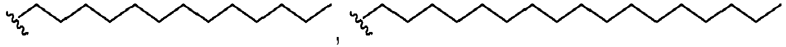

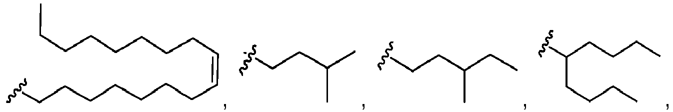

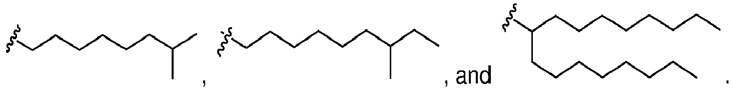

61. The complex of any one of claims 53-60, wherein X¹ and X² are each -S-.

62. The complex of any one of claims 53-61, wherein Y¹ and Y² are each independently an optionally substituted C₁₋₃₀ aliphatic group wherein one or more carbons are independently replaced by -NR^{Y}C(O)NR^{Y}- or -NR^{Y}C(S)NR^{Y}-.

63. The complex of claim 62, wherein R^{Y} is H.

64. The complex of any one of claims 53-63, wherein Y¹ and Y² are each C₁-C₄ aliphatic-NHC(S)NH-C₁-C₄ aliphatic.

65. The complex of claim 64, wherein Y¹ and Y² are each -CH₂-CH₂-NHC(S)NH-CH₂-CH₂-.

66. The complex of any one of claims 53-65, wherein Z¹ and Z² are each independently a cationic or ionizable group comprising an optionally substituted 5- to 14-membered heterocyclyl ring having 1-3 heteroatoms selected from N, O, and S.

67. The complex of any one of claims 53-66, wherein Z¹ and Z² are each independently selected from: 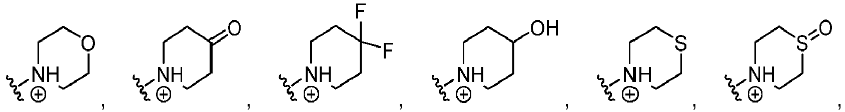 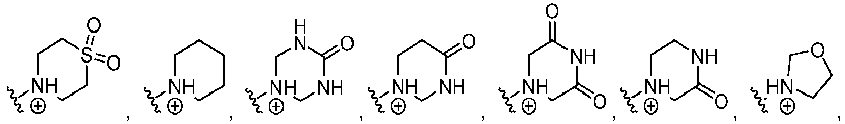 and

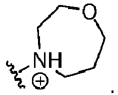

68. The composition of claim 67, wherein Z¹ and Z² are each independently selected from: 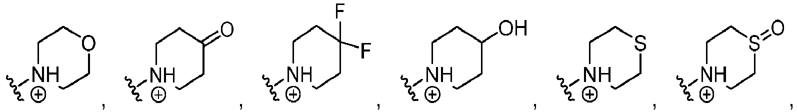 and

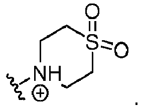

69. The complex of any one of claims 53-68, wherein the cationic oligosaccharide further comprising one or more suitable counterions.

70. The complex of any one of claims 1-53, wherein the cationic oligosaccharide is selected from Table 1, or a pharmaceutically acceptable salt thereof.

71. The complex of any one of claims 1-53, wherein the cationic oligosaccharide is selected from Table 2, or a pharmaceutically acceptable salt thereof.

72. A method of increasing or causing increased expression of RNA in a target in a subject comprising administering to the subject the complex of any one of claims 1-71.

73. The method of claim 72, wherein the target is selected from the lungs, liver, spleen, heart, brain, lymph nodes, bladder, kidneys, and pancreas.

74. A method of treating a disease, disorder, or condition in a subject comprising administering to the subject a complex of any one of claims 1-71.

75. The method of claim 74, wherein the disease, disorder, or condition is an infectious disease, cancer, a genetic disorder, an autoimmune disease, or a rare disease.

76. The method of any one of claims 72-75, wherein the complex is administered intramuscularly.

77. The method of any one of claims 72-75, wherein the complex is administered subcutaneously.

78. A complex of any one of claims 1-71, for use as a medicament.

79. A complex of any one of claims 1-71, for use in the treatment and/or prevention of a disease or disorder, wherein the disease or disorder is an infectious disease, cancer, a genetic disorder, an autoimmune disease, or a rare disease.

80. A composition comprising an oligosaccharide of formula I: 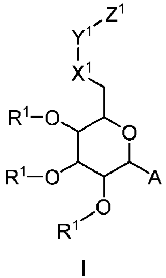 or a pharmaceutically acceptable salt thereof, wherein:
A is A¹, A², or A³: 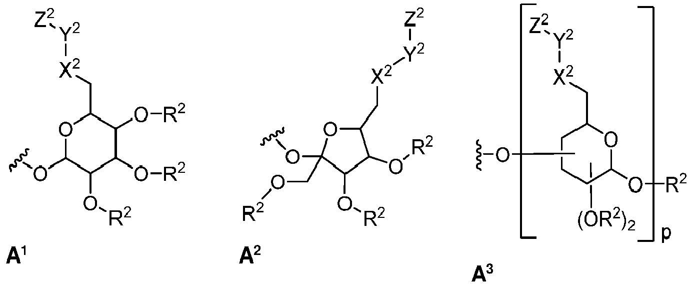
R¹ and R² are each independently selected from H, R^{a}, and -C(O)-R^{a}, wherein at least one instance of R¹ or R² is not H;
each R^{a} is independently selected from C₁-C₂₀ aliphatic, C₃-C₂₀ cycloaliphatic, C₅-C₆ aryl, 3- to 12-membered heterocyclyl comprising 1 to 3 heteroatoms selected from N, O, and S, wherein each R^{a} is optionally substituted with one or more R^{b};
each R^{b} is independently selected from halogen, -N₃, -R^{c}, -OR^{c}, -SR^{c}, -NHR^{c}, -C(O)-R^{c}, -OC(O)R^{c}, -NHC(O)R^{c}, -C(O)NHR^{c}, and -NHC(O)NHR^{c};
each R^{c} is independently selected from optionally substituted C₁-C₂₀ aliphatic, C₃-C₂₀ cycloaliphatic, C₅-C₆ aryl, 3- to 12-membered heterocyclyl comprising 1 to 3 heteroatoms selected from N, O, and S, and 4- to 12-membered heteroaryl comprising 1 to 3 heteroatoms selected from N, O, and S;
X¹ and X² are each independently selected from -S-, -S-S-, and -NH-;
Y¹ and Y² are each independently an optionally substituted C₁₋₃₀ aliphatic group wherein one or more carbons are optionally and independently replaced by -NR^{Y}C(O)NR^{Y}- or NR^{Y}C(S)NR^{Y}-
each R^{Y} is independently selected from H and optionally substituted C₁-C₆ aliphatic;
Z¹ and Z² are each independently selected from: 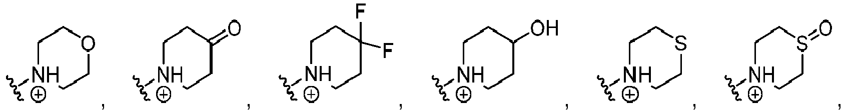 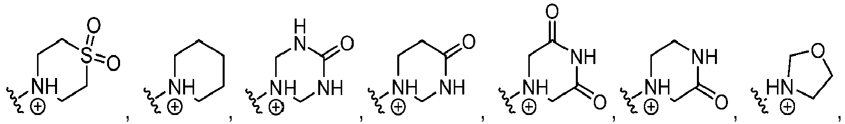 and 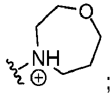 and
p is an integer selected from 1, 2, 3, 4 or 5.

81. The composition of claim 80, wherein Z¹ and Z² are each independently selected from: 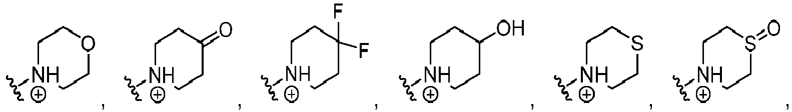 and

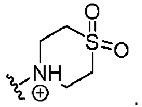

82. The composition of claims 80 or 81, wherein the cationic oligosaccharide is of formula la: 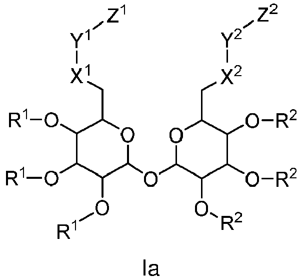 or a pharmaceutically acceptable salt thereof.

83. The composition of claims 80 or 81, wherein the oligosaccharide is of formula Ib: 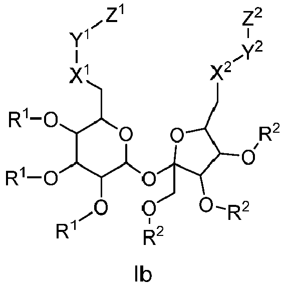 or a pharmaceutically acceptable salt thereof.

84. The composition of claims 80 or 81, wherein the oligosaccharide is of formula Ic: 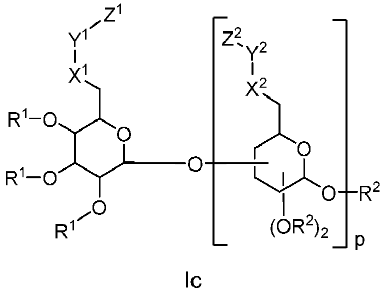 or a pharmaceutically acceptable salt thereof.

85. The composition of any one of claims 80-84, wherein R¹ and R² are each independently selected from R^{a} and -C(O)-R^{a}, and R^{a} is C₁-C₂₀ aliphatic.

86. The composition of any one of claims 80-85, wherein R¹ and R² are each -C(O)-R^{a}, and R^{a} is C₁-C₁₄ aliphatic.

87. The composition of any one of claims 80-86, wherein each R^{a} is C₅-C₁₀ linear alkyl.

88. The composition of any one of claims 80-86, wherein R¹ and R² are each -C(O)-R^{a}, and each R^{a} is independently selected from:

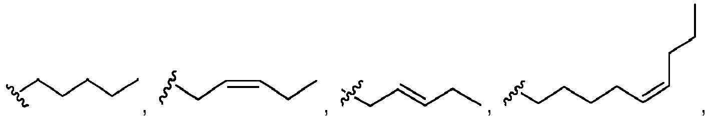

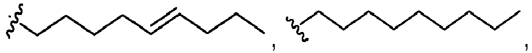

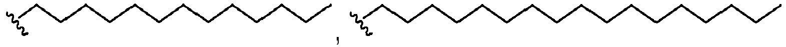

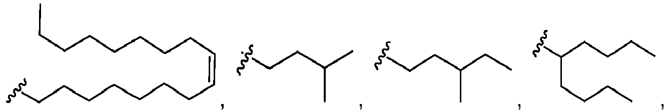

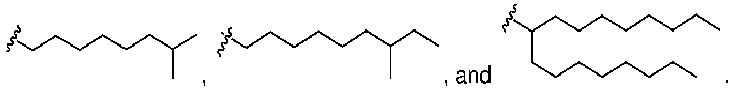

89. The composition of any one of claims 80-88, wherein X¹ and X² are each -S-.

90. The composition of any one of claims 80-89, wherein Y¹ and Y² are each C₁-C₄ aliphatic-NHC(S)NH-C₁-C₄ aliphatic.

91. The composition of claim 90, wherein Y¹ and Y² are each -CH₂-CH₂-NHC(S)NH-CH₂-CH₂-.

92. The composition of any one of claims 80-91, wherein the cationic oligosaccharide further comprising one or more suitable counterions.

93. The composition of claim 80, wherein the cationic oligosaccharide is selected from Table 2.
